(19) Europäisches Patentamt
European Patent Office
Office européen des brevets

(11) **EP 4 699 622 A1**

(12) **EUROPEAN PATENT APPLICATION**
published in accordance with Art. 153(4) EPC

(43) Date of publication:
**25.02.2026 Bulletin 2026/09**

(21) Application number: **24792714.8**

(22) Date of filing: **18.04.2024**

(51) International Patent Classification (IPC):
***A61K 47/68*** (2017.01) ***A61K 31/4184*** (2006.01)
***A61K 31/454*** (2006.01) ***A61K 31/4745*** (2006.01)
***A61K 31/496*** (2006.01) ***A61K 31/519*** (2006.01)
***A61K 31/573*** (2006.01) ***A61K 31/664*** (2006.01)
***A61K 31/704*** (2006.01) ***A61K 39/395*** (2006.01)
***A61K 45/00*** (2006.01) ***A61P 7/00*** (2006.01)
***A61P 35/00*** (2006.01) ***A61P 35/02*** (2006.01)
***A61P 43/00*** (2006.01) ***C07K 7/06*** (2006.01)
***C07K 16/28*** (2006.01)

(52) Cooperative Patent Classification (CPC):
**A61K 31/4184; A61K 31/454; A61K 31/4745; A61K 31/496; A61K 31/519; A61K 31/573; A61K 31/664; A61K 31/704; A61K 39/395; A61K 45/00; A61K 47/68; A61P 7/00; A61P 35/00; A61P 35/02; A61P 43/00;** (Cont.)

(86) International application number:
**PCT/JP2024/015357**

(87) International publication number:
**WO 2024/219442 (24.10.2024 Gazette 2024/43)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **19.04.2023 JP 2023068465**

(71) Applicant: **Daiichi Sankyo Company, Limited**
**Tokyo 103-8426 (JP)**

(72) Inventors:
- **SHINJO, Yuji**
**Tokyo 103-8426 (JP)**
- **SHIROISHI, Machiko**
**Tokyo 103-8426 (JP)**
- **TAKATA, Yoshimi**
**Tokyo 103-8426 (JP)**
- **KURIMOTO, Akiko**
**Tokyo 103-8426 (JP)**
- **SHINADA, Masahiro**
**Tokyo 103-8426 (JP)**

(74) Representative: **Marks & Clerk LLP**
**15 Fetter Lane**
**London EC4A 1BW (GB)**

Remarks:
The complete document including Reference Table(s) and the Sequence Listing(s) can be downloaded from the EPO website

(54) **COMBINATION OF ANTIBODY-DRUG CONJUGATE AND OTHER MEDICINE**

(57) To provide a pharmaceutical composition wherein a specific antibody-drug conjugate and another drug are administered in combination, and/or a method of treatment comprising administering a specific antibody-drug conjugate and another drug in combination to an individual, and the like.

A pharmaceutical composition wherein an anti-CD37 antibody-drug conjugate and another drug (one or more drugs selected from the group consisting of a molecular targeting drug, a drug relating to R-CHOP therapy, a drug relating to R-CHP therapy, an alkylating agent and an immunomodulatory drug) are administered in combination, and the anti-CD37 antibody-drug conjugate is an anti-CD37 antibody-drug conjugate in which a drug-linker represented by the following formula and an anti-CD37 antibody are conjugated via a thioether bond (wherein A represents a connecting position to the anti-CD37 antibody), and/or a method of treatment comprising administering the anti-CD37 antibody-drug conjugate and the other drug in combination to an individual.

EP 4 699 622 A1

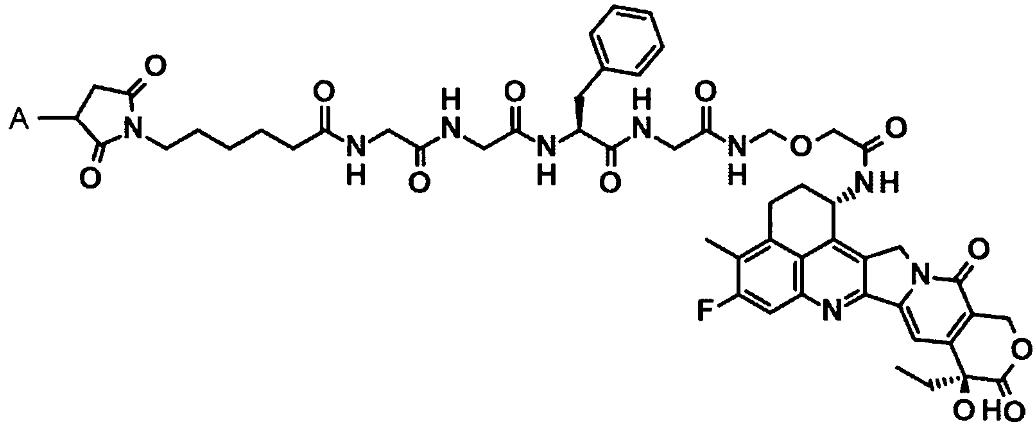

Fig. 11

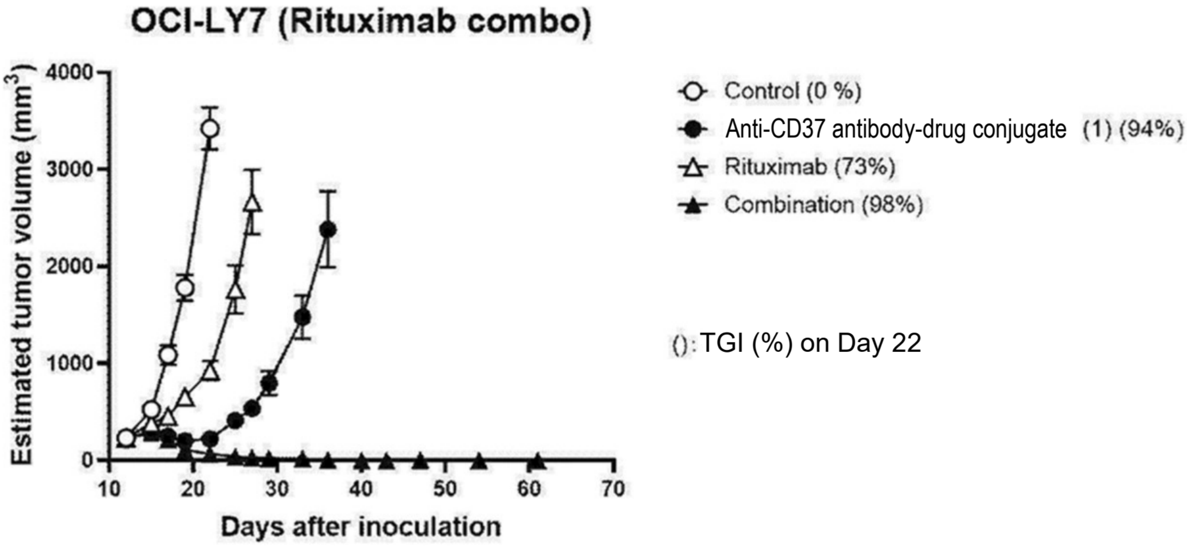


(52) Cooperative Patent Classification (CPC): (Cont.)
**C07K 7/06; C07K 16/28**

## Description

Technical Field

The present invention relates to a pharmaceutical composition wherein a specific antibody-drug conjugate and another drug are administered in combination, and/or a method of treatment comprising administering a specific antibody-drug conjugate and another drug in combination to an individual, and the like.

Background Art

Various therapeutic drugs are under development for B-cell non-Hodgkin's lymphoma (B-NHL) or chronic lymphocytic leukemia (CLL). For example, rituximab is an antibody drug targeting CD20 and was approved as a therapeutic drug for B-NHL by the FDA in 1997 (Non Patent Literature 1). Main mechanisms of action of rituximab are direct induction of apoptosis, ADCC (antibody-dependent cellular cytotoxicity), and CDC (complement-dependent cytotoxicity). Currently, R-CHOP therapy, BR therapy (combination therapy of bendamustine and rituximab), R2 therapy (combination therapy of rituximab and lenalidomide), and the like are widely used for diffuse large B-cell lymphoma (DLBCL), follicular lymphoma (FL), and chronic lymphocytic leukemia (CLL) expressing CD20 targeted by rituximab (Non Patent Literatures 2, 3, and 4). For example, combination therapy of an anti-CD19 antibody tafasitamab and an immunomodulatory drug lenalidomide (Non Patent Literature 5) for DLBCL, and monotherapy with a BTK inhibitor ibrutinib, monotherapy with a BCL-2 inhibitor venetoclax, and VR therapy (combination therapy of venetoclax and rituximab) for CLL (Non Patent Literature 6) are known. Thus, therapeutic drugs having various mechanisms of action and combination therapy thereof are under development for B-NHL or CLL. Nonetheless, some patients exhibit recurrence or resistance, and there is a demand for the development of novel therapeutic drugs or combination therapy.

An antibody-drug conjugate (ADC) in which an antibody capable of binding to an antigen that is expressed on the surface of cancer cells and can be internalized into the cell is conjugated to a drug having cytotoxicity can selectively deliver the drug to cancer cells, and can thereby be expected to kill the cancer cells by accumulating the drug in the cancer cells (Non Patent Literatures 7 to 12).

In recent years, Polivy (registered trademark) (polatuzumab vedotin) targeting CD79b and ZYNLONTA (registered trademark) (loncastuximab tesirine) targeting CD19 have been approved as a therapeutic drug for DLBCL (Non Patent Literatures 13 and 14). Also, naratuximab emtansine targeting CD37 is under clinical trial in combination with rituximab (Non Patent Literature 15).

A known antibody-drug conjugate is an antibody-drug conjugate having an antibody and a topoisomerase I inhibitor exatecan as components (Patent Literatures 1 to 7 and Non Patent Literatures 16 to 19).

Patent Literatures 1 to 7 state that the above-described antibody-drug conjugate can be administered together with various therapeutic agents for cancers.

However, these literatures do not describe any test result showing an excellent combinatorial effect when the above-described antibody-drug conjugate and another drug are used in combination for B-NHL or CLL or any scientific evidence that suggests such a test result.

Citation List

Patent Literature

Patent Literature 1: International Publication No. WO2014/057687
Patent Literature 2: International Publication No. WO2014/061277
Patent Literature 3: International Publication No. WO2015/098099
Patent Literature 4: International Publication No. WO2015/115091
Patent Literature 5: International Publication No. WO2015/146132
Patent Literature 6: International Publication No. WO2015/155976
Patent Literature 7: International Publication No. WO2015/155998

Non Patent Literature

Non Patent Literature 1: Gilles S., et al., Adv Ther (2017) 34 (10): 2232-2273.

Non Patent Literature 2: B Coiffier., et al., Oncogene (2007), 26 (25): 3603-3613.
Non Patent Literature 3: Munakata W., et al., Expert Opin Drug Discov (2016) 11 (11) 1123-1130.
Non Patent Literature 4: Smyth E., et al., Cancer Treat Rev (2023) 113: 102510.
Non Patent Literature 5: Bruce D. Cheson., et al., Blood Cancer, J (2021) 11 (4), 68.
Non Patent Literature 6: Moritz Bewarder., et al., Cancers (Basal) (2021) 13 (10) 2468.
Non Patent Literature 7: Laurent Ducry., et al., Bioconjug Chem (2010) 21 (1): 5-13.
Non Patent Literature 8: Stepen C Alley., et al., Curr Opin ChemBiol (2010) 14 (4): 529-37.
Non Patent Literature 9: Nitin K Damle., et al., Expert Opin Biol Ther (2004) 4 (9): 1445-52.
Non Patent Literature 10: Nitin K Damle., et al., Expert Opin Biol Ther (2004) 4 (9): 1445-52.
Non Patent Literature 11: Peter D Senter., et al., Nat Biotechnol (2012) 30 (7): 631-7.
Non Patent Literature 12: Howard A., et al., J Clin Oncol (2011) 29 (4): 398-405.
Non Patent Literature 13: Laurie H Sehn., Blood Adv (2022) 6 (2): 533-543.
Non Patent Literature 14: Bo Xu., European Journal of Clinical Pharmacology (2022) 78: 707-719.
Non Patent Literature 15: Owen Stretton., et al., Lancet Haematol (2021) 8 (8): e546-e547.
Non Patent Literature 16: Ogitani Y.et al., Clinical Cancer Research (2016) 22 (20): 5097-5108.
Non Patent Literature 17: Ogitani Y.et al., Cancer Science (2016) 107 (7): 1039-46.
Non Patent Literature 18: Doi T, et al., Lancet Oncol (2017) 18 (11): 1512-1522.
Non Patent Literature 19: Takegawa N, et al., Int. J. Cancer (2017) 141 (8), 1682-1689.

## Summary of Invention

### Technical Problem

It is an object to provide a pharmaceutical composition wherein a specific antibody-drug conjugate and another drug are administered in combination, and/or a method of treatment comprising administering a specific antibody-drug conjugate and another drug in combination to an individual, and the like.

### Solution to Problem

The present inventors have conducted intensive studies directed towards achieving the above-described object, and completed the present invention by finding that an anti-CD37 antibody-drug conjugate and one or more other drugs selected from the group consisting of a molecular targeting drug, a drug relating to R-CHOP therapy, a drug relating to R-CHP therapy, an alkylating agent and an immunomodulatory drug when administered in combination exhibit an excellent combinatorial effect.

Specifically, the present invention includes the following aspects of the invention.

[1] A pharmaceutical composition comprising an anti-CD37 antibody-drug conjugate, wherein

the anti-CD37 antibody-drug conjugate is administered in combination with one or more other drugs selected from the group consisting of a molecular targeting drug, a drug relating to R-CHOP therapy, a drug relating to R-CHP therapy, an alkylating agent and an immunomodulatory drug, and

the anti-CD37 antibody-drug conjugate is an anti-CD37 antibody-drug conjugate in which a drug-linker represented by the formula and an anti-CD37 antibody are conjugated via a thioether bond:

[Formula 1]

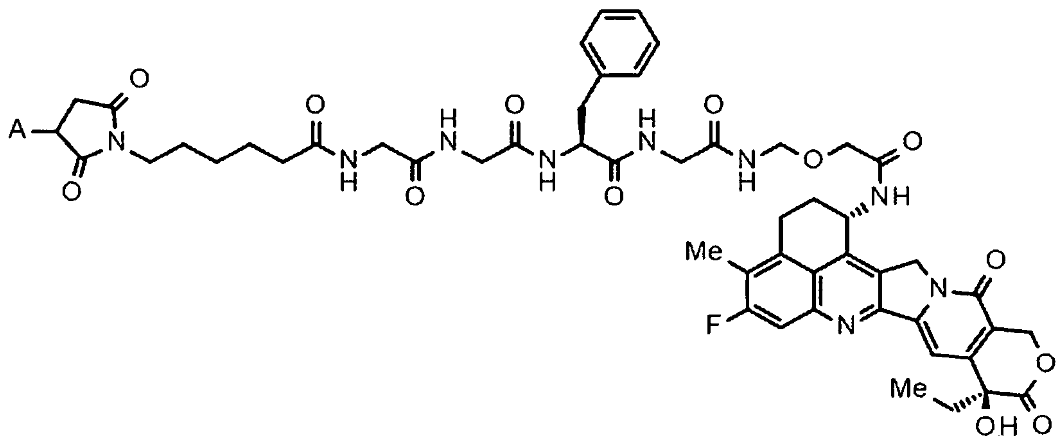

wherein A represents a connecting position to the anti-CD37 antibody.

[2] The pharmaceutical composition according to [1], wherein the anti-CD37 antibody is an antibody comprising a heavy chain variable region and a light chain variable region in any one combination selected from the group consisting of the following combinations (a) to (d):

(a) a light chain variable region consisting of the amino acid sequence at positions 21 to 128 in the light chain full-length amino acid sequence shown in SEQ ID NO: 2 and a heavy chain variable region consisting of the amino acid sequence at positions 20 to 138 in the heavy chain full-length amino acid sequence shown in SEQ ID NO: 4;
(b) a light chain variable region consisting of the amino acid sequence at positions 21 to 128 in the light chain full-length amino acid sequence shown in SEQ ID NO: 2 and a heavy chain variable region consisting of the amino acid sequence at positions 20 to 138 in the heavy chain full-length amino acid sequence shown in SEQ ID NO: 6;
(c) a light chain variable region consisting of the amino acid sequence at positions 21 to 128 in the light chain full-length amino acid sequence shown in SEQ ID NO: 2 and a heavy chain variable region consisting of the amino acid sequence at positions 20 to 138 in the heavy chain full-length amino acid sequence shown in SEQ ID NO: 8; and
(d) a light chain variable region consisting of the amino acid sequence at positions 21 to 128 in the light chain full-length amino acid sequence shown in SEQ ID NO: 2 and a heavy chain variable region consisting of the amino acid sequence at positions 20 to 138 in the heavy chain full-length amino acid sequence shown in SEQ ID NO: 10.

[3] The pharmaceutical composition according to [1] or [2], wherein the anti-CD37 antibody is an antibody comprising a heavy chain and a light chain in any one combination selected from the group consisting of the following combinations (e) to (h):

(e) a light chain consisting of the amino acid sequence at positions 21 to 234 in the light chain full-length amino acid sequence shown in SEQ ID NO: 2 and a heavy chain consisting of the amino acid sequence at positions 20 to 468 in the heavy chain full-length amino acid sequence shown in SEQ ID NO: 4;
(f) a light chain consisting of the amino acid sequence at positions 21 to 234 in the light chain full-length amino acid sequence shown in SEQ ID NO: 2 and a heavy chain consisting of the amino acid sequence at positions 20 to 468 in the heavy chain full-length amino acid sequence shown in SEQ ID NO: 6;
(g) a light chain consisting of the amino acid sequence at positions 21 to 234 in the light chain full-length amino acid sequence shown in SEQ ID NO: 2 and a heavy chain consisting of the amino acid sequence at positions 20 to 468 in the heavy chain full-length amino acid sequence shown in SEQ ID NO: 8; and
(h) a light chain consisting of the amino acid sequence at positions 21 to 234 in the light chain full-length amino acid sequence shown in SEQ ID NO: 2 and a heavy chain consisting of the amino acid sequence at positions 20 to 468 in the heavy chain full-length amino acid sequence shown in SEQ ID NO: 10.

[4] The pharmaceutical composition according to any one of [1] to [3], wherein a lysine residue at the carboxyl terminus of the heavy chain of the anti-CD37 antibody is deleted.
[5] The pharmaceutical composition according to any one of [1] to [4], wherein the average number of units of the drug-linker conjugated per antibody in the anti-CD37 antibody-drug conjugate is in the range of from 2 to 8.

[6] The pharmaceutical composition according to any one of [1] to [5], wherein the average number of units of the drug-linker conjugated per antibody in the anti-CD37 antibody-drug conjugate is in the range of from 7 to 8.
[7] The pharmaceutical composition according to any one of [1] to [5], wherein the average number of units of the drug-linker conjugated per antibody in the anti-CD37 antibody-drug conjugate is in the range of from 3.5 to 4.5.
[8] The pharmaceutical composition according to any one of [1] to [7], wherein the molecular targeting drug is one or more drugs selected from the group consisting of an anti-CD20 antibody, an anti-CD19 antibody, a BCL-2 inhibitor and a BTK inhibitor.
[9] The pharmaceutical composition according to [8], wherein the anti-CD20 antibody is rituximab or obinutuzumab.
[10] The pharmaceutical composition according to [8] or [9], wherein the anti-CD19 antibody is tafasitamab.
[11] The pharmaceutical composition according to any one of [8] to [10], wherein the BCL-2 inhibitor is venetoclax or a pharmacologically acceptable salt thereof.
[12] The pharmaceutical composition according to any one of [8] to [11], wherein the BTK inhibitor is ibrutinib or a pharmacologically acceptable salt thereof.
[13] The pharmaceutical composition according to any one of [1] to [12], wherein the alkylating agent is bendamustine or a pharmacologically acceptable salt thereof.
[14] The pharmaceutical composition according to any one of [1] to [13], wherein the immunomodulatory drug is lenalidomide or a pharmacologically acceptable salt thereof.
[15] The pharmaceutical composition according to any one of [1] to [9] and [13], wherein the other drug is a combination of rituximab and bendamustine or a pharmacologically acceptable salt thereof.
[16] The pharmaceutical composition according to any one of [1] to [8], [10] and [14], wherein the other drug is a combination of tafasitamab and lenalidomide or a pharmacologically acceptable salt thereof.
[17] The pharmaceutical composition according to any one of [1] to [9] and [14], wherein the other drug is a combination of rituximab and lenalidomide or a pharmacologically acceptable salt thereof.
[18] The pharmaceutical composition according to any one of [1] to [9] and [11], wherein the other drug is a combination of rituximab and venetoclax or a pharmacologically acceptable salt thereof.
[19] The pharmaceutical composition according to any one of [1] to [18], wherein the antibody-drug conjugate and the one or more other drugs selected from the group consisting of a molecular targeting drug, a drug relating to R-CHOP therapy, a drug relating to R-CHP therapy, an alkylating agent and an immunomodulatory drug are separately contained as active ingredients in different formulations and administered at the same time or different times.
[20] The pharmaceutical composition according to any one of [1] to [19] for the treatment of at least one disease selected from the group consisting of diffuse large B-cell lymphoma, follicular lymphoma, mantle cell lymphoma, marginal zone lymphoma, Burkitt's lymphoma, chronic lymphocytic leukemia, T-cell lymphoma such as peripheral T-cell lymphoma or cutaneous T-cell lymphoma, myelodysplastic syndrome and acute myeloid leukemia.
[21] The pharmaceutical composition according to [20] for the treatment of diffuse large B-cell lymphoma.
[22] The pharmaceutical composition according to [20] for the treatment of chronic lymphocytic leukemia.
[23] The pharmaceutical composition according to [20] for the treatment of follicular lymphoma.
[24] A pharmaceutical composition comprising an anti-CD37 antibody-drug conjugate, wherein

the anti-CD37 antibody-drug conjugate is administered in combination with one or more other drugs selected from the group consisting of a molecular targeting drug, a drug relating to R-CHOP therapy, a drug relating to R-CHP therapy, an alkylating agent and an immunomodulatory drug, and
the anti-CD37 antibody-drug conjugate is an anti-CD37 antibody-drug conjugate represented by the formula:

[Formula 2]

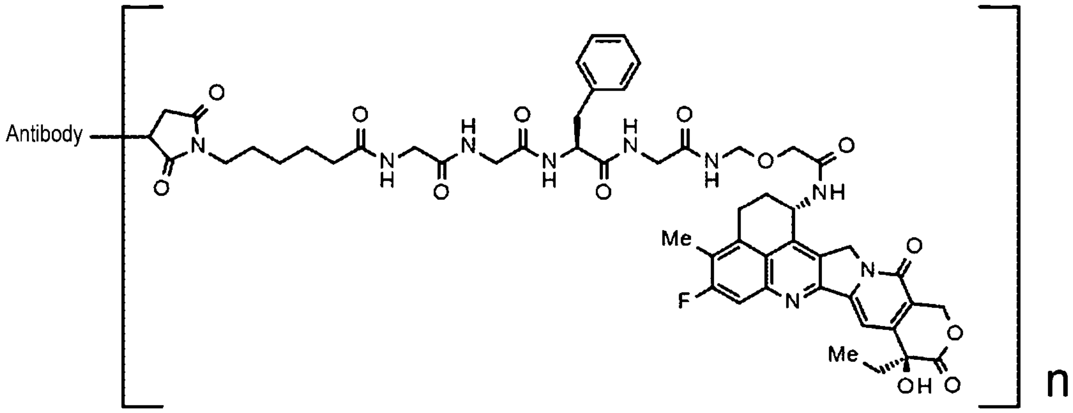

wherein Antibody represents an anti-CD37 antibody, the drug-linker is conjugated to the antibody via a thioether bond, and n represents the average number of units of the drug-linker conjugated per antibody.

[25] The pharmaceutical composition according to [24], wherein the anti-CD37 antibody is an antibody comprising a heavy chain variable region and a light chain variable region in any one combination selected from the group consisting of the following combinations (a) to (d):

(a) a light chain variable region consisting of the amino acid sequence at positions 21 to 128 in the light chain full-length amino acid sequence shown in SEQ ID NO: 2 and a heavy chain variable region consisting of the amino acid sequence at positions 20 to 138 in the heavy chain full-length amino acid sequence shown in SEQ ID NO: 4;
(b) a light chain variable region consisting of the amino acid sequence at positions 21 to 128 in the light chain full-length amino acid sequence shown in SEQ ID NO: 2 and a heavy chain variable region consisting of the amino acid sequence at positions 20 to 138 in the heavy chain full-length amino acid sequence shown in SEQ ID NO: 6;
(c) a light chain variable region consisting of the amino acid sequence at positions 21 to 128 in the light chain full-length amino acid sequence shown in SEQ ID NO: 2 and a heavy chain variable region consisting of the amino acid sequence at positions 20 to 138 in the heavy chain full-length amino acid sequence shown in SEQ ID NO: 8; and
(d) a light chain variable region consisting of the amino acid sequence at positions 21 to 128 in the light chain full-length amino acid sequence shown in SEQ ID NO: 2 and a heavy chain variable region consisting of the amino acid sequence at positions 20 to 138 in the heavy chain full-length amino acid sequence shown in SEQ ID NO: 10.

[26] The pharmaceutical composition according to [24] or [25], wherein the anti-CD37 antibody is an antibody comprising a heavy chain and a light chain in any one combination selected from the group consisting of the following combinations (e) to (h):

(e) a light chain consisting of the amino acid sequence at positions 21 to 234 in the light chain full-length amino acid sequence shown in SEQ ID NO: 2 and a heavy chain consisting of the amino acid sequence at positions 20 to 468 in the heavy chain full-length amino acid sequence shown in SEQ ID NO: 4;
(f) a light chain consisting of the amino acid sequence at positions 21 to 234 in the light chain full-length amino acid sequence shown in SEQ ID NO: 2 and a heavy chain consisting of the amino acid sequence at positions 20 to 468 in the heavy chain full-length amino acid sequence shown in SEQ ID NO: 6;
(g) a light chain consisting of the amino acid sequence at positions 21 to 234 in the light chain full-length amino acid sequence shown in SEQ ID NO: 2 and a heavy chain consisting of the amino acid sequence at positions 20 to 468 in the heavy chain full-length amino acid sequence shown in SEQ ID NO: 8; and
(h) a light chain consisting of the amino acid sequence at positions 21 to 234 in the light chain full-length amino acid sequence shown in SEQ ID NO: 2 and a heavy chain consisting of the amino acid sequence at positions 20 to 468 in the heavy chain full-length amino acid sequence shown in SEQ ID NO: 10.

[27] The pharmaceutical composition according to any one of [24] to [26], wherein a lysine residue at the carboxyl terminus of the heavy chain of the anti-CD37 antibody is deleted.
[28] The pharmaceutical composition according to any one of [24] to [27], wherein the average number of units of the drug-linker conjugated per antibody in the antibody-drug conjugate is in the range of from 2 to 8.

[29] The pharmaceutical composition according to any one of [24] to [28], wherein the average number of units of the drug-linker conjugated per antibody in the antibody-drug conjugate is in the range of from 7 to 8.
[30] The pharmaceutical composition according to any one of [24] to [28], wherein the average number of units of the drug-linker conjugated per antibody in the anti-CD37 antibody-drug conjugate is in the range of from 3.5 to 4.5.
[31] The pharmaceutical composition according to any one of [24] to [30], wherein the molecular targeting drug is one or more drugs selected from the group consisting of an anti-CD20 antibody, an anti-CD19 antibody, a BCL-2 inhibitor and a BTK inhibitor.
[32] The pharmaceutical composition according to [31], wherein the anti-CD20 antibody is rituximab or obinutuzumab.
[33] The pharmaceutical composition according to [31] or [32], wherein the anti-CD19 antibody is tafasitamab.
[34] The pharmaceutical composition according to any one of [31] to [33], wherein the BCL-2 inhibitor is venetoclax or a pharmacologically acceptable salt thereof.
[35] The pharmaceutical composition according to any one of [31] to [34], wherein the BTK inhibitor is ibrutinib or a pharmacologically acceptable salt thereof.
[36] The pharmaceutical composition according to any one of [24] to [35], wherein the alkylating agent is bendamustine or a pharmacologically acceptable salt thereof.
[37] The pharmaceutical composition according to any one of [24] to [36], wherein the immunomodulatory drug is lenalidomide or a pharmacologically acceptable salt thereof.
[38] The pharmaceutical composition according to any one of [24] to [32] and [36], wherein the other drug is a combination of rituximab and bendamustine or a pharmacologically acceptable salt thereof.
[39] The pharmaceutical composition according to any one of [24] to [31], [33] and [37], wherein the other drug is a combination of tafasitamab and lenalidomide or a pharmacologically acceptable salt thereof.
[40] The pharmaceutical composition according to any one of [24] to [32] and [37], wherein the other drug is a combination of rituximab and lenalidomide or a pharmacologically acceptable salt thereof.
[41] The pharmaceutical composition according to any one of [24] to [32] and [34], wherein the other drug is a combination of rituximab and venetoclax or a pharmacologically acceptable salt thereof.
[42] The pharmaceutical composition according to any one of [24] to [41], wherein the antibody-drug conjugate and the one or more other drugs selected from the group consisting of a molecular targeting drug, a drug relating to R-CHOP therapy, a drug relating to R-CHP therapy, an alkylating agent and an immunomodulatory drug are separately contained as active ingredients in different formulations and administered at the same time or different times.
[43] The pharmaceutical composition according to any one of [24] to [42] for the treatment of at least one disease selected from the group consisting of diffuse large B-cell lymphoma, follicular lymphoma, mantle cell lymphoma, marginal zone lymphoma, Burkitt's lymphoma, chronic lymphocytic leukemia, T-cell lymphoma such as peripheral T-cell lymphoma or cutaneous T-cell lymphoma, myelodysplastic syndrome and acute myeloid leukemia.
[44] The pharmaceutical composition according to [43] for the treatment of diffuse large B-cell lymphoma.
[45] The pharmaceutical composition according to [43] for the treatment of chronic lymphocytic leukemia.
[46] The pharmaceutical composition according to [43] for the treatment of follicular lymphoma.
[47] A method of treatment comprising administering an anti-CD37 antibody-drug conjugate and one or more other drugs selected from the group consisting of a molecular targeting drug, a drug relating to R-CHOP therapy, a drug relating to R-CHP therapy, an alkylating agent and an immunomodulatory drug in combination to an individual in need of treatment, wherein the anti-CD37 antibody-drug conjugate is an anti-CD37 antibody-drug conjugate in which a drug-linker represented by the formula and an anti-CD37 antibody are conjugated via a thioether bond:

[Formula 3]

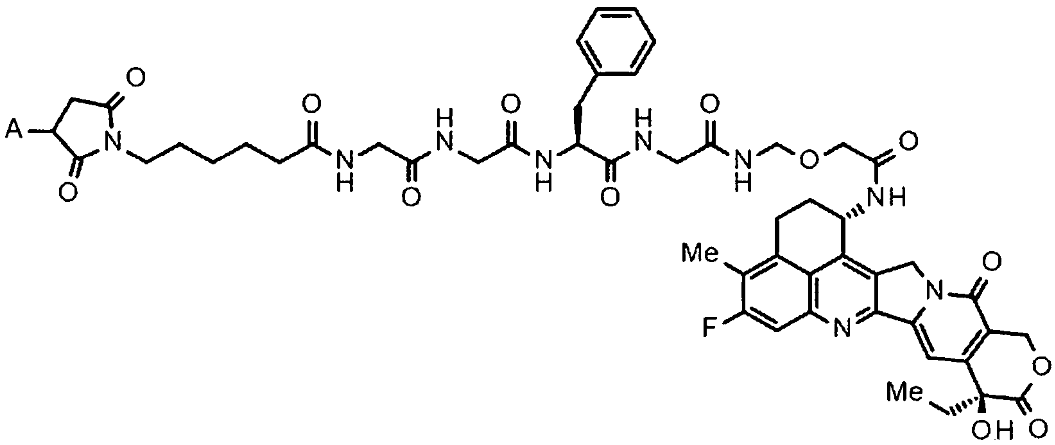

wherein A represents a connecting position to the anti-CD37 antibody.

[48] The method of treatment according to [47], wherein the anti-CD37 antibody is an antibody comprising a heavy chain variable region and a light chain variable region in any one combination selected from the group consisting of the following combinations (a) to (d):

(a) a light chain variable region consisting of the amino acid sequence at positions 21 to 128 in the light chain full-length amino acid sequence shown in SEQ ID NO: 2 and a heavy chain variable region consisting of the amino acid sequence at positions 20 to 138 in the heavy chain full-length amino acid sequence shown in SEQ ID NO: 4;
(b) a light chain variable region consisting of the amino acid sequence at positions 21 to 128 in the light chain full-length amino acid sequence shown in SEQ ID NO: 2 and a heavy chain variable region consisting of the amino acid sequence at positions 20 to 138 in the heavy chain full-length amino acid sequence shown in SEQ ID NO: 6;
(c) a light chain variable region consisting of the amino acid sequence at positions 21 to 128 in the light chain full-length amino acid sequence shown in SEQ ID NO: 2 and a heavy chain variable region consisting of the amino acid sequence at positions 20 to 138 in the heavy chain full-length amino acid sequence shown in SEQ ID NO: 8; and
(d) a light chain variable region consisting of the amino acid sequence at positions 21 to 128 in the light chain full-length amino acid sequence shown in SEQ ID NO: 2 and a heavy chain variable region consisting of the amino acid sequence at positions 20 to 138 in the heavy chain full-length amino acid sequence shown in SEQ ID NO: 10.

[49] The method of treatment according to [47] or [48], wherein the anti-CD37 antibody is an antibody comprising a heavy chain and a light chain in any one combination selected from the group consisting of the following combinations (e) to (h):

(e) a light chain consisting of the amino acid sequence at positions 21 to 234 in the light chain full-length amino acid sequence shown in SEQ ID NO: 2 and a heavy chain consisting of the amino acid sequence at positions 20 to 468 in the heavy chain full-length amino acid sequence shown in SEQ ID NO: 4;
(f) a light chain consisting of the amino acid sequence at positions 21 to 234 in the light chain full-length amino acid sequence shown in SEQ ID NO: 2 and a heavy chain consisting of the amino acid sequence at positions 20 to 468 in the heavy chain full-length amino acid sequence shown in SEQ ID NO: 6;
(g) a light chain consisting of the amino acid sequence at positions 21 to 234 in the light chain full-length amino acid sequence shown in SEQ ID NO: 2 and a heavy chain consisting of the amino acid sequence at positions 20 to 468 in the heavy chain full-length amino acid sequence shown in SEQ ID NO: 8; and
(h) a light chain consisting of the amino acid sequence at positions 21 to 234 in the light chain full-length amino acid sequence shown in SEQ ID NO: 2 and a heavy chain consisting of the amino acid sequence at positions 20 to 468 in the heavy chain full-length amino acid sequence shown in SEQ ID NO: 10.

[50] The method of treatment according to any one of [47] to [49], wherein a lysine residue at the carboxyl terminus of the heavy chain of the anti-CD37 antibody is deleted.

[51] The method of treatment according to any one of [47] to [50], wherein the average number of units of the drug-linker conjugated per antibody in the antibody-drug conjugate is in the range of from 2 to 8.

[52] The method of treatment according to any one of [47] to [51], wherein the average number of units of the drug-linker conjugated per antibody in the antibody-drug conjugate is in the range of from 7 to 8.

[53] The method of treatment according to any one of [47] to [51], wherein the average number of units of the drug-linker conjugated per antibody in the antibody-drug conjugate is in the range of from 3.5 to 4.5.

[54] The method of treatment according to any one of [47] to [53], wherein the molecular targeting drug is one or more drugs selected from the group consisting of an anti-CD20 antibody, an anti-CD19 antibody, a BCL-2 inhibitor and a BTK inhibitor.

[55] The method of treatment according to [54], wherein the anti-CD20 antibody is rituximab or obinutuzumab.

[56] The method of treatment according to [54] or [55], wherein the anti-CD19 antibody is tafasitamab.

[57] The method of treatment according to any one of [54] to [56], wherein the BCL-2 inhibitor is venetoclax or a pharmacologically acceptable salt thereof.

[58] The method of treatment according to any one of [54] to [57], wherein the BTK inhibitor is ibrutinib or a pharmacologically acceptable salt thereof.

[59] The method of treatment according to any one of [47] to [58], wherein the alkylating agent is bendamustine or a pharmacologically acceptable salt thereof.

[60] The method of treatment according to any one of [47] to [59], wherein the immunomodulatory drug is lenalidomide or a pharmacologically acceptable salt thereof.

[61] The method of treatment according to any one of [47] to [55] and [59], wherein the other drug is a combination of

rituximab and bendamustine or a pharmacologically acceptable salt thereof.
[62] The method of treatment according to any one of [47] to [54], [56] and [60], wherein the other drug is a combination of tafasitamab and lenalidomide or a pharmacologically acceptable salt thereof.
[63] The method of treatment according to any one of [47] to [55] and [60], wherein the other drug is a combination of rituximab and lenalidomide or a pharmacologically acceptable salt thereof.
[64] The method of treatment according to any one of [47] to [55] and [57], wherein the other drug is a combination of rituximab and venetoclax or a pharmacologically acceptable salt thereof.
[65] The method of treatment according to any one of [47] to [64], wherein the antibody-drug conjugate and the one or more other drugs selected from the group consisting of a molecular targeting drug, a drug relating to R-CHOP therapy, a drug relating to R-CHP therapy, an alkylating agent and an immunomodulatory drug are separately contained as active ingredients in different formulations and administered at the same time or different times.
[66] The method of treatment according to any one of [47] to [65] for the treatment of at least one disease selected from the group consisting of diffuse large B-cell lymphoma, follicular lymphoma, mantle cell lymphoma, marginal zone lymphoma, Burkitt's lymphoma, chronic lymphocytic leukemia, T-cell lymphoma such as peripheral T-cell lymphoma or cutaneous T-cell lymphoma, myelodysplastic syndrome and acute myeloid leukemia.
[67] The method of treatment according to [66] for the treatment of diffuse large B-cell lymphoma.
[68] The method of treatment according to [66] for the treatment of chronic lymphocytic leukemia.
[69] The method of treatment according to [66] for the treatment of follicular lymphoma.
[70] A method of treatment comprising administering an anti-CD37 antibody-drug conjugate and one or more other drugs selected from the group consisting of a molecular targeting drug, a drug relating to R-CHOP therapy, a drug relating to R-CHP therapy, an alkylating agent and an immunomodulatory drug in combination to an individual in need of treatment, wherein the anti-CD37 antibody-drug conjugate is an anti-CD37 antibody-drug conjugate represented by the formula:

[Formula 4]

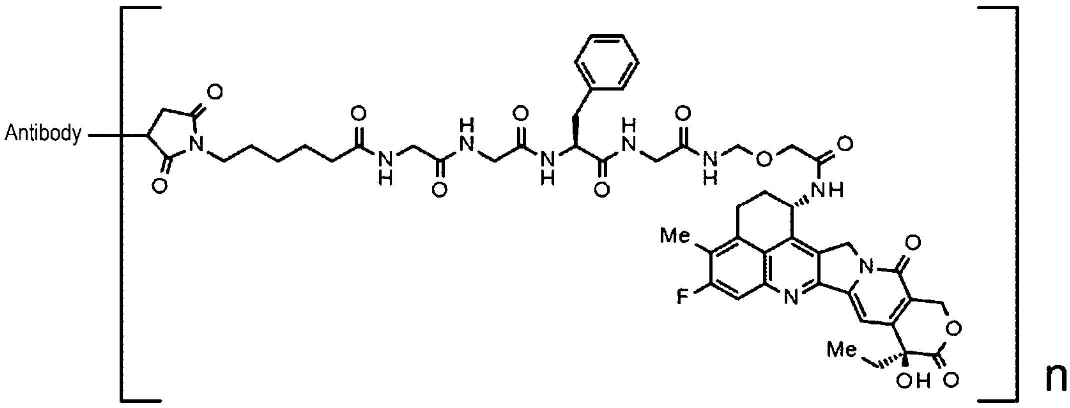

wherein Antibody represents an anti-CD37 antibody, the drug-linker is conjugated to the antibody via a thioether bond, and n represents the average number of units of the drug-linker conjugated per antibody.
[71] The method of treatment according to [70], wherein the anti-CD37 antibody is an antibody comprising a heavy chain variable region and a light chain variable region in any one combination selected from the group consisting of the following combinations (a) to (d):

(a) a light chain variable region consisting of the amino acid sequence at positions 21 to 128 in the light chain full-length amino acid sequence shown in SEQ ID NO: 2 and a heavy chain variable region consisting of the amino acid sequence at positions 20 to 138 in the heavy chain full-length amino acid sequence shown in SEQ ID NO: 4;
(b) a light chain variable region consisting of the amino acid sequence at positions 21 to 128 in the light chain full-length amino acid sequence shown in SEQ ID NO: 2 and a heavy chain variable region consisting of the amino acid sequence at positions 20 to 138 in the heavy chain full-length amino acid sequence shown in SEQ ID NO: 6;
(c) a light chain variable region consisting of the amino acid sequence at positions 21 to 128 in the light chain full-length amino acid sequence shown in SEQ ID NO: 2 and a heavy chain variable region consisting of the amino acid sequence at positions 20 to 138 in the heavy chain full-length amino acid sequence shown in SEQ ID NO: 8; and
(d) a light chain variable region consisting of the amino acid sequence at positions 21 to 128 in the light chain full-length amino acid sequence shown in SEQ ID NO: 2 and a heavy chain variable region consisting of the amino acid

acid sequence at positions 20 to 138 in the heavy chain full-length amino acid sequence shown in SEQ ID NO: 10.

[72] The method of treatment according to [70] or [71], wherein the anti-CD37 antibody is an antibody comprising a heavy chain and a light chain in any one combination selected from the group consisting of the following combinations (e) to (h):

(e) a light chain consisting of the amino acid sequence at positions 21 to 234 in the light chain full-length amino acid sequence shown in SEQ ID NO: 2 and a heavy chain consisting of the amino acid sequence at positions 20 to 468 in the heavy chain full-length amino acid sequence shown in SEQ ID NO: 4;
(f) a light chain consisting of the amino acid sequence at positions 21 to 234 in the light chain full-length amino acid sequence shown in SEQ ID NO: 2 and a heavy chain consisting of the amino acid sequence at positions 20 to 468 in the heavy chain full-length amino acid sequence shown in SEQ ID NO: 6;
(g) a light chain consisting of the amino acid sequence at positions 21 to 234 in the light chain full-length amino acid sequence shown in SEQ ID NO: 2 and a heavy chain consisting of the amino acid sequence at positions 20 to 468 in the heavy chain full-length amino acid sequence shown in SEQ ID NO: 8; and
(h) a light chain consisting of the amino acid sequence at positions 21 to 234 in the light chain full-length amino acid sequence shown in SEQ ID NO: 2 and a heavy chain consisting of the amino acid sequence at positions 20 to 468 in the heavy chain full-length amino acid sequence shown in SEQ ID NO: 10.

[73] The method of treatment according to any one of [70] to [72], wherein a lysine residue at the carboxyl terminus of the heavy chain of the anti-CD37 antibody is deleted.
[74] The method of treatment according to any one of [70] to [73], wherein the average number of units of the drug-linker conjugated per antibody in the antibody-drug conjugate is in the range of from 2 to 8.
[75] The method of treatment according to any one of [70] to [74], wherein the average number of units of the drug-linker conjugated per antibody in the antibody-drug conjugate is in the range of from 7 to 8.
[76] The method of treatment according to any one of [70] to [74], wherein the average number of units of the drug-linker conjugated per antibody in the antibody-drug conjugate is in the range of from 3.5 to 4.5.
[77] The method of treatment according to any one of [70] to [76], wherein the molecular targeting drug is one or more drugs selected from the group consisting of an anti-CD20 antibody, an anti-CD19 antibody, a BCL-2 inhibitor and a BTK inhibitor.
[78] The method of treatment according to [77], wherein the anti-CD20 antibody is rituximab or obinutuzumab.
[79] The method of treatment according to [77] or [78], wherein the anti-CD19 antibody is tafasitamab.
[80] The method of treatment according to any one of [77] to [79], wherein the BCL-2 inhibitor is venetoclax or a pharmacologically acceptable salt thereof.
[81] The method of treatment according to any one of [77] to [80], wherein the BTK inhibitor is ibrutinib or a pharmacologically acceptable salt thereof.
[82] The method of treatment according to any one of [70] to [81], wherein the alkylating agent is bendamustine or a pharmacologically acceptable salt thereof.
[83] The method of treatment according to any one of [70] to [82], wherein the immunomodulatory drug is lenalidomide or a pharmacologically acceptable salt thereof.
[84] The method of treatment according to any one of [70] to [78] and [82], wherein the other drug is a combination of rituximab and bendamustine or a pharmacologically acceptable salt thereof.
[85] The method of treatment according to any one of [70] to [77], [79] and [83], wherein the other drug is a combination of tafasitamab and lenalidomide or a pharmacologically acceptable salt thereof.
[86] The method of treatment according to any one of [70] to [78] and [83], wherein the other drug is a combination of rituximab and lenalidomide or a pharmacologically acceptable salt thereof.
[87] The method of treatment according to any one of [70] to [78] and [80], wherein the other drug is a combination of rituximab and venetoclax or a pharmacologically acceptable salt thereof.
[88] The method of treatment according to any one of [70] to [87], wherein the antibody-drug conjugate and the one or more other drugs selected from the group consisting of a molecular targeting drug, a drug relating to R-CHOP therapy, a drug relating to R-CHP therapy, an alkylating agent and an immunomodulatory drug are separately contained as active ingredients in different formulations and administered at the same time or different times.
[89] The method of treatment according to any one of [70] to [88] for the treatment of at least one disease selected from the group consisting of diffuse large B-cell lymphoma, follicular lymphoma, mantle cell lymphoma, marginal zone lymphoma, Burkitt's lymphoma, chronic lymphocytic leukemia, T-cell lymphoma such as peripheral T-cell lymphoma or cutaneous T-cell lymphoma, myelodysplastic syndrome and acute myeloid leukemia.
[90] The method of treatment according to [89] for the treatment of diffuse large B-cell lymphoma.
[91] The method of treatment according to [89] for the treatment of chronic lymphocytic leukemia.

[92] The method of treatment according to [89] for the treatment of follicular lymphoma.
[93] An anti-CD37 antibody-drug conjugate for the treatment of cancer, for use in combination with another drug, wherein the anti-CD37 antibody-drug conjugate is an anti-CD37 antibody-drug conjugate in which a drug-linker represented by the formula is conjugated to an anti-CD37 antibody via a thioether bond:

[Formula 5]

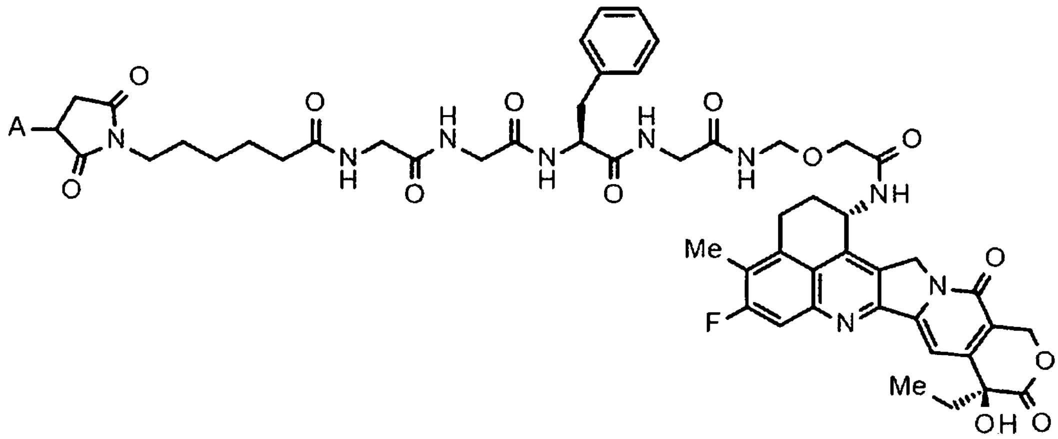

wherein A represents a connecting position to the anti-CD37 antibody, and
the other drug is one or more other drugs selected from the group consisting of a molecular targeting drug, a drug relating to R-CHOP therapy, a drug relating to R-CHP therapy, an alkylating agent and an immunomodulatory drug.

[94] The antibody-drug conjugate according to [93], wherein the anti-CD37 antibody is an antibody comprising a heavy chain variable region and a light chain variable region in any one combination selected from the group consisting of the following combinations (a) to (d):

(a) a light chain variable region consisting of the amino acid sequence at positions 21 to 128 in the light chain full-length amino acid sequence shown in SEQ ID NO: 2 and a heavy chain variable region consisting of the amino acid sequence at positions 20 to 138 in the heavy chain full-length amino acid sequence shown in SEQ ID NO: 4;
(b) a light chain variable region consisting of the amino acid sequence at positions 21 to 128 in the light chain full-length amino acid sequence shown in SEQ ID NO: 2 and a heavy chain variable region consisting of the amino acid sequence at positions 20 to 138 in the heavy chain full-length amino acid sequence shown in SEQ ID NO: 6;
(c) a light chain variable region consisting of the amino acid sequence at positions 21 to 128 in the light chain full-length amino acid sequence shown in SEQ ID NO: 2 and a heavy chain variable region consisting of the amino acid sequence at positions 20 to 138 in the heavy chain full-length amino acid sequence shown in SEQ ID NO: 8; and
(d) a light chain variable region consisting of the amino acid sequence at positions 21 to 128 in the light chain full-length amino acid sequence shown in SEQ ID NO: 2 and a heavy chain variable region consisting of the amino acid sequence at positions 20 to 138 in the heavy chain full-length amino acid sequence shown in SEQ ID NO: 10.

[95] The antibody-drug conjugate according to [93] or [94], wherein the anti-CD37 antibody is an antibody comprising a heavy chain and a light chain in any one combination selected from the group consisting of the following combinations (e) to (h):

(e) a light chain consisting of the amino acid sequence at positions 21 to 234 in the light chain full-length amino acid sequence shown in SEQ ID NO: 2 and a heavy chain consisting of the amino acid sequence at positions 20 to 468 in the heavy chain full-length amino acid sequence shown in SEQ ID NO: 4;
(f) a light chain consisting of the amino acid sequence at positions 21 to 234 in the light chain full-length amino acid sequence shown in SEQ ID NO: 2 and a heavy chain consisting of the amino acid sequence at positions 20 to 468 in the heavy chain full-length amino acid sequence shown in SEQ ID NO: 6;
(g) a light chain consisting of the amino acid sequence at positions 21 to 234 in the light chain full-length amino acid sequence shown in SEQ ID NO: 2 and a heavy chain consisting of the amino acid sequence at positions 20 to 468 in the heavy chain full-length amino acid sequence shown in SEQ ID NO: 8; and

(h) a light chain consisting of the amino acid sequence at positions 21 to 234 in the light chain full-length amino acid sequence shown in SEQ ID NO: 2 and a heavy chain consisting of the amino acid sequence at positions 20 to 468 in the heavy chain full-length amino acid sequence shown in SEQ ID NO: 10.

[96] The antibody-drug conjugate according to any one of [93] to [95], wherein a lysine residue at the carboxyl terminus of the heavy chain of the anti-CD37 antibody is deleted.
[97] The antibody-drug conjugate according to any one of [93] to [96], wherein the average number of units of the drug-linker conjugated per antibody in the anti-CD37 antibody-drug conjugate is in the range of from 2 to 8.
[98] The antibody-drug conjugate according to any one of [93] to [97], wherein the average number of units of the drug-linker conjugated per antibody in the anti-CD37 antibody-drug conjugate is in the range of from 7 to 8.
[99] The antibody-drug conjugate according to any one of [93] to [97], wherein the average number of units of the drug-linker conjugated per antibody in the anti-CD37 antibody-drug conjugate is in the range of from 3.5 to 4.5.
[100] The antibody-drug conjugate according to any one of [93] to [99], wherein the molecular targeting drug is one or more drugs selected from the group consisting of an anti-CD20 antibody, an anti-CD19 antibody, a BCL-2 inhibitor and a BTK inhibitor.
[101] The antibody-drug conjugate according to [100], wherein the anti-CD20 antibody is rituximab or obinutuzumab.
[102] The antibody-drug conjugate according to [100] or [101], wherein the anti-CD19 antibody is tafasitamab.
[103] The antibody-drug conjugate according to any one of [100] to [102], wherein the BCL-2 inhibitor is venetoclax or a pharmacologically acceptable salt thereof.
[104] The antibody-drug conjugate according to any one of [100] to [103], wherein the BTK inhibitor is ibrutinib or a pharmacologically acceptable salt thereof.
[105] The antibody-drug conjugate according to any one of [93] to [104], wherein the alkylating agent is bendamustine or a pharmacologically acceptable salt thereof.
[106] The antibody-drug conjugate according to any one of [93] to [105], wherein the immunomodulatory drug is lenalidomide or a pharmacologically acceptable salt thereof.
[107] The antibody-drug conjugate according to any one of [93] to [101] and [105], wherein the other drug is a combination of rituximab and bendamustine or a pharmacologically acceptable salt thereof.
[108] The antibody-drug conjugate according to any one of [93] to [100], [102] and [106], wherein the other drug is a combination of tafasitamab and lenalidomide or a pharmacologically acceptable salt thereof.
[109] The antibody-drug conjugate according to any one of [93] to [101] and [106], wherein the other drug is a combination of rituximab and lenalidomide or a pharmacologically acceptable salt thereof.
[110] The antibody-drug conjugate according to any one of [93] to [101] and [103], wherein the other drug is a combination of rituximab and venetoclax or a pharmacologically acceptable salt thereof.
[111] The antibody-drug conjugate according to any one of [93] to [110], wherein the antibody-drug conjugate and the one or more other drugs selected from the group consisting of a molecular targeting drug, a drug relating to R-CHOP therapy, a drug relating to R-CHP therapy, an alkylating agent and an immunomodulatory drug are separately contained as active ingredients in different formulations and administered at the same time or different times.
[112] The antibody-drug conjugate according to any one of [93] to [111] for the treatment of at least one disease selected from the group consisting of diffuse large B-cell lymphoma, follicular lymphoma, mantle cell lymphoma, marginal zone lymphoma, Burkitt's lymphoma, chronic lymphocytic leukemia, T-cell lymphoma such as peripheral T-cell lymphoma or cutaneous T-cell lymphoma, myelodysplastic syndrome and acute myeloid leukemia.
[113] The antibody-drug conjugate according to [112] for the treatment of diffuse large B-cell lymphoma.
[114] The antibody-drug conjugate according to [112] for the treatment of chronic lymphocytic leukemia.
[115] The antibody-drug conjugate according to [112] for the treatment of follicular lymphoma.
[116] An anti-CD37 antibody-drug conjugate for use in combination with another drug for the treatment of cancer, wherein

the anti-CD37 antibody-drug conjugate is an anti-CD37 antibody-drug conjugate represented by the formula:

[Formula 6]

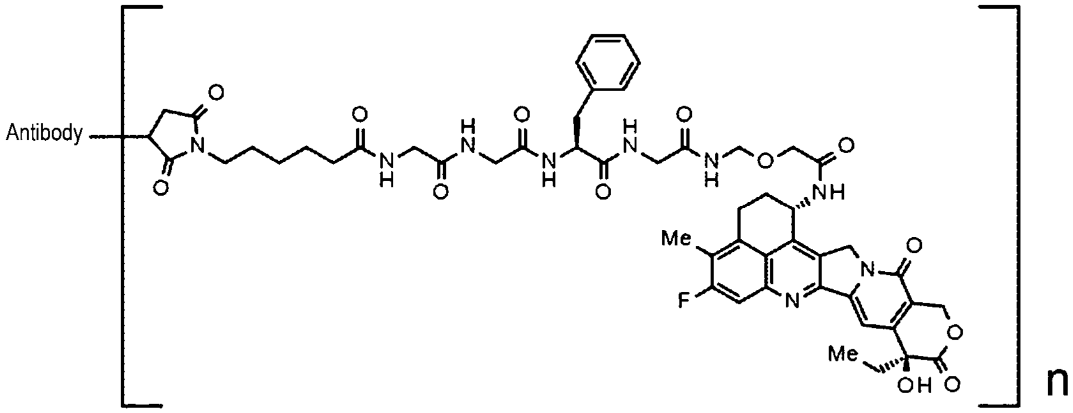

wherein Antibody represents an anti-CD37 antibody conjugated to the drug linker through a thioether bond, and n represents the average number of units of the drug-linker conjugated per antibody, and

the other drug is one or more other drugs selected from the group consisting of a molecular targeting drug, a drug relating to R-CHOP therapy, a drug relating to R-CHP therapy, an alkylating agent and an immunomodulatory drug.

[117] The antibody-drug conjugate according to [116], wherein the anti-CD37 antibody is an antibody comprising a heavy chain variable region and a light chain variable region in any one combination selected from the group consisting of the following combinations (a) to (d):

(a) a light chain variable region consisting of the amino acid sequence at positions 21 to 128 in the light chain full-length amino acid sequence shown in SEQ ID NO: 2 and a heavy chain variable region consisting of the amino acid sequence at positions 20 to 138 in the heavy chain full-length amino acid sequence shown in SEQ ID NO: 4;
(b) a light chain variable region consisting of the amino acid sequence at positions 21 to 128 in the light chain full-length amino acid sequence shown in SEQ ID NO: 2 and a heavy chain variable region consisting of the amino acid sequence at positions 20 to 138 in the heavy chain full-length amino acid sequence shown in SEQ ID NO: 6;
(c) a light chain variable region consisting of the amino acid sequence at positions 21 to 128 in the light chain full-length amino acid sequence shown in SEQ ID NO: 2 and a heavy chain variable region consisting of the amino acid sequence at positions 20 to 138 in the heavy chain full-length amino acid sequence shown in SEQ ID NO: 8; and
(d) a light chain variable region consisting of the amino acid sequence at positions 21 to 128 in the light chain full-length amino acid sequence shown in SEQ ID NO: 2 and a heavy chain variable region consisting of the amino acid sequence at positions 20 to 138 in the heavy chain full-length amino acid sequence shown in SEQ ID NO: 10.

[118] The antibody-drug conjugate according to [116] or [117], wherein the anti-CD37 antibody is an antibody comprising a heavy chain and a light chain in any one combination selected from the group consisting of the following combinations (e) to (h):

(e) a light chain consisting of the amino acid sequence at positions 21 to 234 in the light chain full-length amino acid sequence shown in SEQ ID NO: 2 and a heavy chain consisting of the amino acid sequence at positions 20 to 468 in the heavy chain full-length amino acid sequence shown in SEQ ID NO: 4;
(f) a light chain consisting of the amino acid sequence at positions 21 to 234 in the light chain full-length amino acid sequence shown in SEQ ID NO: 2 and a heavy chain consisting of the amino acid sequence at positions 20 to 468 in the heavy chain full-length amino acid sequence shown in SEQ ID NO: 6;
(g) a light chain consisting of the amino acid sequence at positions 21 to 234 in the light chain full-length amino acid sequence shown in SEQ ID NO: 2 and a heavy chain consisting of the amino acid sequence at positions 20 to 468 in the heavy chain full-length amino acid sequence shown in SEQ ID NO: 8; and
(h) a light chain consisting of the amino acid sequence at positions 21 to 234 in the light chain full-length amino acid sequence shown in SEQ ID NO: 2 and a heavy chain consisting of the amino acid sequence at positions 20 to 468 in the heavy chain full-length amino acid sequence shown in SEQ ID NO: 10.

[119] The antibody-drug conjugate according to any one of [116] to [118], wherein a lysine residue at the carboxyl

terminus of the heavy chain of the anti-CD37 antibody is deleted.

[120] The antibody-drug conjugate according to any one of [116] to [119], wherein the average number of units of the drug-linker conjugated per antibody in the antibody-drug conjugate is in the range of from 2 to 8.

[121] The antibody-drug conjugate according to any one of [116] to [120], wherein the average number of units of the drug-linker conjugated per antibody in the antibody-drug conjugate is in the range of from 7 to 8.

[122] The antibody-drug conjugate according to any one of [116] to [120], wherein the average number of units of the drug-linker conjugated per antibody in the antibody-drug conjugate is in the range of from 3.5 to 4.5.

[123] The antibody-drug conjugate according to any one of [116] to [122], wherein the molecular targeting drug is one or more drugs selected from the group consisting of an anti-CD20 antibody, an anti-CD19 antibody, a BCL-2 inhibitor and a BTK inhibitor.

[124] The antibody-drug conjugate according to [123], wherein the anti-CD20 antibody is rituximab or obinutuzumab.

[125] The antibody-drug conjugate according to [123] or [124], wherein the anti-CD19 antibody is tafasitamab.

[126] The antibody-drug conjugate according to any one of [123] to [125], wherein the BCL-2 inhibitor is venetoclax or a pharmacologically acceptable salt thereof.

[127] The antibody-drug conjugate according to any one of [123] to [126], wherein the BTK inhibitor is ibrutinib or a pharmacologically acceptable salt thereof.

[128] The antibody-drug conjugate according to any one of [116] to [127], wherein the alkylating agent is bendamustine or a pharmacologically acceptable salt thereof.

[129] The antibody-drug conjugate according to any one of [116] to [128], wherein the immunomodulatory drug is lenalidomide or a pharmacologically acceptable salt thereof.

[130] The antibody-drug conjugate according to any one of [116] to [124] and [128], wherein the other drug is a combination of rituximab and bendamustine or a pharmacologically acceptable salt thereof.

[131] The antibody-drug conjugate according to any one of [116] to [123], [125] and [129], wherein the other drug is a combination of tafasitamab and lenalidomide or a pharmacologically acceptable salt thereof.

[132] The antibody-drug conjugate according to any one of [116] to [124] and [129], wherein the other drug is a combination of rituximab and lenalidomide or a pharmacologically acceptable salt thereof.

[133] The antibody-drug conjugate according to any one of [116] to [124] and [126], wherein the other drug is a combination of rituximab and venetoclax or a pharmacologically acceptable salt thereof.

[134] The antibody-drug conjugate according to any one of [116] to [133], wherein the antibody-drug conjugate and the one or more other drugs selected from the group consisting of a molecular targeting drug, a drug relating to R-CHOP therapy, a drug relating to R-CHP therapy, an alkylating agent and an immunomodulatory drug are separately contained as active ingredients in different formulations and administered at the same time or different times.

[135] The antibody-drug conjugate according to any one of [116] to [134] for the treatment of at least one disease selected from the group consisting of diffuse large B-cell lymphoma, follicular lymphoma, mantle cell lymphoma, marginal zone lymphoma, Burkitt's lymphoma, chronic lymphocytic leukemia, T-cell lymphoma such as peripheral T-cell lymphoma or cutaneous T-cell lymphoma, myelodysplastic syndrome and acute myeloid leukemia.

[136] The antibody-drug conjugate according to [135] for the treatment of diffuse large B-cell lymphoma.

[137] The antibody-drug conjugate according to [135] for the treatment of chronic lymphocytic leukemia.

[138] The antibody-drug conjugate according to [135] for the treatment of follicular lymphoma.

[139] Use of an anti-CD37 antibody-drug conjugate in combination with another drug in the production of a medicament for the treatment of cancer, wherein the anti-CD37 antibody-drug conjugate is an anti-CD37 antibody-drug conjugate in which a drug-linker represented by the formula and an anti-CD37 antibody are conjugated via a thioether bond:

[Formula 7]

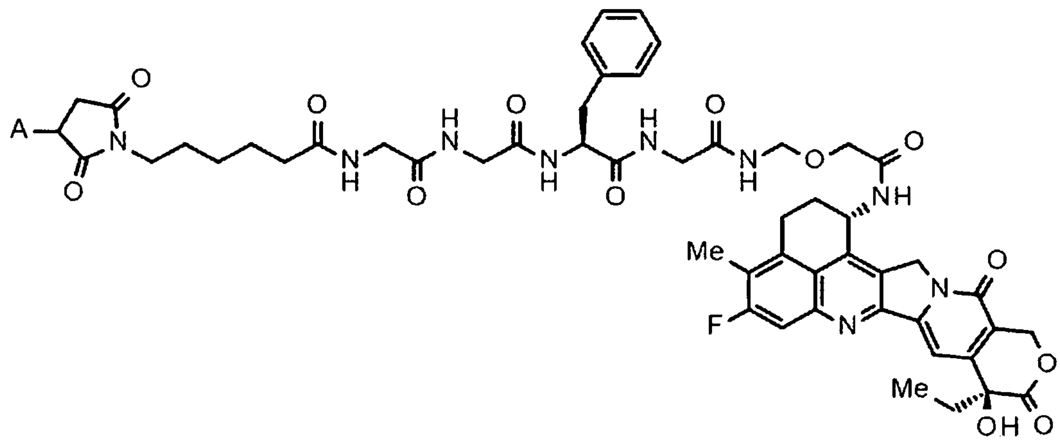

wherein A represents a connecting position to the anti-CD37 antibody, and
the other drug is one or more other drugs selected from the group consisting of a molecular targeting drug, a drug relating to R-CHOP therapy, a drug relating to R-CHP therapy, an alkylating agent and an immunomodulatory drug.

[140] The use according to [139], wherein the anti-CD37 antibody is an antibody comprising a heavy chain variable region and a light chain variable region in any one combination selected from the group consisting of the following combinations (a) to (d):

(a) a light chain variable region consisting of the amino acid sequence at positions 21 to 128 in the light chain full-length amino acid sequence shown in SEQ ID NO: 2 and a heavy chain variable region consisting of the amino acid sequence at positions 20 to 138 in the heavy chain full-length amino acid sequence shown in SEQ ID NO: 4;
(b) a light chain variable region consisting of the amino acid sequence at positions 21 to 128 in the light chain full-length amino acid sequence shown in SEQ ID NO: 2 and a heavy chain variable region consisting of the amino acid sequence at positions 20 to 138 in the heavy chain full-length amino acid sequence shown in SEQ ID NO: 6;
(c) a light chain variable region consisting of the amino acid sequence at positions 21 to 128 in the light chain full-length amino acid sequence shown in SEQ ID NO: 2 and a heavy chain variable region consisting of the amino acid sequence at positions 20 to 138 in the heavy chain full-length amino acid sequence shown in SEQ ID NO: 8; and
(d) a light chain variable region consisting of the amino acid sequence at positions 21 to 128 in the light chain full-length amino acid sequence shown in SEQ ID NO: 2 and a heavy chain variable region consisting of the amino acid sequence at positions 20 to 138 in the heavy chain full-length amino acid sequence shown in SEQ ID NO: 10.

[141] The use according to [139] or [140], wherein the anti-CD37 antibody is an antibody comprising a heavy chain and a light chain in any one combination selected from the group consisting of the following combinations (e) to (h):

(e) a light chain consisting of the amino acid sequence at positions 21 to 234 in the light chain full-length amino acid sequence shown in SEQ ID NO: 2 and a heavy chain consisting of the amino acid sequence at positions 20 to 468 in the heavy chain full-length amino acid sequence shown in SEQ ID NO: 4;
(f) a light chain consisting of the amino acid sequence at positions 21 to 234 in the light chain full-length amino acid sequence shown in SEQ ID NO: 2 and a heavy chain consisting of the amino acid sequence at positions 20 to 468 in the heavy chain full-length amino acid sequence shown in SEQ ID NO: 6;
(g) a light chain consisting of the amino acid sequence at positions 21 to 234 in the light chain full-length amino acid sequence shown in SEQ ID NO: 2 and a heavy chain consisting of the amino acid sequence at positions 20 to 468 in the heavy chain full-length amino acid sequence shown in SEQ ID NO: 8; and
(h) a light chain consisting of the amino acid sequence at positions 21 to 234 in the light chain full-length amino acid sequence shown in SEQ ID NO: 2 and a heavy chain consisting of the amino acid sequence at positions 20 to 468 in the heavy chain full-length amino acid sequence shown in SEQ ID NO: 10.

[142] The use according to any one of [139] to [141], wherein a lysine residue at the carboxyl terminus of the heavy chain of the anti-CD37 antibody is deleted.

[143] The use according to any one of [139] to [142], wherein the average number of units of the drug-linker conjugated per antibody in the anti-CD37 antibody-drug conjugate is in the range of from 2 to 8.

[144] The use according to any one of [139] to [143], wherein the average number of units of the drug-linker conjugated per antibody in the anti-CD37 antibody-drug conjugate is in the range of from 7 to 8.

[145] The use according to any one of [139] to [143], wherein the average number of units of the drug-linker conjugated per antibody in the anti-CD37 antibody-drug conjugate is in the range of from 3.5 to 4.5.

[146] The use according to any one of [139] to [145], wherein the molecular targeting drug is one or more drugs selected from the group consisting of an anti-CD20 antibody, an anti-CD19 antibody, a BCL-2 inhibitor and a BTK inhibitor.

[147] The use according to [146], wherein the anti-CD20 antibody is rituximab or obinutuzumab.

[148] The use according to [146] or [147], wherein the anti-CD19 antibody is tafasitamab.

[149] The use according to any one of [146] to [148], wherein the BCL-2 inhibitor is venetoclax or a pharmacologically acceptable salt thereof.

[150] The use according to any one of [146] to [149], wherein the BTK inhibitor is ibrutinib or a pharmacologically acceptable salt thereof.

[151] The use according to any one of [139] to [150], wherein the alkylating agent is bendamustine or a pharmacologically acceptable salt thereof.

[152] The use according to any one of [139] to [151], wherein the immunomodulatory drug is lenalidomide or a pharmacologically acceptable salt thereof.

[153] The use according to any one of [139] to [147] and [151], wherein the other drug is a combination of rituximab and bendamustine or a pharmacologically acceptable salt thereof.

[154] The use according to any one of [139] to [146], [148] and [152], wherein the other drug is a combination of tafasitamab and lenalidomide or a pharmacologically acceptable salt thereof.

[155] The use according to any one of [139] to [147] and [152], wherein the other drug is a combination of rituximab and lenalidomide or a pharmacologically acceptable salt thereof.

[156] The use according to any one of [139] to [147] and [149], wherein the other drug is a combination of rituximab and venetoclax or a pharmacologically acceptable salt thereof.

[157] The use according to any one of [139] to [156], wherein the antibody-drug conjugate and the one or more other drugs selected from the group consisting of a molecular targeting drug, a drug relating to R-CHOP therapy, a drug relating to R-CHP therapy, an alkylating agent and an immunomodulatory drug are separately contained as active ingredients in different formulations and administered at the same time or different times.

[158] The use according to any one of [139] to [157] for the treatment of at least one disease selected from the group consisting of diffuse large B-cell lymphoma, follicular lymphoma, mantle cell lymphoma, marginal zone lymphoma, Burkitt's lymphoma, chronic lymphocytic leukemia, T-cell lymphoma such as peripheral T-cell lymphoma or cutaneous T-cell lymphoma, myelodysplastic syndrome and acute myeloid leukemia.

[159] The use according to [158] for the treatment of diffuse large B-cell lymphoma.

[160] The use according to [158] for the treatment of chronic lymphocytic leukemia.

[161] The use according to [158] for the treatment of follicular lymphoma.

[162] Use of an anti-CD37 antibody-drug conjugate in combination with another drug in the production of a medicament for the treatment of cancer, wherein the anti-CD37 antibody-drug conjugate is an anti-CD37 antibody-drug conjugate represented by the formula:

[Formula 8]

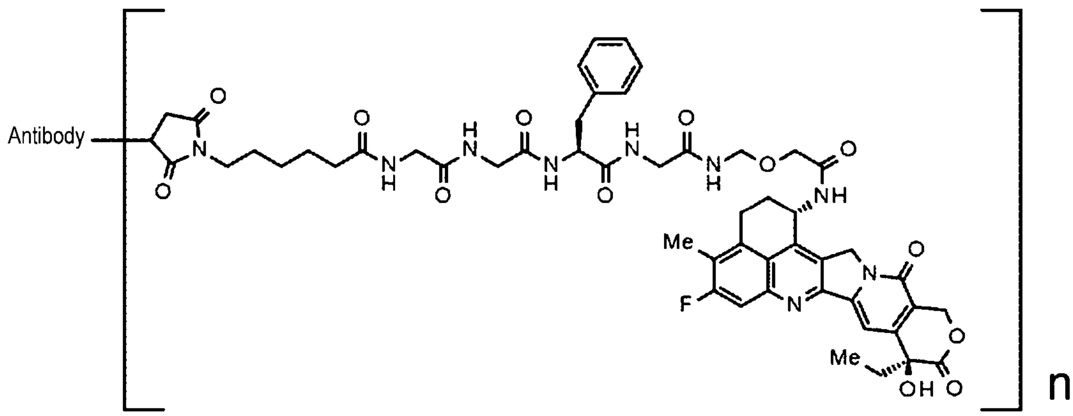

wherein Antibody represents an anti-CD37 antibody, the drug linker is conjugated to the antibody through a thioether bond, and n represents the average number of units of the drug-linker conjugated per antibody, and the other drug is one or more other drugs selected from the group consisting of a molecular targeting drug, a drug relating to R-CHOP therapy, a drug relating to R-CHP therapy, an alkylating agent and an immunomodulatory drug.
[163] The use according to [162], wherein the anti-CD37 antibody is an antibody comprising a heavy chain variable region and a light chain variable region in any one combination selected from the group consisting of the following combinations (a) to (d):

(a) a light chain variable region consisting of the amino acid sequence at positions 21 to 128 in the light chain full-length amino acid sequence shown in SEQ ID NO: 2 and a heavy chain variable region consisting of the amino acid sequence at positions 20 to 138 in the heavy chain full-length amino acid sequence shown in SEQ ID NO: 4;
(b) a light chain variable region consisting of the amino acid sequence at positions 21 to 128 in the light chain full-length amino acid sequence shown in SEQ ID NO: 2 and a heavy chain variable region consisting of the amino acid sequence at positions 20 to 138 in the heavy chain full-length amino acid sequence shown in SEQ ID NO: 6;
(c) a light chain variable region consisting of the amino acid sequence at positions 21 to 128 in the light chain full-length amino acid sequence shown in SEQ ID NO: 2 and a heavy chain variable region consisting of the amino acid sequence at positions 20 to 138 in the heavy chain full-length amino acid sequence shown in SEQ ID NO: 8; and
(d) a light chain variable region consisting of the amino acid sequence at positions 21 to 128 in the light chain full-length amino acid sequence shown in SEQ ID NO: 2 and a heavy chain variable region consisting of the amino acid sequence at positions 20 to 138 in the heavy chain full-length amino acid sequence shown in SEQ ID NO: 10.

[164] The use according to [162] or [163], wherein the anti-CD37 antibody is an antibody comprising a heavy chain and a light chain in any one combination selected from the group consisting of the following combinations (e) to (h):

(e) a light chain consisting of the amino acid sequence at positions 21 to 234 in the light chain full-length amino acid sequence shown in SEQ ID NO: 2 and a heavy chain consisting of the amino acid sequence at positions 20 to 468 in the heavy chain full-length amino acid sequence shown in SEQ ID NO: 4;
(f) a light chain consisting of the amino acid sequence at positions 21 to 234 in the light chain full-length amino acid sequence shown in SEQ ID NO: 2 and a heavy chain consisting of the amino acid sequence at positions 20 to 468 in the heavy chain full-length amino acid sequence shown in SEQ ID NO: 6;
(g) a light chain consisting of the amino acid sequence at positions 21 to 234 in the light chain full-length amino acid sequence shown in SEQ ID NO: 2 and a heavy chain consisting of the amino acid sequence at positions 20 to 468 in the heavy chain full-length amino acid sequence shown in SEQ ID NO: 8; and
(h) a light chain consisting of the amino acid sequence at positions 21 to 234 in the light chain full-length amino acid sequence shown in SEQ ID NO: 2 and a heavy chain consisting of the amino acid sequence at positions 20 to 468 in the heavy chain full-length amino acid sequence shown in SEQ ID NO: 10.

[165] The use according to any one of [162] to [164], wherein a lysine residue at the carboxyl terminus of the heavy chain of the anti-CD37 antibody is deleted.
[166] The use according to any one of [162] to [165], wherein the average number of units of the drug-linker conjugated per antibody in the antibody-drug conjugate is in the range of from 2 to 8.
[167] The use according to any one of [162] to [166], wherein the average number of units of the drug-linker conjugated per antibody in the antibody-drug conjugate is in the range of from 7 to 8.
[168] The use according to any one of [162] to [166], wherein the average number of units of the drug-linker conjugated per antibody in the antibody-drug conjugate is in the range of from 3.5 to 4.5.
[169] The use according to any one of [162] to [168], wherein the molecular targeting drug is one or more drugs selected from the group consisting of an anti-CD20 antibody, an anti-CD19 antibody, a BCL-2 inhibitor and a BTK inhibitor.
[170] The use according to [169], wherein the anti-CD20 antibody is rituximab or obinutuzumab.
[171] The use according to [169] or [170], wherein the anti-CD19 antibody is tafasitamab.
[172] The use according to any one of [169] to [171], wherein the BCL-2 inhibitor is venetoclax or a pharmacologically acceptable salt thereof.
[173] The use according to any one of [169] to [172], wherein the BTK inhibitor is ibrutinib or a pharmacologically acceptable salt thereof.
[174] The use according to any one of [162] to [173], wherein the alkylating agent is bendamustine or a pharmacologically acceptable salt thereof.
[175] The use according to any one of [162] to [174], wherein the immunomodulatory drug is lenalidomide or a

pharmacologically acceptable salt thereof.
[176] The use according to any one of [162] to [170] and [174] , wherein the other drug is a combination of rituximab and bendamustine or a pharmacologically acceptable salt thereof.
[177] The use according to any one of [162] to [169], [171] and [175], wherein the other drug is a combination of tafasitamab and lenalidomide or a pharmacologically acceptable salt thereof.
[178] The use according to any one of [162] to [170] and [175], wherein the other drug is a combination of rituximab and lenalidomide or a pharmacologically acceptable salt thereof.
[179] The use according to any one of [162] to [170] and [172], wherein the other drug is a combination of rituximab and venetoclax or a pharmacologically acceptable salt thereof.
[180] The use according to any one of [162] to [179], wherein the antibody-drug conjugate and the one or more other drugs selected from the group consisting of a molecular targeting drug, a drug relating to R-CHOP therapy, a drug relating to R-CHP therapy, an alkylating agent and an immunomodulatory drug are separately contained as active ingredients in different formulations and administered at the same time or different times.
[181] The use according to any one of [162] to [180] for the treatment of at least one disease selected from the group consisting of diffuse large B-cell lymphoma, follicular lymphoma, mantle cell lymphoma, marginal zone lymphoma, Burkitt's lymphoma, chronic lymphocytic leukemia, T-cell lymphoma such as peripheral T-cell lymphoma or cutaneous T-cell lymphoma, myelodysplastic syndrome and acute myeloid leukemia.
[182] The use according to [181] for the treatment of diffuse large B-cell lymphoma.
[183] The use according to [181] for the treatment of chronic lymphocytic leukemia.
[184] The use according to [181] for the treatment of follicular lymphoma.

The present invention further includes the following aspects of the invention.

[A1] A pharmaceutical composition comprising an anti-CD37 antibody-drug conjugate, wherein the anti-CD37 antibody-drug conjugate is administered in combination with one or more other drugs selected from the group consisting of a molecular targeting drug, a drug relating to R-CHOP therapy, a drug relating to R-CHP therapy, an alkylating agent and an immunomodulatory drug.
[A2] The pharmaceutical composition according to [A1], wherein the drug of the anti-CD37 antibody-drug conjugate is a topoisomerase I inhibitor.
[A3] The pharmaceutical composition according to [A2], wherein a drug represented by the following formula:

[Formula 9]

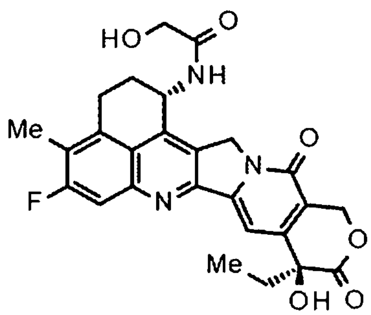

is released and thereby exerts antitumor activity.
[A4] The pharmaceutical composition according to [A2], wherein the anti-CD37 antibody is conjugated to an antitumor compound represented by the following formula:

[Formula 10]

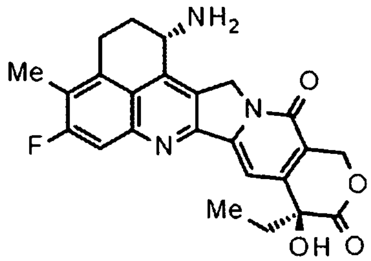

via a linker with the nitrogen atom of the amino group at position 1 as a connecting position.

[A5] The pharmaceutical composition according to [A4], wherein the anti-CD37 antibody is conjugated to the antitumor compound via a linker through a thioether bond formed in a disulfide bond moiety present in the anti-CD37 antibody.

[A6] The pharmaceutical composition according to [A4] or [A5], wherein the linker comprises a tetrapeptide residue of -Gly-Gly-Phe-Gly-.

[A7] The pharmaceutical composition according to any one of [A1] to [A6], wherein the anti-CD37 antibody-drug conjugate is administered in combination with one or more other drugs selected from the group consisting of a molecular targeting drug, a drug relating to R-CHOP therapy, a drug relating to R-CHP therapy, an alkylating agent and an immunomodulatory drug, and

the anti-CD37 antibody-drug conjugate is an anti-CD37 antibody-drug conjugate in which a drug-linker represented by the formula and an anti-CD37 antibody are conjugated via a thioether bond:

[Formula 11]

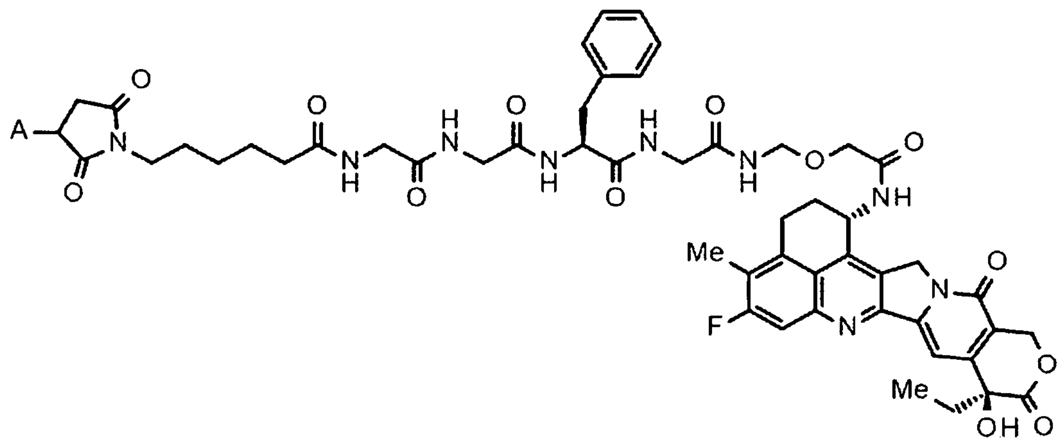

wherein A represents a connecting position to the anti-CD37 antibody.

[A8] The pharmaceutical composition according to [A7], wherein the anti-CD37 antibody is an antibody comprising a heavy chain variable region and a light chain variable region in any one combination selected from the group consisting of the following combinations (a) to (d):

(a) a light chain variable region consisting of the amino acid sequence at positions 21 to 128 in the light chain full-length amino acid sequence shown in SEQ ID NO: 2 and a heavy chain variable region consisting of the amino acid sequence at positions 20 to 138 in the heavy chain full-length amino acid sequence shown in SEQ ID NO: 4;
(b) a light chain variable region consisting of the amino acid sequence at positions 21 to 128 in the light chain full-length amino acid sequence shown in SEQ ID NO: 2 and a heavy chain variable region consisting of the amino acid sequence at positions 20 to 138 in the heavy chain full-length amino acid sequence shown in SEQ ID NO: 6;
(c) a light chain variable region consisting of the amino acid sequence at positions 21 to 128 in the light chain full-length amino acid sequence shown in SEQ ID NO: 2 and a heavy chain variable region consisting of the amino acid sequence at positions 20 to 138 in the heavy chain full-length amino acid sequence shown in SEQ ID NO: 8; and
(d) a light chain variable region consisting of the amino acid sequence at positions 21 to 128 in the light chain full-length amino acid sequence shown in SEQ ID NO: 2 and a heavy chain variable region consisting of the amino acid sequence at positions 20 to 138 in the heavy chain full-length amino acid sequence shown in SEQ ID NO: 10.

[A9] The pharmaceutical composition according to [A7] or [A8], wherein the anti-CD37 antibody is an antibody comprising a heavy chain variable region and a light chain variable region in the following combination (a):

(a) a light chain variable region consisting of the amino acid sequence at positions 21 to 128 in the light chain full-length amino acid sequence shown in SEQ ID NO: 2 and a heavy chain variable region consisting of the amino acid sequence at positions 20 to 138 in the heavy chain full-length amino acid sequence shown in SEQ ID NO: 4.
[A10] The pharmaceutical composition according to [A7] or [A8], wherein the anti-CD37 antibody is an antibody comprising a heavy chain variable region and a light chain variable region in the following combination (b):
(b) a light chain variable region consisting of the amino acid sequence at positions 21 to 128 in the light chain full-length amino acid sequence shown in SEQ ID NO: 2 and a heavy chain variable region consisting of the amino acid sequence at positions 20 to 138 in the heavy chain full-length amino acid sequence shown in SEQ ID NO: 6.
[A11] The pharmaceutical composition according to [A7] or [A8], wherein the anti-CD37 antibody is an antibody comprising a heavy chain variable region and a light chain variable region in the following combination (c):
(c) a light chain variable region consisting of the amino acid sequence at positions 21 to 128 in the light chain full-length amino acid sequence shown in SEQ ID NO: 2 and a heavy chain variable region consisting of the amino acid sequence at positions 20 to 138 in the heavy chain full-length amino acid sequence shown in SEQ ID NO: 8.
[A12] The pharmaceutical composition according to [A7] or [A8], wherein the anti-CD37 antibody is an antibody comprising a heavy chain variable region and a light chain variable region in the following combination (d):
(d) a light chain variable region consisting of the amino acid sequence at positions 21 to 128 in the light chain full-length amino acid sequence shown in SEQ ID NO: 2 and a heavy chain variable region consisting of the amino acid sequence at positions 20 to 138 in the heavy chain full-length amino acid sequence shown in SEQ ID NO: 10.

[A13] The pharmaceutical composition according to [A7] or [A8], wherein the anti-CD37 antibody is an antibody comprising a heavy chain and a light chain in any one combination selected from the group consisting of the following combinations (e) to (h):

(e) a light chain consisting of the amino acid sequence at positions 21 to 234 in the light chain full-length amino acid sequence shown in SEQ ID NO: 2 and a heavy chain consisting of the amino acid sequence at positions 20 to 468 in the heavy chain full-length amino acid sequence shown in SEQ ID NO: 4;
(f) a light chain consisting of the amino acid sequence at positions 21 to 234 in the light chain full-length amino acid sequence shown in SEQ ID NO: 2 and a heavy chain consisting of the amino acid sequence at positions 20 to 468 in the heavy chain full-length amino acid sequence shown in SEQ ID NO: 6;
(g) a light chain consisting of the amino acid sequence at positions 21 to 234 in the light chain full-length amino acid sequence shown in SEQ ID NO: 2 and a heavy chain consisting of the amino acid sequence at positions 20 to 468 in the heavy chain full-length amino acid sequence shown in SEQ ID NO: 8; and
(h) a light chain consisting of the amino acid sequence at positions 21 to 234 in the light chain full-length amino acid sequence shown in SEQ ID NO: 2 and a heavy chain consisting of the amino acid sequence at positions 20 to 468 in the heavy chain full-length amino acid sequence shown in SEQ ID NO: 10.

[A14] The pharmaceutical composition according to any one of [A7] to [A9] and [A13], wherein the anti-CD37 antibody is an antibody comprising a heavy chain and a light chain in the following combination (e):
(e) a light chain consisting of the amino acid sequence at positions 21 to 234 in the light chain full-length amino acid sequence shown in SEQ ID NO: 2 and a heavy chain consisting of the amino acid sequence at positions 20 to 468 in the heavy chain full-length amino acid sequence shown in SEQ ID NO: 4.
[A15] The pharmaceutical composition according to any one of [A7], [A8], [A10] and [A13], wherein the anti-CD37 antibody is an antibody comprising a heavy chain and a light chain in the following combination (f):
(f) a light chain consisting of the amino acid sequence at positions 21 to 234 in the light chain full-length amino acid sequence shown in SEQ ID NO: 2 and a heavy chain consisting of the amino acid sequence at positions 20 to 468 in the heavy chain full-length amino acid sequence shown in SEQ ID NO: 6.
[A16] The pharmaceutical composition according to any one of [A7], [A8], [A11] and [A13], wherein the anti-CD37 antibody is an antibody comprising a heavy chain and a light chain in the following combination (g):
(g) a light chain consisting of the amino acid sequence at positions 21 to 234 in the light chain full-length amino acid sequence shown in SEQ ID NO: 2 and a heavy chain consisting of the amino acid sequence at positions 20 to 468 in the heavy chain full-length amino acid sequence shown in SEQ ID NO: 8.
[A17] The pharmaceutical composition according to any one of [A7], [A8], [A12] and [A13], wherein the anti-CD37 antibody is an antibody comprising a heavy chain and a light chain in the following combination (h):
(h) a light chain consisting of the amino acid sequence at positions 21 to 234 in the light chain full-length amino acid sequence shown in SEQ ID NO: 2 and a heavy chain consisting of the amino acid sequence at positions 20 to 468 in the heavy chain full-length amino acid sequence shown in SEQ ID NO: 10.
[A18] The pharmaceutical composition according to any one of [A7] to [A17], wherein a lysine residue at the carboxyl terminus of the heavy chain of the anti-CD37 antibody is deleted.
[A19] The pharmaceutical composition according to any one of [A7] to [A18], wherein the average number of units of the drug-linker conjugated per antibody in the anti-CD37 antibody-drug conjugate is in the range of from 2 to 8.
[A20] The pharmaceutical composition according to any one of [A7] to [A19], wherein the average number of units of the drug-linker conjugated per antibody in the anti-CD37 antibody-drug conjugate is in the range of from 7 to 8.
[A21] The pharmaceutical composition according to any one of [A7] to [A20], wherein the average number of units of the drug-linker conjugated per antibody in the anti-CD37 antibody-drug conjugate is approximately 8.

[A22] The pharmaceutical composition according to any one of [A7] to [A21], wherein the molecular targeting drug is one or more drugs selected from the group consisting of an anti-CD20 antibody, an anti-CD19 antibody, a BCL-2 inhibitor and a BTK inhibitor.

[A23] The pharmaceutical composition according to [A22], wherein the anti-CD20 antibody is rituximab.

[A24] The pharmaceutical composition according to [A22], wherein the anti-CD20 antibody is obinutuzumab.

[A25] The pharmaceutical composition according to any one of [A22] to [A24], wherein the anti-CD19 antibody is tafasitamab.

[A26] The pharmaceutical composition according to any one of [A22] to [A25], wherein the BCL-2 inhibitor is venetoclax or a pharmacologically acceptable salt thereof.

[A27] The pharmaceutical composition according to any one of [A22] to [A26], wherein the BTK inhibitor is ibrutinib or a pharmacologically acceptable salt thereof.

[A28] The pharmaceutical composition according to any one of [A22] to [A26], wherein the BTK inhibitor is acalabrutinib or a pharmacologically acceptable salt thereof.

[A29] The pharmaceutical composition according to any one of [A22] to [A26], wherein the BTK inhibitor is zanubrutinib or a pharmacologically acceptable salt thereof.

[A30] The pharmaceutical composition according to any one of [A7] to [A29], wherein the alkylating agent is bendamustine or a pharmacologically acceptable salt thereof.

[A31] The pharmaceutical composition according to any one of [A7] to [A30], wherein the immunomodulatory drug is lenalidomide or a pharmacologically acceptable salt thereof.

[A32] The pharmaceutical composition according to any one of [A7] to [A21], wherein the other drug is a combination of rituximab and bendamustine or a pharmacologically acceptable salt thereof.

[A33] The pharmaceutical composition according to any one of [A7] to [A21], wherein the other drug is a combination of tafasitamab and lenalidomide or a pharmacologically acceptable salt thereof.

[A34] The pharmaceutical composition according to any one of [A7] to [A21], wherein the other drug is a combination of rituximab and lenalidomide or a pharmacologically acceptable salt thereof.

[A35] The pharmaceutical composition according to any one of [A7] to [A21], wherein the other drug is a combination of rituximab and venetoclax or a pharmacologically acceptable salt thereof.

[A36] The pharmaceutical composition according to any one of [A7] to [A21], wherein the other drug is a drug relating to R-CHOP therapy.

[A37] The pharmaceutical composition according to any one of [A7] to [A21], wherein the other drug is a drug relating to R-CHP therapy.

[A38] The pharmaceutical composition according to any one of [A7] to [A37], wherein the anti-CD37 antibody-drug conjugate and the one or more other drugs selected from the group consisting of a molecular targeting drug, a drug relating to R-CHOP therapy, a drug relating to R-CHP therapy, an alkylating agent and an immunomodulatory drug are separately contained as active ingredients in different formulations and administered at the same time or different times.

[A39] The pharmaceutical composition according to any one of [A7] to [A37], wherein the anti-CD37 antibody-drug conjugate and the one or more other drugs selected from the group consisting of a molecular targeting drug, a drug relating to R-CHOP therapy, a drug relating to R-CHP therapy, an alkylating agent and an immunomodulatory drug are contained as active ingredients in one formulation.

[A40] The pharmaceutical composition according to any one of [A7] to [A39] for the treatment of at least one disease selected from the group consisting of diffuse large B-cell lymphoma, follicular lymphoma, mantle cell lymphoma, marginal zone lymphoma, Burkitt's lymphoma, chronic lymphocytic leukemia, peripheral T-cell lymphoma, cutaneous T-cell lymphoma, myelodysplastic syndrome and acute myeloid leukemia.

[A41] The pharmaceutical composition according to [A40] for the treatment of diffuse large B-cell lymphoma.

[A42] The pharmaceutical composition according to [A40] for the treatment of chronic lymphocytic leukemia.

[A43] The pharmaceutical composition according to [A40] for the treatment of follicular lymphoma.

[A44] The pharmaceutical composition according to any one of [A1] to [A6], comprising the anti-CD37 antibody-drug conjugate, wherein

the anti-CD37 antibody-drug conjugate is administered in combination with one or more other drugs selected from the group consisting of a molecular targeting drug, a drug relating to R-CHOP therapy, a drug relating to R-CHP therapy, an alkylating agent and an immunomodulatory drug, and

the anti-CD37 antibody-drug conjugate is an anti-CD37 antibody-drug conjugate represented by the formula:

[Formula 12]

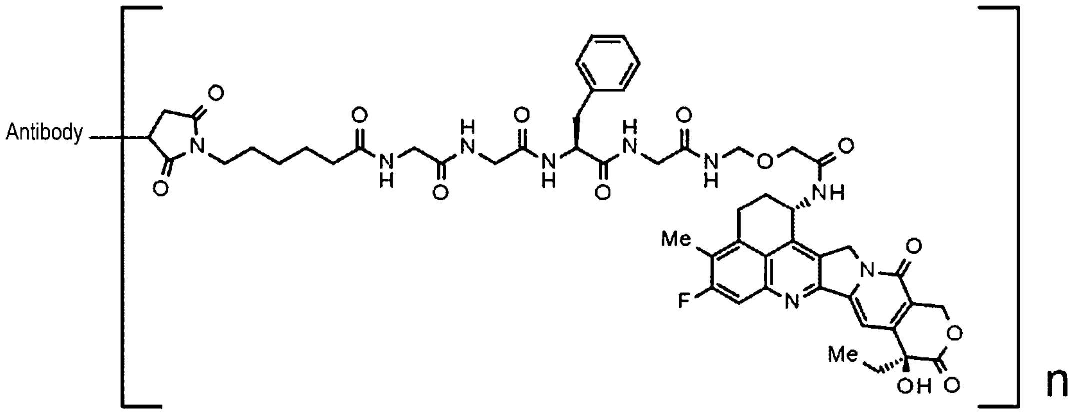

wherein Antibody represents an anti-CD37 antibody, the drug linker is conjugated to the antibody through a thioether bond, and n represents the average number of units of the drug-linker conjugated per antibody.

[A45] The pharmaceutical composition according to [A44], wherein the anti-CD37 antibody is an antibody comprising a heavy chain variable region and a light chain variable region in any one combination selected from the group consisting of the following combinations (a) to (d):

(a) a light chain variable region consisting of the amino acid sequence at positions 21 to 128 in the light chain full-length amino acid sequence shown in SEQ ID NO: 2 and a heavy chain variable region consisting of the amino acid sequence at positions 20 to 138 in the heavy chain full-length amino acid sequence shown in SEQ ID NO: 4;
(b) a light chain variable region consisting of the amino acid sequence at positions 21 to 128 in the light chain full-length amino acid sequence shown in SEQ ID NO: 2 and a heavy chain variable region consisting of the amino acid sequence at positions 20 to 138 in the heavy chain full-length amino acid sequence shown in SEQ ID NO: 6;
(c) a light chain variable region consisting of the amino acid sequence at positions 21 to 128 in the light chain full-length amino acid sequence shown in SEQ ID NO: 2 and a heavy chain variable region consisting of the amino acid sequence at positions 20 to 138 in the heavy chain full-length amino acid sequence shown in SEQ ID NO: 8; and
(d) a light chain variable region consisting of the amino acid sequence at positions 21 to 128 in the light chain full-length amino acid sequence shown in SEQ ID NO: 2 and a heavy chain variable region consisting of the amino acid sequence at positions 20 to 138 in the heavy chain full-length amino acid sequence shown in SEQ ID NO: 10.

[A46] The pharmaceutical composition according to [A44] or [A45], wherein the anti-CD37 antibody is an antibody comprising a heavy chain variable region and a light chain variable region in the following combination (a):

(a) a light chain variable region consisting of the amino acid sequence at positions 21 to 128 in the light chain full-length amino acid sequence shown in SEQ ID NO: 2 and a heavy chain variable region consisting of the amino acid sequence at positions 20 to 138 in the heavy chain full-length amino acid sequence shown in SEQ ID NO: 4.

[A47] The pharmaceutical composition according to [A44] or [A45], wherein the anti-CD37 antibody is an antibody comprising a heavy chain variable region and a light chain variable region in the following combination (b):
(b) a light chain variable region consisting of the amino acid sequence at positions 21 to 128 in the light chain full-length amino acid sequence shown in SEQ ID NO: 2 and a heavy chain variable region consisting of the amino acid sequence at positions 20 to 138 in the heavy chain full-length amino acid sequence shown in SEQ ID NO: 6.
[A48] The pharmaceutical composition according to [A44] or [A45], wherein the anti-CD37 antibody is an antibody comprising a heavy chain variable region and a light chain variable region in the following combination (c):

(c) a light chain variable region consisting of the amino acid sequence at positions 21 to 128 in the light chain full-length amino acid sequence shown in SEQ ID NO: 2 and a heavy chain variable region consisting of the amino acid sequence at positions 20 to 138 in the heavy chain full-length amino acid sequence shown in SEQ ID NO: 8.

[A49] The pharmaceutical composition according to [A44] or [A45], wherein the anti-CD37 antibody is an antibody comprising a heavy chain variable region and a light chain variable region in the following combination (d):

(d) a light chain variable region consisting of the amino acid sequence at positions 21 to 128 in the light chain full-length amino acid sequence shown in SEQ ID NO: 2 and a heavy chain variable region consisting of the amino acid sequence at positions 20 to 138 in the heavy chain full-length amino acid sequence shown in SEQ ID NO: 10.

[A50] The pharmaceutical composition according to [A44] or [A45], wherein the anti-CD37 antibody is an antibody comprising a heavy chain and a light chain in any one combination selected from the group consisting of the following combinations (e) to (h):

(e) a light chain consisting of the amino acid sequence at positions 21 to 234 in the light chain full-length amino acid sequence shown in SEQ ID NO: 2 and a heavy chain consisting of the amino acid sequence at positions 20 to 468 in the heavy chain full-length amino acid sequence shown in SEQ ID NO: 4;
(f) a light chain consisting of the amino acid sequence at positions 21 to 234 in the light chain full-length amino acid sequence shown in SEQ ID NO: 2 and a heavy chain consisting of the amino acid sequence at positions 20 to 468 in the heavy chain full-length amino acid sequence shown in SEQ ID NO: 6;
(g) a light chain consisting of the amino acid sequence at positions 21 to 234 in the light chain full-length amino acid sequence shown in SEQ ID NO: 2 and a heavy chain consisting of the amino acid sequence at positions 20 to 468 in the heavy chain full-length amino acid sequence shown in SEQ ID NO: 8; and
(h) a light chain consisting of the amino acid sequence at positions 21 to 234 in the light chain full-length amino acid sequence shown in SEQ ID NO: 2 and a heavy chain consisting of the amino acid sequence at positions 20 to 468 in the heavy chain full-length amino acid sequence shown in SEQ ID NO: 10.

[A51] The pharmaceutical composition according to any one of [A44] to [A46] and [A50], wherein the anti-CD37 antibody is an antibody comprising a heavy chain and a light chain in the following combination (e):
(e) a light chain consisting of the amino acid sequence at positions 21 to 234 in the light chain full-length amino acid sequence shown in SEQ ID NO: 2 and a heavy chain consisting of the amino acid sequence at positions 20 to 468 in the heavy chain full-length amino acid sequence shown in SEQ ID NO: 4.
[A52] The pharmaceutical composition according to any one of [A44], [A45], [A47] and [A50], wherein the anti-CD37 antibody is an antibody comprising a heavy chain and a light chain in the following combination (f):
(f) a light chain consisting of the amino acid sequence at positions 21 to 234 in the light chain full-length amino acid sequence shown in SEQ ID NO: 2 and a heavy chain consisting of the amino acid sequence at positions 20 to 468 in the heavy chain full-length amino acid sequence shown in SEQ ID NO: 6.
[A53] The pharmaceutical composition according to any one of [A44], [A45], [A48] and [A50], wherein the anti-CD37 antibody is an antibody comprising a heavy chain and a light chain in the following combination (g):
(g) a light chain consisting of the amino acid sequence at positions 21 to 234 in the light chain full-length amino acid sequence shown in SEQ ID NO: 2 and a heavy chain consisting of the amino acid sequence at positions 20 to 468 in the heavy chain full-length amino acid sequence shown in SEQ ID NO: 8.
[A54] The pharmaceutical composition according to any one of [A44], [A45], [A49] and [A50], wherein the anti-CD37 antibody is an antibody comprising a heavy chain and a light chain in the following combination (h):
(h) a light chain consisting of the amino acid sequence at positions 21 to 234 in the light chain full-length amino acid sequence shown in SEQ ID NO: 2 and a heavy chain consisting of the amino acid sequence at positions 20 to 468 in the heavy chain full-length amino acid sequence shown in SEQ ID NO: 10.
[A55] The pharmaceutical composition according to any one of [A44] to [A54], wherein a lysine residue at the carboxyl terminus of the heavy chain of the anti-CD37 antibody is deleted.
[A56] The pharmaceutical composition according to any one of [A44] to [A55], wherein the average number of units of the drug-linker conjugated per antibody in the anti-CD37 antibody-drug conjugate is in the range of from 2 to 8.
[A57] The pharmaceutical composition according to any one of [A44] to [A56], wherein the average number of units of the drug-linker conjugated per antibody in the anti-CD37 antibody-drug conjugate is in the range of from 7 to 8.
[A58] The pharmaceutical composition according to any one of [A44] to [A57], wherein the average number of units of the drug-linker conjugated per antibody in the anti-CD37 antibody-drug conjugate is approximately 8.
[A59] The pharmaceutical composition according to any one of [A44] to [A58], wherein the molecular targeting drug is one or more drugs selected from the group consisting of an anti-CD20 antibody, an anti-CD19 antibody, a BCL-2 inhibitor and a BTK inhibitor.
[A60] The pharmaceutical composition according to [A59], wherein the anti-CD20 antibody is rituximab.
[A61] The pharmaceutical composition according to [A59], wherein the anti-CD20 antibody is obinutuzumab.
[A62] The pharmaceutical composition according to any one of [A59] to [A61], wherein the anti-CD19 antibody is tafasitamab.
[A63] The pharmaceutical composition according to any one of [A59] to [A62], wherein the BCL-2 inhibitor is venetoclax or a pharmacologically acceptable salt thereof.
[A64] The pharmaceutical composition according to any one of [A59] to [A63], wherein the BTK inhibitor is ibrutinib or a

pharmacologically acceptable salt thereof.

[A65] The pharmaceutical composition according to any one of [A59] to [A63], wherein the BTK inhibitor is acalabrutinib or a pharmacologically acceptable salt thereof.

[A66] The pharmaceutical composition according to any one of [A59] to [A63], wherein the BTK inhibitor is zanubrutinib or a pharmacologically acceptable salt thereof.

[A67] The pharmaceutical composition according to any one of [A44] to [A66], wherein the alkylating agent is bendamustine or a pharmacologically acceptable salt thereof.

[A68] The pharmaceutical composition according to any one of [A44] to [A67], wherein the immunomodulatory drug is lenalidomide or a pharmacologically acceptable salt thereof.

[A69] The pharmaceutical composition according to any one of [A44] to [A58], wherein the other drug is a combination of rituximab and bendamustine or a pharmacologically acceptable salt thereof.

[A70] The pharmaceutical composition according to any one of [A44] to [A58], wherein the other drug is a combination of tafasitamab and lenalidomide or a pharmacologically acceptable salt thereof.

[A71] The pharmaceutical composition according to any one of [A44] to [A58], wherein the other drug is a combination of rituximab and lenalidomide or a pharmacologically acceptable salt thereof.

[A72] The pharmaceutical composition according to any one of [A44] to [A58], wherein the other drug is a combination of rituximab and venetoclax or a pharmacologically acceptable salt thereof.

[A44] The pharmaceutical composition according to any one of [A44] to [A58], wherein the other drug is a drug relating to R-CHOP therapy.

[A73] The pharmaceutical composition according to any one of [A44] to [A58], wherein the other drug is a drug relating to R-CHP therapy.

[A74] The pharmaceutical composition according to any one of [A44] to [A73], wherein the anti-CD37 antibody-drug conjugate and the one or more other drugs selected from the group consisting of a molecular targeting drug, a drug relating to R-CHOP therapy, a drug relating to R-CHP therapy, an alkylating agent and an immunomodulatory drug are separately contained as active ingredients in different formulations and administered at the same time or different times.

[A75] The pharmaceutical composition according to any one of [A44] to [A73], wherein the anti-CD37 antibody-drug conjugate and the one or more other drugs selected from the group consisting of a molecular targeting drug, a drug relating to R-CHOP therapy, a drug relating to R-CHP therapy, an alkylating agent and an immunomodulatory drug are contained as active ingredients in one formulation.

[A76] The pharmaceutical composition according to any one of [A44] to [A75] for the treatment of at least one disease selected from the group consisting of diffuse large B-cell lymphoma, follicular lymphoma, mantle cell lymphoma, marginal zone lymphoma, Burkitt's lymphoma, chronic lymphocytic leukemia, peripheral T-cell lymphoma, cutaneous T-cell lymphoma, myelodysplastic syndrome and acute myeloid leukemia.

[A77] The pharmaceutical composition according to [A76] for the treatment of diffuse large B-cell lymphoma.

[A78] The pharmaceutical composition according to [A76] for the treatment of chronic lymphocytic leukemia.

[A79] The pharmaceutical composition according to [A76] for the treatment of follicular lymphoma.

[B1] A method of treatment comprising administering an anti-CD37 antibody-drug conjugate and one or more other drugs selected from the group consisting of a molecular targeting drug, a drug relating to R-CHOP therapy, a drug relating to R-CHP therapy, an alkylating agent and an immunomodulatory drug in combination to an individual in need of treatment, wherein the anti-CD37 antibody-drug conjugate and the other drugs are defined in any one of [A1] to [A37] and [A44] to [A73].

[B2] The method of treatment according to [B1], wherein the anti-CD37 antibody-drug conjugate and the one or more other drugs selected from the group consisting of a molecular targeting drug, a drug relating to R-CHOP therapy, a drug relating to R-CHP therapy, an alkylating agent and an immunomodulatory drug are separately contained as active ingredients in different formulations and administered at the same time or different times.

[B3] The method of treatment according to [B1], wherein the anti-CD37 antibody-drug conjugate and the one or more other drugs selected from the group consisting of a molecular targeting drug, a drug relating to R-CHOP therapy, a drug relating to R-CHP therapy, an alkylating agent and an immunomodulatory drug are contained as active ingredients in one formulation.

[B4] The method of treatment according to any one of [B1] to [B3] for the treatment of at least one disease selected from the group consisting of diseases defined in [A40] or [A76].

[C1] An anti-CD37 antibody-drug conjugate for the treatment of a disease by administration in combination with one or more other drugs selected from the group consisting of a molecular targeting drug, a drug relating to R-CHOP therapy, a drug relating to R-CHP therapy, an alkylating agent and an immunomodulatory drug, wherein the anti-CD37 antibody-drug conjugate and the other drugs are defined in any one of [A1] to [A37] and [A44] to [A73].

[C2] The anti-CD37 antibody-drug conjugate according to [C1], wherein the anti-CD37 antibody-drug conjugate and the one or more other drugs selected from the group consisting of a molecular targeting drug, a drug relating to R-CHOP therapy, a drug relating to R-CHP therapy, an alkylating agent and an immunomodulatory drug are separately contained as active ingredients in different formulations and administered at the same time or different times.

[C3] The anti-CD37 antibody-drug conjugate according to [C1], wherein the anti-CD37 antibody-drug conjugate and the one or more other drugs selected from the group consisting of a molecular targeting drug, a drug relating to R-CHOP therapy, a drug relating to R-CHP therapy, an alkylating agent and an immunomodulatory drug are contained as active ingredients in one formulation.

[C4] The anti-CD37 antibody-drug conjugate according to any one of [C1] to [C3] for the treatment of at least one disease selected from the group consisting of diseases defined in [A40] or [A76].

[D1] Use of an anti-CD37 antibody-drug conjugate for the production of a medicament for the treatment of a disease by administration in combination with one or more other drugs selected from the group consisting of a molecular targeting drug, a drug relating to R-CHOP therapy, a drug relating to R-CHP therapy, an alkylating agent and an immunomodulatory drug, wherein the anti-CD37 antibody-drug conjugate and the other drugs are defined in any one of [A1] to [A37] and [A44] to [A73].

[D2] The use according to [D1], wherein the anti-CD37 antibody-drug conjugate and the one or more other drugs selected from the group consisting of a molecular targeting drug, a drug relating to R-CHOP therapy, a drug relating to R-CHP therapy, an alkylating agent and an immunomodulatory drug are separately contained as active ingredients in different formulations and administered at the same time or different times.

[D3] The use according to [D1], wherein the anti-CD37 antibody-drug conjugate and the one or more other drugs selected from the group consisting of a molecular targeting drug, a drug relating to R-CHOP therapy, a drug relating to R-CHP therapy, an alkylating agent and an immunomodulatory drug are contained as active ingredients in one formulation.

[D4] The use according to any one of [D1] to [D3] for the treatment of at least one disease selected from the group consisting of diseases defined in [A40] or [A76].

[E1] A pharmaceutical product comprising an anti-CD37 antibody-drug conjugate and one or more other drugs selected from the group consisting of a molecular targeting drug, a drug relating to R-CHOP therapy, a drug relating to R-CHP therapy, an alkylating agent and an immunomodulatory drug for administration in combination, wherein the anti-CD37 antibody-drug conjugate and the other drugs are defined in any one of [A1] to [A37] and [A44] to [A73].

[E2] The pharmaceutical product according to [E1], wherein the anti-CD37 antibody-drug conjugate and the one or more other drugs selected from the group consisting of a molecular targeting drug, a drug relating to R-CHOP therapy, a drug relating to R-CHP therapy, an alkylating agent and an immunomodulatory drug are separately contained as active ingredients in different formulations and administered at the same time or different times.

[E3] The pharmaceutical product according to [E1], wherein the anti-CD37 antibody-drug conjugate and the one or more other drugs selected from the group consisting of a molecular targeting drug, a drug relating to R-CHOP therapy, a drug relating to R-CHP therapy, an alkylating agent and an immunomodulatory drug are contained as active ingredients in one formulation.

[E4] The pharmaceutical product according to any one of [E1] to [E3] for the treatment of at least one disease selected from the group consisting of diseases defined in [A40] or [A76].

[F1] A combination medicament comprising an anti-CD37 antibody-drug conjugate and one or more other drugs selected from the group consisting of a molecular targeting drug, a drug relating to R-CHOP therapy, a drug relating to R-CHP therapy, an alkylating agent and an immunomodulatory drug for administration in combination, wherein the anti-CD37 antibody-drug conjugate and the other drugs are defined in any one of [A1] to [A37] and [A44] to [A73].

[F2] The combination medicament according to [F1], wherein the anti-CD37 antibody-drug conjugate and the one or more other drugs selected from the group consisting of a molecular targeting drug, a drug relating to R-CHOP therapy, a drug relating to R-CHP therapy, an alkylating agent and an immunomodulatory drug are separately contained as active ingredients in different formulations and administered at the same time or different times.

[F3] The combination medicament according to [F1], wherein the anti-CD37 antibody-drug conjugate and the one or more other drugs selected from the group consisting of a molecular targeting drug, a drug relating to R-CHOP therapy, a drug relating to R-CHP therapy, an alkylating agent and an immunomodulatory drug are contained as active ingredients in one formulation.

[F4] The combination medicament according to any one of [F1] to [F3] for the treatment of at least one disease selected from the group consisting of diseases defined in [A40] or [A76].

[G1] A pharmaceutical combination comprising an anti-CD37 antibody-drug conjugate and one or more other drugs selected from the group consisting of a molecular targeting drug, a drug relating to R-CHOP therapy, a drug relating to R-CHP therapy, an alkylating agent and an immunomodulatory drug for administration in combination, wherein the anti-CD37 antibody-drug conjugate and the other drugs are defined in any one of [A1] to [A37] and [A44] to [A73].

[G2] The pharmaceutical combination according to [G1], wherein the anti-CD37 antibody-drug conjugate and the one or more other drugs selected from the group consisting of a molecular targeting drug, a drug relating to R-CHOP therapy, a drug relating to R-CHP therapy, an alkylating agent and an immunomodulatory drug are separately

contained as active ingredients in different formulations and administered at the same time or different times.

[G3] The pharmaceutical combination according to [G1], wherein the anti-CD37 antibody-drug conjugate and the one or more other drugs selected from the group consisting of a molecular targeting drug, a drug relating to R-CHOP therapy, a drug relating to R-CHP therapy, an alkylating agent and an immunomodulatory drug are contained as active ingredients in one formulation.

[G4] The pharmaceutical combination according to any one of [G1] to [G3] for the treatment of at least one disease selected from the group consisting of diseases defined in [A40] or [A76].

[H1] Use of an anti-CD37 antibody-drug conjugate for the treatment of a disease, in combination with one or more other drugs selected from the group consisting of a molecular targeting drug, a drug relating to R-CHOP therapy, a drug relating to R-CHP therapy, an alkylating agent and an immunomodulatory drug, wherein the anti-CD37 antibody-drug conjugate and the other drugs are defined in any one of [A1] to [A37] and [A44] to [A73].

[H2] The use according to [H1], wherein the anti-CD37 antibody-drug conjugate and the one or more other drugs selected from the group consisting of a molecular targeting drug, a drug relating to R-CHOP therapy, a drug relating to R-CHP therapy, an alkylating agent and an immunomodulatory drug are separately contained as active ingredients in different formulations and administered at the same time or different times.

[H3] The use according to [H1], wherein the anti-CD37 antibody-drug conjugate and the one or more other drugs selected from the group consisting of a molecular targeting drug, a drug relating to R-CHOP therapy, a drug relating to R-CHP therapy, an alkylating agent and an immunomodulatory drug are contained as active ingredients in one formulation.

[H4] The use according to any one of [H1] to [H3] for the treatment of at least one disease selected from the group consisting of diseases defined in [A40] or [A76].

[I1] A medicament comprising an anti-CD37 antibody-drug conjugate and one or more other drugs selected from the group consisting of a molecular targeting drug, a drug relating to R-CHOP therapy, a drug relating to R-CHP therapy, an alkylating agent and an immunomodulatory drug, wherein the anti-CD37 antibody-drug conjugate and the other drugs are administered in combination, and the anti-CD37 antibody-drug conjugate and the other drugs are defined in any one of [A1] to [A37] and [A44] to [A73].

[I2] The medicament according to [I1], wherein the anti-CD37 antibody-drug conjugate and the one or more other drugs selected from the group consisting of a molecular targeting drug, a drug relating to R-CHOP therapy, a drug relating to R-CHP therapy, an alkylating agent and an immunomodulatory drug are separately contained as active ingredients in different formulations and administered at the same time or different times.

[I3] The medicament according to [I1], wherein the anti-CD37 antibody-drug conjugate and the one or more other drugs selected from the group consisting of a molecular targeting drug, a drug relating to R-CHOP therapy, a drug relating to R-CHP therapy, an alkylating agent and an immunomodulatory drug are contained as active ingredients in one formulation.

[I4] The medicament according to any one of [I1] to [I3] for the treatment of at least one disease selected from the group consisting of diseases defined in [A40] or [A76].

Advantageous Effects of Invention

The present invention can provide a pharmaceutical composition wherein a specific antibody-drug conjugate and another drug are administered in combination, and/or a method of treatment comprising administering a specific antibody-drug conjugate and another drug in combination to an individual, and the like.

Brief Description of Drawings

[Figure 1] Figure 1 is a diagram showing a nucleotide sequence encoding a hmAb-L11 light chain.

[Figure 2] Figure 2 is a diagram showing the full-length amino acid sequence of the hmAb-L11 light chain.

[Figure 3] Figure 3 is a diagram showing a nucleotide sequence encoding a hmAb-H11 heavy chain.

[Figure 4] Figure 4 is a diagram showing the full-length amino acid sequence of the hmAb-H11 heavy chain.

[Figure 5] Figure 5 is a diagram showing a nucleotide sequence encoding a hmAb-H541 heavy chain.

[Figure 6] Figure 6 is a diagram showing the full-length amino acid sequence of the hmAb-H541 heavy chain.

[Figure 7] Figure 7 is a diagram showing a nucleotide sequence encoding a hmAb-H551 heavy chain.

[Figure 8] Figure 8 is a diagram showing the full-length amino acid sequence of the hmAb-H551 heavy chain.

[Figure 9] Figure 9 is a diagram showing a nucleotide sequence encoding a hmAb-H11a heavy chain.

[Figure 10] Figure 10 is a diagram showing the full-length amino acid sequence of the hmAb-H11a heavy chain.

[Figure 11] Figure 11 is a diagram showing the *in vivo* antitumor effects of a humanized anti-CD37 antibody-drug

conjugate and/or Rituximab against SCID mice inoculated with CD37-positive human diffuse large B-cell lymphoma cell line OCI-LY7 was inoculated.

[Figure 12] Figure 12 is a diagram showing the rate of change in tumor volume on Day 22 for each individual, using the tumor volume on Day 12 as a baseline, resulting from the *in vivo* antitumor effect of a humanized anti-CD37 antibody-drug conjugate and/or rituximab against SCID mice inoculated with CD37-positive human diffuse large B-cell lymphoma cell line OCI-LY7.

[Figure 13] Figure 13 is a diagram showing the *in vivo* antitumor effects of a humanized anti-CD37 antibody-drug conjugate, and/or Rituximab against SCID mice inoculated with CD37-positive human diffuse large B-cell lymphoma cell line SU-DHL-4.

[Figure 14] Figure 14 is a diagram showing the *in vivo* antitumor effect of a humanized anti-CD37 antibody-drug conjugate and/or Rituximab+Bendamustine(RB) against SCID mice inoculated with CD37-positive human diffuse large B-cell lymphoma cell line SU-DHL-4.

[Figure 15] Figure 15 is a diagram showing the *in vivo* antitumor effect of a humanized anti-CD37 antibody-drug conjugate and/or tafasitamab against SCID mice inoculated with CD37-positive human diffuse large B-cell lymphoma cell line SU-DHL-4.

[Figure 16] Figure 16 is a diagram showing the *in vivo* antitumor effect of a humanized anti-CD37 antibody-drug conjugate and/or tafasitamab-lenalidomide against SCID mice inoculated with CD37-positive human diffuse large B-cell lymphoma cell line SU-DHL-4.

[Figure 17] Figure 17 is a diagram showing the rate of change in tumor volume on Day 38 for each individual, using the tumor volume on Day 16 as a baseline, resulting from the *in vivo* antitumor effect of a humanized anti-CD37 antibody-drug conjugate and/or rituximab, the humanized anti-CD37 antibody-drug conjugate and/or rituximab + bendamustine (RB), the humanized anti-CD37 antibody-drug conjugate and/or tafasitamab, or the humanized anti-CD37 antibody-drug conjugate and/or tafasitamab-lenalidomide against SCID mice inoculated with CD37-positive human diffuse large B-cell lymphoma cell line SU-DHL-4.

[Figure 18] Figure 18 is a diagram showing the *in vivo* antitumor effect of a humanized anti-CD37 antibody-drug conjugate and/or rituximab against SCID mice inoculated with CD37-positive human follicular lymphoma cell line DOHH-2.

[Figure 19] Figure 19 is a diagram showing the *in vivo* antitumor effect of a humanized anti-CD37 antibody-drug conjugate and/or rituximab + bendamustine (RB) against SCID mice inoculated with CD37-positive human follicular lymphoma cell line DOHH-2.

[Figure 20] Figure 20 is a diagram showing the *in vivo* antitumor effect of a humanized anti-CD37 antibody-drug conjugate and/or rituximab + lenalidomide (R-lenalidomide) against SCID mice inoculated with CD37-positive human follicular lymphoma cell line DOHH-2.

[Figure 21] Figure 21 is a diagram showing the rate of change in tumor volume on Day 44 for each individual, using the tumor volume on Day 23 as a baseline, resulting from the *in vivo* antitumor effect of a humanized anti-CD37 antibody-drug conjugate and/or rituximab, the humanized anti-CD37 antibody-drug conjugate and/or rituximab + bendamustine (RB), or the humanized anti-CD37 antibody-drug conjugate and/or rituximab + lenalidomide (R-lenalidomide) against SCID mice inoculated with CD37-positive human follicular lymphoma cell line DOHH-2.

[Figure 22] Figure 22 is a diagram showing the *in vivo* antitumor effect of a humanized anti-CD37 antibody-drug conjugate and/or rituximab against SCID mice inoculated with CD37-positive human chronic lymphocytic leukemia cell line JVM-13.

[Figure 23] Figure 23 is a diagram showing the *in vivo* antitumor effect of a humanized anti-CD37 antibody-drug conjugate and/or rituximab + bendamustine (RB) against SCID mice inoculated with CD37-positive human chronic lymphocytic leukemia cell line JVM-13.

[Figure 24] Figure 24 is a diagram showing the *in vivo* antitumor effect of a humanized anti-CD37 antibody-drug conjugate and/or venetoclax against SCID mice inoculated with CD37-positive human chronic lymphocytic leukemia cell line JVM-13.

[Figure 25] Figure 25 is a diagram showing the *in vivo* antitumor effect of a humanized anti-CD37 antibody-drug conjugate and/or rituximab + venetoclax (R-venetoclax) against SCID mice inoculated with CD37-positive human chronic lymphocytic leukemia cell line JVM-13.

[Figure 26] Figure 26 is a diagram showing the *in vivo* antitumor effect of a humanized anti-CD37 antibody-drug conjugate and/or ibrutinib against SCID mice inoculated with CD37-positive human chronic lymphocytic leukemia cell line JVM-13.

[Figure 27] Figure 27 is a diagram showing the rate of change in tumor volume on Day 35 for each individual, using the tumor volume on Day 14 as a baseline, resulting from the *in vivo* antitumor effect of a humanized anti-CD37 antibody-drug conjugate and/or rituximab, the humanized anti-CD37 antibody-drug conjugate and/or rituximab + bendamustine (RB), the humanized anti-CD37 antibody-drug conjugate and/or venetoclax, the humanized anti-CD37 antibody-drug conjugate and/or rituximab + venetoclax (R-venetoclax), or the humanized anti-CD37 antibody-drug conjugate

and/or ibrutinib against SCID mice inoculated with CD37-positive human chronic lymphocytic leukemia cell line JVM-13.

[Figure 28] Figure 28 is a diagram showing the *in vitro* cellular internalization rate of anti-CD37 antibody-drug conjugate (1) when a fluorescently labeled anti-CD37 antibody-drug conjugate (1) in the presence of rituximab or IgG1 were added to CD37-positive human diffuse large B-cell lymphoma cell line OCI-LY7.

[Figure 29] Figure 29 is a diagram showing the *in vitro* cellular internalization rate of anti-CD37 antibody-drug conjugate (1) when a fluorescently labeled anti-CD37 antibody-drug conjugate (1) in the presence of rituximab or IgG1 were added to CD37-positive human diffuse large B-cell lymphoma cell line SU-DHL-4.

[Figure 30] Figure 30 is a diagram showing the *in vivo* antitumor effect of a humanized anti-CD37 antibody-drug conjugate and/or rituximab against SCID mice inoculated with CD37-positive human diffuse large B-cell lymphoma cell line OCI-LY7.

[Figure 31] Figure 31 is a diagram showing the *in vivo* antitumor effect of a humanized anti-CD37 antibody-drug conjugate and/or R-CHP against SCID mice inoculated with CD37-positive human diffuse large B-cell lymphoma cell line OCI-LY7.

[Figure 32] Figure 32 is a diagram showing the *in vivo* antitumor effect of a humanized anti-CD37 antibody-drug conjugate and/or R-CHOP against SCID mice inoculated with CD37-positive human diffuse large B-cell lymphoma cell line OCI-LY7.

[Figure 33] Figure 33 is a diagram showing the rate of change in tumor volume on Day 18 for each individual, using the tumor volume on Day 10 as a baseline, resulting from the *in vivo* antitumor effect of a humanized anti-CD37 antibody-drug conjugate and/or rituximab, the humanized anti-CD37 antibody-drug conjugate and/or R-CHP, or the humanized anti-CD37 antibody-drug conjugate and/or R-CHOP against SCID mice inoculated with CD37-positive human diffuse large B-cell lymphoma cell line OCI-LY7.

[Figure 34] Figure 34 is a diagram showing the *in vivo* antitumor effect of a humanized anti-CD37 antibody-drug conjugate and/or rituximab against SCID mice inoculated with CD37-positive human chronic lymphocytic leukemia cell line JVM-3.

[Figure 35] Figure 35 is a diagram showing the *in vivo* antitumor effect of a humanized anti-CD37 antibody-drug conjugate and/or obinutuzumab against SCID mice inoculated with CD37-positive human chronic lymphocytic leukemia cell line JVM-3.

[Figure 36] Figure 36 is a diagram showing the rate of change in tumor volume on Day 28 for each individual, using the tumor volume on Day 13 as a baseline, resulting from the *in vivo* antitumor effect of a humanized anti-CD37 antibody-drug conjugate and/or rituximab or the humanized anti-CD37 antibody-drug conjugate and/or obinutuzumab against SCID mice inoculated with CD37-positive human chronic lymphocytic leukemia cell line JVM-3.

[Figure 37] Figure 37 is a diagram showing the *in vivo* antitumor effect of a humanized anti-CD37 antibody-drug conjugate and/or obinutuzumab against SCID mice inoculated with CD37-positive human chronic lymphocytic leukemia cell line JVM-13.

[Figure 38] Figure 38 is a diagram showing the rate of change in tumor volume on Day 25 for each individual, using the tumor volume on Day 10 as a baseline, resulting from the *in vivo* antitumor effect of a humanized anti-CD37 antibody-drug conjugate and/or obinutuzumab against SCID mice inoculated with CD37-positive human chronic lymphocytic leukemia cell line JVM-13.

## Description of Embodiments

Hereinafter, the preferred modes for carrying out the present invention will be described. It is to be noted that the embodiments described below merely illustrate examples of the representative embodiments of the present invention, and the scope of the present invention shall not be narrowly interpreted due to these examples.

### 1. Definition

In the present invention, the term "gene" means a nucleic acid molecule comprising a nucleotide sequence encoding the amino acids of a protein, or its complementary strand. The term "gene" is meant to include, for example, a polynucleotide, an oligonucleotide, DNA, mRNA, cDNA, and cRNA comprising a nucleotide sequence encoding the amino acids of a protein or a nucleotide sequence complementary thereto. Such a gene is a single-stranded, double-stranded, or triple or more stranded nucleotide. The term "gene" is also meant to include an association of DNA and RNA strands, a mixture of ribonucleotides (RNAs) and deoxyribonucleotides (DNAs) on one nucleotide strand, and a double-stranded or triple or more stranded nucleotide comprising such a nucleotide strand. Examples of the "CD37 gene" of the present invention can include DNA, mRNA, cDNA, and cRNA comprising a nucleotide sequence encoding the amino acid sequence of the CD37 protein.

In the present invention, the term "nucleotide" has the same meaning as "nucleic acid" and "nucleic acid

molecule", and is also meant to include, for example, DNA, RNA, a probe, an oligonucleotide, a polynucleotide, and a primer. Such a nucleotide is a single-stranded, double-stranded, or triple or more stranded nucleotide. The term "nucleotide" is also meant to include an association of DNA and RNA strands, a mixture of ribonucleotides (RNAs) and deoxyribonucleotides (DNAs) on one nucleotide strand, and an association of two strands or three or more strands comprising such a nucleotide strand.

In the present invention, the terms "polypeptide", "peptide", and "protein" have the same meaning.

In the present invention, the term "protein" refers to a "protein" from any given vertebrate source including mammals such as primates (e.g., humans and monkeys) and rodents (e.g., mice and rats) unless otherwise specified.

In the present invention, the term "full-length amino acid sequence of an antibody heavy chain " or "full-length amino acid sequence of an antibody light chain" refers to the amino acid sequence of a protein obtained by transcription and translation (or by translation) from a nucleotide comprising a nucleotide sequence encoding the antibody heavy chain or the antibody light chain. These sequences may comprise the amino acid sequence of a signal sequence. When the antibody heavy chain and the antibody light chain together form an antibody molecule, the signal sequence may be cleaved off.

In the present invention, the term "antigen" has the same meaning as "immunogen".

In the present invention, the term "cell" also includes, for example, various cells derived from individual animals, subcultured cells, primary cultured cells, cell lines, recombinant cells, and microbial cells.

In the present invention, the "site" to which an antibody binds, i.e., the "site" recognized by an antibody, means a partial peptide or a partial conformation on an antigen that is bound or recognized by the antibody. In the present invention, such a site is also referred to as an epitope or a binding site of the antibody. Examples of the site on the CD37 protein that is bound or recognized by the anti-CD37 antibody of the present invention can include a partial peptide or a partial conformation on the CD37 protein.

In the present invention, the term "CDR" means a complementarity determining region, and the term "FR" means a framework region. The heavy and light chains of an antibody molecule are known to each have three CDRs. The CDRs are also called hypervariable domains. These regions are located in the variable regions of the antibody heavy and light chains. These sites have a particularly highly variable primary structure and are usually separated into three positions on the respective primary structures of the heavy and light chain polypeptide strands. In the present invention, the complementarity determining regions of the antibody are referred to as CDRH1, CDRH2, and CDRH3 from the amino terminus of the heavy chain amino acid sequence for the complementarity determining regions of the heavy chain and as CDRL1, CDRL2, and CDRL3 from the amino terminus of the light chain amino acid sequence for the complementarity determining regions of the light chain. These sites are proximal to each other on the three-dimensional structure and determine the specificity for the antigen to be bound. The portions other than CDRH1 to CDRH3 in the heavy chain variable region amino acid sequence are called FRs, and the portions from the amino terminus up to but not including CDRH1, from just after CDRH1 up to but not including CDRH2, from just after CDRH2 up to but not including CDRH3, and from just after CDRH3 to the carboxyl terminus are respectively called FRH1 to FRH4. Likewise, the portions other than CDRL1 to CDRL3 in the light chain variable region amino acid sequence are also FRs, and the portions from the amino terminus up to but not including CDRL1, from just after CDRL1 up to but not including CDRL2, from just after CDRL2 up to but not including CDRL3, and from just after CDRL3 to the carboxyl terminus are respectively called FRL1 to FRL4. That is, in (the amino acid sequence(s) of) the heavy chain and light chain variable regions, FRH1-CDRH1-FRH2-CDRH2-FRH3-CDRH3-FRH4 and FRL1-CDRL1-FRL2-CDRL2-FRL3-CDRL3-FRL4 are continuously aligned from the amino terminal side toward the carboxyl terminus in this order.

In the present invention, the term "antigen-binding fragment of an antibody" means an antibody fragment that exerts at least a part of the antigen-biding activity exerted by the original antibody. Examples of the "antigen binding fragment of the antibody" can include, but are not limited to, Fab, F(ab')2, scFv, Fab', and single chain immunoglobulin. Such an antigen binding fragment of the antibody may be obtained by treating a full-length molecule of the antibody protein with an enzyme such as papain or pepsin or may be a recombinant protein produced in an appropriate host cell using a recombinant gene.

In the present invention, the term "another drug" refers to a drug that is administered in combination with a specific antibody-drug conjugate. The specific antibody-drug conjugate according to the present invention is preferably an anti-CD37 antibody-drug conjugate. The other drug according to the present invention is preferably one or more drugs selected from the group consisting of a molecular targeting drug, a drug relating to R-CHOP therapy, a drug relating to R-CHP therapy, an alkylating agent and an immunomodulatory drug.

In the present invention, the term "pharmacologically acceptable salt" refers to a salt that has no marked toxicity and may be used as a pharmaceutical composition. A compound having an acidic substituent can form a salt through reaction with a base. Examples of the salt can include, but are not limited to: alkali metal salts such as a sodium salt, a potassium salt, and a lithium salt; alkaline earth metal salts such as a calcium salt and a magnesium salt; metal salts such as an aluminum salt and an iron salt; inorganic salts such as an ammonium salt; amine salts including organic salts such as a tert-butylamine salt, a tert-octylamine salt, a dibenzylamine salt, a morpholine salt, a glucosamine salt, a phenylglycine

alkyl ester salt, an ethylenediamine salt, an N-methylglucamine salt, a guanidine salt, a diethylamine salt, a triethylamine salt, a dicyclohexylamine salt, an N,N'-dibenzylethylenediamine salt, a chloroprocaine salt, a procaine salt, a diethanolamine salt, an N-benzylphenethylamine salt, a piperazine salt, a tetramethylammonium salt, and a tris(hydroxymethyl) aminomethane salt; and amino acid salts such as a glycine salt, a lysine salt, an arginine salt, an ornithine salt, glutamate, and aspartate.

A compound having a basic substituent can form a salt through reaction with an acid. Examples of the salt can include, but are not limited to: salts of inorganic acids such as hydrochloric acid, hydrobromic acid, hydroiodic acid, nitric acid, sulfuric acid, and phosphoric acid; salts of organic carboxylic acids such as acetic acid, fumaric acid, maleic acid, succinic acid, citric acid, tartaric acid, adipic acid, lactic acid, and trifluoroacetic acid; salts of organic sulfonic acids such as methanesulfonic acid, trifluoromethanesulfonic acid, benzenesulfonic acid, p-toluenesulfonic acid, and naphthalenesulfonic acid; and amino acid salts such as a glycine salt, a lysine salt, an arginine salt, an ornithine salt, glutamate, and aspartate.

The other drug and the pharmacologically acceptable salt thereof used in the present invention may exist as solvates. These solvates are also included in the other drug and the pharmacologically acceptable salt thereof used in the present invention.

In the present invention, the term "prodrug" is a derivative of the compound of the present invention that has a chemically or metabolically degradable group and exhibits inherent medicinal effects by restoring the original compound, for example, through hydrolysis, solvolysis, or degradation under physiological conditions after being administered to the living body. The prodrug also includes a complex independent from a covalent bond, and a salt. The prodrug is utilized, for example, for improvement in absorption for oral administration or targeting to a target site. Examples of the modification site include highly reactive functional groups such as a hydroxy group, a carboxy group, and an amino group in the compound of the present invention.

In the present invention, the terms "cancer" and "tumor" have the same meaning. The "cancer" or the "tumor" includes malignant lymphoma.

In the present invention, the term "malignant lymphoma" is a blood cancer (hematopoietic tumor) and refers to a disease in which lymphocytes among leukocytes become cancerous. The malignant lymphoma is broadly classified into B-cell lymphoma, T-cell lymphoma, NK-cell lymphoma, and Hodgkin's lymphoma. The B-cell lymphoma, the T-cell lymphoma, and the NK-cell lymphoma are also collectively referred to as non-Hodgkin's lymphoma.

In the present invention, the term "several" means 1 to 10, 1 to 9, 1 to 8, 1 to 7, 1 to 6, 1 to 5, 1 to 4, 1 to 3, or 1 or 2.

In the present description, the term "approximately" refers to a value which may vary up to plus or minus 10%, 8%, 6%, 5%, 4%, 3%, 2%, or 1% from the reference value. Preferably, the term "approximately" refers to the range of plus or minus 10%, 5%, or 1% from the reference value.

## 2. CD37

CD37 is a four-pass transmembrane protein of the tetraspanin superfamily (Charrin S., et al., J Cell Sci. 3641-3648, 127, 2014). CD37 is a membrane protein composed of 281 amino acids and has both amino-terminal and carboxy-terminal intracellular domains, and its sequence can be referred to under, for example, accession Nos. NM_001774 and NP_001765 (NCBI).

The CD37 protein used in the present invention can be directly purified from the CD37-expressing cells of a human or a non-human mammal (e.g., a rat, a mouse or a monkey) and can then be used, or a cell membrane fraction of the aforementioned cells can be prepared and can be used as the CD37 protein. Alternatively, CD37 can also be obtained by synthesizing it *in vitro,* or by allowing host cells to produce CD37 by genetic manipulation. According to such genetic manipulation, the CD37 protein can be obtained, specifically, by incorporating CD37 cDNA into a vector capable of expressing the CD37 cDNA, and then synthesizing CD37 in a solution containing enzymes, substrate and energy substances necessary for transcription and translation, or by transforming the host cells of other prokaryotes or eukaryotes, so as to allow them to express CD37. Also, CD37-expressing cells based on the above-described genetic manipulation, or a cell line expressing CD37 may be used as the CD37 protein. Alternatively, the expression vector into which CD37 cDNA has been incorporated can be directly administered to an animal to be immunized, and CD37 can be expressed in the body of the animal thus immunized.

Moreover, a protein which consists of an amino acid sequence comprising a substitution, deletion and/or addition of one or several amino acids in the above-described amino acid sequence of the CD37 protein, and has a biological activity equivalent to that of the CD37 protein, is also included within the term "CD37".

The human CD37 protein has the amino acid sequence shown in SEQ ID NO: 18. The extracellular region of the human CD37 protein is composed of extracellular domain 1 (in the present description, also referred to as EC1) having the amino acid sequence at positions 39 to 59 in the amino acid sequence shown in SEQ ID NO: 18, and extracellular domain 2 (in the present description, also referred to as EC2) having the amino acid sequence at positions 112 to 241 in the amino acid sequence shown in SEQ ID NO: 18. For the sequence of the human CD37 protein, also see the following link (https://

www.uniprot.org/uniprot/P11049).

SEQ ID NO: 18

```
MSAQESCLSL IKYFLFVFNL FFFVLGSLIF CFGIWILIDK TSFVSFVGLA
FVPLQIWSKV LAISGIFTMG IALLGCVGAL KELRCLLGLY FGMLLLLFAT
QITLGILIST QRAQLERSLR DVVEKTIQKY GTNPEETAAE ESWDYVQFQL
RCCGWHYPQD WFQVLILRGN GSEAHRVPCS CYNLSATNDS TILDKVILPQ
LSRLGHLARS RHSADICAVP AESHIYREGC AQGLQKWLHN NLISIVGICL
GVGLLELGFM TLSIFLCRNL DHVYNRLARY R
```

3. Anti-CD37 antibody

(1) Antibody

In the present invention, both the antibody binding to CD37 and the antibody recognizing CD37 are also referred to as an "anti-CD37 antibody" or also abbreviated to a "CD37 antibody".

The anti-CD37 antibody of the present invention may be derived from any species. Preferred examples of the species can include humans, rats, mice and rabbits. When the anti-CD37 antibody of the present invention is derived from a species other than humans, it is desirable to chimerize or humanize the anti-CD37 antibody by a known technique. The antibody of the present invention may be a polyclonal antibody or may be a monoclonal antibody, and a monoclonal antibody is preferred.

The anti-CD37 antibody of the present invention is an antibody that can target tumor cells. Specifically, the anti-CD37 antibody of the present invention possesses the property of being able to recognize tumor cells, the property of being able to bind to tumor cells, and the property of being internalized into tumor cells by cellular uptake, and the like. Accordingly, the anti-CD37 antibody of the present invention can be conjugated to a compound having antitumor activity via a linker to prepare an antibody-drug conjugate.

The binding activity of an antibody against tumor cells can be confirmed by flow cytometry. The uptake of an antibody into tumor cells can be confirmed by (1) an assay of visualizing a cellularly taken-up antibody under a fluorescent microscope using a secondary antibody (fluorescently labeled) binding to the therapeutic antibody (Cell Death and Differentiation (2008) 15, 751-761), (2) an assay of measuring the amount of cellularly taken-up fluorescence using a secondary antibody (fluorescently labeled) binding to the therapeutic antibody (Molecular Biology of the Cell Vol. 15, 5268-5282, December 2004) or (3) a Mab-ZAP assay using an immunotoxin binding to the therapeutic antibody, wherein the toxin is released upon cellular uptake, so as to suppress cell growth (Bio Techniques 28: 162-165, January 2000). A recombinant conjugated protein of a catalytic region of diphtheria toxin and protein G may be used as the immunotoxin.

In the present description, the term "high internalization ability" is used to mean that the survival rate (which is indicated by a ratio relative to a cell survival rate without antibody addition defined as 100%) of CD37-expressing cells to which the aforementioned antibody and a saporin-labeled anti-rat IgG antibody have been administered is 80% or less, preferably 70% or less, and more preferably 60% or less.

The antitumor antibody-drug conjugate of the present invention comprises a conjugated compound exerting an antitumor effect. Therefore, it is preferred, but not essential, that the antibody itself should have an antitumor effect. For the purpose of specifically and selectively exerting the cytotoxicity of the antitumor compound in tumor cells, it is important and preferred that the antibody should have a property of being internalized and transferred into tumor cells.

The anti-CD37 antibody can be obtained by immunizing an animal with a polypeptide serving as an antigen by a method performed in this field, and then collecting and purifying an antibody produced in a living body thereof. It is preferred to use CD37 retaining its three-dimensional structure as an antigen because CD37 is a four-pass transmembrane protein. Examples of such a method can include DNA immunization.

The origin of the antigen is not limited to a human, and thus, an animal can also be immunized with an antigen derived from a non-human animal such as a mouse or a rat. In this case, an antibody applicable to the disease of a human can be selected by examining the cross-reactivity of the obtained antibody binding to the heterologous antigen with the human antigen.

Furthermore, antibody-producing cells that produce an antibody against the antigen can be fused with myeloma

cells according to a known method (e.g., Kohler and Milstein, Nature (1975) 256, p. 495-497; and Kennet, R. ed., Monoclonal Antibodies, p. 365-367, Plenum Press, N. Y. (1980)) to establish hybridomas, so as to obtain a monoclonal antibody.

Hereinafter, the method for obtaining an antibody against CD37 will be specifically described.

(I) Preparation of antigen

The antigen can be obtained by allowing host cells to produce a gene encoding the antigen protein according to genetic manipulation. Specifically, a vector capable of expressing the antigen gene is produced, and the vector is then introduced into host cells, so that the gene is expressed therein, and thereafter, the expressed antigen may be purified. The antibody can also be obtained by a method of immunizing an animal with the antigen-expressing cells based on the above-described genetic manipulation, or a cell line expressing the antigen.

Alternatively, the antibody can also be obtained, without the use of the antigen protein, by incorporating cDNA of the antigen protein into an expression vector, then administering the expression vector to an animal to be immunized, and expressing the antigen protein in the body of the animal thus immunized, so that an antibody against the antigen protein is produced therein.

(II) Production of anti-CD37 monoclonal antibody

The anti-CD37 antibody used in the present invention is not particularly limited. For example, an antibody specified by an amino acid sequence shown in the sequence listing of the present application can be suitably used. The anti-CD37 antibody used in the present invention is desirably an antibody having the following properties:

(i) an antibody having the following properties:

specifically binding to CD37, and
· having the activity of being internalized into CD37-expressing cells by binding to CD37;

(ii) the antibody according to the above (i), wherein the CD37 is human CD37; or
(iii) the antibody according to the above (i) or (ii) which recognizes the conformation of CD37.

The method for obtaining the antibody against CD37 of the present invention is not particularly limited as long as an anti-CD37 antibody can be obtained. It is preferred to use CD37 retaining its conformation as an antigen because CD37 is a transmembrane protein.

One preferred example of the method for obtaining the antibody can include a DNA immunization method. The DNA immunization method is an approach which involves transfecting an individual animal (e.g., mouse or rat) with an expression plasmid of an antigen, and then expressing the antigen in the animal to induce immunity against the antigen. The transfection approach includes a method of directly injecting the plasmid into a muscle, a method of injecting a transfection reagent such as a liposome or polyethylenimine into a vein, an approach using a viral vector, an approach of injecting gold particles attached to the plasmid using a gene gun, a hydrodynamic method of rapidly injecting a plasmid solution in a large amount into a vein, and the like. With regard to the transfection method of injecting the expression plasmid into a muscle, a technique called *in vivo* electroporation, which involves applying electroporation to the intramuscular injection site of the plasmid, is known as an approach for improving expression levels (Aihara H, Miyazaki J. Nat Biotechnol. 1998 Sep; 16 (9): 867-70 or Mir LM, Bureau MF, Gehl J, Rangara R, Rouy D, Caillaud JM, Delaere P, Branellec D, Schwartz B, Scherman D. Proc Natl Acad Sci U S A. 1999 Apr 13; 96 (8): 4262-7). This approach further improves the expression level by treating the muscle with hyaluronidase before the intramuscular injection of the plasmid (McMahon JM1, Signori E, Wells KE, Fazio VM, Wells DJ., Gene Ther. 2001 Aug; 8 (16): 1264-70). Furthermore, hybridoma production can be performed by a known method, and can also be performed using, for example, a Hybrimune Hybridoma Production System (Cyto Pulse Sciences, Inc.).

Specific examples of obtaining a monoclonal antibody can also include the following procedures:

(a) immune response can be induced by incorporating CD37 cDNA into an expression vector, and directly administering the vector to an animal to be immunized by a method such as electroporation or a gene gun, so as to express CD37 in the body of the animal. The administration of the vector by electroporation or the like may be performed one or more times, preferably a plurality of times, if necessary for enhancing antibody titer;
(b) collection of tissue (e.g., a lymph node) containing antibody-producing cells from the aforementioned animal in which the immune response has been induced;
(c) preparation of myeloma cells (hereinafter, referred to as "myelomas");

(d) cell fusion between the antibody-producing cells and the myelomas;
(e) selection of a hybridoma group producing an antibody of interest;
(f) division into single cell clones (cloning);
(g) the culture of hybridomas for the mass production of monoclonal antibodies, or the breeding of animals into which the hybridomas are inoculated; and
(h) study of the physiological activity (internalization activity) and binding specificity of the monoclonal antibody thus produced, or examination of the properties of the antibody as a labeling reagent.

The resulting monoclonal antibody has high antigen specificity for CD37. Examples of the aforementioned monoclonal antibody can include, but are not particularly limited to, anti-CD37 mouse monoclonal antibody HH1 (Smeland E, et al., Scand J Immunol, 21 (3), 205-214 (1985)).
Examples of the method for measuring the antibody titer used in (a) can include, but are not limited to, flow cytometry and Cell-ELISA.
Furthermore, in the case where the steps (a) to (h) in the specific examples of obtaining a monoclonal antibody described in the above "3. Production of anti-CD37 antibody" are carried out again to obtain independently a monoclonal antibody separately and also in the case where a monoclonal antibody is obtained separately by other methods, an antibody having cytotoxic activity equivalent to that of the anti-CD37 antibody obtained in the step (g) can be obtained. One example of such an antibody can include an antibody binding to the same epitope to which the anti-CD37 antibody obtained in the step (g) binds. If a newly prepared monoclonal antibody binds to a partial peptide or a partial three-dimensional structure to which the anti-CD37 antibody binds, it can be determined that the monoclonal antibody binds to the same epitope to which the anti-CD37 antibody binds. Moreover, by confirming that the monoclonal antibody competes with the anti-CD37 antibody in the binding of the antibody to CD37 (i.e., the monoclonal antibody interferes with the binding of the anti-CD37 antibody to CD37), it can be determined that the monoclonal antibody binds to the same epitope to which the anti-CD37 binds, even if the specific sequence or structure of the epitope has not been determined. When it is confirmed that the monoclonal antibody binds to the same epitope to which the anti-CD37 antibody binds, then it is strongly expected that the monoclonal antibody should have antigen-binding ability or biological activity equivalent to that of the anti-CD37 antibody.

(III) Other antibodies

The antibody of the present invention also includes genetically recombinant antibodies that have been artificially modified for the purpose of, for example, reducing heterogenetic antigenicity to humans or improving the physical properties of the antibody-drug conjugate, such as a chimeric antibody, a humanized antibody and a human antibody, as well as the above-described monoclonal antibody against CD37. These antibodies can be produced by known methods.
Examples of the chimeric antibody can include antibodies in which a variable region and a constant region are heterologous to each other, such as a chimeric antibody formed by conjugating the variable region of a mouse- or rat-derived antibody to a human-derived constant region (see Proc. Natl. Acad. Sci. U.S.A., 81, 6851-6855, (1984)).
Examples of the humanized antibody can include an antibody formed by incorporating only CDRs into a human-derived antibody (see Nature (1986) 321, p. 522-525), an antibody formed by incorporating the amino acid residues from some frameworks, as well as CDR sequences, into a human antibody according to a CDR grafting method (International Publication No. WO90/07861), and an antibody formed by modifying the amino acid sequences of some CDRs while maintaining antigen-binding ability.
Concrete examples of the humanized antibody of the anti-CD37 mouse monoclonal antibody HH1 include an antibody comprising the light chain variable region of hmAb-L11 and the heavy chain variable region of hmAb-H11, hmAb-H541, hmAb-H551, or hmAb-H11a. The amino acid sequence of hmAb-L11 full-length light chain is shown in SEQ ID NO: 2, and the amino acid sequences of hmAb-H11, hmAb-H541, hmAb-H551, and hmAb-H11a full-length heavy chain are shown in SEQ ID NOs: 4, 6, 8, and 10, respectively. In hmAb, the light chain variable region consists of the sequence represented by amino acid positions 21 to 128 in the amino acid sequence of full-length light chain shown in SEQ ID NO: 2, and the heavy chain variable region consists of the sequence represented by amino acid positions 20 to 138 in the amino acid sequence of full-length heavy chain shown in the corresponding SEQ ID NO. The antibody of the present invention further includes an antibody comprising the light chain of hmAb-L11 and the heavy chain of hmAb-H11, hmAb-H541, hmAb-H551, or hmAb-H11a. The amino acid sequence of the light chain of hmAb-L11 comprises the sequence represented by amino acid positions 21 to 234 in the full-length amino acid sequence of the hmAb-L11 light chain shown in SEQ ID NO: 2, and the amino acid sequences of the heavy chains of hmAb-H11, hmAb-H541, hmAb-H551, and hmAb-H11a comprise the sequence represented by amino acid positions 20 to 468 in the full-length amino acid sequences of the hmAb-H11, hmAb-H541, hmAb-H551 and hmAb-H11a heavy chains shown in SEQ ID NOs: 4, 6, 8, and 10, respectively. Specific examples of the humanized antibody of the anti-CD37 mouse monoclonal antibody HH1 can include hmAb-H11L11, hmAb-H541L11, hmAb-H551L11, and hmAb-H11aL11.

In SEQ ID NO: 2, the sequence consisting of the amino acid residues at positions 44 to 54 (KASQDVSTAVD: SEQ ID NO: 19) corresponds to CDRL1, the sequence consisting of the amino acid residues at positions 70 to 76 (WASTRHT: SEQ ID NO: 20) corresponds to CDRL2, and the sequence consisting of the amino acid residues at positions 109 to 117 (RQHYSTPFT: SEQ ID NO: 21) corresponds to CDRL3. In SEQ ID NOs: 4, 6, 8, and 10, the sequence consisting of the amino acid residues at positions 45 to 54 (GYSFTDYNMY: SEQ ID NO: 22) corresponds to CDRH1, the sequence consisting of the amino acid residues at positions 69 to 78 (YIDPYNGDTT:SEQ ID NO: 23) corresponds to CDRH2, and the sequence consisting of the amino acid residues at positions 118 to 127 (SPYGHYAMDY: SEQ ID NO: 24) corresponds to CDRH3. These CDR sequences are described in accordance with the AbM definition (Handbook of Therapeutic Antibodies, Chapter 5, Bioinformatics Tools for Antibody Engineering, Andrew C. R. Martin, James Allen, 2007).

The amino acid substitution in the present description is preferably a conservative amino acid substitution. The conservative amino acid substitution is a substitution occurring within an amino acid group associated with certain amino acid side chains. Preferred amino acid groups are the following: acidic group = aspartic acid and glutamic acid; basic group = lysine, arginine, and histidine; non-polar group = alanine, valine, leucine, isoleucine, proline, phenylalanine, methionine, and tryptophan; and uncharged polar family = glycine, asparagine, glutamine, cysteine, serine, threonine, and tyrosine. Other preferred amino acid groups are the following: aliphatic hydroxy group = serine and threonine; amide-containing group = asparagine and glutamine; aliphatic group = alanine, valine, leucine and isoleucine; and aromatic group = phenylalanine, tryptophan and tyrosine. Such amino acid substitution is preferably carried out without impairing the properties of a substance having the original amino acid sequence.

By combining together sequences showing a high homology to the above-described heavy chain amino acid sequences and light chain amino acid sequences, it is possible to select an antibody having a biological activity equivalent to that of each of the above-described antibodies. Such a homology is a homology of generally 80% or more, preferably 85% or more, more preferably 90% or more, further preferably 95% or more, and most preferably 99% or more. Moreover, also by combining amino acid sequences of a heavy chain and a light chain comprising a substitution, deletion or addition of one or several amino acid residues thereof with respect to the amino acid sequence of a heavy chain or a light chain, it is possible to select an antibody having a biological activity equivalent to that of each of the above-described antibodies.

The homology between two types of amino acid sequences can be determined using default parameters of Blast algorithm version 2.2.2 (Altschul, Stephen F., Thomas L. Madden, Alejandro A. Schaffer, Jinghui Zhang, Zheng Zhang, Webb Miller, and David J. Lipman (1997), "Gapped BLAST and PSI-BLAST: a new generation of protein database search programs", Nucleic Acids Res. 25: 3389-3402). The Blast algorithm can also be used by accessing www.ncbi.nlm.nih.gov/blast through the internet.

In the amino acid sequence of the full-length light chain shown in SEQ ID NO: 2 in the sequence listing, the amino acid sequence consisting of the amino acid residues at positions 1 to 20 corresponds to a signal sequence, the amino acid sequence consisting of the amino acid residues at positions 21 to 128 corresponds to a variable region, and the amino acid sequence consisting of the amino acid residues at positions 129 to 234 corresponds to a constant region. The sequence of SEQ ID NO: 2 is described in Figure 2.

Also, in the amino acid sequence of the full-length heavy chain shown in SEQ ID NO: 4, 6, 8, or 10, the amino acid sequence consisting of the amino acid sequence at positions 1 to 19 corresponds to a signal sequence, the amino acid sequence consisting of the amino acid residues at positions 20 to 138 corresponds to a variable region, and the amino acid sequence consisting of the amino acid residues at positions 139 to 468 corresponds to a constant region. The sequence of SEQ ID NO: 4, 6, 8, or 10 is described in Figure 4, 6, 8, or 10.

Further examples of the antibody of the present invention can include a human antibody binding to CD37. The anti-CD37 human antibody means a human antibody having only the gene sequence of an antibody derived from human chromosomes. The anti-CD37 human antibody can be obtained by a method using a human antibody-producing mouse having a human chromosomal fragment comprising the heavy chain and light chain genes of a human antibody (see Tomizuka, K. et al., Nature Genetics (1997) 16, p. 133-143; Kuroiwa, Y. et al., Nucl. Acids Res. (1998) 26, p. 3447-3448; Yoshida, H. et al., Animal Cell Technology: Basic and Applied Aspects vol. 10, p. 69-73 (Kitagawa, Y., Matsuda, T. and Iijima, S. eds.), Kluwer Academic Publishers, 1999; Tomizuka, K. et al., Proc. Natl. Acad. Sci. USA (2000) 97, p. 722-727; etc.).

Such a human antibody-producing mouse can be specifically produced by using a genetically modified animal, the gene loci of endogenous immunoglobulin heavy chain and light chain of which have been disrupted and instead the gene loci of the human immunoglobulin heavy chain and light chain have been then introduced using a yeast artificial chromosome (YAC) vector or the like, then producing a knock-out animal and a transgenic animal from such a genetically modified animal, and then breeding such animals with one another.

Otherwise, the anti-CD37 human antibody can also be obtained by transforming eukaryotic cells with cDNA encoding each of the heavy chain and light chain of such a human antibody, or preferably with a vector comprising such cDNA, according to genetic recombination techniques, and then culturing the transformed cells and producing a genetically modified human monoclonal antibody, so that the antibody can be obtained from the culture supernatant.

In this context, eukaryotic cells, and preferably, mammalian cells such as CHO cells, lymphocytes or myelomas

can, for example, be used as a host.

Furthermore, a method of obtaining a phage display-derived human antibody that has been selected from a human antibody library (see Wormstone, I. M. et al., Investigative Ophthalmology & Visual Science. (2002) 43 (7), p. 2301-2308; Carmen, S. et al., Briefings in Functional Genomics and Proteomics (2002), 1 (2), p. 189-203; Siriwardena, D. et al., Ophthalmology (2002) 109 (3), p. 427-431; etc.) is also known.

For example, a phage display method, which comprises allowing the variable regions of a human antibody to express as a single chain antibody (scFv) on the surface of phages, and then selecting a phage binding to an antigen, can be applied (Nature Biotechnology (2005), 23, (9), p. 1105-1116).

By analyzing the phage gene that has been selected because of its binding ability to the antigen, DNA sequences encoding the variable regions of a human antibody binding to the antigen can be determined.

Once the DNA sequence of scFv binding to the antigen is determined, an expression vector comprising the aforementioned sequence is produced, and the produced expression vector is then introduced into an appropriate host and can be allowed to express therein, thereby obtaining a human antibody (International Publication No. WO92/01047, International Publication No. WO92/20791, International Publication No. WO93/06213, International Publication No. WO93/11236, International Publication No. WO93/19172, International Publication No. WO95/01438, and International Publication No. WO95/15388, Annu. Rev. Immunol (1994) 12, p. 433-455, Nature Biotechnology (2005) 23 (9), p. 1105-1116).

If a newly produced human antibody binds to a partial peptide or a partial three-dimensional structure to which the anti-CD37 antibody described in the present description binds, it can be determined that the human antibody binds to the same epitope. Moreover, by confirming that the human antibody competes with the anti-CD37 antibody described in the present description in the binding of the antibody to CD37 (i.e., the human antibody interferes with the binding of the anti-CD37 antibody described in the present description to CD37), it can be determined that the human antibody binds to the same epitope to which the anti-CD37 antibody described in the present description binds, even if the specific sequence or structure of the epitope has not been determined. When it is confirmed that the human antibody binds to the same epitope to which the anti-CD37 antibody described in the present description binds, then it is strongly expected that the human antibody should have antigen-binding ability or biological activity equivalent to that of the anti-CD37 antibody described in the present description.

The chimeric antibodies, the humanized antibodies, or the human antibodies obtained by the above-described methods are evaluated for their binding activity against the antigen according to a known method, etc., so that a preferred antibody can be selected.

One example of another indicator for comparison of the properties of antibodies can include the stability of an antibody. A differential scanning calorimeter (DSC) is an apparatus capable of promptly and exactly measuring a thermal denaturation midpoint (Tm) serving as a good indicator for the relative structural stability of a protein. By using DSC to measure Tm values and making a comparison regarding the obtained values, differences in thermal stability can be compared. It is known that the preservation stability of an antibody has a certain correlation with the thermal stability of the antibody (Lori Burton, et al., Pharmaceutical Development and Technology (2007) 12, p. 265-273), and thus, a preferred antibody can be selected using thermal stability as an indicator. Other examples of an indicator for selection of an antibody can include high yield in suitable host cells and low agglutination in an aqueous solution. For example, since an antibody with the highest yield does not always exhibit the highest thermal stability, it is necessary to select an antibody most suitable for administration to a human by comprehensively determining it based on the aforementioned indicators.

The antibody of the present invention also includes a modification of an antibody. A modification is used to mean an antibody of the present invention, which is chemically or biologically modified. Examples of such a chemical modification include the binding of a chemical moiety to an amino acid skeleton, and the chemical modification of an N-linked or O-linked carbohydrate chain. Examples of such a biological modification include antibodies which have undergone a post-translational modification (e.g., N-linked or O-linked glycosylation, amino-terminal or carboxyl-terminal processing, deamidation, isomerization of aspartic acid, oxidation of methionine, and oxidation of tryptophan), and antibodies, to the amino terminus of which a methionine residue is added as a result of having been allowed to be expressed using prokaryote host cells. In addition, such a modification is also meant to include labeled antibodies for enabling detection or isolation of the antibody of the present invention or an antigen, for example, an enzymatically labeled antibody, a fluorescently labeled antibody, and an affinity-labeled antibody. Such a modification of the antibody of the present invention is useful for the improvement of the stability and retention in blood of an antibody; a reduction in antigenicity; detection or isolation of an antibody or an antigen; etc.

Moreover, by regulating a sugar chain modification (glycosylation, de-fucosylation, etc.) that binds to the antibody of the present invention, antibody-dependent cellular cytotoxic activity can be enhanced. As techniques of regulating the sugar chain modification of an antibody, those described in International Publication Nos. WO1999/54342, WO2000/61739, WO2002/31140, WO2007/133855, and WO2013/120066, etc. are known, though the techniques are not limited thereto. The antibody of the present invention also includes antibodies in respect of which the aforementioned sugar chain modification has been regulated.

(2) Method for producing antibody

Once an antibody gene is isolated, the gene can be introduced into an appropriate host to produce an antibody, using an appropriate combination of a host and an expression vector. A specific example of the antibody gene can be a combination of a gene encoding the heavy chain sequence of the antibody described in the present description and a gene encoding the light chain sequence of the antibody described therein. Upon transformation of host cells, such a heavy chain sequence gene and a light chain sequence gene may be inserted into a single expression vector, or these genes may instead each be inserted into different expression vectors.

In a preferred aspect of the antibody gene of the present invention, the light chain sequence gene comprises the nucleotide sequence shown in SEQ ID NO: 1. The sequence of SEQ ID NO: 1 is described in Figure 1.

In a preferred aspect of the antibody gene of the present invention, the heavy chain sequence gene comprises the nucleotide sequence shown in SEQ ID NO: 3, 5, 7 or 9. The sequence of SEQ ID NO: 3, 5, 7 or 9 is described in Figure 3, 5, 7 or 9.

In a particularly preferred aspect of the antibody gene of the present invention, the light chain sequence gene has the nucleotide sequence shown in SEQ ID NO: 1, and the heavy chain sequence gene comprises the nucleotide sequence shown in SEQ ID NO: 3, 5, 7 or 9. Specifically, the antibody gene of the present invention is particularly preferably any one of the following antibody genes (i) to (iv):

(i) an antibody gene in which the light chain sequence gene has the nucleotide sequence shown in SEQ ID NO: 1, and the heavy chain sequence gene comprises the nucleotide sequence shown in SEQ ID NO: 3,
(ii) an antibody gene in which the light chain sequence gene has the nucleotide sequence shown in SEQ ID NO: 1, and the heavy chain sequence gene comprises the nucleotide sequence shown in SEQ ID NO: 5,
(iii) an antibody gene in which the light chain sequence gene has the nucleotide sequence shown in SEQ ID NO: 1, and the heavy chain sequence gene comprises the nucleotide sequence shown in SEQ ID NO: 7, and
(iv) an antibody gene in which the light chain sequence gene has the nucleotide sequence shown in SEQ ID NO: 1, and the heavy chain sequence gene comprises the nucleotide sequence shown in SEQ ID NO: 9.

When eukaryotic cells are used as hosts, animal cells, plant cells or eukaryotic microorganisms can be used. In particular, examples of the animal cells can include mammalian cells such as COS cells which are monkey cells (Gluzman, Y., Cell (1981) 23, p. 175-182, ATCC CRL-1650), mouse fibroblasts NIH3T3 (ATCC No. CRL-1658), a dihydrofolate reductase-deficient cell line of Chinese hamster ovary cells (CHO cells, ATCC CCL-61) (Urlaub, G. and Chasin, L. A. Proc. Natl. Acad. Sci. U.S.A. (1980) 77, p. 4126-4220), and FreeStyle 293F cells (Invitrogen Corp.).

When prokaryotic cells are used as hosts, *Escherichia coli* or *Bacillus subtilis* can be used, for example.

An antibody gene of interest is introduced into these cells for transformation, and the transformed cells are then cultured *in vitro* to obtain an antibody. In the aforementioned culture, there are cases where yield is different depending on the sequence of the antibody, and thus, it is possible to select an antibody, which is easily produced as a medicament, from antibodies having equivalent binding activity, using the yield as an indicator. Accordingly, the antibody of the present invention also includes an antibody obtained by the above-described method for producing an antibody, which comprises a step of culturing the transformed host cells and a step of collecting an antibody of interest or an antigen binding fragment of the antibody from the culture obtained in the aforementioned step.

It is known that the lysine residue at the carboxyl terminus of the heavy chain of an antibody produced by cultured mammalian cells is deleted (Journal of Chromatography A, 705: 129-134 (1995)), and also, it is known that the two amino acid residues at the heavy chain carboxyl terminus, glycine and lysine, are deleted, and that the proline residue newly positioned at the carboxyl terminus is amidated (Analytical Biochemistry, 360: 75-83 (2007)). However, such deletion and modification of these heavy chain sequences does not have an influence on the antigen-binding activity and effector function (activation of complement, antibody-dependent cellular cytotoxicity, etc.) of an antibody. Accordingly, the antibody according to the present invention also includes an antibody that has undergone the aforementioned modification, and an antigen binding fragment of the antibody, and specific examples of such an antibody include a deletion mutant comprising a deletion of 1 or 2 amino acids at the heavy chain carboxyl terminus, and a deletion mutant formed by amidating the aforementioned deletion mutant (e.g., a heavy chain in which the proline residue at the carboxyl-terminal site is amidated). However, deletion mutants involving a deletion at the carboxyl terminus of the heavy chain of the antibody according to the present invention are not limited to the above-described deletion mutants, as long as they retain antigen-binding activity and/or effector function. Two heavy chains constituting the antibody according to the present invention may be any one type of heavy chain selected from the group consisting of a full-length antibody and the above-described deletion mutants, or may be a combination of any two types selected from the aforementioned group. The ratio of individual deletion mutants can be influenced by the types of cultured mammalian cells that produce the antibody according to the present invention, and the culture conditions. Examples of the main ingredient of the antibody according to the present invention can include antibodies where one amino acid residue is deleted at each of the carboxyl termini of the two heavy chains.

Examples of the isotype of the antibody of the present invention can include IgG (IgG1, IgG2, IgG3, and IgG4). Among others, IgG1 and IgG2 are preferable.

Examples of the biological activity of an antibody can generally include antigen-binding activity, activity of being internalized into cells expressing an antigen by binding to the antigen, activity of neutralizing the activity of an antigen, activity of enhancing the activity of an antigen, antibody-dependent cellular cytotoxic (ADCC) activity, complement-dependent cytotoxic (CDC) activity, and antibody-dependent cellular phagocytosis (ADCP). The function of the antibody according to the present invention is binding activity against CD37 and is preferably the activity of being internalized into CD37-expressing cells by binding to CD37. Moreover, the antibody of the present invention may have ADCC activity, CDC activity and/or ADCP activity, as well as cellular internalization activity.

The obtained antibody can be purified to a homogenous state. For separation and purification of the antibody, separation and purification methods used for ordinary proteins may be used. For example, column chromatography, filtration, ultrafiltration, salting-out, dialysis, preparative polyacrylamide gel electrophoresis, and isoelectric focusing are appropriately selected and combined with one another, so that the antibody can be separated and purified (Strategies for Protein Purification and Characterization: A Laboratory Course Manual, Daniel R. Marshak et al. eds., Cold Spring Harbor Laboratory Press (1996); and Antibodies: A Laboratory Manual. Ed Harlow and David Lane, Cold Spring Harbor Laboratory (1988)), though examples of the separation and purification methods are not limited thereto.

Examples of the chromatography can include affinity chromatography, ion exchange chromatography, hydrophobic chromatography, gel filtration chromatography, reverse phase chromatography, and absorption chromatography.

These chromatographic techniques can be carried out using liquid chromatography such as HPLC or FPLC.

Examples of the column used in the affinity chromatography can include a Protein A column and a Protein G column. Examples of columns that can be used as a Protein A column can include Hyper D, POROS, and Sepharose F. F. (Pharmacia).

Also, using an antigen-immobilized carrier, the antibody can be purified by utilizing the binding activity of the antibody to the antigen.

4. Anti-CD37 antibody-drug conjugate

The anti-CD37 antibody-drug conjugate used in the present invention is an anti-CD37 antibody-drug conjugate in which a drug-linker represented by the following formula and an anti-CD37 antibody are conjugated via a thioether bond:

[Formula 13]

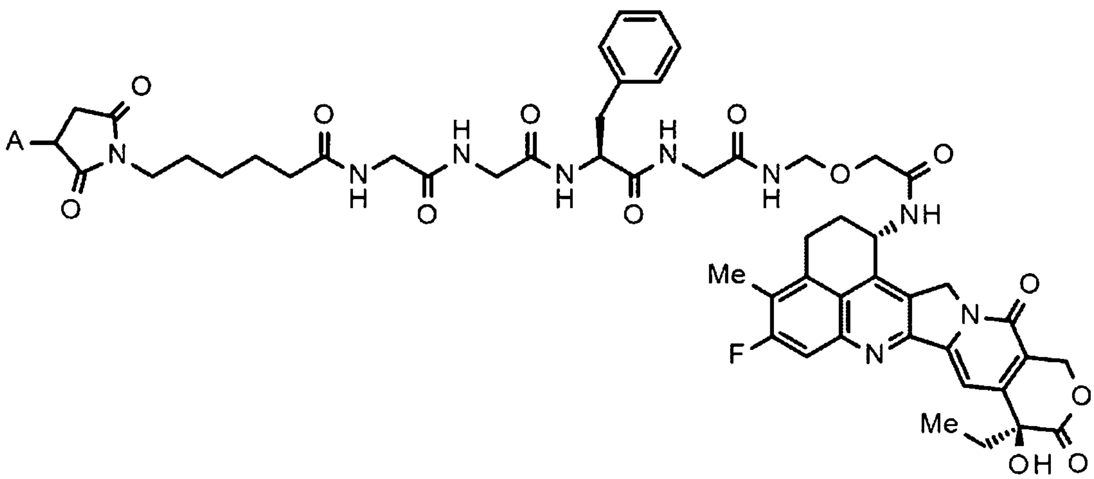

wherein A represents a connecting position to the anti-CD37 antibody.

In the present invention, the partial structure consisting of a linker and a drug in the anti-CD37 antibody-drug conjugate is referred to as a "drug linker". This drug linker is connected to a thiol group (in other words, the sulfur atom of a cysteine residue) formed at an interchain disulfide bond site (two sites between heavy chains and two sites between a heavy chain and a light chain) in the antibody.

The drug linker of the present invention comprises a topoisomerase I inhibitor exatecan (IUPAC name: (1S,9S)-1-amino-9-ethyl-5-fluoro-1,2,3,9,12,15-hexahydro-9-hydroxy-4-methyl-10H,13H-benzo[de]pyrano[3',4':6,7]indolizino[1,2-b]quinoline-10,13-dione, (chemical name: (1S,9S)-1-amino-9-ethyl-5-fluoro-2,3-dihydro-9-hydroxy-4-methyl-1H,12H-benzo[de]pyrano[3',4':6,7]indolizino[1,2-b]quinoline-10,13(9H,15H)-dione)) as a component. Exatecan is represented by the following formula:

[Formula 14]

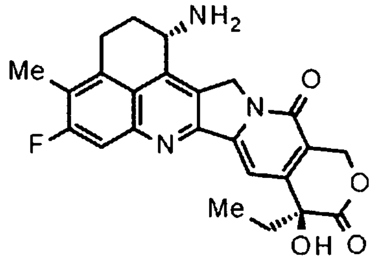

and is a camptothecin derivative having an antitumor effect.

The anti-CD37 antibody-drug conjugate used in the present invention can also be represented by the following formula:

[Formula 15]

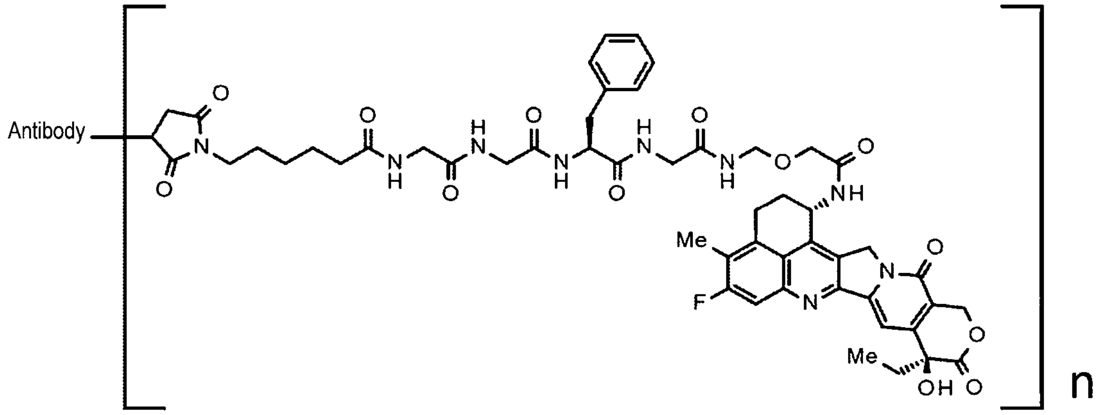

In this context, the antibody is conjugated to a drug linker through a thioether bond. Also, n has the same meaning as that of the so-called drug-to-antibody ratio (DAR), and represents the average number of units of the drug linker conjugated per antibody.

After migrating into cancer cells, the anti-CD37 antibody-drug conjugate used in the present invention is cleaved at the linker moiety so that a compound represented by the following formula:

[Formula 16]

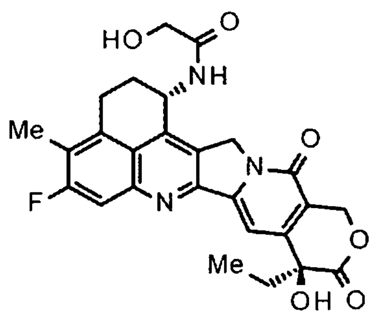

is released.

The above-described compound is considered as the original source of the antitumor activity of the anti-CD37 antibody-drug conjugate used in the present invention, and has been confirmed to have a topoisomerase I inhibitory effect (Ogitani Y. et al., Clinical Cancer Research, 2016, Oct 15; 22 (20): 5097-5108, Epub 2016 Mar 29).

It is to be noted that the drug linker of the anti-CD37 antibody-drug conjugate used in the present invention is also known to have a bystander effect (Ogitani Y. et al., Cancer Science (2016) 107, 1039-1046).

This bystander antitumor effect is exerted through a process in which the anti-CD37 antibody-drug conjugate used in the present invention is internalized into target-expressing cancer cells where the above-described compound is

then released so as to also exert an antitumor effect on non-target-expressing cancer cells present therearound.

This bystander antitumor effect is also exerted as an excellent antitumor effect when the anti-CD37 antibody-drug conjugate according to the present invention is used in combination with another drug.

(1) Drug

The anti-CD37 antibody obtained in the above "3. Production of anti-CD37 antibody" can be conjugated to a drug via a linker structure moiety to prepare an anti-CD37 antibody-drug conjugate. The drug is not particularly limited as long as it has a substituent or a partial structure that can be connected to a linker structure. The anti-CD37 antibody-drug conjugate can be used for various purposes according to the conjugated drug. Examples of such a drug can include substances having antitumor activity, substances effective for blood diseases, substances effective for autoimmune diseases, anti-inflammatory substances, antimicrobial substances, antifungal substances, antiparasitic substances, antiviral substances, and anti-anesthetic substances.

(1)-1 Antitumor compound

An example using an antitumor compound as a compound to be conjugated in the anti-CD37 antibody-drug conjugate of the present invention will be described below. The antitumor compound is not particularly limited as long as the compound has an antitumor effect and has a substituent or a partial structure that can be connected to a linker structure. Upon cleavage of a part or the whole of the linker in tumor cells, the antitumor compound moiety is released so that the antitumor compound exhibits an antitumor effect. As the linker is cleaved at a connecting position with the drug, the antitumor compound is released in its original structure to exert its original antitumor effect.

As one example of the antitumor compound used in the present invention, exatecan, a camptothecin derivative ((1S,9S)-1-amino-9-ethyl-5-fluoro-2,3-dihydro-9-hydroxy-4-methyl-1H,12H-benzo[de]pyrano[3',4':6,7]indolizino[1,2-b]quinoline-10,13(9H,15H)-dione represented by the following formula) can preferably be used.

[Formula 17]

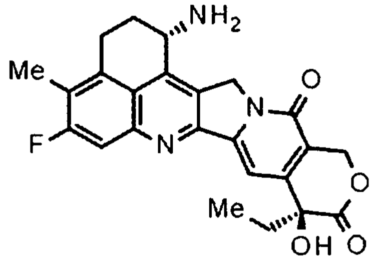

The compound can be easily obtained by, for example, a method described in U.S. Patent Publication No. 2016/0297890 or other known methods, and the amino group at position 1 can be preferably used as a connecting position to the linker structure. Further, exatecan may be released into tumor cells while a part of the linker is still attached thereto. However, the compound exerts an excellent antitumor effect even in such a state.

Since exatecan has a camptothecin structure, it is known that the equilibrium shifts to a structure with a formed lactone ring (closed ring) in an acidic aqueous medium (e.g., of the order of pH 3) whereas the equilibrium shifts to a structure with an opened lactone ring (open ring) in a basic aqueous medium (e.g., of the order of pH 10). A drug conjugate into which exatecan residues corresponding to such a closed ring structure and an open ring structure have been introduced is also expected to have an equivalent antitumor effect, and a drug conjugate into which an exatecan group corresponding to any structure has been introduced is included within the scope of the present invention.

Other examples of the antitumor compound can include antitumor compounds described in the literature (Pharmacological Reviews, 68, p. 3-19, 2016). Specific examples thereof can include doxorubicin, calicheamicin, dolastatin 10, auristatins such as monomethyl auristatin E (MMAE) and monomethyl auristatin F (MMAF), maytansinoids such as DM1 and DM4, a pyrrolobenzodiazepine dimer SG2000 (SJG-136), a camptothecin derivative SN-38, duocarmycins such as CC-1065, amanitin, daunorubicin, mitomycin C, bleomycin, cyclocytidine, vincristine, vinblastine, methotrexate, platinum-based antitumor agents (cisplatin and derivatives thereof), and Taxol and derivatives thereof.

In the antibody-drug conjugate, the number of conjugated drug molecules per antibody molecule is a key factor having an influence on the efficacy and safety thereof. The production of the antibody-drug conjugate is carried out by specifying reaction conditions such as the amounts of starting materials and reagents used for reaction, so as to attain a constant number of conjugated drug molecules. Unlike the chemical reaction of a low-molecular-weight compound, a mixture containing different numbers of conjugated drug molecules is usually obtained. The number of conjugated drug

molecules per antibody molecule is defined and indicated as an average value, i.e., the average number of conjugated drug molecules. Unless otherwise specified, i.e., except in the case of representing an antibody-drug conjugate having a specific number of conjugated drug molecules that is included in an antibody-drug conjugate mixture having different numbers of conjugated drug molecules, the number of conjugated drug molecules according to the present invention also means an average value as a rule. The number of exatecan molecules conjugated to an antibody molecule is controllable, and as an average number of conjugated drug molecules per antibody, approximately 1 to 10 exatecan molecules can be conjugated. The number of exatecan molecules is preferably 2 to 8, more preferably 5 to 8, further preferably 7 to 8, still further preferably about 8 or 8. It is to be noted that a person skilled in the art can design a reaction for conjugating a required number of drug molecules to an antibody molecule based on the description of Examples of the present application, and can obtain an antibody-drug conjugate with a controlled number of conjugated exatecan molecules.

(2) Linker structure

The linker structure which conjugates the drug to the anti-CD37 antibody in the anti-CD37 antibody-drug conjugate of the present invention will be described.

In the antibody-drug conjugate of the present application, the linker structure which conjugates the anti-CD37 antibody to the drug is not particularly limited as long as the resulting antibody-drug conjugate can be used. The linker structure may be appropriately selected and used according to the purpose of use. Examples of the linker structure can include a linker described in known literature (Pharmacol Rev 68: 3-19, January 2016, Protein Cell DOI 10.1007/s13238-016-0323-0, etc.). Further specific examples thereof can include VC (valine-citrulline), MC (maleimidocaproyl), SMCC (succinimidyl 4-(N-maleimidomethyl) cyclohexane-1-carboxylate), SPP (N-succinimidyl 4-(2-pyridyldithio)pentanoate, SS (disulfide), SPDB (N-succinimidyl 4-(2-pyridyldithio)butyrate, SS/hydrazone, hydrazone and carbonate.

Another example thereof can include a linker structure described in U.S. Patent Publication No. 2016/0297890 (for example, those described in the paragraphs [0260] to [0289] in the aforementioned patent publication literature). Any linker structure given below can be preferably used. It is to be noted that the left terminus of the structure is a connecting position to the antibody, and the right terminus thereof is a connecting position to the drug. Furthermore, GGFG in the linker structures given below represents an amino acid sequence consisting of glycine-glycine-phenylalanine-glycine (GGFG) linked through peptide bonds.

- (Succinimid-3-yl-N)-$CH_2CH_2$-C(=O)-GGFG-NH-$CH_2CH_2CH_2$-C(=O)-,
- (Succinimid-3-yl-N)-$CH_2CH_2CH_2CH_2CH_2$-C(=O)-GGFG-NH-$CH_2CH_2CH_2$-C(=O) -,
- (Succinimid-3-yl-N)-$CH_2CH_2CH_2CH_2CH_2$-C(=O) -GGFG-NH-$CH_2$-O-$CH_2$-C(=O) -,
- (Succinimid-3-yl-N)-$CH_2CH_2CH_2CH_2CH_2$-C(=O)-GGFG-NH-$CH_2CH_2$-O-$CH_2$-C(=O) -,
- (Succinimid-3-yl-N)-$CH_2CH_2$-C(=O) -NH-$CH_2CH_2$O-$CH_2CH_2$O-$CH_2CH_2$-C(=O)-GGFG-NH-$CH_2CH_2CH_2$-C(=O) -, and
- (Succinimid-3-yl-N)-$CH_2CH_2$-C(=O)-NH-$CH_2CH_2$O-$CH_2CH_2$O-$CH_2CH_2$O-$CH_2CH_2$O-$CH_2CH_2$-C(=O)-GGFG-NH-$CH_2CH_2CH_2$-C(=O)-.

More preferred are the following:

- (Succinimid-3-yl-N)-$CH_2CH_2CH_2CH_2CH_2$-C(=O) -GGFG-NH-$CH_2$-O-$CH_2$-C(=O) -,
- (Succinimid-3-yl-N)-$CH_2CH_2CH_2CH_2CH_2$-C(=O)-GGFG-NH-$CH_2CH_2$-O-$CH_2$-C(=O) -, and
- (Succinimid-3-yl-N)-$CH_2CH_2$-C(=O)-NH-$CH_2CH_2$O-$CH_2CH_2$O-$CH_2CH_2$-C(=O)-GGFG-NH-$CH_2CH_2CH_2$-C(=O) -.

Still more preferred are the following:

- (Succinimid-3-yl-N)-$CH_2CH_2CH_2CH_2CH_2$-C(=O)-GGFG-NH-$CH_2$-O-$CH_2$-C(=O) -, and
- (Succinimid-3-yl-N)-$CH_2CH_2$-C(=O)-NH-$CH_2CH_2$O-$CH_2CH_2$O-$CH_2CH_2$-C(=O)-GGFG-NH-$CH_2CH_2CH_2$-C(=O) -.

The antibody is connected to the terminus of - (Succinimid-3-yl-N)(e.g., a terminus opposite (left terminus) to the terminus to which -$CH_2CH_2CH_2CH_2CH_2$- is connected in "-(Succinimid-3-yl-N)-$CH_2CH_2CH_2CH_2CH_2$-C(=O)-GGFG-NH-$CH_2$-O-$CH_2$-C(=O)-"), and the antitumor compound is connected to a terminus (the carbonyl group of $CH_2$-O-$CH_2$-C(=O)- at the right terminus in the above-described example) opposite to the terminus to which the antibody is connected to -(Succinimid-3-yl-N). "- (Succinimid-3-yl-N)-" has a structure represented by the following formula:

[Formula 18]

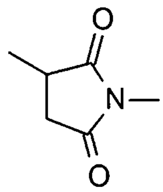

Position 3 of this partial structure is the connecting position to the anti-CD37 antibody. This connection to the antibody at position 3 is characterized by forming a thioether bond. The nitrogen atom at position 1 of this structural moiety is connected to the carbon atom of methylene which is present within the linker including this structure.

In the antibody-drug conjugate of the present invention having exatecan as the drug, a drug-linker structural moiety having any structure given below is preferred for conjugation to the antibody. For these drug-linker structural moieties, the average number conjugated per antibody may be 1 to 10 and is preferably 2 to 8, more preferably 5 to 8, further preferably 7 to 8, and still further preferably approximately 8 or 8.

- (Succinimid-3-yl-N)-$CH_2CH_2$-C(=O)-GGFG-NH-$CH_2CH_2CH_2$-C(=O)-(NH-DX),
- (Succinimid-3-yl-N)-$CH_2CH_2CH_2CH_2CH_2$-C(=O) -GGFG-NH-$CH_2CH_2CH_2$-C(=O) - (NH-DX),
- (Succinimid-3-yl-N)-$CH_2CH_2CH_2CH_2CH_2$-C(=O) -GGFG-NH-$CH_2$-O-$CH_2$-C(=O) - (NH-DX),
- (Succinimid-3-yl-N)-$CH_2CH_2CH_2CH_2CH_2$-C(=O)-GGFG-NH-$CH_2CH_2$-O-$CH_2$-C(=O)-(NH-DX),
- (Succinimid-3-yl-N)-$CH_2CH_2$-C(=O)-NH-$CH_2CH_2O$-$CH_2CH_2O$-$CH_2CH_2$-C(=O)-GGFG-NH-$CH_2CH_2CH_2$-C(=O) - (NH-DX), and
- (Succinimid-3-yl-N)-$CH_2CH_2$-C(=O)-NH-$CH_2CH_2O$-$CH_2CH_2O$-$CH_2CH_2O$-$CH_2CH_2O$-$CH_2CH_2$-C(=O)-GGFG-NH-$CH_2CH_2CH_2$-C(=O)-(NH-DX) .

More preferred are the following:

- (Succinimid-3-yl-N)-$CH_2CH_2CH_2CH_2CH_2$-C(=O)-GGFG-NH-$CH_2$-O-$CH_2$-C(=O) - (NH-DX),
- (Succinimid-3-yl-N)-$CH_2CH_2CH_2CH_2CH_2$-C(=O) -GGFG-NH-$CH_2CH_2$-O-$CH_2$-C(=O)-(NH-DX), and
- (Succinimid-3-yl-N)-$CH_2CH_2$-C(=O)-NH-$CH_2CH_2O$-$CH_2CH_2O$-$CH_2CH_2$-C(=O)-GGFG-NH-$CH_2CH_2CH_2$-C(=O)-(NH-DX) .

Still more preferred are the following:

- (Succinimid-3-yl-N)-$CH_2CH_2CH_2CH_2CH_2$-C(=O)-GGFG-NH-$CH_2$-O-$CH_2$-C(=O)-(NH-DX), and
- (Succinimid-3-yl-N)-$CH_2CH_2$-C(=O)-NH-$CH_2CH_2O$-$CH_2CH_2O$-$CH_2CH_2$-C(=O)-GGFG-NH-$CH_2CH_2CH_2$-C(=O)-(NH-DX) .

-(NH-DX) has a structure represented by the following formula:

[Formula 19]

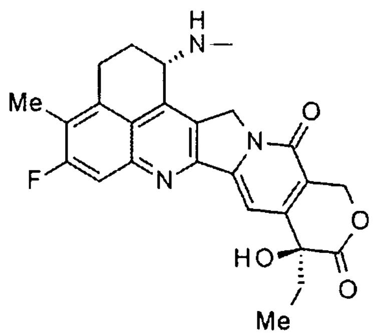

and it represents a group that is derived by removing one hydrogen atom from the amino group at position 1 of exatecan.

(3) Method for producing antibody-drug conjugate

The antibody that can be used in the antibody-drug conjugate of the present invention is not particularly limited as long as it is an anti-CD37 antibody having internalization activity or an antigen binding fragment of the antibody, as described in the above section "3. Production of anti-CD37 antibody" and the Examples.

Next, a typical method for producing the antibody-drug conjugate of the present invention will be described. It is to be noted that, in the description below, "compound or antibody-drug conjugate No." shown in each reaction scheme is used to represent a compound or an antibody-drug conjugate. Specifically, each compound or antibody-drug conjugate is referred to as a "compound of formula (1)", "antibody-drug conjugate of formula (1)", "compound (1)", "antibody-drug conjugate (1)", or the like. The same holds true for the other compound or antibody-drug conjugate Nos.

(3)-1 Production method 1

The antibody-drug conjugate of formula (1) given below in which the anti-CD37 antibody is connected to the linker structure via a thioether can be produced by reacting antibody AB having a sulfhydryl group converted from a disulfide bond by the reduction of the anti-CD37 antibody, with the compound (2) obtainable by a known method (e.g., obtainable by a method described in U.S. Patent Publication No. 2016/297890 (e.g., a method described in the paragraphs [0336] to [0374] in the aforementioned patent publication literature)). This antibody-drug conjugate can be produced by the following method, for example.

[Expression 1]

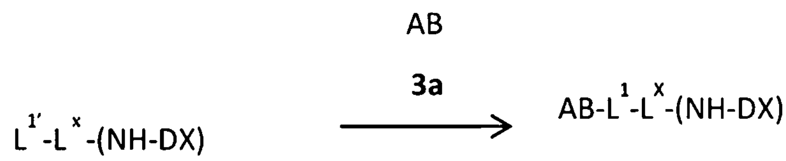


(2) (1)

wherein AB represents an antibody with a sulfhydryl group, wherein
$L^1$ has a structure represented by -(Succinimid-3-yl-N)-, and
$L^{1'}$ represents a maleimidyl group represented by the following formula.

[Formula 20]

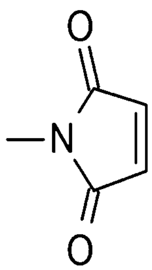

$-L^1-L^X$ has a structure represented by any of the following formulas:

- (Succinimid-3-yl-N)-$CH_2CH_2$-C(=O)-GGFG-NH-$CH_2CH_2CH_2$-C(=O)-,
- (Succinimid-3-yl-N)-$CH_2CH_2CH_2CH_2CH_2$-C(=O) -GGFG-NH-$CH_2CH_2CH_2$-C(=O) -,
- (Succinimid-3-yl-N)-$CH_2CH_2CH_2CH_2CH_2$-C(=O) -GGFG-NH-$CH_2$-O-$CH_2$-C(=O) -,
- (Succinimid-3-yl-N)-$CH_2CH_2CH_2CH_2CH_2$-C(=O) -GGFG-NH-$CH_2CH_2$-O-$CH_2$-C(=O) -,
- (Succinimid-3-yl-N)-$CH_2CH_2$-C(=O)-NH-$CH_2CH_2O$-$CH_2CH_2O$-$CH_2CH_2$-C(=O)-GGFG-NH-$CH_2CH_2CH_2$-C(=O) -, and
- (Succinimid-3-yl-N)-$CH_2CH_2$-C(=O) -NH-$CH_2CH_2O$-$CH_2CH_2O$-$CH_2CH_2O$-$CH_2CH_2O$-$CH_2CH_2$-C(=O)-GGFG-NH-$CH_2CH_2CH_2$-C(=O)-.

More preferred are the following:

- (Succinimid-3-yl-N)-$CH_2CH_2CH_2CH_2CH_2$-C(=O)-GGFG-NH-$CH_2$-O-$CH_2$-C(=O) -,
- (Succinimid-3-yl-N)-$CH_2CH_2CH_2CH_2CH_2$-C(=O)-GGFG-NH-$CH_2CH_2$-O-$CH_2$-C(=O) -, and
- (Succinimid-3-yl-N)-$CH_2CH_2$-C(=O)-NH-$CH_2CH_2O$-$CH_2CH_2O$-$CH_2CH_2$-C(=O)-GGFG-NH-$CH_2CH_2CH_2$-C(=O)-.

Still more preferred are the following:

- (Succinimid-3-yl-N)-$CH_2CH_2CH_2CH_2CH_2$-C(=O)-GGFG-NH-$CH_2$-O-$CH_2$-C(=O) -, and
- (Succinimid-3-yl-N)-$CH_2CH_2$-C(=O)-NH-$CH_2CH_2O$-$CH_2CH_2O$-$CH_2CH_2$-C(=O)-GGFG-NH-$CH_2CH_2CH_2$-C(=O)-.

In the above-described reaction scheme, the antibody-drug conjugate (1) can be understood as having a structure in which one structure moiety from the drug to the linker terminus is connected to one antibody. However, this description is given for the sake of convenience, and there are actually many cases in which a plurality of the aforementioned structural moieties is connected to one antibody molecule. The same holds true for the explanation of the production method described below.

Specifically, the antibody-drug conjugate (1) can be produced by reacting the compound (2) obtainable by a known method (e.g., obtainable by a method described in U.S. Patent Publication No. 2016/297890 (e.g., a method described in the paragraphs [0336] to [0374] in the aforementioned patent publication literature)), with the antibody having a sulfhydryl group.

The antibody having a sulfhydryl group can be obtained by a method well known to a person skilled in the art (Hermanson, G. T, Bioconjugate Techniques, pp. 56-136, pp. 456-493, Academic Press (1996)). Examples of the method can include, but are not limited to: Traut's reagent being reacted with the amino group of the antibody; N-succinimidyl S-acetylthioalkanoates being reacted with the amino group of the antibody followed by reaction with hydroxylamine; N-succinimidyl 3-(pyridyldithio)propionate being reacted with the antibody, followed by reaction with a reducing agent; the antibody being reacted with a reducing agent such as dithiothreitol, 2-mercaptoethanol, or tris(2-carboxyethyl)phosphine hydrochloride (TCEP) to reduce the interchain disulfide bond in the antibody, so as to form a sulfhydryl group.

Specifically, an antibody with interchain disulfide bonds partially or completely reduced can be obtained by using 0.3 to 3 molar equivalents of TCEP as a reducing agent per interchain disulfide bond in the antibody, and reacting the reducing agent with the antibody in a buffer solution containing a chelating agent. Examples of the chelating agent can include ethylenediaminetetraacetic acid (EDTA) and diethylenetriaminepentaacetic acid (DTPA). The chelating agent can be used at a concentration of 1 mM to 20 mM. A solution of sodium phosphate, sodium borate, sodium acetate, or the like can be used as the buffer solution. As a specific example, the antibody having partially or completely reduced sulfhydryl groups can be obtained by reacting the antibody with TCEP at 4°C to 37°C for 1 hour to 4 hours. In this context, by carrying out an addition reaction of a sulfhydryl group to a drug-linker moiety, the drug-linker moiety can be conjugated by a thioether bond.

Then, using 2 to 20 molar equivalents of the compound (2) per antibody having a sulfhydryl group, the antibody-drug conjugate (1) in which 2 to 8 drug molecules are conjugated per antibody can be produced. Specifically, a solution containing the compound (2) dissolved therein may be added to a buffer solution containing the antibody having a sulfhydryl group for the reaction. In this context, a sodium acetate solution, sodium phosphate, sodium borate, or the like can be used as the buffer solution. pH for the reaction is 5 to 9, and more preferably, the reaction may be performed near pH 7. An organic solvent such as dimethyl sulfoxide (DMSO), dimethylformamide (DMF), dimethylacetamide (DMA), or N-methyl-2-pyrrolidone (NMP) can be used as a solvent for dissolving the compound (2). The reaction may be performed by adding the solution containing the compound (2) dissolved in the organic solvent at 1 to 20% v/v to a buffer solution containing the antibody having a sulfhydryl group. The reaction temperature is 0°C to 37°C, more preferably 10°C to 25°C, and the reaction time is 0.5 hours to 2 hours. The reaction can be terminated by deactivating the reactivity of unreacted compound (2) with a thiol-containing reagent. The thiol-containing reagent is, for example, cysteine or N-acetyl-L-cysteine (NAC). More specifically, the reaction can be terminated by adding 1 to 2 molar equivalents of NAC to the compound (2) used, and incubating the obtained mixture at room temperature for 10 minutes to 30 minutes.

(4) Identification of antibody-drug conjugate

The produced antibody-drug conjugate (1) can be subjected to concentration, buffer exchange, purification, and measurement of antibody concentration and the average number of conjugated drug molecules per antibody molecule according to common procedures described below, to identify the antibody-drug conjugate (1).

(4)-1 Common procedure A: Concentration of aqueous solution of antibody or antibody-drug conjugate

To an Amicon Ultra (50,000 MWCO, Millipore Corporation) container, a solution of an antibody or an antibody-drug conjugate is added, and the solution of the antibody or the antibody-drug conjugate is concentrated by centrifugation (centrifugation at 2000 G to 3800 G for 5 to 20 minutes) using a centrifuge (Allegra X-15R, Beckman Coulter, Inc.)

(4)-2 Common procedure B: Measurement of antibody concentration

Using a UV detector (Nanodrop 1000, Thermo Fisher Scientific Inc.), measurement of the antibody concentration is carried out according to the method defined by the manufacturer. In this respect, 280 nm absorption coefficient differing among antibodies (1.3 $mLmg^{-1}cm^{-1}$ to 1.8 $mLmg^{-1}cm^{-1}$) is used.

(4)-3 Common procedure C: Buffer exchange for antibody

A NAP-25 column (Cat. No. 17-0852-02, GE Healthcare Japan Corporation) using Sephadex G-25 carrier is equilibrated with a phosphate buffer (50 mM, pH 6.0) (referred to as PBS6.0/EDTA in the present description) containing sodium chloride (50 mM) and EDTA (2 mM) according to the method defined by the manufacturer. An aqueous solution of the antibody is applied in an amount of 2.5 mL per NAP-25 column, and thereafter, a fraction (3.5 mL) eluted with 3.5 mL of PBS6.0/EDTA is collected. This fraction is concentrated by common procedure A. After measurement of the concentration of the antibody using common procedure B, the antibody concentration is adjusted to 10 mg/mL using PBS6.0/EDTA.

(4)-4 Common procedure D: Purification of antibody-drug conjugate

A NAP-25 column is equilibrated with any commercially available buffer solution such as an acetate buffer containing sorbitol (5%) (10 mM, pH 5.5; referred to as ABS in the present description). An aqueous reaction solution of the antibody-drug conjugate (approximately 2.5 mL) is applied to the NAP-25 column, and thereafter, elution is carried out with the buffer solution in an amount defined by the manufacturer, so as to collect an antibody fraction. A gel filtration purification process, in which the collected fraction is applied again to the NAP-25 column, and elution is carried out with the buffer solution, is repeated a total of 2 or 3 times to obtain the antibody-drug conjugate excluding non-conjugated drug linker and low-molecular-weight compounds (tris(2-carboxyethyl)phosphine hydrochloride (TCEP), N-acetyl-L-cysteine (NAC), and dimethyl sulfoxide).

(4)-5 Common procedure E: Measurement (1) of antibody concentration in antibody-drug conjugate and average number of conjugated drug molecules per antibody molecule

The conjugated drug concentration in the antibody-drug conjugate can be calculated by measuring UV absorbance of an aqueous solution of the antibody-drug conjugate at two wavelengths of 280 nm and 370 nm, and thereafter performing the calculation shown below.

The total absorbance at any given wavelength is equal to the sum of the absorbance of all light-absorbing chemical species that are present in a system [additivity of absorbance]. Therefore, based on the hypothesis that the molar absorption coefficients of the antibody and the drug do not vary between before and after conjugation between the antibody and the drug, the antibody concentration and the drug concentration in the antibody-drug conjugate are represented by the following equations.

$$A_{280} = A_{D,280} + A_{A,280} = \varepsilon_{D,280}C_D + \varepsilon_{A,280}C_A \quad \text{Equation (1)}$$

$$A_{370} = A_{D,370} + A_{A,370} = \varepsilon_{D,370}C_D + \varepsilon_{A,370}C_A \quad \text{Equation (2)}$$

In this context, $A_{280}$ represents the absorbance of an aqueous solution of the antibody-drug conjugate at 280 nm, $A_{370}$ represents the absorbance of an aqueous solution of the antibody-drug conjugate at 370 nm, $A_{A,280}$ represents the absorbance of the antibody at 280 nm, $A_{A,370}$ represents the absorbance of the antibody at 370 nm, $A_{D,280}$ represents the absorbance of a conjugate precursor at 280 nm, $A_{D,370}$ represents the absorbance of a conjugate precursor at 370 nm, $\varepsilon_{A,280}$ represents the molar absorption coefficient of the antibody at 280 nm, $\varepsilon_{A,370}$ represents the molar absorption coefficient of the antibody at 370 nm, $\varepsilon_{D,280}$ represents the molar absorption coefficient of a conjugate precursor at 280 nm, $\varepsilon_{D,370}$ represents the molar absorption coefficient of a conjugate precursor at 370 nm, $C_A$ represents the antibody concentration in the antibody-drug conjugate, and $C_D$ represents the drug concentration in the antibody-drug conjugate.

In this context, with regard to $\varepsilon_{A,280}$, $\varepsilon_{A,370}$, $\varepsilon_{D,280}$, and $\varepsilon_{D,370}$, preliminarily prepared values (estimated values

based on calculation or measurement values obtained by UV measurement of the compound) are used. For example, $\varepsilon_{A,280}$ can be estimated from the amino acid sequence of the antibody by a known calculation method (Protein Science, 1995, vol. 4, 2411-2423). $\varepsilon_{A,370}$ is generally zero. $\varepsilon_{D,280}$ and $\varepsilon_{D,370}$ can be obtained according to Lambert-Beer's law (Absorbance = Molar concentration × Molar absorption coefficient × Cell path length) by measuring the absorbance of a solution in which the conjugate precursor used is dissolved at a certain molar concentration. $C_A$ and $C_D$ can be determined by measuring $A_{280}$ and $A_{370}$ of an aqueous solution of the antibody-drug conjugate, and then solving the simultaneous equations (1) and (2) by substitution of these values. Further, by dividing $C_D$ by $C_A$, the average number of conjugated drug molecules per antibody can be determined.

(4)-6 Common procedure F: Measurement of average number of conjugated drug molecules per antibody molecule in antibody-drug conjugate - (2)

The average number of conjugated drug molecules per antibody molecule in the antibody-drug conjugate can also be determined by high-performance liquid chromatography (HPLC) analysis using the following method, in addition to the aforementioned "(4)-5 Common procedure E". Hereinafter, the method for measuring the average number of conjugated drug molecules by HPLC when the antibody is conjugated to the drug linker by a disulfide bond will be described. A person skilled in the art is capable of appropriately measuring the average number of conjugated drug molecules by HPLC, depending on the connecting manner between the antibody and the drug linker, with reference to this method.

F-1. Preparation of sample for HPLC analysis

(Reduction of antibody-drug conjugate)

An antibody-drug conjugate solution (approximately 1 mg/mL, 60 μL) is mixed with an aqueous solution of dithiothreitol (DTT) (100 mM, 15 μL). By incubating the mixture at 37°C for 30 minutes, the disulfide bond between the light chain and heavy chain of the antibody-drug conjugate is cleaved. The resulting sample is used in HPLC analysis.

F-2. HPLC analysis

The HPLC analysis is carried out under the following measurement conditions.

HPLC system: Agilent 1290 HPLC system (Agilent Technologies, Inc.)
Detector: Ultraviolet absorption spectrometer (measurement wavelength: 280 nm)
Column: ACQUITY UPLC BEH Phenyl (2.1 × 50 mm, 1.7 μm, 130 angstroms; Waters Corp., P/N 186002884)
Column temperature: 80°C
Mobile phase A: Aqueous solution containing 0.10% trifluoroacetic acid (TFA) and 15% 2-propanol
Mobile phase B: Acetonitrile solution containing 0.075% TFA and 15% 2-propanol
Gradient program: 14%-36% (0 min-15 min), 36%-80% (15 min-17 min), 80%-14% (17 min-17.01 min), and 14% (17.01 min-25 min)
Sample injection: 10 μL

F-3. Data analysis

F-3-1. Compared with non-conjugated antibody light (L0) and heavy (H0) chains, a light chain bound to drug molecule(s) (light chain bound to i drug molecule(s): $L_i$) and a heavy chain bound to drug molecule(s) (heavy chain bound to i drug molecule(s): $H_i$) exhibit higher hydrophobicity in proportion to the number of conjugated drug molecules and thus have a larger retention time. These chains are therefore eluted in the order of, for example, L0 and L1 or H0, H1, H2, and H3. Detection peaks can be assigned to any of L0, L1, H0, H1, H2, and H3 by the comparison of retention times with L0 and H0. The number of conjugated drug molecules can be defined by a person skilled in the art, but is preferably L0, L1, H0, H1, H2, and H3.

F-3-2. Since the drug linker has UV absorption, peak area values are corrected in response to the number of conjugated drug linker molecules according to the following expression using the molar absorption coefficients of the light chain or heavy chain and the drug linker.

[Expression 2]

$$\text{Corrected value of peak area of light chain bound to i drug molecule(s)}(A_{Li}) =$$

$$\text{Peak area} \times \frac{\text{Molar absorption coefficient of light chain}}{\text{Molar absorption coefficient of light chain} + \text{The number of conjugated drug molecules (i)} \times \text{Molar absorption coefficient of drug linker}}$$

[Expression 3]

$$\text{Corrected value of peak area of heavy chain bound to i drug molecule(s)}(A_{Hi}) =$$

$$\text{Peak area} \times \frac{\text{Molar absorption coefficient of heavy chain}}{\text{Molar absorption coefficient of heavy chain} + \text{The number of conjugated drug molecules (i)} \times \text{Molar absorption coefficient of drug linker}}$$

In this context, a value estimated from the amino acid sequence of the light chain or heavy chain of each antibody by a known calculation method (Protein Science, 1995, vol. 4, 2411-2423) can be used as the molar absorption coefficient (280 nm) of the light chain or heavy chain of the antibody. In the case of H01L02, a molar absorption coefficient of 31710 and a molar absorption coefficient of 79990 were used as estimated values for the light chain and heavy chain, respectively, according to the amino acid sequence of the antibody. The actually measured molar absorption coefficient (280 nm) of a compound in which the maleimide group has been converted to succinimide thioether by the reaction of each drug linker with mercaptoethanol or N-acetylcysteine was used as the molar absorption coefficient (280 nm) of the drug linker. The wavelength for absorbance measurement can be appropriately set by a person skilled in the art, but is preferably a wavelength at which the peak of the antibody can be measured, and more preferably 280 nm.

F-3-3. The peak area ratio (%) of each chain is calculated for the total of the corrected values of peak areas according to the following expressions.

[Expression 4]

$$\text{Peak area ratio of light chain bound to i drug molecule(s)} = \frac{A_{Li}}{A_{L0} + A_{L1}} \times 100$$

[Expression 5]

$$\text{Peak area ratio of heavy chain bound to i drug molecule(s)} = \frac{A_{Hi}}{A_{H0} + A_{H1} + A_{H2} + A_{H3}} \times 100$$

$A_{Li}$, $A_{Hi}$: Corrected values of peak areas of Li and Hi, respectively

F-3-4. The average number of conjugated drug molecules per antibody molecule in the antibody-drug conjugate is calculated according to the following expression.

Average number of conjugated drug molecules = ($L_0$ peak area ratio x 0 + $L_1$ peak area ratio x 1 + $H_0$ peak area ratio x 0 + $H_1$ peak area ratio x 1 + $H_2$ peak area ratio x 2 + $H_3$ peak area ratio x 3) / 100 x 2

It is to be noted that, in order to secure the amount of the antibody-drug conjugate, a plurality of antibody-drug conjugates having almost the same average number of conjugated drug molecules (e.g., on the order of ±1), which have

been produced under similar conditions, can be mixed to prepare a new lot. In this case, the average number of conjugated drug molecules of the new lot falls between the average numbers of conjugated drug molecules before the mixing.

One specific example of the antibody-drug conjugate of the present invention can include an antibody-drug conjugate having a structure represented by the following formula:

[Formula 21]

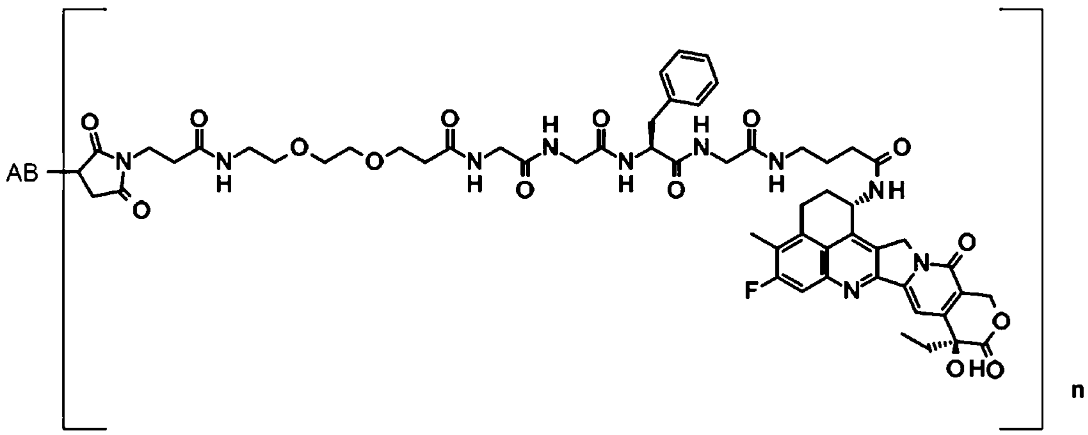

or the following formula:

[Formula 22]

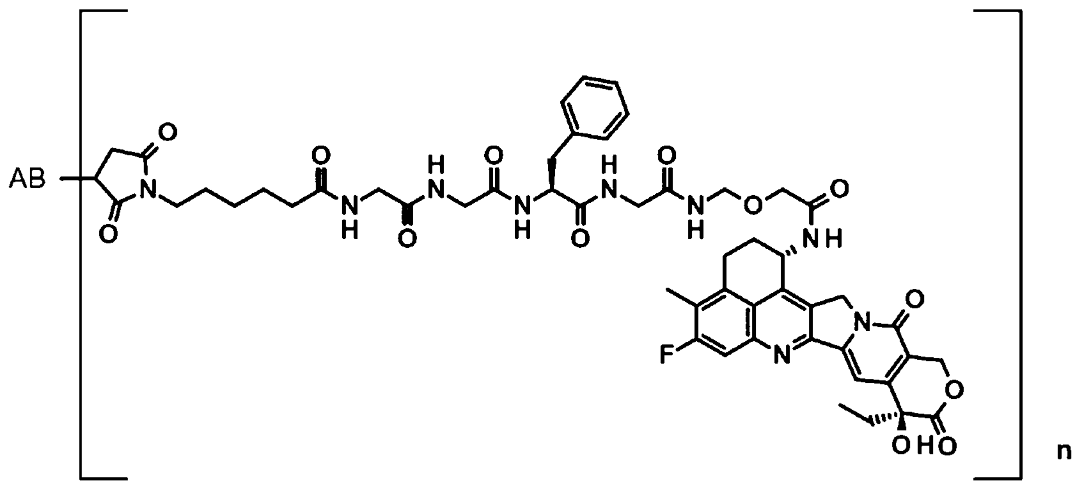

In this context, AB represents the anti-CD37 antibody disclosed in the present description, and the antibody is conjugated to the drug linker via a sulfhydryl group stemming from the antibody. In this context, n has the same meaning as that of the so-called DAR (drug-to-antibody Ratio), and represents a drug-to-antibody ratio per antibody. Specifically, n represents the number of conjugated drug molecules per antibody molecule, which is a numeric value defined and indicated as an average value, i.e., the average number of conjugated drug molecules. In the case of the antibody-drug conjugate represented by Formula 21 or Formula 22 of the present invention, n can be 2 to 8 and is preferably 5 to 8, more preferably 7 to 8, and still more preferably 8 or about 8, in measurement by common procedure F.

In other embodiments, the number of drug molecules or units of the drug linker conjugated per antibody molecule in the anti-CD37 antibody-drug conjugate used in the present invention is preferably an integer within the range of from 2 to 8, more preferably 2, 4, 6 or 8, and still more preferably 8.

Examples of the antibody-drug conjugate of the present invention can include an antibody-drug conjugate having a structure represented by the above-described formula Formula 21 or Formula 22 wherein the antibody represented by AB comprises any one antibody consisting of heavy chain and light chain selected from the group consisting of the following antibodies (a) to (e), or an antigen binding fragment of the antibody, or a pharmacologically acceptable salt of the antibody-drug conjugate:

(a) an antibody consisting of a light chain consisting of the amino acid sequence at positions 21 to 234 in the light chain full-length amino acid sequence shown in SEQ ID NO: 2 and a heavy chain consisting of the amino acid sequence at positions 20 to 468 in the heavy chain full-length amino acid sequence shown in SEQ ID NO: 4;
(b) an antibody consisting of a light chain consisting of the amino acid sequence at positions 21 to 234 in the light chain full-length amino acid sequence shown in SEQ ID NO: 2 and a heavy chain consisting of the amino acid sequence at positions 20 to 468 in the heavy chain full-length amino acid sequence shown in SEQ ID NO: 6;
(c) an antibody consisting of a light chain consisting of the amino acid sequence at positions 21 to 234 in the light chain full-length amino acid sequence shown in SEQ ID NO: 2 and a heavy chain consisting of the amino acid sequence at positions 20 to 468 in the heavy chain full-length amino acid sequence shown in SEQ ID NO: 8;
(d) an antibody consisting of a light chain consisting of the amino acid sequence at positions 21 to 234 in the light chain full-length amino acid sequence shown in SEQ ID NO: 2 and a heavy chain consisting of the amino acid sequence at positions 20 to 468 in the heavy chain full-length amino acid sequence shown in SEQ ID NO: 10; and
(e) any one antibody selected from the group consisting of the antibodies (a) to (d), wherein the heavy chain or the light chain comprises one or more modifications selected from the group consisting of post-translational modifications typified by N-linked glycosylation, O-linked glycosylation, amino-terminal processing, carboxyl-terminal processing, deamidation, isomerization of aspartic acid, oxidation of methionine, oxidation of tryptophan, addition of a methionine residue to the amino terminus, amidation of a proline residue, and conversion of amino-terminal glutamine or amino-terminal glutamic acid to pyroglutamic acid, and a deletion of one or two amino acids from the carboxyl terminus.

The anti-CD37 antibody-drug conjugate used in the present invention is known in the art. Such an anti-CD37 antibody-drug conjugate is not particularly limited as long as the anti-CD37 antibody is conjugated to an antitumor compound or a radionuclide via a linker structure moiety. Examples thereof include naratuximab emtansine (IMGN529), AGS67E and $^{177}$Lu-satetraxetan-lilotomab (Betalutin). The anti-CD37 antibody-drug conjugate is also described in International Publication No. WO2011/112978, U.S. Patent No. 8765917, International Publication No. WO2015/017552, U.S. Patent No. 9925273, International Publication No. WO2013/088363 and U.S. Patent Application Publication No. 2014/0348745, all of which are incorporated herein by reference.

5. Molecular targeting drug

In the present invention, the term "molecular targeting drug" refers to a drug that acts on a specific molecule in cancer cells and exerts an anticancer effect. The molecular targeting drug is classified depending on the mechanism of action thereof. In the present invention, examples of the molecular targeting drug that can be used as the other drug include antibodies, BCL-2 inhibitors, and BTK inhibitors.

In the present invention, examples of the antibody that may be used as the other drug include, but are not particularly limited to, polyclonal antibodies, monoclonal antibodies, and antigen-binding fragments of the antibodies. An anti-CD20 antibody or an anti-CD19 antibody, which is a monoclonal antibody, is preferred.

In the present invention, the term "anti-CD20 antibody", "CD20 antibody" or "antibody that binds to CD20" refers to an antibody having the ability to bind to CD20 with sufficient affinity. The anti-CD20 antibody according to the present invention is not particularly limited as long as it is a drug having the above-described properties. Examples thereof include rituximab, ofatumumab, ocrelizumab, veltuzumab, obinutuzumab, AME-133v (LY 2469298), ocaratuzumab, PRO131921, tositumomab, ibritumomab tiuxetan BLX-301, and PRO70769 (described in WO2004/056312). Rituximab or obinutuzumab is preferred. A drug that is a prodrug of each above-described drug and is converted to the above-described drug *in vivo* is also included in the anti-CD20 antibody of the present invention.

When the anti-CD37 antibody-drug conjugate of the present invention is administered in combination with the anti-CD20 antibody, the property of being internalized into tumor cells by cellular uptake (i.e., an internalization rate) of the anti-CD37 antibody-drug conjugate is significantly increased. The mechanism of action of enhancing drug efficacy by the combination is presumably based on an increase in the internalization rate of the anti-CD37 antibody-drug conjugate into tumor cells. The increase or decrease in the internalization rate can be confirmed by, for example, an experiment described in the subsequent paragraph. In Examples of the present invention, it was confirmed by an experiment described in Example 4 that the internalization rate of the anti-CD37 antibody-drug conjugate into tumor cells is significantly increased by combination with rituximab. Likewise, the internalization rate of the anti-CD37 antibody-drug conjugate into tumor cells is increased by combination with obinutuzumab, and the anti-CD37 antibody-drug conjugate and obinutuzumab administered in combination enhance drug efficacy.

The fluorescently labeled anti-CD37 antibody-drug conjugate of the present invention and the anti-CD20 antibody or an antibody that causes no change in the internalization rate (e.g., Human IgG1, Myeloma) are added at their respective suitable concentrations to tumor cells. As negative controls, an antibody-drug conjugate prepared from human IgG1 that recognizes an antigen unrelated to CD37 and the anti-CD20 antibody or the antibody that causes no increase in_the internalization rate are added at their respective suitable concentrations to tumor cells. The cells are

cultured at 4°C for 1 hour or at 37°C for 1 to 6 hours and then washed, and a buffer solution or an anti-FITC monoclonal antibody is added thereto. After incubation at 4°C for 30 minutes, a fixative solution is added to the cells, which are then fixed at 4°C. Then, measurement is performed using a flow cytometer. A plurality of wells is used in culture under each condition, and an average value of internalization rates calculated according to the expression given below is compared between the anti-CD20 antibody-supplemented group and the group supplemented with the antibody that causes no change in internalization rate, to thereby confirm an effect by which the internalization rate is increased or decreased by the anti-CD20 antibody. MFI represents mean fluorescence intensity.

Internalization rate (%) = (MFI of the wells treated with the anti-FITC monoclonal antibody after culture at 37°C for 1 to 6 hours - Average value of MFI of the wells treated with the anti-FITC monoclonal antibody after culture at 4°C for 1 hour) / (Average value of MFI of the wells treated with the buffer solution after culture at 37°C for 1 to 6 hours - Average value of MFI of the negative control wells treated with the buffer solution after culture at 4°C for 1 hour) $\times$ 100

In the present invention, the term "anti-CD19 antibody", "CD19 antibody" or "antibody that binds to CD19" refers to an antibody having the ability to bind to CD19 with sufficient affinity. The anti-CD19 antibody according to the present invention is not particularly limited as long as it is a drug having the above-described properties. Examples thereof include tafasitamab and inebilizumab. Tafasitamab is preferred. A drug that is a prodrug of each above-described drug and is converted to the above-described drug *in vivo* is also included in the anti-CD19 antibody of the present invention.

In the present invention, the term "BCL-2 inhibitor" refers to an agent that inhibits an apoptosissuppressing protein B-cell lymphoma-2 (BCL-2). The BCL-2 inhibitor according to the present invention may have an inhibitory effect on a protein other than BCL-2. The BCL-2 inhibitor according to the present invention is not particularly limited as long as it is a drug having the above-described properties. Examples thereof include venetoclax, 4-[4-[[2-(4-chlorophenyl)-5,5-dimethyl-1-cyclohexen-1-yl]methyl]-1-piperazinyl]-N-[[4-[[(1R)-3-(4-morpholinyl)-1-[(phenylthio)methyl]propyl]amino]-3-[(trifluoromethyl)sulfonyl]phenyl]sulfonyl]benzamide (ABT-263, described in WO2009/155386), tetrocarcin A, antimycin, gossypol (BL 193), obatoclax, ethyl-2-amino-6-cyclopentyl-4-(1-cyano-2-ethoxy-2-oxoethyl)-4H-chromone-3-carboxylate (HA14-1), oblimersen, Bak BH3 peptide, (-)-gossypol acetic acid (AT-101), 4-[4-[(4'-chloro[1,1'-biphenyl]-2-yl)methyl]-1-piperazinyl]-N-[[4-[[(1R)-3-(dimethylamino)-1-[(phenylthio)methyl]propyl]amino]-3-nitrophenyl]sulfonyl]-benzamide (ABT-737, CAS852808-04-9), navitoclax, and pharmacologically acceptable salts of these drugs. Venetoclax or a pharmacologically acceptable salt thereof is preferred. A drug that is a prodrug of each above-described drug and is converted to the above-described drug *in vivo* is also included in the BCL-2 inhibitor of the present invention.

In the present invention, the term "BTK inhibitor" refers to an agent that inhibits Bruton's tyrosine kinase (BTK), an enzyme called protein kinase. The BTK inhibitor according to the present invention may have an inhibitory effect on a kinase other than BTK. The BTK inhibitor according to the present invention is not particularly limited as long as it is a drug having the above-described properties. Examples thereof include spebrutinib (AVL-292, CC-292), trabrutinib (ONO-4059), ibrutinib, acalabrutinib, zanubrutinib, and pharmacologically acceptable salts of these drugs. Ibrutinib, acalabrutinib, zanubrutinib or a pharmacologically acceptable salt thereof is preferred. A drug that is a prodrug of each above-described drug and is converted to the above-described drug *in vivo* is also included in the BTK inhibitor of the present invention.

6. Drug relating to R-CHOP therapy

In the present invention, the term "drug relating to R-CHOP therapy" refers to each of five drugs for use in R-CHOP therapy and specifically refers to each of rituximab, cyclophosphamide, doxorubicin, vincristine and prednisolone (or prednisone). In the present invention, the term "R-CHOP therapy" is a cancer chemotherapy and is a method of treatment in which the five drugs rituximab, cyclophosphamide, doxorubicin, vincristine and prednisolone (or prednisone) are administered in combination. In a preferred aspect of the present invention, the other drug is a drug relating to R-CHOP therapy.

In the present invention, obinutuzumab may be used in place of rituximab as the "drug relating to R-CHOP therapy" from the viewpoint of antitumor activity, safety, toxicity, and the like. Specifically, five drugs obinutuzumab, cyclophosphamide, doxorubicin, vincristine and prednisolone (or prednisone) may be used as the other drug. These five drugs are referred to as "drugs relating to G-CHOP therapy". In the present invention, the term "G-CHOP therapy" is a cancer chemotherapy and is a method of treatment in which the five drugs obinutuzumab, cyclophosphamide, doxorubicin, vincristine and prednisolone (or prednisone) are administered in combination.

7. Drug relating to R-CHP therapy

In the present invention, the term "drug relating to R-CHP therapy" refers to each of four drugs for use in R-CHP

therapy and specifically refers to each of rituximab, cyclophosphamide, doxorubicin and prednisolone (or prednisone). In the present invention, the term "R-CHP therapy" is a cancer chemotherapy and is a method of treatment in which the four drugs rituximab, cyclophosphamide, doxorubicin and prednisolone are administered in combination. In a preferred aspect of the present invention, the other drug is a drug relating to R-CHP therapy.

In the present invention, obinutuzumab may be used in place of rituximab as the "drug relating to R-CHP therapy" from the viewpoint of antitumor activity, safety, toxicity, and the like. Specifically, four drugs obinutuzumab, cyclophosphamide, doxorubicin and prednisolone (or prednisone) may be used as the other drug. These four drugs are referred to as "drugs relating to G-CHP therapy". In the present invention, the term "G-CHP therapy" is a cancer chemotherapy and is a method of treatment in which the four drugs obinutuzumab, cyclophosphamide, doxorubicin and prednisolone (or prednisone) are administered in combination.

8. Alkylating agent

In the present invention, the term "alkylating agent" refers to a drug that exerts an anticancer effect by alkylating DNA of cancer cells and interfering with the growth of the cells. The alkylating agent is not particularly limited as long as it is a drug having the above-described properties. Examples thereof include bendamustine, cyclophosphamide (CPA), ifosfamide (IFO), dacarbazine (DTIC, DIC), temozolomide (TMZ), nimustine (ACNU), busulfan (BSF, BUS), procarbazine, melphalan (L-PAM), ranimustine (MCNU), and pharmacologically acceptable salts of these drugs. Bendamustine or a pharmacologically acceptable salt thereof is preferred. A drug that is a prodrug of each above-described drug and is converted to the above-described drug *in vivo* is also included in the alkylating agent of the present invention.

9. Immunomodulatory drug

In the present invention, the term "immunomodulatory drug" refers to a drug that exerts an anticancer effect by modulating immune functions. The immunomodulatory drug is not particularly limited as long as it is a drug having the above-described properties. Examples thereof include thalidomide, lenalidomide (LEN), and pharmacologically acceptable salts of these drugs. Lenalidomide or a pharmacologically acceptable salt thereof is preferred. A drug that is a prodrug of each above-described drug and is converted to the above-described drug *in vivo* is also included in the immunomodulatory drug of the present invention.

10. Medicament

Hereinafter, the pharmaceutical composition and the treatment method, and the like, comprising administering an anti-CD37 antibody-drug conjugate and another drug in combination according to the present invention will be described.

The pharmaceutical composition of the present invention may be administered as a pharmaceutical composition comprising a pharmaceutically acceptable carrier, diluent, solubilizer, emulsifier, preservative, adjuvant, and the like. The "pharmaceutically acceptable carrier" and the like can be appropriately selected from a wide range depending on the type of a target disease or the dosage form of the drug. Examples of the "pharmaceutically acceptable carrier" and the like include sterilized liquids. In this context, the liquid includes, for example, water and oils (petroleum and oils of animal origin, plant origin, or synthetic origin). The oil can be, for example, peanut oil, soybean oil, mineral oil, or sesame oil. Water is more typical when the pharmaceutical composition is intravenously administered. An aqueous saline solution, an aqueous dextrose solution, and an aqueous glycerol solution can also be used as the liquid, in particular, for an injection solution. Suitable pharmaceutical vehicles can be appropriately selected from those known in the art. Examples of suitable "pharmaceutically acceptable carriers" and the like are disclosed in "Remington's Pharmaceutical Sciences" by E. W. Martin. The prescription corresponds to an administration mode.

A method for administering the pharmaceutical composition of the present invention can be appropriately selected. For example, administration by injection can be performed, and local injection, intraperitoneal injection, selective intravenous injection, intravenous injection, subcutaneous injection, organ perfusate injection, or the like can be adopted. Administration can be made by injection or bolus injection, for example. A solution for injection can be formulated using a carrier formed from a salt solution, a glucose solution, or a mixture of salt water and a glucose solution, various buffer solutions, and the like. Alternatively, the solution for injection may be prepared by mixing a formulation in a powder state with the liquid carrier upon use. According to a specific preferred embodiment, the administration of the anti-CD37 antibody-drug conjugate and the other drug used in the present invention is performed by injection. Parenteral administration is a preferred administration route.

Other administration methods can also be appropriately selected along with the development of a formulation. In the case of oral administration, for example, an oral solution, a powder, a pill, a capsule, and a tablet are applicable. The oral solution can be produced as an oral liquid preparation such as a suspension and a syrup using, for example: water;

sugars such as sucrose, sorbitol, and fructose; glycols such as polyethylene glycol; oils such as sesame oil and soybean oil; antiseptics such as alkyl p-hydroxybenzoate; and flavors such as a strawberry flavor and peppermint. The powder, the pill, the capsule, and the tablet can be formulated using, for example: an excipient such as lactose, glucose, sucrose, or mannitol; a disintegrant such as starch or sodium alginate; a lubricant such as magnesium stearate or talc; a binder such as polyvinyl alcohol, hydroxypropylcellulose, or gelatin; a surfactant such as fatty acid ester; and/or a plasticizer such as glycerin. The tablet and the capsule are preferred unit dosage forms because of the ease of administration. A solid pharmaceutical carrier is used in producing the tablet or the capsule.

A feature of the pharmaceutical composition and the method of treatment of the present invention may be that the anti-CD37 antibody-drug conjugate and the other drug are separately contained as active ingredients in different formulations and administered at the same time or different times, or may be that the anti-CD37 antibody-drug conjugate and the other drug are contained as an active ingredient in a single formulation for administration.

In the pharmaceutical composition and the method of treatment of the present invention, two or more types of other drugs used in the present invention may be administered in combination.

In the present invention, the other drug that is administered in combination with the anti-CD37 antibody-drug conjugate is not particularly limited and is, for example, another drug or a combination of two or more other drugs selected from the group consisting of a molecular targeting drug, a drug relating to R-CHOP therapy, a drug relating to R-CHP therapy, an alkylating agent and an immunomodulatory drug, preferably another drug or a combination of two or more other drugs selected from the group consisting of an anti-CD20 antibody, an anti-CD19 antibody, a BCL-2 inhibitor, a BTK inhibitor, a drug relating to R-CHOP therapy, a drug relating to R-CHP therapy, an alkylating agent and an immunomodulatory drug, more preferably another drug or a combination of two or more other drugs selected from the group consisting of rituximab, obinutuzumab, tafasitamab, venetoclax, ibrutinib, acalabrutinib, zanubrutinib, a drug relating to R-CHOP therapy, a drug relating to R-CHP therapy, bendamustine and lenalidomide, and still more preferably a combination with rituximab, a combination with rituximab and bendamustine or a pharmacologically acceptable salt thereof, a combination with tafasitamab, a combination with tafasitamab and lenalidomide or a pharmacologically acceptable salt thereof, a combination with rituximab and lenalidomide or a pharmacologically acceptable salt thereof, a combination with venetoclax or a pharmacologically acceptable salt thereof, a combination with rituximab and venetoclax or a pharmacologically acceptable salt thereof, a combination with ibrutinib or a pharmacologically acceptable salt thereof, a combination with acalabrutinib or a pharmacologically acceptable salt thereof, a combination with zanubrutinib or a pharmacologically acceptable salt thereof, a combination with obinutuzumab, a combination with obinutuzumab and bendamustine or a pharmacologically acceptable salt thereof, a combination with obinutuzumab and lenalidomide or a pharmacologically acceptable salt thereof, a combination with obinutuzumab and venetoclax or a pharmacologically acceptable salt thereof, a combination with a drug relating to R-CHOP therapy, a combination with a drug relating to R-CHP therapy, a combination with a drug relating to G-CHOP therapy, or a combination with a drug relating to G-CHP therapy.

The pharmaceutical composition and the method of treatment of the present invention can be used for the treatment of cancer, can be preferably used for the treatment of non-Hodgkin's lymphoma (NHL), for example, B-cell non-Hodgkin's lymphoma (B-NHL) or T-cell lymphoma (TCL), can be more preferably used for the treatment of at least one cancer selected from the group consisting of diffuse large B-cell lymphoma (DLBCL), follicular lymphoma (FL), mantle cell lymphoma (MCL), marginal zone lymphoma (MZL), Burkitt's lymphoma (BL), and chronic lymphocytic leukemia (CLL) classified into B-NHL, and peripheral T-cell lymphoma (PTCL) and cutaneous T-cell lymphoma (CTCL) classified into TCL, and can be still more preferably used for the treatment of at least one cancer selected from the group consisting of diffuse large B-cell lymphoma, chronic lymphocytic leukemia, and follicular lymphoma.

The pharmaceutical composition and the method of treatment of the present invention can be used for the treatment of the above-described cancer and in addition, can be used for the treatment of myelodysplastic syndrome (MDS) and acute myeloid leukemia (AML).

The presence or absence of a tumor marker of CD37 can be confirmed, for example, by collecting tumor tissues from a cancer patient, preparing formalin-fixed paraffin-embedded (FFPE) samples, and conducting a test at the gene product (protein) level by immunohistochemistry (IHC), a flow cytometer, Western blot, or the like, or a test at the gene transcription level by *in situ* hybridization (ISH), quantitative PCR (q-PCR), microarray analysis, or the like, or can also be confirmed by collecting cell-free blood circulating tumor DNA (ctDNA) from a cancer patient, and conducting a test using a method such as a next-generation sequencer (NGS).

The pharmaceutical composition and the method of treatment of the present invention can be preferably used for a mammal, and can be more preferably used for a human.

The antitumor effect of the pharmaceutical composition and the method of treatment of the present invention can be confirmed, for example, by inoculating cancer cells into a test animal to prepare a model, applying thereto the pharmaceutical composition and the method of treatment of the present invention, and measuring a decrease in tumor volume or a life-extending effect, and can be determined from an indicator such as estimated tumor volume, tumor growth inhibition (TGI), change in tumor volume from a baseline, or complete response (CR). Then, the combinatorial effect of the anti-CD37 antibody-drug conjugate and the other drug used in the present invention can be confirmed by comparing the

above-described antitumor effect with the respective antitumor effects of single administrations of the anti-CD37 antibody-drug conjugate and the other drug used in the present invention.

The toxicity of the pharmaceutical composition and the method of treatment of the present invention can be confirmed, for example, from weight loss of the model between before and after the pharmaceutical composition and the method of treatment of the present invention are applied thereto. As for the weight loss, the model is preferably free of 50% or more weight loss, more preferably free of 40% or more weight loss, still more preferably free of 30% or more weight loss, and particularly preferably free of 20% or more weight loss. The toxicity of the combination of the anti-CD37 antibody-drug conjugate and the other drug used in the present invention can be confirmed by comparing the above-described antitumor effect with the respective toxicities of single administrations of the anti-CD37 antibody-drug conjugate and the other drug used in the present invention.

The pharmaceutical composition and the method of treatment of the present invention preferably have a combinatorial effect of the anti-CD37 antibody-drug conjugate and the other drug used in the present invention, without increasing toxicity.

Also, the antitumor effect of the pharmaceutical composition and the method of treatment of the present invention can be confirmed by evaluation of response evaluation criteria in solid tumors (RECIST), WHO evaluation, Macdonald evaluation, body weight measurement, and other approaches in clinical trials, and can be determined from an indicator such as complete response (CR), partial response (PR), progressive disease (PD), objective response rate (ORR), duration of response (DoR), progression-free survival (PFS), or an overall survival (OS).

By the above-described methods, the pharmaceutical composition and the method of treatment of the present invention can be confirmed to be superior in antitumor effect to existing pharmaceutical compositions for cancer treatments and treatment methods.

The pharmaceutical composition and the method of treatment of the present invention can delay development of cancer cells, inhibit growth thereof, and further destroy cancer cells. These effects can allow cancer patients to be free from symptoms caused by cancer or achieve improvement in QOL of cancer patients and attain a therapeutic effect by sustaining the lives of the cancer patients. Even if the pharmaceutical composition or the method of treatment does not accomplish destruction of cancer cells, it can achieve higher QOL of cancer patients while achieving longer-term survival, by suppressing or controlling the growth of cancer cells.

The pharmaceutical composition of the present invention may be administered as a composition comprising one or more pharmaceutically compatible components. The pharmaceutically compatible components can be appropriately selected, for use, from pharmaceutical additives and the like usually used in the art, depending on the doses, administration concentrations, and the like of the anti-CD37 antibody-drug conjugate and the other drug used in the present invention. For example, the anti-CD37 antibody-drug conjugate used in the present invention may be administered as a pharmaceutical composition containing a buffer such as a histidine buffer, an excipient such as sucrose or trehalose, and a surfactant such as polysorbate 80 or 20. The pharmaceutical composition comprising the anti-CD37 antibody-drug conjugate used in the present invention can be preferably used as an injection, can be more preferably used as an aqueous injection or a freeze-dried injection, and can be still more preferably used as a freeze-dried injection.

In the case of an aqueous injection, the pharmaceutical composition comprising the anti-CD37 antibody-drug conjugate used in the present invention can be preferably diluted with a suitable diluent, and then administered intravenously. Examples of the diluent can include glucose solutions and physiological saline, can preferably include glucose solutions, and can more preferably include 5% glucose solutions.

In the case of a freeze-dried injection, the pharmaceutical composition comprising the anti-CD37 antibody-drug conjugate used in the present invention can be preferably dissolved in injectable water, then diluted in a necessary amount with a suitable diluent, and then administered intravenously. Examples of the diluent can include glucose solutions and physiological saline, can preferably include glucose solutions, and can more preferably include 5% glucose solutions.

Examples of the administration route that may be used for administering the pharmaceutical composition of the present invention can include intravenous, intradermal, subcutaneous, intramuscular, and intraperitoneal routes, and can preferably include intravenous routes.

The anti-CD37 antibody-drug conjugate used in the present invention can be administered to a human at intervals of 1 to 180 days, can be preferably administered at intervals of 1 week, 2 weeks, 3 weeks, or 4 weeks, can be more preferably administered at intervals of 3 weeks. The anti-CD37 antibody-drug conjugate used in the present invention can be administered at a dose of approximately 0.001 to 100 mg/kg per administration, and can be administered at preferably 0.8 to 12.4 mg/kg per administration.

When the molecular targeting drug used in the present invention is rituximab, it can be preferably administered intravenously at a dose of 125 $mg/m^2$, 250 $mg/m^2$, 375 $mg/m^2$, or 500 $mg/m^2$ per administration at intervals of 1 week.

When the molecular targeting drug used in the present invention is obinutuzumab, it can be preferably administered intravenously at a dose of 200 mg, 400 mg, 800 mg, 1000 mg, 1200 mg or 2000 mg per administration at intervals of 1 to 2 weeks.

When the molecular targeting drug used in the present invention is tafasitamab, it can be preferably administered

intravenously at a dose of 3 mg/kg, 6 mg/kg, 12 mg/kg or 24 mg/kg per administration at intervals of 1 week.

When the molecular targeting drug used in the present invention is venetoclax or a pharmacologically acceptable salt thereof, it can be preferably administered by an oral route at a dose of 20 mg, 50 mg, 100 mg, 200 mg, 400 mg or 600 mg per administration at intervals of once daily.

When the molecular targeting drug used in the present invention is ibrutinib or a pharmacologically acceptable salt thereof, it can be preferably administered by an oral route at a dose of 140 mg, 280 mg, 420 mg, 560 mg or 700 mg per administration at intervals of once daily.

When the molecular targeting drug used in the present invention is acalabrutinib or a pharmacologically acceptable salt thereof, it can be preferably administered by an oral route at a dose of 50 mg, 100 mg, 150 mg or 200 mg per administration at intervals of twice daily.

When the molecular targeting drug used in the present invention is zanubrutinib or a pharmacologically acceptable salt thereof, it can be preferably administered by an oral route at a dose of 40 mg, 80 mg, 160 mg, 240 mg, 320 mg, 400 mg or 480 mg per administration at intervals of once or twice daily.

When the alkylating agent used in the present invention is bendamustine or a pharmacologically acceptable salt thereof, it can be preferably administered intravenously at a dose of 50 $mg/m^2$, 60 $mg/m^2$, 75 $mg/m^2$, 90 $mg/m^2$, 100 $mg/m^2$, 120 $mg/m^2$ or 125 $mg/m^2$ per administration at intervals of once daily.

When the immunomodulatory drug used in the present invention is lenalidomide or a pharmacologically acceptable salt thereof, it can be preferably administered by an oral route at a dose of 10 mg, 15 mg, 20 mg or 25 mg per administration at intervals of once daily.

The pharmaceutical composition and the method of treatment of the present invention may further comprise a therapeutic agent for a cancer other than the antibody-drug conjugate and the other drug according to the present invention. The pharmaceutical composition and the method of treatment of the present invention may attain administration in combination with an additional therapeutic agent for a cancer, and can thereby enhance an antitumor effect. The additional therapeutic agent for a cancer used for such a purpose may be administered to an individual, simultaneously, separately, or continuously with the pharmaceutical composition of the present invention. Alternatively, the additional therapeutic agent and the pharmaceutical composition may be administered at different administration intervals. Such a therapeutic agent for a cancer is not limited as long as it is a drug having antitumor activity. Examples thereof can include at least one agent selected from the group consisting of irinotecan (CPT-11), cisplatin, carboplatin, oxaliplatin, fluorouracil (5-FU), gemcitabine, capecitabine, paclitaxel, docetaxel, doxorubicin, epirubicin, cyclophosphamide, mitomycin C, tegafur/gimeracil/oteracil, cetuximab, panitumumab, bevacizumab, ramucirumab, regorafenib, trifluridine/tipiracil, gefitinib, erlotinib, afatinib, methotrexate, pemetrexed, tamoxifen, toremifene, fulvestrant, leuprorelin, goserelin, letrozole, anastrozole, progesterone formulations, trastuzumab, pertuzumab, and lapatinib.

The pharmaceutical composition and the method of treatment of the present invention may be used in combination with radiotherapy. For example, a cancer patient receives radiotherapy before and/or after or simultaneously with treatment with the pharmaceutical composition of the present invention.

The pharmaceutical composition and the method of treatment of the present invention may be used as adjunctive chemotherapy in combination with surgery. The pharmaceutical composition of the present invention may be administered for the purpose of decreasing a tumor size before surgery (neoadjuvant chemotherapy), or may be administered for the purpose of preventing tumor recurrence after surgery (adjuvant chemotherapy).

The pharmaceutical composition and the method of treatment of the present invention may be used as maintenance therapy. For example, after initial chemotherapy, treatment is continued for the purpose of preventing recurrence.

The present invention includes a method of treatment comprising administering the anti-CD37 antibody-drug conjugate of the present application and the other drug of the present application in combination to an individual in need of treatment. The present invention includes the anti-CD37 antibody-drug conjugate of the present application for the treatment of a disease by administration in combination with the other drug of the present application. The present invention includes use of the anti-CD37 antibody-drug conjugate of the present application for the production of a medicament for the treatment of a disease by administration in combination with the other drug of the present application. The present invention includes a pharmaceutical product comprising the anti-CD37 antibody-drug conjugate of the present application and the other drug of the present application for administration in combination. The present invention includes a combination medicament comprising the anti-CD37 antibody-drug conjugate of the present application and the other drug of the present application. The present invention includes a pharmaceutical combination comprising the anti-CD37 antibody-drug conjugate of the present application and the other drug of the present application. The present invention includes use of the anti-CD37 antibody-drug conjugate of the present application for the treatment of a disease, in combination with the other drug of the present application. The present invention includes a medicament comprising the anti-CD37 antibody-drug conjugate of the present application and the other drug of the present application.

In the present description, a term described in a singular form encompasses its plural form, and vice versa, without violating the context, without making description in a particularly limited manner, and without causing technical

contradiction.

Examples

Hereinafter, the present invention will be specifically described with reference to examples given below. However, the present invention is not limited to these examples. Furthermore, these examples should not be construed in a limited manner by any means.

[Example 1: Production of humanized anti-CD37 antibody]

1)-1 Design of anti-CD37 humanized antibody

1)-1-1 Molecular modeling of variable region of anti-CD37 antibody.

A known method (Methods in Enzymology, 203, 121-153, (1991)) was exploited as homology modeling. The commercially available protein three-dimensional structure analysis program DiscoveryStudio (manufactured by Dassault Systèmes S.E.) was employed to search for structures registered in the Protein Data Bank (Nuc. Acid Res. 35, D301-D303 (2007)) with a high sequence homology to variable regions. Three-dimensional model structures were generated using the identified heavy chain, light chain, and interface structure between the heavy chain and the light chain as templates.

1)-1-2 Design method for humanization

Construction of a humanized antibody of the anti-CD37 mouse monoclonal antibody HH1 (Smeland E, et al., Scand J Immunol, 21 (3), 205-214 (1985)) was performed by a method generally known as CDR grafting (Proc. Natl. Acad. Sci. USA 86, 10029-10033 (1989)). The consensus sequences of human κ chain subgroup 1 and human γ chain subgroup 1 determined by KABAT et al. (Sequences of Proteins of Immunological Interest, 5th Ed. Public Health Service National Institutes of Health, Bethesda, MD. (1991)) had a high homology to the framework regions of an anti-CD37 human chimeric antibody, and based on this, they were selected as acceptors for the light chain and the heavy chain of the anti-CD37 human chimeric antibody, respectively. Also, human γ chain IGHV1-2*02 and IGHJ6*01 determined by IMGT (registered trademark) (THE INTERNATIONAL IMMUNOGENETICS INFORMATION SYSTEM) were selected as acceptors for the heavy chain for the purpose of improving the physicochemical properties of an anti-CD37 humanized antibody-drug conjugate. Donor residues to be grafted onto the acceptors were uniquely designed according to each sequence by analyzing three-dimensional models with reference to, for example, the criteria given by Queen et al. (Proc. Natl. Acad. Sci. USA 86, 10029-10033 (1989)).

1)-1-3 Humanization of anti-CD37 human chimeric antibody light chain

A humanized antibody light chain comprising a human IgG1 κ chain constant region connected to the variable region of the designed anti-CD37 humanized antibody light chain was designed and named hmAb-L11. The full-length amino acid sequence of hmAb-L11 is shown in SEQ ID NO: 2. A nucleotide sequence encoding the amino acid sequence of SEQ ID NO: 2 is shown in SEQ ID NO: 1.

1)-1-4 Humanization of anti-CD37 human chimeric antibody heavy chain hmAb-H11

A humanized antibody heavy chain comprising a human IgG1 γ chain constant region connected to the variable region of the anti-CD37 humanized antibody heavy chain designed by grafting into the consensus sequence of human γ chain subgroup 1 having the highest homology to the anti-CD37 human chimeric antibody was designed and named hmAb-H11. The full-length amino acid sequence of hmAb-H11 is shown in SEQ ID NO: 4. A nucleotide sequence encoding the amino acid sequence of SEQ ID NO: 4 is shown in SEQ ID NO: 3.

1)-1-5 Humanization of anti-CD37 human chimeric antibody heavy chain hmAb-H541, hmAb-H551, hmAb-H11a

Humanized antibody heavy chains comprising a human IgG1 γ chain constant region connected to the variable regions of the anti-CD37 humanized antibody heavy chains designed for the purpose of improving the physical properties of an anti-CD37 humanized antibody-drug conjugate were named hmAb-H541, hmAb-H551, and hmAb-H11a, respectively. The full-length amino acid sequence of hmAb-H541 is shown in SEQ ID NO: 6. A nucleotide sequence encoding the amino acid sequence of SEQ ID NO: 6 is shown in SEQ ID NO: 5. The full-length amino acid sequence of hmAb-H551 is shown in SEQ ID NO: 8. A nucleotide sequence encoding the amino acid sequence of SEQ ID NO: 8 is shown in SEQ ID

NO: 7. The full-length amino acid sequence of hmAb-H11a is shown in SEQ ID NO: 10. A nucleotide sequence encoding the amino acid sequence of SEQ ID NO: 10 is shown in SEQ ID NO: 9.

1)-2 Construction of anti-CD37 humanized antibody expression vector and preparation of antibody

1)-2-1 Construction of light chain expression vector pCMA-LK

An approx. 5.4-kb fragment, which had been obtained by digesting plasmid pcDNA3.3-TOPO/LacZ (manufactured by Thermo Fisher Scientific Inc.) with the restriction enzymes XbaI and PmeI, was ligated to a DNA fragment comprising a nucleotide sequence (SEQ ID NO: 11) encoding a human light chain signal sequence and a human κ chain constant region, using an In-Fusion HD PCR cloning kit (manufactured by Takra Bio USA), to produce pcDNA3.3/LK. A neomycin resistance gene was removed from pcDNA3.3/LK to construct pCMA-LK.

1)-2-1-1 Construction of hmAb-L11 expression vector

A DNA fragment having the nucleotide sequence of the hmAb-L11 variable region shown in SEQ ID NO: 12 was synthesized (manufactured by Thermo Fisher Scientific Inc.). Using an In-Fusion HD PCR cloning kit, the synthesized DNA fragment was inserted into a site of pCMA-LK constructed in Example 1)-2-1 that had been cleaved with the restriction enzyme BsiWI, so as to construct a hmAb-L11 expression vector.

1)-2-2 Construction of heavy chain expression vector pCMA-G1

A DNA fragment comprising a nucleotide sequence (SEQ ID NO: 13) encoding a heavy chain signal sequence and a human heavy chain G1 constant region was synthesized (manufactured by Eurofins Genomics K.K.). This DNA fragment was cleaved with the restriction enzymes XbaI and PmeI, and a 1.1-kb DNA fragment was then excised by agarose gel electrophoresis and purified using Wizard SV Gel and PCR Clean-Up System (manufactured by Promega Corp.). An approx. 3.4-kb fragment, which had been obtained by digesting pCMA-LK constructed in Example 1)-2-1 with the restriction enzymes XbaI and PmeI, was ligated to the 1.1-kb DNA fragment, using Ligation High (manufactured by Toyobo Co., Ltd.), to construct pCMA-G1.

1)-2-2-1 Construction of hmAb-H11 expression vector

A DNA fragment having the nucleotide sequence of hmAb-H11 shown in SEQ ID NO: 14 was synthesized. Using an In-Fusion HD PCR cloning kit, the synthesized DNA fragment was inserted into a site of pCMA-G1 constructed in Example 1)-2-2 that had been cleaved with the restriction enzyme BlpI, so as to construct a hmAb-H11 expression vector.

1)-2-2-2 Construction of hmAb-H541 expression vector

A DNA fragment having the nucleotide sequence of hmAb-H541 shown in SEQ ID NO: 15 was synthesized. Using an In-Fusion HD PCR cloning kit, the synthesized DNA fragment was inserted into a site of pCMA-G1 constructed in Example 1)-2-2 that had been cleaved with the restriction enzyme BlpI, so as to construct a hmAb-H541 expression vector.

1)-2-2-3 Construction of hmAb-H551 expression vector

A DNA fragment having the nucleotide sequence of hmAb-H551 shown in SEQ ID NO: 16 was synthesized. Using an In-Fusion HD PCR cloning kit, the synthesized DNA fragment was inserted into a site of pCMA-G1 constructed in Example 1)-2-2 that had been cleaved with the restriction enzyme BlpI, so as to construct a hmAb-H551 expression vector.

1)-2-2-4 Construction of hmAb-H11a expression vector

A DNA fragment having the nucleotide sequence of hmAb-H11a shown in SEQ ID NO: 17 was synthesized. Using an In-Fusion HD PCR cloning kit, the synthesized DNA fragment was inserted into a site of pCMA-G1 constructed in Example 1)-2-2 that had been cleaved with the restriction enzyme BlpI, so as to construct a hmAb-H11a expression vector.

1)-2-2-5 Combination of heavy chain expression vector and light chain expression vector of anti-CD37 humanized antibody

An anti-CD37 humanized antibody having hmAb-H11 as a heavy chain and hmAb-L11 as a light chain was

named hmAb-H11L11. An anti-CD37 humanized antibody having hmAb-H541 as a heavy chain and hmAb-L11 as a light chain was named hmAb-H541L11. An anti-CD37 humanized antibody having hmAb-H551 as a heavy chain and hmAb-L11 as a light chain was named hmAb-H551L11. An anti-CD37 humanized antibody having hmAb-H11a as a heavy chain and hmAb-L11 as a light chain was named hmAb-H11aL11.

1)-2-3 Production of anti-CD37 humanized antibody

In accordance with the manual, FreeStyle 293F cells (manufactured by Thermo Fisher Scientific Inc.) were cultured and passaged. FreeStyle 293F cells in the logarithmic growth phase were adjusted to $2.0 \times 10^6$ cells/mL by dilution with FreeStyle 293 expression medium (manufactured by Thermo Fisher Scientific Inc.), and 600 mL of the dilution was seeded on a 3-L Fernbach Erlenmeyer Flask (manufactured by Corning Inc.). To 20 mL of Opti-Pro SFM medium (manufactured by Thermo Fisher Scientific Inc.), 1.8 mg of Polyethyleneimine (manufactured by Polysciences Inc.) was added. Next, to 20 mL of Opti-Pro SFM medium, 300 μg of the heavy chain expression vector and 300 μg of the light chain expression vector were added. To the Polyethyleneimine/Opti-Pro SFM mixed solution, the expression vector/Opti-Pro SFM mixed solution was added, and the obtained mixture was gently stirred, further left standing for 5 minutes, and then added to the FreeStyle 293F cells. The cells were shake-cultured at 95 rpm in an 8% $CO_2$ incubator at 37°C for 4 hours, and thereafter, 600 mL of EX-CELL VPRO medium (manufactured by SAFC Biosciences Inc.), and 30 mL of 43.4 g/L BD Recharge CD (manufactured by BD Biosciences) were added to the culture. The cells were further shake-cultured at 95 rpm in an 8% $CO_2$ incubator at 37°C for 6 days. The obtained culture supernatant was filtered through a bottle top filter having a pore size of 0.2 μm (manufactured by Thermo Fisher Scientific Inc.).

1)-2-4 Purification of anti-CD37 humanized antibody

The antibody was purified from the culture supernatant obtained in Example 1)-2-3, by a two-step process, namely, by rProtein A affinity chromatography and ceramic hydroxyapatite. The culture supernatant was equilibrated with PBS and applied to a column that had been packed with MabSelectSuRe (manufactured by Cytiva Corp.), and thereafter, the column was washed with PBS in an amount of two or more times the volume of the column. Subsequently, the antibody was eluted using a 2 M arginine hydrochloride solution (pH 4.0). A fraction containing the antibody was dialyzed using Slide-A-Lyzer Dialysis Cassette (manufactured by Thermo Fisher Scientific Inc.), so that the buffer was replaced with PBS. The antibody solution was 5-fold diluted with a buffer of 5 mM sodium phosphate/50 mM MES/pH 7.0, and then applied to a ceramic hydroxyapatite column (manufactured by Bio-Rad Laboratories, Inc.) that had been equilibrated with a buffer of 5 mM NaPi/50 mM MES/30 mM NaCl/pH 7.0. Elution was carried out on a linear concentration gradient of sodium chloride, so that a fraction containing an antibody was collected. This fraction was dialyzed using Dialysis Cassettes, so that the buffer was replaced with HBSor (25 mM histidine/5% sorbitol, pH 6.0). The antibody was concentrated with VIVASPIN20 (molecular weight cutoff: UF10K, manufactured by Sartorius Stedim Biotech Inc.), thereby adjusting the IgG concentration to 20 to 25 mg/ml. Finally, the antibody was filtered through a Minisart Plus (manufactured by Sartorius Stedim Biotech Inc.) to obtain a purified sample.

[Example 2: Production of anti-CD37 antibody-drug conjugate]

2)-1 Production of antibody-drug conjugate - (1) hmAb-H11L11-DXd

hmAb-H11L11-DXd was synthesized by the following step.

[Formula 23]

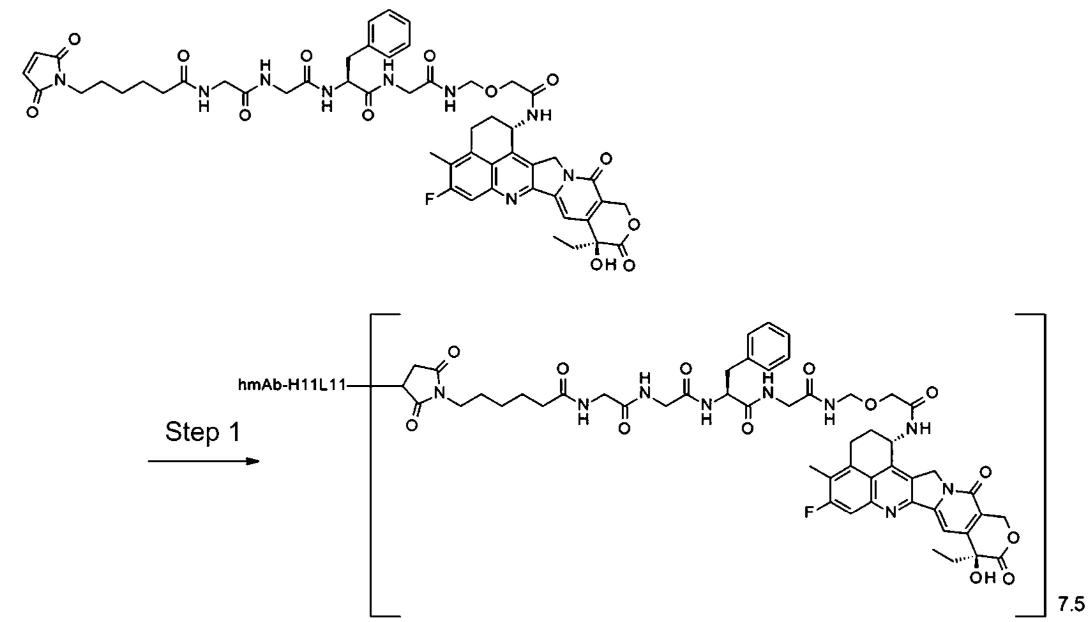


Reduction of antibody: hmAb-H11L11 prepared in Example 1)-2 was adjusted to 10.67 mg/mL with PBS6.0/EDTA by using common procedures B (using 1.50 $mLmg^{-1}cm^{-1}$ as 280 nm absorption coefficient) and C described in production method 1. To this solution (0.5 mL), a 1 M aqueous dipotassium hydrogen phosphate solution (Nacalai Tesque, Inc.; 0.0075 mL) and an aqueous solution of 10 mM TCEP (Tokyo Chemical Industry Co., Ltd.) (0.022 mL; 6.0 equivalents per antibody molecule) were added. After confirming that the solution had a pH within 7.0 $\pm$ 0.1, the interchain disulfide bond in the antibody was reduced by incubating the solution at 37°C for 2 hours.

Conjugation between antibody and drug linker: The above-described solution was incubated at 15°C for 10 minutes. Subsequently, a 10 mM solution of N-[6-(2,5-dioxo-2,5-dihydro-1H-pyrrol-1-yl)hexanoyl]glycylglycyl-L-phenylalanyl-N-[(2-{[(1S,9S)-9-ethyl-5-fluoro-9-hydroxy-4-methyl-10,13-dioxo-2,3,9,10,13,15-hexahydro-1H,12H-benzo[de]pyrano[3',4':6,7]indolizino[1,2-b]quinolin-1-yl]amino}-2-oxoethoxy)methyl]glycinamide in dimethyl sulfoxide (0.0367 mL; 10.0 equivalents per antibody molecule) was added thereto, and the obtained mixture was incubated at 15°C for 1 hour to conjugate the drug linker to the antibody. Subsequently, an aqueous solution of 100 mM NAC (Sigma-Aldrich Co. LLC) (0.0037 mL; 10.0 equivalents per antibody molecule) was added thereto, and the obtained mixture was stirred and then further left standing at room temperature for 20 minutes to terminate the reaction of the drug linker.

Purification: The above-described solution was purified by common procedure D described in production method 1 to obtain 3.5 mL of a solution containing the title antibody-drug conjugate "hmAb-H11L11-ADC".

Characterization: Using common procedures E and F (using $\varepsilon_{D,280}$ = 5440 and $\varepsilon_{D,370}$ = 21800) described in production method 1, the following characteristic values were obtained.

Antibody concentration: 1.30 mg/mL, antibody yield: 4.57 mg (86%), average number of conjugated drug molecules (n) per antibody molecule measured by common procedure E: 5.6, and average number of conjugated drug molecules (n) per antibody molecule measured by common procedure F: 7.5.

2)-2 Production of antibody-drug conjugate - (2) hmAb-H11L11-DXd

hmAb-H11L11-DXd was synthesized by the following step.

[Formula 24]

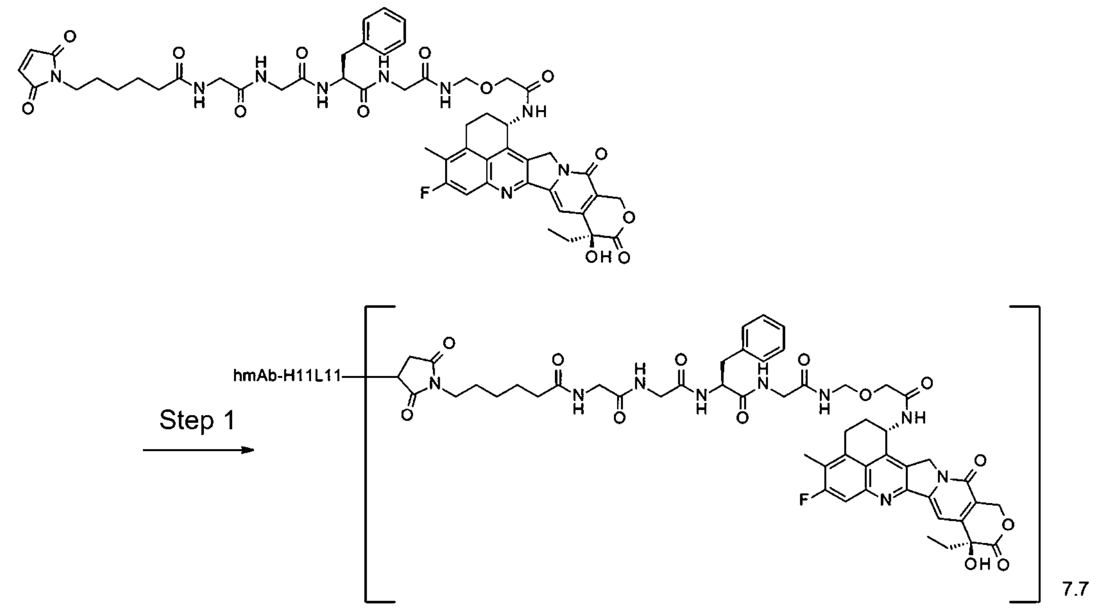


Reduction of antibody: hmAb-H11L11 prepared in Example 1)-2 was adjusted to 10.67 mg/mL with PBS6.0/EDTA by using common procedures B (using 1.50 $mLmg^{-1}cm^{-1}$ as 280 nm absorption coefficient) and C described in production method 1. To this solution (0.5 mL), a 1 M aqueous dipotassium hydrogen phosphate solution (Nacalai Tesque, Inc.; 0.0075 mL) and an aqueous solution of 10 mM TCEP (Tokyo Chemical Industry Co., Ltd.) (0.0294 mL; 8.0 equivalents per antibody molecule) were added. After confirming that the solution had a pH within 7.0 ± 0.1, the interchain disulfide bond in the antibody was reduced by incubating the solution at 37°C for 2 hours.

Conjugation between antibody and drug linker: The above-described solution was incubated at 15°C for 10 minutes. Subsequently, a 10 mM solution of N-[6-(2,5-dioxo-2,5-dihydro-1H-pyrrol-1-yl)hexanoyl]glycylglycyl-L-phenylalanyl-N-[(2-{[(1S,9S)-9-ethyl-5-fluoro-9-hydroxy-4-methyl-10,13-dioxo-2,3,9,10,13,15-hexahydro-1H,12H-benzo[de]pyrano[3',4':6,7]indolizino[1,2-b]quinolin-1-yl]amino}-2-oxoethoxy)methyl]glycinamide in dimethyl sulfoxide (0.0441 mL; 12.0 equivalents per antibody molecule) was added thereto, and the obtained mixture was incubated at 15°C for 1 hour to conjugate the drug linker to the antibody. Subsequently, an aqueous solution of 100 mM NAC (Sigma-Aldrich Co. LLC) (0.0044 mL; 12.0 equivalents per antibody molecule) was added thereto and stirred, and thereafter, the obtained mixture was further left standing at room temperature for 20 minutes to terminate the reaction of the drug linker.

Purification: The above-described solution was purified by common procedure D described in production method 1 to obtain 3.5 mL of a solution containing the title antibody-drug conjugate "hmAb-H11L11-ADC".

Characterization: Using common procedures E and F (using $\varepsilon_{D,280}$ = 5440 and $\varepsilon_{D,370}$ = 21800) described in production method 1, the following characteristic values were obtained.

Antibody concentration: 1.34 mg/mL, antibody yield: 4.69 mg (88%), average number of conjugated drug molecules (n) per antibody molecule measured by common procedure E: 5.9, and average number of conjugated drug molecules (n) per antibody molecule measured by common procedure F: 7.7.

2)-3 Production of antibody-drug conjugate - (3) hmAb-H11L11-DXd

hmAb-H11L11-DXd was synthesized by the following step.

[Formula 25]

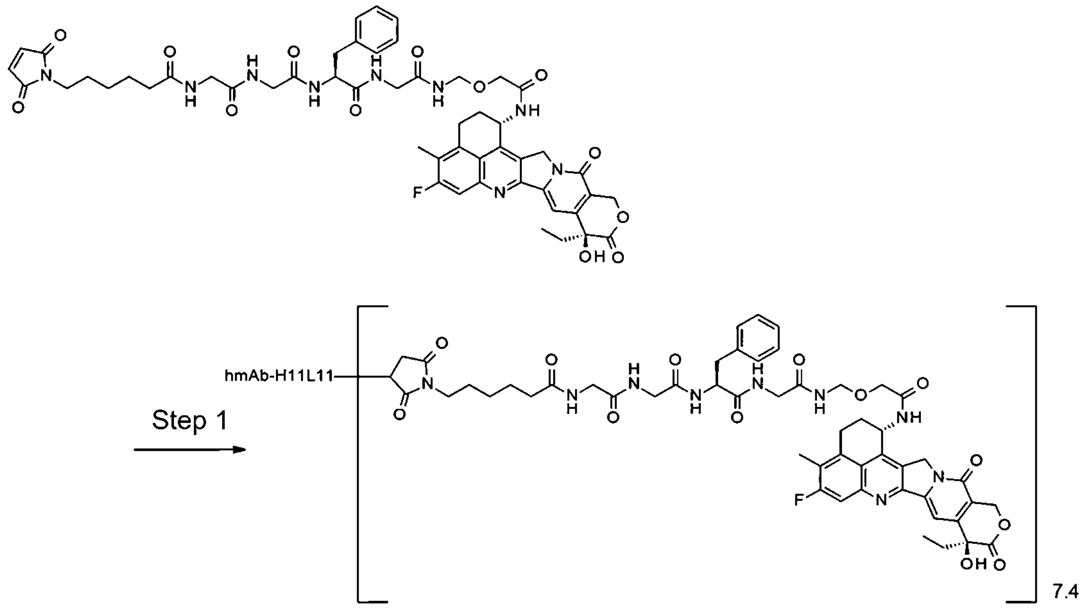


Reduction of antibody: hmAb-H11L11 prepared in Example 1)-2 was adjusted to 10.67 mg/mL with PBS6.0/EDTA by using common procedures B (using 1.50 $mLmg^{-1}cm^{-1}$ as 280 nm absorption coefficient) and C described in production method 1. To this solution (8.3 mL), a 1 M aqueous dipotassium hydrogen phosphate solution (Nacalai Tesque, Inc.; 0.124 mL) and an aqueous solution of 10 mM TCEP (Tokyo Chemical Industry Co., Ltd.) (0.486 mL; 8.0 equivalents per antibody molecule) were added. After confirming that the solution had a pH within 7.0 ± 0.1, the interchain disulfide bond in the antibody was reduced by incubating the solution at 37°C for 2 hours.

Conjugation between antibody and drug linker: The above-described solution was incubated at 15°C for 10 minutes. Subsequently, a 10 mM solution of N-[6-(2,5-dioxo-2,5-dihydro-1H-pyrrol-1-yl)hexanoyl]glycylglycyl-L-phenylalanyl-N-[(2-{[(1S,9S)-9-ethyl-5-fluoro-9-hydroxy-4-methyl-10,13-dioxo-2,3,9,10,13,15-hexahydro-1H,12H-benzo[de]pyrano[3',4' :6,7]indolizino[1,2-b]quinolin-1-yl]amino}-2-oxoethoxy)methyl]glycinamide in dimethyl sulfoxide (0.728 mL; 12.0 equivalents per antibody molecule) was added thereto, and the obtained mixture was incubated at 15°C for 1 hour to conjugate the drug linker to the antibody. Subsequently, an aqueous solution of 100 mM NAC (Sigma-Aldrich Co. LLC) (0.073 mL; 12.0 equivalents per antibody molecule) was added thereto and stirred, and thereafter, the obtained mixture was further left standing at room temperature for 20 minutes to terminate the reaction of the drug linker.

Purification: The above-described solution was purified by common procedure D described in production method 1 to obtain 31.5 mL of a solution containing the title antibody-drug conjugate "hmAb-H11L11-ADC".

Characterization: Using common procedures E and F (using $\varepsilon_{D,280}$ = 5440 and $\varepsilon_{D,370}$ = 21800) described in production method 1, the following characteristic values were obtained.

Antibody concentration: 2.23 mg/mL, antibody yield: 70.29 mg (80%), average number of conjugated drug molecules (n) per antibody molecule measured by common procedure E: 5.6, and average number of conjugated drug molecules (n) per antibody molecule measured by common procedure F: 7.4.

2)-4 Production of antibody-drug conjugate - (4) hmAb-H541L11-DXd

hmAb-H541L11-DXd (hereinafter, also referred to as "anti-CD37 antibody-drug conjugate (1)") was synthesized by the following step.

[Formula 26]

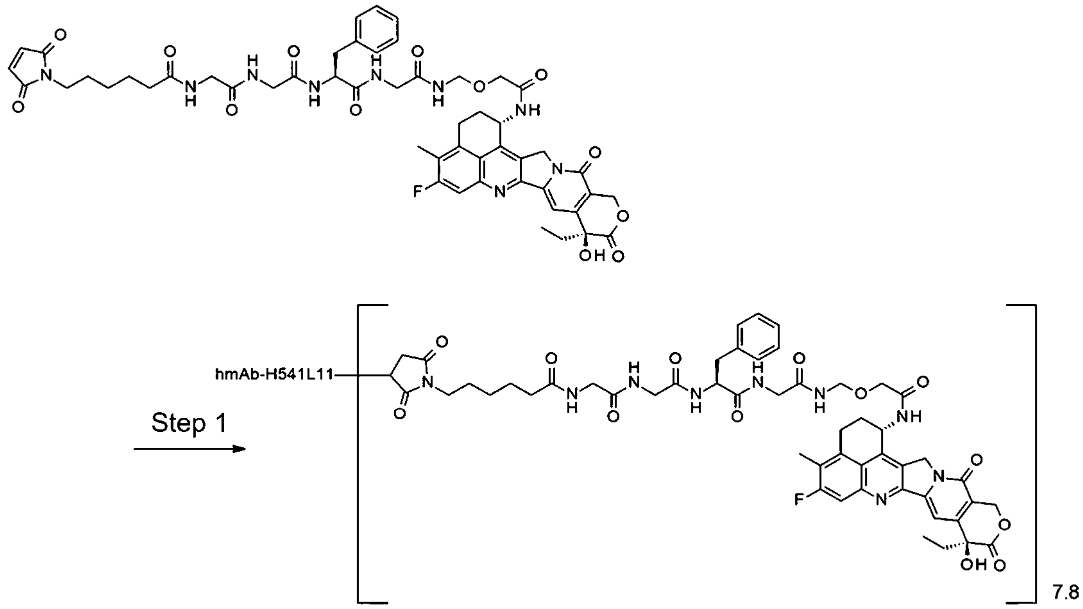


Reduction of antibody: hmAb-H541L11 prepared in Example 1)-2 was adjusted to 10.63 mg/mL with PBS6.0/EDTA by using common procedures B (using 1.50 $mLmg^{-1}cm^{-1}$ as 280 nm absorption coefficient) and C described in production method 1. To this solution (8.9 mL), a 1 M aqueous dipotassium hydrogen phosphate solution (Nacalai Tesque, Inc.; 0.133 mL) and an aqueous solution of 10 mM TCEP (Tokyo Chemical Industry Co., Ltd.) (0.389 mL; 6.0 equivalents per antibody molecule) were added. After confirming that the solution had a pH within 7.0 ± 0.1, the interchain disulfide bond in the antibody was reduced by incubating the solution at 37°C for 2 hours.

Conjugation between antibody and drug linker: The above-described solution was incubated at 15°C for 10 minutes. Subsequently, a 10 mM solution of N-[6-(2,5-dioxo-2,5-dihydro-1H-pyrrol-1-yl)hexanoyl]glycylglycyl-L-phenylalanyl-N-[(2-{[(1S,9S)-9-ethyl-5-fluoro-9-hydroxy-4-methyl-10,13-dioxo-2,3,9,10,13,15-hexahydro-1H,12H-benzo[de]pyrano[3',4':6,7]indolizino[1,2-b]quinolin-1-yl]amino}-2-oxoethoxy)methyl]glycinamide in dimethyl sulfoxide (0.649 mL; 10.0 equivalents per antibody molecule) was added thereto, and the obtained mixture was incubated at 15°C for 1 hour to conjugate the drug linker to the antibody. Subsequently, an aqueous solution of 100 mM NAC (Sigma-Aldrich Co. LLC) (0.065 mL; 10.0 equivalents per antibody molecule) was added thereto and stirred, and thereafter, the obtained mixture was further left standing at room temperature for 20 minutes to terminate the reaction of the drug linker.

Purification: The above-described solution was purified by common procedure D described in production method 1 to obtain 31.5 mL of a solution containing the title antibody-drug conjugate "hmAb-H541L11-ADC".

Characterization: Using common procedures E and F (using $\varepsilon_{D,280}$ = 5440 and $\varepsilon_{D,370}$ = 21800) described in production method 1, the following characteristic values were obtained.

Antibody concentration: 2.68 mg/mL, antibody yield: 84.26 mg (89%), average number of conjugated drug molecules (n) per antibody molecule measured by common procedure E: 5.9, and average number of conjugated drug molecules (n) per antibody molecule measured by common procedure F: 7.8.

2)-5 Production of antibody-drug conjugate - (5) hmAb-H551L11-DXd

hmAb-H551L11-DXd was synthesized by the following step.

[Formula 27]

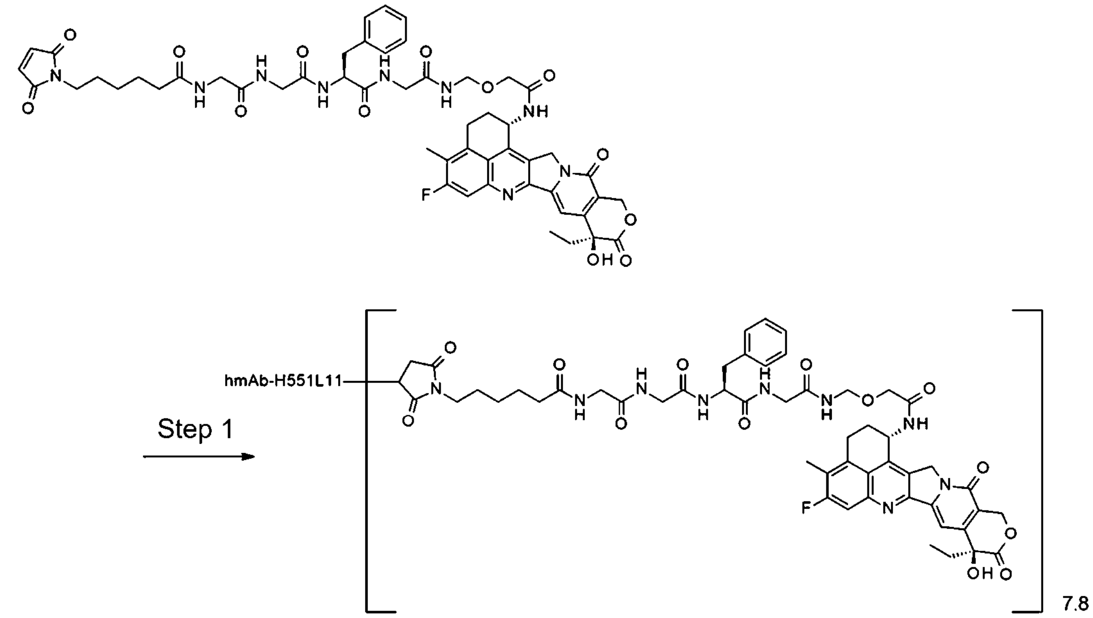


Reduction of antibody: hmAb-H551L11 prepared in Example 1)-2 was adjusted to 10.62 mg/mL with PBS6.0/EDTA by using common procedures B (using 1.50 $mLmg^{-1}cm^{-1}$ as 280 nm absorption coefficient) and C described in production method 1. To this solution (9.4 mL), a 1 M aqueous dipotassium hydrogen phosphate solution (Nacalai Tesque, Inc.; 0.141 mL) and an aqueous solution of 10 mM TCEP (Tokyo Chemical Industry Co., Ltd.) (0.411 mL; 6.0 equivalents per antibody molecule) were added. After confirming that the solution had a pH within 7.0 $\pm$ 0.1, the interchain disulfide bond in the antibody was reduced by incubating the solution at 37°C for 2 hours.

Conjugation between antibody and drug linker: The above-described solution was incubated at 15°C for 10 minutes. Subsequently, a 10 mM solution of N-[6-(2,5-dioxo-2,5-dihydro-1H-pyrrol-1-yl)hexanoyl]glycylglycyl-L-phenylalanyl-N-[(2-{[(1S,9S)-9-ethyl-5-fluoro-9-hydroxy-4-methyl-10,13-dioxo-2,3,9,10,13,15-hexahydro-1H,12H-benzo[de]pyrano[3',4':6,7]indolizino[1,2-b]quinolin-1-yl]amino}-2-oxoethoxy)methyl]glycinamide in dimethyl sulfoxide (0.686 mL; 10.0 equivalents per antibody molecule) was added thereto, and the obtained mixture was incubated at 15°C for 1 hour to conjugate the drug linker to the antibody. Subsequently, an aqueous solution of 100 mM NAC (Sigma-Aldrich Co. LLC) (0.069 mL; 10.0 equivalents per antibody molecule) was added thereto and stirred, and thereafter, the obtained mixture was further left standing at room temperature for 20 minutes to terminate the reaction of the drug linker.

Purification: The above-described solution was purified by common procedure D described in production method 1 to obtain 35.0 mL of a solution containing the title antibody-drug conjugate "hmAb-H551L11-ADC".

Characterization: Using common procedures E and F (using $\varepsilon_{D,280}$ = 5440 and $\varepsilon_{D,370}$ = 21800) described in production method 1, the following characteristic values were obtained.

Antibody concentration: 2.43 mg/mL, antibody yield: 85.08 mg (85%), average number of conjugated drug molecules (n) per antibody molecule measured by common procedure E: 5.7, and average number of conjugated drug molecules (n) per antibody molecule measured by common procedure F: 7.8.

2)-6 Production of antibody-drug conjugate - (6) hmAb-H11aL11-DXd

hmAb-H11aL11-DXd was synthesized by the following step.

[Formula 28]

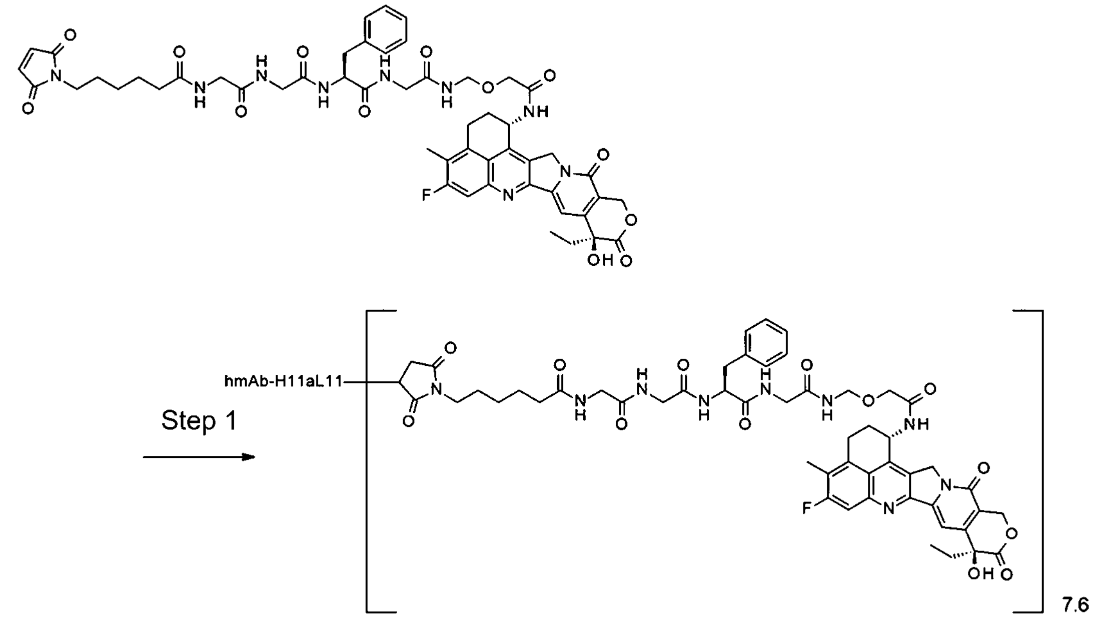


Reduction of antibody: hmAb-H11aL11 prepared in Example 1)-2 was adjusted to 10.59 mg/mL with PBS6.0/EDTA by using common procedures B (using 1.50 $mLmg^{-1}cm^{-1}$ as 280 nm absorption coefficient) and C described in production method 1. To this solution (10.0 mL), a 1 M aqueous dipotassium hydrogen phosphate solution (Nacalai Tesque, Inc.; 0.150 mL) and an aqueous solution of 10 mM TCEP (Tokyo Chemical Industry Co., Ltd.) (0.510 mL; 7.0 equivalents per antibody molecule) were added. After confirming that the solution had a pH within 7.0 $\pm$ 0.1, the interchain disulfide bond in the antibody was reduced by incubating the solution at 37°C for 2 hours.

Conjugation between antibody and drug linker: The above-described solution was incubated at 15°C for 10 minutes. Subsequently, a 10 mM solution of N-[6-(2,5-dioxo-2,5-dihydro-1H-pyrrol-1-yl)hexanoyl]glycylglycyl-L-phenylalanyl-N-[(2-{[(1S,9S)-9-ethyl-5-fluoro-9-hydroxy-4-methyl-10,13-dioxo-2,3,9,10,13,15-hexahydro-1H,12H-benzo[de]pyrano[3',4':6,7]indolizino[1,2-b]quinolin-1-yl]amino}-2-oxoethoxy)methyl]glycinamide in dimethyl sulfoxide (0.801 mL; 11.0 equivalents per antibody molecule) was added thereto, and the obtained mixture was incubated at 15°C for 1 hour to conjugate the drug linker to the antibody. Subsequently, an aqueous solution of 100 mM NAC (Sigma-Aldrich Co. LLC) (0.080 mL; 11.0 equivalents per antibody molecule) was added thereto and stirred, and thereafter, the obtained mixture was further left standing at room temperature for 20 minutes to terminate the reaction of the drug linker.

Purification: The above-described solution was purified by common procedure D described in production method 1 to obtain 35.0 mL of a solution containing the title antibody-drug conjugate "hmAb-H11aL11-ADC".

Characterization: Using common procedures E and F (using $\varepsilon_{D,280}$ = 5440 and $\varepsilon_{D,370}$ = 21800) described in production method 1, the following characteristic values were obtained.

Antibody concentration: 2.62 mg/mL, antibody yield: 91.57 mg (86%), average number of conjugated drug molecules (n) per antibody molecule measured by common procedure E: 5.7, and average number of conjugated drug molecules (n) per antibody molecule measured by common procedure F: 7.6.

[Example 3: Antitumor test - 1]

In an antitumor test, evaluation was performed using animal models derived from immunodeficient mice by the inoculation of CD37-positive human tumor cell line cells. SCID mice (CB17/Icr-Prkdc[scid]/CrlCrlj: Charles River Laboratories Japan Inc., The Jackson Laboratory Japan, Inc., and C.B-17/Icr-scid/scidJcl: CLEA Japan, Inc.) obtained at the age of 4 to 6 weeks were acclimatized for 3 days or longer under SPF conditions before use in the experiment. The mice were fed with a sterilized solid diet (FR-2, Funabashi Farms Co., Ltd) and given sterilized tap water (prepared with a sodium hypochlorite solution to attain a final concentration of 3 ppm). The long diameter and short diameter of the inoculated tumor were measured twice or three times a week using electronic digital calipers (CD-15CX, Mitutoyo Corp.), and the estimated tumor volume was then calculated according to the following formula. It is to be noted that in the present description, the estimated tumor volume is also simply referred to as a "tumor volume".

Estimated tumor volume ($mm^3$) = 1/2 $\times$ Long diameter (mm) $\times$ [Short diameter (mm)]$^2$

The anti-CD37 antibody-drug conjugate (1) was diluted with ABS buffer (10 mM acetate buffer, 5% sorbitol, pH 5.5) (Nacalai Tesque, Inc.) or histidine buffer (10 mM histidine buffer, pH 5.5, 9% (w/v) sucrose, 0.03% (w/v) PS80), and the dilution was intravenously administered at the dose shown in each Example via the tail vein of each mouse. Rituximab (manufactured by Zenyaku Kogyo Co., Ltd.), bendamustine (manufactured by SymBio Pharmaceuticals Ltd.), tafasitamab (manufactured by MorphoSys AG), lenalidomide (manufactured by AdooQ Bioscience LLC), ibrutinib (synthesized by a method well known to those skilled in the art), or venetoclax (synthesized by a method well known to those skilled in the art) was administered at the usage and dose shown in each Example. The antitumor test was conducted using the anti-CD37 antibody-drug conjugate (1) prepared by the method described in Example 2)-5, or the anti-CD37 antibody-drug conjugate (1) prepared by a method with a minor change made in the method described in Example 2)-5 appropriate for the preparation scale. Five to six mice per group were used in the experiment.

3)-1 Antitumor test - (1)

CD37-positive human diffuse large B-cell lymphoma cell line OCI-LY7 (DSMZ) was suspended in 50% Matrigel (Corning Inc., diluted with PBS), and the cell suspension was subcutaneously inoculated with $1 \times 10^7$ cells to the right flank region of each female SCID mouse (Day 0). On Day 12, the mice were randomly grouped. On the day of grouping, the anti-CD37 antibody-drug conjugate (1) and rituximab were intravenously administered at doses of 1 mg/kg and 10 mg/kg, respectively, via the tail vein of each mouse. Each monotherapy group, a combination group, and a vehicle administration group as a control group were established.

Figure 11 shows results of the combination of the anti-CD37 antibody-drug conjugate (1) and rituximab. Tumor regrowth was observed in each monotherapy group, whereas the combination group exhibited a better tumor growth inhibitory effect than that of the monotherapy group and induced tumor regression. Tumor growth inhibition (%) (hereinafter, referred to as "TGI") on Day 22 was calculated according to the following formula, and is described in Table 1.

Tumor growth inhibition (%) = (1 - Average estimated tumor volume for each group / Average estimated tumor volume for a vehicle control) $\times$ 100

Change from baseline (%) (rate of change in tumor volume on Day 22 of each individual from the tumor volume on Day 12 as a baseline) was calculated according to the following formula, and is shown in Table 1 and Figure 12. The combination group of the anti-CD37 antibody-drug conjugate (1) and rituximab exhibited an excellent tumor regression effect as compared with the control group, the anti-CD37 antibody-drug conjugate (1) administration group, and the rituximab administration group.

Change from baseline(%) = [(Estimated tumor volume on Day 22 for each individual - Estimated tumor volume on Day 12) / Estimated tumor volume on Day 12)] $\times$ 100

On the final day of observation (Day 61), CR (complete response) (%) was calculated according to the formula given below, and is described in Table 1. CR represents, as shown in the formula given below, the ratio (%) of the number of individuals having an estimated tumor volume of 0 $mm^3$ in each group to the total number of individuals in each group. The tumor sizes of the control group, the anti-CD37 antibody-drug conjugate (1) administration group, and the rituximab administration group reached the humane endpoint in the middle of the observation period, whereas the combination group of the anti-CD37 antibody-drug conjugate (1) and rituximab exhibited an excellent tumor regression effect with CR in all the individuals up to the end date of observation. None of the mouse individuals given each compound exhibited 20% or more weight loss. Accordingly, the combination of the anti-CD37 antibody-drug conjugate (1) and rituximab increased effectiveness without increasing toxicity.

CR (%) = (The number of individuals having an estimated tumor volume of 0 $mm^3$ in each group / The total number of individuals in each group) $\times$ 100

[Table 1]

| Compound | TGI (%) | Change from baseline (%) | CR (%) on final day of observation |
|---|---|---|---|
| Control | 0 | 1381.7 | 0* |

(continued)

| Compound | TGI (%) | Change from baseline (%) | CR (%) on final day of observation |
|---|---|---|---|
| Anti-CD37 antibody-drug conjugate (1) | 94 | -4.8 | 0* |
| Rituximab | 73 | 298.8 | 0* |
| Anti-CD37 antibody-drug conjugate (1) and Rituximab | 98 | -71.6 | 100 |
| * : All individuals were euthanized in the middle of the observation period due to reaching the humane endpoint. | | | |

3)-2 Antitumor test - (2)

CD37-positive human diffuse large B-cell lymphoma cell line SU-DHL-4 (DSMZ) was suspended in 50% Matrigel, and the cell suspension was subcutaneously inoculated with $1 \times 10^7$ cells to the right flank region of each female SCID mouse (Day 0). On Day 16, the mice were randomly grouped. Each monotherapy group, a combination group, and a vehicle administration group as a control group were established. TGI and the rate of change in tumor volume on Day 38 were calculated and are shown in Table 2.

Figure 13 shows results of the combination of the anti-CD37 antibody-drug conjugate (1) and rituximab. On the day of grouping, the anti-CD37 antibody-drug conjugate (1) and rituximab were intravenously administered at doses of 3 mg/kg and 10 mg/kg, respectively, via the tail vein of each mouse. The anti-CD37 antibody-drug conjugate (1) administration group and the rituximab administration group had TGI of 44% and 38%, respectively, on Day 38, whereas the combination group exhibited a better tumor growth inhibitory effect (TGI, 70%) than that of each monotherapy group.

Figure 14 shows results of the combination of the anti-CD37 antibody-drug conjugate (1) and rituximab + bendamustine (RB). The anti-CD37 antibody-drug conjugate (1) was intravenously administered at a dose of 3 mg/kg via the tail vein of each mouse at the time of grouping. On the day of grouping, rituximab was intravenously administered at a dose of 10 mg/kg via the tail vein of each mouse. On the day of grouping and the next day, bendamustine was intraperitoneally administered at a dose of 12.5 mg/kg. The anti-CD37 antibody-drug conjugate (1) administration group and the RB administration group had TGI of 44% and 28%, respectively, on Day 38, whereas the combination group exhibited a better tumor growth inhibitory effect (TGI, 78%) than that of each monotherapy group.

Figure 15 shows results of the combination of the anti-CD37 antibody-drug conjugate (1) and tafasitamab. The anti-CD37 antibody-drug conjugate (1) was intravenously administered at a dose of 3 mg/kg via the tail vein of each mouse at the time of grouping. Tafasitamab was intravenously administered at a dose of 30 mg/kg via the tail vein of each mouse every week from the day of grouping. The anti-CD37 antibody-drug conjugate (1) administration group and the tafasitamab administration group had TGI of 44% and 39%, respectively, on Day 38, whereas the combination group exhibited a better tumor growth inhibitory effect (TGI, 76%) than that of each monotherapy group.

Figure 16 shows results of the combination of the anti-CD37 antibody-drug conjugate (1) and tafasitamab-lenalidomide. The anti-CD37 antibody-drug conjugate (1) was intravenously administered at a dose of 3 mg/kg via the tail vein of each mouse at the time of grouping. Tafasitamab was intravenously administered at a dose of 30 mg/kg via the tail vein of each mouse every week from the day of grouping. Lenalidomide was orally administered at a dose of 10 mg/kg at intervals of 5 times/week from the time of grouping. The anti-CD37 antibody-drug conjugate (1) administration group and the tafasitamab-lenalidomide administration group had TGI of 44% and 45%, respectively, on Day 38, whereas the combination group exhibited a better tumor growth inhibitory effect (TGI, 74%) than that of each monotherapy group.

Figure 17 shows the rate of change in tumor volume on Day 38. The combination of the anti-CD37 antibody-drug conjugate (1) and each drug was confirmed to have an evident additive effect on drug efficacy. None of the mouse individuals given each compound exhibited 20% or more weight loss. Accordingly, the combination of the anti-CD37 antibody-drug conjugate (1) and the other drug increased effectiveness without increasing toxicity.

[Table 2]

| Compound | TGI (%) | Change from baseline (%) | CR (%) on final day of observation |
|---|---|---|---|
| Control | 0 | 1262.0 | 0 |
| Anti-CD37 antibody-drug conjugate (1) | 44 | 673.2 | 0 |
| Rituximab | 38 | 715.8 | 0 |

(continued)

| Compound | TGI (%) | Change from baseline (%) | CR (%) on final day of observation |
|---|---|---|---|
| Anti-CD37 antibody-drug conjugate (1) and Rituximab | 70 | 303.7 | 0 |
| RB | 28 | 859. 5 | 0 |
| Anti-CD37 antibody-drug conjugate (1) and RB | 78 | 197.2 | 0 |
| Tafasitamab | 39 | 734.9 | 0 |
| Anti-CD37 antibody-drug conjugate (1) and Tafasitamab | 76 | 228.4 | 0 |
| Tafasitamab -Lenalidomide | 45 | 674.1 | 0 |
| Anti-CD37 antibody-drug conjugate (1) and Tafasitamab -Lenalidomide | 74 | 256.7 | 0 |

3)-3 Antitumor effect - (3)

CD37-positive human follicular lymphoma cell line DOHH-2 (DSMZ) was suspended in 50% Matrigel, and the cell suspension was subcutaneously inoculated with 3 × $10^6$ cells to the right flank region of each female SCID mouse (Day 0). On Day 23, the mice were randomly grouped. Each monotherapy group, a combination group, and a non-administration group as a control group were established. TGI and the rate of change in tumor volume on Day 44 were calculated and are shown in Table 3.

Figure 18 shows results of the combination of the anti-CD37 antibody-drug conjugate (1) and rituximab. On the day of grouping, the anti-CD37 antibody-drug conjugate (1) and rituximab were intravenously administered at doses of 0.3 mg/kg and 1 mg/kg, respectively, via the tail vein of each mouse. The anti-CD37 antibody-drug conjugate (1) administration group and the rituximab administration group had TGI of 26% and 51%, respectively, on Day 44, whereas the combination group exhibited a better tumor growth inhibitory effect (TGI, 67%) than that of each monotherapy group.

Figure 19 shows results of the combination of the anti-CD37 antibody-drug conjugate (1) and rituximab + bendamustine (RB). The anti-CD37 antibody-drug conjugate (1) was intravenously administered at a dose of 0.3 mg/kg via the tail vein of each mouse at the time of grouping. On the day of grouping, rituximab was intravenously administered at a dose of 1 mg/kg via the tail vein of each mouse. On the day of grouping and the next day, bendamustine was intraperitoneally administered at a dose of 12.5 mg/kg. The anti-CD37 antibody-drug conjugate (1) administration group and the RB administration group had TGI of 26% and 68%, respectively, on Day 44, whereas the combination group exhibited a better tumor growth inhibitory effect (TGI, 94%) than that of each monotherapy group. Some individuals in the combination group exhibited CR (one out of 5 cases).

Figure 20 shows results of the combination of the anti-CD37 antibody-drug conjugate (1) and rituximab + lenalidomide (R-lenalidomide). The anti-CD37 antibody-drug conjugate (1) was intravenously administered at a dose of 0.3 mg/kg via the tail vein of each mouse at the time of grouping. On the day of grouping, rituximab was intravenously administered at a dose of 1 mg/kg via the tail vein of each mouse. Lenalidomide was orally administered at a dose of 10 mg/kg at intervals of 5 times/week from the time of grouping. The anti-CD37 antibody-drug conjugate (1) administration group and the R-lenalidomide group had TGI of 26% and 54%, respectively, on Day 44, whereas the combination group exhibited a better tumor growth inhibitory effect (TGI, 74%) than that of each monotherapy group.

Figure 21 shows the rate of change in tumor volume on Day 44. The combination of the anti-CD37 antibody-drug conjugate (1) and each drug was confirmed to have an evident additive effect on drug efficacy. Particularly, the combination of the anti-CD37 antibody-drug conjugate (1) and RB was confirmed to have a tumor regression effect. None of the mouse individuals given each compound exhibited 20% or more weight loss. Accordingly, the combination of the anti-CD37 antibody-drug conjugate (1) and the other drug increased effectiveness without increasing toxicity.

[Table 3]

| Compound | TGI (%) | Change from baseline (%) | CR (%) on final day of observation |
|---|---|---|---|
| Control | 0 | 524.3 | 0 |
| Anti-CD37 antibody-drug conjugate (1) | 26 | 374.6 | 0 |
| Rituximab | 51 | 215.5 | 0 |

(continued)

| Compound | TGI (%) | Change from baseline (%) | CR (%) on final day of observation |
|---|---|---|---|
| Anti-CD37 antibody-drug conjugate (1) and Rituximab | 67 | 109.5 | 0 |
| RB | 68 | 97.5 | 0 |
| Anti-CD37 antibody-drug conjugate (1) and RB | 94 | -66.1 | 20 |
| R-Lenalidomide | 54 | 194.5 | 0 |
| Anti-CD37 antibody-drug conjugate (1) and R-Lenalidomide | 74 | 67.3 | 0 |

3)-4 antitumor effect - (4)

CD37-positive human chronic lymphocytic leukemia cell line JVM-13 (ATCC) was suspended in 50% Matrigel, and the cell suspension was subcutaneously inoculated with $1 \times 10^7$ cells to the right flank region of each female SCID mouse (Day 0). On Day 14, the mice were randomly grouped. Each monotherapy group, a combination group, and a non-administration group as a control group were established. TGI and the rate of change in tumor volume on Day 35 were calculated and are shown in Table 4.

Figure 22 shows results of the combination of the anti-CD37 antibody-drug conjugate (1) and rituximab. On the day of grouping, the anti-CD37 antibody-drug conjugate (1) and rituximab were intravenously administered at doses of 0.3 mg/kg and 10 mg/kg, respectively, via the tail vein of each mouse. The anti-CD37 antibody-drug conjugate (1) administration group and the rituximab administration group had TGI of 27% and 0%, respectively, on Day 35, whereas the combination group exhibited a better tumor growth inhibitory effect (TGI, 68%) than that of each monotherapy group.

Figure 23 shows results of the combination of the anti-CD37 antibody-drug conjugate (1) and rituximab + bendamustine (RB). The anti-CD37 antibody-drug conjugate (1) was intravenously administered at a dose of 0.3 mg/kg via the tail vein of each mouse at the time of grouping. On the day of grouping, rituximab was intravenously administered at a dose of 10 mg/kg via the tail vein of each mouse. On the day of grouping and the next day, bendamustine was intraperitoneally administered at a dose of 12.5 mg/kg. The anti-CD37 antibody-drug conjugate (1) administration group and the RB administration group had TGI of 27% and 51%, respectively, on Day 35, whereas the combination group exhibited a better tumor growth inhibitory effect (TGI, 81%) than that of each monotherapy group.

Figure 24 shows results of the combination of the anti-CD37 antibody-drug conjugate (1) and venetoclax. The anti-CD37 antibody-drug conjugate (1) was intravenously administered at a dose of 0.3 mg/kg via the tail vein of each mouse at the time of grouping. Venetoclax was orally administered at a dose of 100 mg/kg every day from the day of grouping. The anti-CD37 antibody-drug conjugate (1) administration group and the venetoclax administration group had TGI of 27% and 25%, respectively, on Day 35, whereas the combination group exhibited a better tumor growth inhibitory effect (TGI, 49%) than that of each monotherapy group.

Figure 25 shows results of the combination of the anti-CD37 antibody-drug conjugate (1) and rituximab + venetoclax (R-venetoclax). The anti-CD37 antibody-drug conjugate (1) was intravenously administered at a dose of 0.3 mg/kg via the tail vein of each mouse at the time of grouping. On the day of grouping, rituximab was intravenously administered at a dose of 10 mg/kg via the tail vein of each mouse. Venetoclax was orally administered at a dose of 100 mg/kg every day from the day of grouping. The anti-CD37 antibody-drug conjugate (1) administration group and the R-venetoclax administration group had TGI of 27% and 44%, respectively, on Day 35, whereas the combination group exhibited a better tumor growth inhibitory effect (TGI, 88%) than that of each monotherapy group.

Figure 26 shows results of the combination of the anti-CD37 antibody-drug conjugate (1) and ibrutinib. The anti-CD37 antibody-drug conjugate (1) was intravenously administered at a dose of 0.3 mg/kg via the tail vein of each mouse at the time of grouping. Ibrutinib was orally administered at a dose of 200 mg/kg every day from the day of grouping. The anti-CD37 antibody-drug conjugate (1) administration group and the ibrutinib administration group had TGI of 27% and -12%, respectively, on Day 35, whereas the combination group exhibited a better tumor growth inhibitory effect (TGI, 53%) than that of each monotherapy group.

Figure 27 shows the rate of change in tumor volume on Day 35. The combination of the anti-CD37 antibody-drug conjugate (1) and each drug was confirmed to have an evident additive effect on drug efficacy. Particularly, the combination of the anti-CD37 antibody-drug conjugate (1) and R-venetoclax was confirmed to have a tumor regression effect. None of the mouse individuals given each compound exhibited 20% or more weight loss. Accordingly, the combination of the anti-CD37 antibody-drug conjugate (1) and the other drug increased effectiveness without increasing toxicity.

[Table 4]

| Compound | TGI (%) | Change from baseline (%) | CR (%) on final day of observation |
|---|---|---|---|
| Control | 0 | 707.4 | 0 |
| Anti-CD37 antibody-drug conjugate (1) | 27 | 480.4 | 0 |
| Rituximab | 0 | 708.4 | 0 |
| Anti-CD37 antibody-drug conjugate (1) and Rituximab | 68 | 149.5 | 0 |
| RB | 51 | 289.6 | 0 |
| Anti-CD37 antibody-drug conjugate (1) and RB | 8 1 | 56.5 | 0 |
| Venetoclax | 25 | 490.9 | 0 |
| Anti-CD37 antibody-drug conjugate (1) and Venetoclax | 49 | 303.9 | 0 |
| R-Venetoclax | 44 | 335.8 | 0 |
| Anti-CD37 antibody-drug conjugate (1) and R-Venetoclax | 88 | -5.2 | 0 |
| Ibrutinib | -12 | 776.7 | 0 |
| Anti-CD37 antibody-drug conjugate (1) and Ibrutinib | 53 | 274.9 | 0 |

[Example 4: Internalization evaluation]

Cells of each line were inoculated at $5 \times 10^4$ cells/well into a 96-well Clear Round Bottom TC-treated Microplate (Corning Inc.). The anti-CD37 antibody-drug conjugate (1) fluorescently labeled using Alexa Fuluor (registered trademark) 488 Antibody Labeling Kit (Thermo Fisher Scientific Inc.), and rituximab or Human IgG1, Myeloma (Merk Millipore; hereinafter, referred to as "IgG1") were added to each wall to attain a final concentration of 1.5 μg/mL. Wells supplemented with fluorescently labeled antibody-drug conjugate (2) in the presence of rituximab or IgG1 were prepared as a negative control. The antibody-drug conjugate (2) is an antibody-drug conjugate prepared from human IgG1 that recognizes an antigen unrelated to CD37. Each plate was cultured at 4°C for 1 hour or at 37°C for 1 to 6 hours and then washed, and ice-cooled PBS or Anti-Alexa Fuluor (registered trademark) 488, rabbit IgG fraction (Thermo Fisher Scientific Inc.) diluted into 25 μg/mL was added thereto. After incubation at 4°C for 30 minutes, 2% PFA/PBS was added to the cells, and the cells were fixed at 4°C. The fixed cells were measured using NovoCyte flow cytometer system. Two wells were used in culture under each condition, and an average value of internalization rates calculated according to the formula given below is shown. MFI represents mean fluorescence intensity.

Internalization rate (%) = (MFI of the wells treated with Anti-Alexa 488 after culture at 37°C for 1 to 6 hours - Average value of MFI of the wells treated with Anti-Alexa 488 after culture at 4°C for 1 hour) / (Average value of MFI of the wells treated with PBS after culture at 37°C for 1 to 6 hours - Average value of MFI of the negative control wells treated with PBS after culture at 37°C for 1 to 6 hours) $\times$ 100

4)-1 Internalization evaluation - 1

Figure 28 shows evaluation results obtained using OCI-LY7 (DSMZ) cells. The rituximab-supplemented group significantly increased the internalization rate of the anti-CD37 antibody-drug conjugate (1) as compared with the IgG1-supplemented group. This suggested enhancement in drug efficacy by elevated cellular uptake of the anti-CD37 antibody-drug conjugate (1) as the mechanism of action of the combination of rituximab and the anti-CD37 antibody-drug conjugate (1).

4)-2 Internalization evaluation - 2

Figure 29 shows evaluation results obtained using SU-DHL-4 (DSMZ) cells. The rituximab-supplemented group significantly increased the internalization rate of the anti-CD37 antibody-drug conjugate (1) as compared with the IgG1-supplemented group. This suggested enhancement in drug efficacy by elevated cellular uptake of the anti-CD37 antibody-drug conjugate (1) as the mechanism of action of the combination of rituximab and the anti-CD37 antibody-drug conjugate (1).

[Example 5: Antitumor test - 2]

The antitumor test was conducted by the same method as in Example 3. The anti-CD37 antibody-drug conjugate (1) was diluted with histidine buffer (10 mM histidine buffer, pH 5.5, 9% (w/v) sucrose, 0.03% (w/v) PS80), and the dilution was intravenously administered at a dose shown in each Example via the tail vein of each mouse. Rituximab (manufactured by Zenyaku Kogyo Co., Ltd.), cyclophosphamide (manufactured by Shionogi & Co., Ltd.), doxorubicin (manufactured by Kyowa Kirin Co., Ltd.), vincristine (manufactured by Nippon Kayaku Co., Ltd.), predonisone (manufactured by FUJIFILM Wako Pure Chemical Corp.), or obinutuzumab (manufactured by Roche) was administered at the usage and dose shown in each Example. The antitumor test was conducted using the anti-CD37 antibody-drug conjugate (1) prepared by the method described in Example 2)-5, or the anti-CD37 antibody-drug conjugate (1) prepared by a method with a minor change made in the method described in Example 2)-5 appropriate for the preparation scale. Six mice per group were used in the experiment.

5)-1 Antitumor test - (5)

CD37-positive human diffuse large B-cell lymphoma cell line OCI-LY7 (DSMZ) was suspended in 50% Matrigel (Corning Inc., diluted with PBS), and the cell suspension was subcutaneously inoculated with $1 \times 10^7$ cells to the right flank region of each female SCID mouse (Day 0). On Day 10, the mice were randomly grouped. Each monotherapy group, a combination group, and a non-administration group as a control group were established. TGI and the rate of change in tumor volume on Day 18 and CR on the final day of observation (Day 42) were calculated and are shown in Table 5.

Figure 30 shows results of the combination of the anti-CD37 antibody-drug conjugate (1) and rituximab. On the day of grouping, the anti-CD37 antibody-drug conjugate (1) and rituximab were intravenously administered at doses of 1 mg/kg and 3 mg/kg, respectively, via the tail vein of each mouse. The anti-CD37 antibody-drug conjugate (1) administration group, the rituximab administration group, and the combination group had TGI of 85%, 55%, and 84%, respectively, on Day 18, whereas the combination group suppressed tumor growth more than each monotherapy group did on Day 18 and thereafter.

Figure 31 shows results of the combination of the anti-CD37 antibody-drug conjugate (1) and R-CHP (rituximab + cyclophosphamide + doxorubicin + predonisone). On the day of grouping, the anti-CD37 antibody-drug conjugate (1) and rituximab were intravenously administered at doses of 1 mg/kg and 3 mg/kg, respectively, via the tail vein of each mouse. On the day following grouping, cyclophosphamide at a dose of 30 mg/kg and doxorubicin at a dose of 2.5 mg/kg were intravenously administered via the tail vein of each mouse. Predonisone was orally administered at a dose of 0.15 mg/kg once a day for 5 days from the day following grouping. The anti-CD37 antibody-drug conjugate (1) administration group and the R-CHP administration group had TGI of 85% and 76%, respectively, on Day 18, whereas the combination group exhibited a better tumor growth inhibitory effect (TGI, 93%) than that of each monotherapy group. Furthermore, CR on the final day of observation was 100%.

Figure 32 shows results of the combination of the anti-CD37 antibody-drug conjugate (1) and R-CHOP (rituximab + cyclophosphamide + doxorubicin + vincristine + predonisone). On the day of grouping, the anti-CD37 antibody-drug conjugate (1) and rituximab were intravenously administered at doses of 1 mg/kg and 3 mg/kg, respectively, via the tail vein of each mouse. On the day following grouping, cyclophosphamide at a dose of 30 mg/kg, doxorubicin at a dose of 2.5 mg/kg, and vincristine at a dose of 0.375 mg/kg were intravenously administered via the tail vein of each mouse. Predonisone was orally administered at a dose of 0.15 mg/kg once a day for 5 days from the day following grouping. The anti-CD37 antibody-drug conjugate (1) administration group and the R-CHP administration group had TGI of 85% and 81%, respectively, on Day 18, whereas the combination group exhibited a better tumor growth inhibitory effect (TGI, 95%) than that of each monotherapy group. Furthermore, CR on the final day of observation was 100%.

Figure 33 shows the rate of change in tumor volume on Day 18. The addition of CHP or CHOP to the anti-CD37 antibody-drug conjugate (1) and rituximab exhibited a significant combinatorial effect that induced a tumor regression effect. None of the mouse individuals given each compound exhibited 20% or more weight loss. Accordingly, the combination of the anti-CD37 antibody-drug conjugate (1) and the other drug increased effectiveness without increasing toxicity.

[Table 5]

| Compound | TGI (%) | Change from baseline (%) | CR (%) on final day of observation |
|---|---|---|---|
| Control | 0 | 563.5 | 0* |
| Anti-CD37 antibody-drug conjugate (1) | 85 | -0.9 | 0* |
| Rituximab | 55 | 187.9 | 0* |

(continued)

| Compound | TGI (%) | Change from baseline (%) | CR (%) on final day of observation |
|---|---|---|---|
| Anti-CD37 antibody-drug conjugate (1) and Rituximab | 84 | 2.6 | 0 * |
| R-CHP | 76 | 50.9 | 0* |
| Anti-CD37 antibody-drug conjugate (1) and R-CHP | 93 | -57.1 | 100 |
| R-CHOP | 81 | 26.4 | 0* |
| Anti-CD37 antibody-drug conjugate (1) and R-CHOP | 95 | -64.1 | 100 |
| * : All individuals were euthanized in the middle of the observation period due to reaching the humane endpoint. | | | |

5)-2 Antitumor test - (6)

CD37-positive human chronic lymphocytic leukemia cell line JVM-3 (DSMZ) was suspended in 50% Matrigel, and the cell suspension was subcutaneously inoculated with $3 \times 10^6$ cells to the right flank region of each female SCID mouse (Day 0). On Day 13, the mice were randomly grouped. Each monotherapy group, a combination group, and a non-administration group as a control group were established. TGI and the rate of change in tumor volume on Day 28 were calculated and are shown in Table 6.

Figure 34 shows results of the combination of the anti-CD37 antibody-drug conjugate (1) and rituximab. On the day of grouping, the anti-CD37 antibody-drug conjugate (1) and rituximab were intravenously administered at doses of 0.3 mg/kg and 10 mg/kg, respectively, via the tail vein of each mouse. The anti-CD37 antibody-drug conjugate (1) administration group and the rituximab administration group had TGI of 47% and 32%, respectively, on Day 28, whereas the combination group exhibited a better tumor growth inhibitory effect (TGI, 66%) than that of each monotherapy group.

Figure 35 shows results of the combination of the anti-CD37 antibody-drug conjugate (1) and obinutuzumab. The anti-CD37 antibody-drug conjugate (1) was intravenously administered at a dose of 0.3 mg/kg via the tail vein of each mouse at the time of grouping. On the day of grouping, obinutuzumab was intravenously administered at a dose of 30 mg/kg via the tail vein of each mouse. The anti-CD37 antibody-drug conjugate (1) administration group and the obinutuzumab administration group had TGI of 47% and 44%, respectively, on Day 28, whereas the combination group exhibited a better tumor growth inhibitory effect (TGI, 82%) than that of each monotherapy group.

Figure 36 shows the rate of change in tumor volume on Day 28. The combination of the anti-CD37 antibody-drug conjugate (1) and rituximab or obinutuzumab was confirmed to have an evident additive effect on drug efficacy. None of the mouse individuals given each compound exhibited 20% or more weight loss. Accordingly, the combination of the anti-CD37 antibody-drug conjugate (1) and the other drug increased effectiveness without increasing toxicity.

[Table 6]

| Compound | TGI (%) | Change from baseline (%) | CR (%) on final day of observation |
|---|---|---|---|
| Control | 0 | 1096. 8 | 0 |
| Anti-CD37 antibody-drug conjugate (1) | 47 | 538.7 | 0 |
| Rituximab | 32 | 694.9 | 0 |
| Anti-CD37 antibody-drug conjugate (1) and Rituximab | 66 | 325.7 | 0 |
| Obinutuzumab | 44 | 560.7 | 0 |
| Anti-CD37 antibody-drug conjugate (1) and Obinutuzumab | 82 | 118.2 | 0 |

5)-3 Antitumor test - (7)

CD37-positive human chronic lymphocytic leukemia cell line JVM-13 (ATCC) was suspended in 50% Matrigel, and the cell suspension was subcutaneously inoculated with $1 \times 10^7$ cells to the right flank region of each female SCID mouse (Day 0). On Day 10, the mice were randomly grouped. Each monotherapy group, a combination group, and a non-administration group as a control group were established. TGI and the rate of change in tumor volume on Day 25 were calculated and are shown in Table 7.

Figure 37 shows results of the combination of the anti-CD37 antibody-drug conjugate (1) and obinutuzumab. The

anti-CD37 antibody-drug conjugate (1) was intravenously administered at a dose of 0.3 mg/kg via the tail vein of each mouse at the time of grouping. On the day of grouping, obinutuzumab was intravenously administered at a dose of 30 mg/kg via the tail vein of each mouse. The anti-CD37 antibody-drug conjugate (1) administration group and the obinutuzumab administration group had TGI of 58% and 50%, respectively, on Day 25, whereas the combination group exhibited a better tumor growth inhibitory effect (TGI, 86%) than that of each monotherapy group.

Figure 38 shows the rate of change in tumor volume on Day 25. The combination of the anti-CD37 antibody-drug conjugate (1) and obinutuzumab was confirmed to have an evident additive effect on drug efficacy. None of the mouse individuals given each compound exhibited 20% or more weight loss. Accordingly, the combination of the anti-CD37 antibody-drug conjugate (1) and obinutuzumab increased effectiveness without increasing toxicity.

[Table 7]

| Compound | TGI (%) | Change from baseline (%) | CR (%) on final day of observation |
|---|---|---|---|
| Control | 0 | 597.3 | 0 |
| Anti-CD37 antibody-drug conjugate (1) | 58 | 190.6 | 0 |
| Obinutuzumab | 50 | 244.9 | 0 |
| Anti-CD37 antibody-drug conjugate (1) and Obinutuzumab | 86 | -0.1 | 0 |

[Example 6: Evaluation of cell growth inhibition activity by combination of anti-CD37 antibody-drug conjugate (1) and each BTK inhibitor]

CD37-positive human chronic lymphocytic leukemia cell lines JVM-2 (DSMZ), JVM-3 (DSMZ), or JVM-13 (ATCC) were inoculated into a 96-well culture plate for cell culture (Corning Inc.), and the anti-CD37 antibody-drug conjugate (1) diluted into varying concentrations was added thereto. At the same time therewith, a DMSO solution of ibrutinib (synthesized by a method well known to those skilled in the art), acalabrutinib (prepared by purifying a formulation obtained from AstraZeneca K.K. by a method well known to those skilled in the art) or zanubrutinib (prepared by purifying a formulation obtained from BeiGene, Ltd. by a method well known to those skilled in the art) (hereinafter, collectively referred to as "each BTK inhibitor") diluted into varying concentrations, or DMSO was added thereto (DMSO concentration: 0.1% or lower), and the cells were cultured for 6 days. During the culture period, the cells were cultured under conditions of 37°C and 5% $CO_2$ in all the cases. The cells thus cultured for 6 days were reacted using Celltiter-Glo 2.0 Cell Viability Assay (Promega Corp,) in accordance with the attached manual, followed by the measurement of the amount of luminescence from each well using a plate reader (Envision 2105 XCite multimode plate reader, PerkinElmer, Inc.). Cell growth inhibition rates were calculated according to the following formula from the measured amounts of luminescence of the groups supplemented with the varying concentrations of the anti-CD37 antibody-drug conjugate (1) and each BTK inhibitor- (T) and the DMSO-supplemented group (C).

$$\text{Cell growth inhibition rate (\%)} = (T / C) \times 100$$

The calculated cell growth inhibition rates were subjected to curve fitting with four-parameter logistic models after converting the concentrations of the anti-CD37 antibody-drug conjugate (1) to log values, to calculate respective 50% cell growth inhibition concentrations ($IC_{50}$) of the anti-CD37 antibody-drug conjugate (1) alone, each BTK inhibitor alone, and combinations of the varying concentrations of the anti-CD37 antibody-drug conjugate (1) and each BTK inhibitor.

From the calculated 50% cell growth inhibition concentrations (anti-CD37 antibody-drug conjugate (1) alone: D1, each BTK inhibitor alone: D2, anti-CD37 antibody-drug conjugate (1) in the combination: d1, each BTK inhibitor drug in the combination: d2), a combination index (CI) was calculated according to the following formula, and assessed as a synergistic effect with CI < 1, an additive effect with CI = 1, and an antagonizing effect with CI > 1 (see Adv. Enzyme Regul. 1984, P. 2227-55) .

$$CI = (d1 / D1) + (d2 / D2)$$

The results are shown in Table 8. The combination of the anti-CD37 antibody-drug conjugate (1) and each BTK inhibitor exhibited a synergistic effect with CI < 1 against all the cell lines.

[Table 8]

| | | JVM-2 | JVM-3 | JVM-13 |
|---|---|---|---|---|
| Anti-CD37 antibody-drug conjugate (1) | Concentration range | 0.0024-10 (μg/mL) | | |
| +Ibrutinib | Concentration range | 0.0024-10 (μM) | | |
| | D1 | 0.65 | 0.18 | 2.09 |
| | D2 | 1.19 | 0.27 | 0.58 |
| | d1 | 0.16 | 0.039 | 0.63 |
| | d2 | 0.32 | 0.097 | 0.16 |
| | Combination index | 0.51 | 0.58 | 0.58 |
| +Acalabrutinib | Concentratio, range | 0.024-100 (μM) | | |
| | D1 | 0.49 | 0.44 | 2.30 |
| | D2 | 25.80 | 16.62 | 7.15 |
| | d1 | 0.16 | 0.16 | 0.63 |
| | d2 | 3.79 | 5.74 | 3.39 |
| | Combination index | 0.47 | 0.70 | 0.75 |
| +Zanubrutinib | Concentration range | 0.012-50 (μM) | | |
| | D1 | 0.41 | 0.38 | 1.25 |
| | D2 | 6.86 | 17.91 | 2.83 |
| | d1 | 0.16 | 0.16 | 0.63 |
| | d2 | 0.53 | 1.86 | 0.21 |
| | Combination index | 0.46 | 0.52 | 0.58 |

Industrial Applicability

The present invention can be used in a pharmaceutical composition wherein an anti-CD37 antibody-drug conjugate and another drug are administered in combination, and/or a method of treatment comprising administering an anti-CD37 antibody-drug conjugate and another drug in combination to an individual, and the like.

Sequence Listing Free Text

SEQ ID NO: 1: Nucleotide sequence encoding a hmAb-L11 light chain
SEQ ID NO: 2: Full-length amino acid sequence of the hmAb-L11 light chain
SEQ ID NO: 3: Nucleotide sequence encoding a hmAb-H11 heavy chain
SEQ ID NO: 4: Full-length amino acid sequence of the hmAb-H11 heavy chain
SEQ ID NO: 5: Nucleotide sequence encoding a hmAb-H541 heavy chain
SEQ ID NO: 6: Full-length amino acid sequence of the hmAb-H541 heavy chain
SEQ ID NO: 7: Nucleotide sequence encoding a hmAb-H551 heavy chain
SEQ ID NO: 8: Full-length amino acid sequence of the hmAb-H551 heavy chain
SEQ ID NO: 9: Nucleotide sequence encoding a hmAb-H11a heavy chain
SEQ ID NO: 10: Full-length amino acid sequence of the hmAb-H11a heavy chain
SEQ ID NO: 11: Nucleotide fragment comprising a nucleotide sequence encoding a light chain signal sequence and a human κ light chain constant region
SEQ ID NO: 12: Nucleotide sequence encoding the variable region of the hmAb-L11 light chain
SEQ ID NO: 13: Nucleotide fragment comprising a nucleotide sequence encoding a heavy chain signal sequence and a human G1 heavy chain constant region
SEQ ID NO: 14: Nucleotide sequence encoding the variable region of the hmAb-H11 heavy chain
SEQ ID NO: 15: Nucleotide sequence encoding the variable region of the hmAb-H541 heavy chain

SEQ ID NO: 16: Nucleotide sequence encoding the variable region of the hmAb-H551 heavy chain
SEQ ID NO: 17: Nucleotide sequence encoding the variable region of the hmAb-H11a heavy chain
SEQ ID NO: 18: Amino acid sequence of human CD37
SEQ ID NO: 19: CDRL1 sequence of a humanized anti-CD37 antibody
SEQ ID NO: 20: CDRL2 sequence of the humanized anti-CD37 antibody
SEQ ID NO: 21: CDRL3 sequence of the humanized anti-CD37 antibody
SEQ ID NO: 22: CDRH1 sequence of the humanized anti-CD37 antibody
SEQ ID NO: 23: CDRH2 sequence of the humanized anti-CD37 antibody
SEQ ID NO: 24: CDRH3 sequence of the humanized anti-CD37 antibody

## Claims

1. A pharmaceutical composition comprising an anti-CD37 antibody-drug conjugate, wherein

   the anti-CD37 antibody-drug conjugate is administered in combination with one or more other drugs selected from the group consisting of a molecular targeting drug, a drug relating to R-CHOP therapy, a drug relating to R-CHP therapy, an alkylating agent and an immunomodulatory drug, and
   the anti-CD37 antibody-drug conjugate is an anti-CD37 antibody-drug conjugate in which a drug-linker represented by the formula and an anti-CD37 antibody are conjugated via a thioether bond:

   [Formula 1]

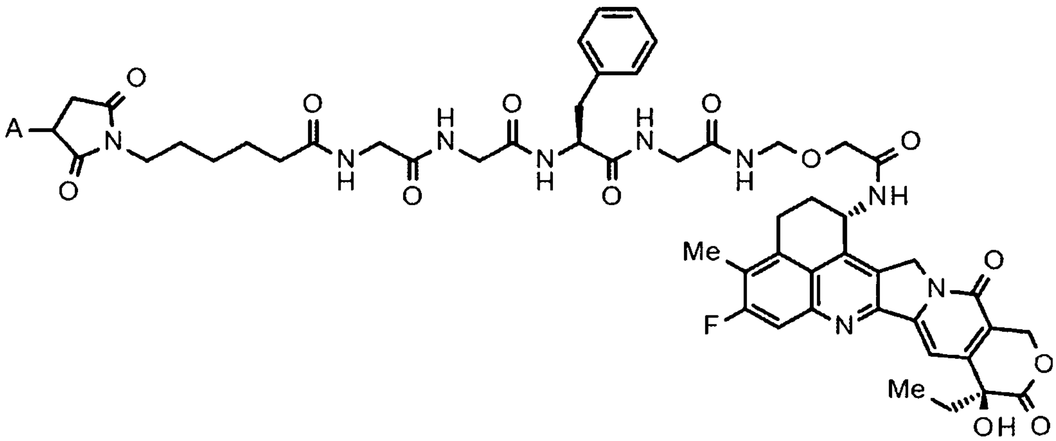

   Wherein A represents a connecting position to the anti-CD37 antibody.

2. The pharmaceutical composition according to claim 1, wherein the anti-CD37 antibody is an antibody comprising a heavy chain variable region and a light chain variable region in any one combination selected from the group consisting of the following combinations (a) to (d):

   (a) a light chain variable region consisting of the amino acid sequence at positions 21 to 128 in the light chain full-length amino acid sequence shown in SEQ ID NO: 2 and a heavy chain variable region consisting of the amino acid sequence at positions 20 to 138 in the heavy chain full-length amino acid sequence shown in SEQ ID NO: 4;
   (b) a light chain variable region consisting of the amino acid sequence at positions 21 to 128 in the light chain full-length amino acid sequence shown in SEQ ID NO: 2 and a heavy chain variable region consisting of the amino acid sequence at positions 20 to 138 in the heavy chain full-length amino acid sequence shown in SEQ ID NO: 6;
   (c) a light chain variable region consisting of the amino acid sequence at positions 21 to 128 in the light chain full-length amino acid sequence shown in SEQ ID NO: 2 and a heavy chain variable region consisting of the amino acid sequence at positions 20 to 138 in the heavy chain full-length amino acid sequence shown in SEQ ID NO: 8; and
   (d) a light chain variable region consisting of the amino acid sequence at positions 21 to 128 in the light chain full-length amino acid sequence shown in SEQ ID NO: 2 and a heavy chain variable region consisting of the amino acid sequence at positions 20 to 138 in the heavy chain full-length amino acid sequence shown in SEQ ID NO: 10.

3. The pharmaceutical composition according to claim 1 or 2, wherein the anti-CD37 antibody is an antibody comprising a heavy chain variable region and a light chain variable region in the following combination (a):

(a) a light chain variable region consisting of the amino acid sequence at positions 21 to 128 in the light chain full-length amino acid sequence shown in SEQ ID NO: 2 and a heavy chain variable region consisting of the amino acid sequence at positions 20 to 138 in the heavy chain full-length amino acid sequence shown in SEQ ID NO: 4.

4. The pharmaceutical composition according to claim 1 or 2, wherein the anti-CD37 antibody is an antibody comprising a heavy chain variable region and a light chain variable region in the following combination (b):
(b) a light chain variable region consisting of the amino acid sequence at positions 21 to 128 in the light chain full-length amino acid sequence shown in SEQ ID NO: 2 and a heavy chain variable region consisting of the amino acid sequence at positions 20 to 138 in the heavy chain full-length amino acid sequence shown in SEQ ID NO: 6.

5. The pharmaceutical composition according to claim 1 or 2, wherein the anti-CD37 antibody is an antibody comprising a heavy chain variable region and a light chain variable region in the following combination (c):
(c) a light chain variable region consisting of the amino acid sequence at positions 21 to 128 in the light chain full-length amino acid sequence shown in SEQ ID NO: 2 and a heavy chain variable region consisting of the amino acid sequence at positions 20 to 138 in the heavy chain full-length amino acid sequence shown in SEQ ID NO: 8.

6. The pharmaceutical composition according to claim 1 or 2, wherein the anti-CD37 antibody is an antibody comprising a heavy chain variable region and a light chain variable region in the following combination (d):
(d) a light chain variable region consisting of the amino acid sequence at positions 21 to 128 in the light chain full-length amino acid sequence shown in SEQ ID NO: 2 and a heavy chain variable region consisting of the amino acid sequence at positions 20 to 138 in the heavy chain full-length amino acid sequence shown in SEQ ID NO: 10.

7. The pharmaceutical composition according to claim 1 or 2, wherein the anti-CD37 antibody is an antibody comprising a heavy chain and a light chain in any one combination selected from the group consisting of the following combinations (e) to (h):

(e) a light chain consisting of the amino acid sequence at positions 21 to 234 in the light chain full-length amino acid sequence shown in SEQ ID NO: 2 and a heavy chain consisting of the amino acid sequence at positions 20 to 468 in the heavy chain full-length amino acid sequence shown in SEQ ID NO: 4;
(f) a light chain consisting of the amino acid sequence at positions 21 to 234 in the light chain full-length amino acid sequence shown in SEQ ID NO: 2 and a heavy chain consisting of the amino acid sequence at positions 20 to 468 in the heavy chain full-length amino acid sequence shown in SEQ ID NO: 6;
(g) a light chain consisting of the amino acid sequence at positions 21 to 234 in the light chain full-length amino acid sequence shown in SEQ ID NO: 2 and a heavy chain consisting of the amino acid sequence at positions 20 to 468 in the heavy chain full-length amino acid sequence shown in SEQ ID NO: 8; and
(h) a light chain consisting of the amino acid sequence at positions 21 to 234 in the light chain full-length amino acid sequence shown in SEQ ID NO: 2 and a heavy chain consisting of the amino acid sequence at positions 20 to 468 in the heavy chain full-length amino acid sequence shown in SEQ ID NO: 10.

8. The pharmaceutical composition according to any one of claims 1 to 3 and 7, wherein the anti-CD37 antibody is an antibody comprising a heavy chain and a light chain in the following combination (e):
(e) a light chain consisting of the amino acid sequence at positions 21 to 234 in the light chain full-length amino acid sequence shown in SEQ ID NO: 2 and a heavy chain consisting of the amino acid sequence at positions 20 to 468 in the heavy chain full-length amino acid sequence shown in SEQ ID NO: 4.

9. The pharmaceutical composition according to any one of claims 1, 2, 4 and 7, wherein the anti-CD37 antibody is an antibody comprising a heavy chain and a light chain in the following combination (f):
(f) a light chain consisting of the amino acid sequence at positions 21 to 234 in the light chain full-length amino acid sequence shown in SEQ ID NO: 2 and a heavy chain consisting of the amino acid sequence at positions 20 to 468 in the heavy chain full-length amino acid sequence shown in SEQ ID NO: 6.

10. The pharmaceutical composition according to any one of claims 1, 2, 5 and 7, wherein the anti-CD37 antibody is an antibody comprising a heavy chain and a light chain in the following combination (g):
(g) a light chain consisting of the amino acid sequence at positions 21 to 234 in the light chain full-length amino acid sequence shown in SEQ ID NO: 2 and a heavy chain consisting of the amino acid sequence at positions 20 to 468 in

the heavy chain full-length amino acid sequence shown in SEQ ID NO: 8.

**11.** The pharmaceutical composition according to any one of claims 1, 2, 6 and 7, wherein the anti-CD37 antibody is an antibody comprising a heavy chain and a light chain in the following combination (h):
(h) a light chain consisting of the amino acid sequence at positions 21 to 234 in the light chain full-length amino acid sequence shown in SEQ ID NO: 2 and a heavy chain consisting of the amino acid sequence at positions 20 to 468 in the heavy chain full-length amino acid sequence shown in SEQ ID NO: 10.

**12.** The pharmaceutical composition according to any one of claims 1 to 11, wherein a lysine residue at the carboxyl terminus of the heavy chain of the anti-CD37 antibody is deleted.

**13.** The pharmaceutical composition according to any one of claims 1 to 12, wherein the average number of units of the drug-linker conjugated per antibody in the anti-CD37 antibody-drug conjugate is in the range of from 2 to 8.

**14.** The pharmaceutical composition according to any one of claims 1 to 13, wherein the average number of units of the drug-linker conjugated per antibody in the anti-CD37 antibody-drug conjugate is in the range of from 7 to 8.

**15.** The pharmaceutical composition according to any one of claims 1 to 14, wherein the average number of units of the drug-linker conjugated per antibody in the anti-CD37 antibody-drug conjugate is approximately 8.

**16.** The pharmaceutical composition according to any one of claims 1 to 15, wherein the molecular targeting drug is one or more drugs selected from the group consisting of an anti-CD20 antibody, an anti-CD19 antibody, a BCL-2 inhibitor and a BTK inhibitor.

**17.** The pharmaceutical composition according to claim 16, wherein the anti-CD20 antibody is rituximab.

**18.** The pharmaceutical composition according to claim 16, wherein the anti-CD20 antibody is obinutuzumab.

**19.** The pharmaceutical composition according to any one of claims 16 to 18, wherein the anti-CD19 antibody is tafasitamab.

**20.** The pharmaceutical composition according to any one of claims 16 to 19, wherein the BCL-2 inhibitor is venetoclax or a pharmacologically acceptable salt thereof.

**21.** The pharmaceutical composition according to any one of claims 16 to 20, wherein the BTK inhibitor is ibrutinib or a pharmacologically acceptable salt thereof.

**22.** The pharmaceutical composition according to any one of claims 16 to 20, wherein the BTK inhibitor is acalabrutinib or a pharmacologically acceptable salt thereof.

**23.** The pharmaceutical composition according to any one of claims 16 to 20, wherein the BTK inhibitor is zanubrutinib or a pharmacologically acceptable salt thereof.

**24.** The pharmaceutical composition according to any one of claims 1 to 23, wherein the alkylating agent is bendamustine or a pharmacologically acceptable salt thereof.

**25.** The pharmaceutical composition according to any one of claims 1 to 24, wherein the immunomodulatory drug is lenalidomide or a pharmacologically acceptable salt thereof.

**26.** The pharmaceutical composition according to any one of claims 1 to 15, wherein the other drug is a combination of rituximab and bendamustine or a pharmacologically acceptable salt thereof.

**27.** The pharmaceutical composition according to any one of claims 1 to 15, wherein the other drug is a combination of tafasitamab and lenalidomide or a pharmacologically acceptable salt thereof.

**28.** The pharmaceutical composition according to any one of claims 1 to 15, wherein the other drug is a combination of rituximab and lenalidomide or a pharmacologically acceptable salt thereof.

**29.** The pharmaceutical composition according to any one of claims 1 to 15, wherein the other drug is a combination of rituximab and venetoclax or a pharmacologically acceptable salt thereof.

**30.** The pharmaceutical composition according to any one of claims 1 to 15, wherein the other drug is a drug relating to R-CHOP therapy.

**31.** The pharmaceutical composition according to any one of claims 1 to 15, wherein the other drug is a drug relating to R-CHP therapy.

**32.** The pharmaceutical composition according to any one of claims 1 to 31, wherein the anti-CD37 antibody-drug conjugate and the one or more other drugs selected from the group consisting of a molecular targeting drug, a drug relating to R-CHOP therapy, a drug relating to R-CHP therapy, an alkylating agent and an immunomodulatory drug are separately contained as active ingredients in different formulations and administered at the same time or different times.

**33.** The pharmaceutical composition according to any one of claims 1 to 31, wherein the anti-CD37 antibody-drug conjugate and the one or more other drugs selected from the group consisting of a molecular targeting drug, a drug relating to R-CHOP therapy, a drug relating to R-CHP therapy, an alkylating agent and an immunomodulatory drug are contained as active ingredients in one formulation.

**34.** The pharmaceutical composition according to any one of claims 1 to 33 for the treatment of at least one disease selected from the group consisting of diffuse large B-cell lymphoma, follicular lymphoma, mantle cell lymphoma, marginal zone lymphoma, Burkitt's lymphoma, chronic lymphocytic leukemia, peripheral T-cell lymphoma, cutaneous T-cell lymphoma, myelodysplastic syndrome and acute myeloid leukemia.

**35.** The pharmaceutical composition according to claim 34 for the treatment of diffuse large B-cell lymphoma.

**36.** The pharmaceutical composition according to claim 34 for the treatment of chronic lymphocytic leukemia.

**37.** The pharmaceutical composition according to claim 34 for the treatment of follicular lymphoma.

**38.** A pharmaceutical composition comprising an anti-CD37 antibody-drug conjugate, wherein

the anti-CD37 antibody-drug conjugate is administered in combination with one or more other drugs selected from the group consisting of a molecular targeting drug, a drug relating to R-CHOP therapy, a drug relating to R-CHP therapy, an alkylating agent and an immunomodulatory drug, and
the anti-CD37 antibody-drug conjugate is an anti-CD37 antibody-drug conjugate represented by the formula:

[Formula 2]

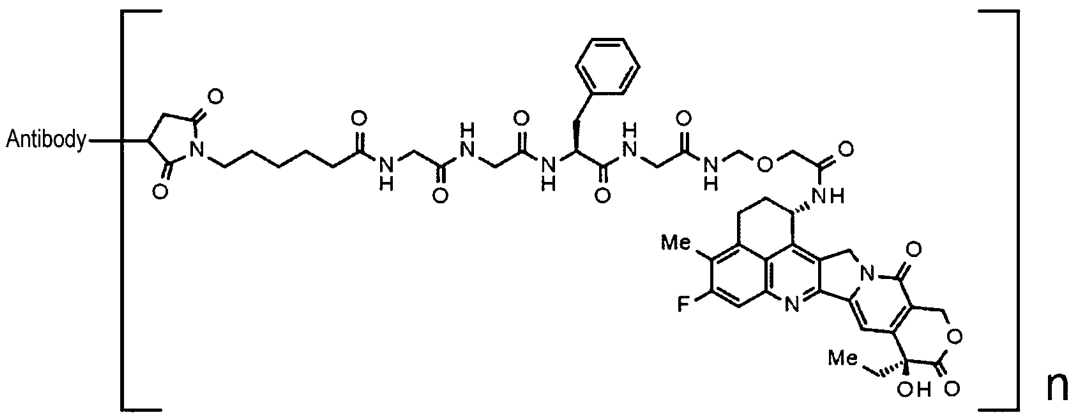

wherein Antibody represents an anti-CD37 antibody, the drug linker is conjugated to the antibody via a thioether bond, and n represents the average number of units of the drug-linker conjugated per antibody.

**39.** The pharmaceutical composition according to claim 38, wherein the anti-CD37 antibody is an antibody comprising a heavy chain and a light chain in the following combination (e):
(e) a light chain consisting of the amino acid sequence at positions 21 to 234 in the light chain full-length amino acid sequence shown in SEQ ID NO: 2 and a heavy chain consisting of the amino acid sequence at positions 20 to 468 in the heavy chain full-length amino acid sequence shown in SEQ ID NO: 4.

**40.** The pharmaceutical composition according to claim 38, wherein the anti-CD37 antibody is an antibody comprising a heavy chain and a light chain in the following combination (f):
(f) a light chain consisting of the amino acid sequence at positions 21 to 234 in the light chain full-length amino acid sequence shown in SEQ ID NO: 2 and a heavy chain consisting of the amino acid sequence at positions 20 to 468 in the heavy chain full-length amino acid sequence shown in SEQ ID NO: 6.

**41.** The pharmaceutical composition according to claim 38, wherein the anti-CD37 antibody is an antibody comprising a heavy chain and a light chain in the following combination (g):
(g) a light chain consisting of the amino acid sequence at positions 21 to 234 in the light chain full-length amino acid sequence shown in SEQ ID NO: 2 and a heavy chain consisting of the amino acid sequence at positions 20 to 468 in the heavy chain full-length amino acid sequence shown in SEQ ID NO: 8.

**42.** The pharmaceutical composition according to claim 38, wherein the anti-CD37 antibody is an antibody comprising a heavy chain and a light chain in the following combination (h):
(h) a light chain consisting of the amino acid sequence at positions 21 to 234 in the light chain full-length amino acid sequence shown in SEQ ID NO: 2 and a heavy chain consisting of the amino acid sequence at positions 20 to 468 in the heavy chain full-length amino acid sequence shown in SEQ ID NO: 10.

**43.** The pharmaceutical composition according to any one of claims 38 to 42, wherein a lysine residue at the carboxyl terminus of the heavy chain of the anti-CD37 antibody is deleted.

**44.** The pharmaceutical composition according to any one of claims 38 to 43, wherein the average number of units of the drug-linker conjugated per antibody in the anti-CD37 antibody-drug conjugate is in the range of from 7 to 8.

**45.** The pharmaceutical composition according to any one of claims 38 to 44, wherein the average number of units of the drug-linker conjugated per antibody in the anti-CD37 antibody-drug conjugate is approximately 8.

**46.** The pharmaceutical composition according to any one of claims 38 to 45, wherein the molecular targeting drug is one or more drugs selected from the group consisting of an anti-CD20 antibody, an anti-CD19 antibody, a BCL-2 inhibitor and a BTK inhibitor.

**47.** The pharmaceutical composition according to claim 46, wherein the anti-CD20 antibody is rituximab.

**48.** The pharmaceutical composition according to claim 46, wherein the anti-CD20 antibody is obinutuzumab.

**49.** The pharmaceutical composition according to any one of claims 46 to 48, wherein the anti-CD19 antibody is tafasitamab.

**50.** The pharmaceutical composition according to any one of claims 46 to 49, wherein the BCL-2 inhibitor is venetoclax or a pharmacologically acceptable salt thereof.

**51.** The pharmaceutical composition according to any one of claims 46 to 50, wherein the BTK inhibitor is ibrutinib or a pharmacologically acceptable salt thereof.

**52.** The pharmaceutical composition according to any one of claims 46 to 50, wherein the BTK inhibitor is acalabrutinib or a pharmacologically acceptable salt thereof.

**53.** The pharmaceutical composition according to any one of claims 46 to 50, wherein the BTK inhibitor is zanubrutinib or a pharmacologically acceptable salt thereof.

**54.** The pharmaceutical composition according to any one of claims 38 to 53, wherein the alkylating agent is bendamustine or a pharmacologically acceptable salt thereof.

**55.** The pharmaceutical composition according to any one of claims 38 to 54, wherein the immunomodulatory drug is lenalidomide or a pharmacologically acceptable salt thereof.

**56.** The pharmaceutical composition according to any one of claims 38 to 45, wherein the other drug is a combination of rituximab and bendamustine or a pharmacologically acceptable salt thereof.

**57.** The pharmaceutical composition according to any one of claims 38 to 45, wherein the other drug is a combination of tafasitamab and lenalidomide or a pharmacologically acceptable salt thereof.

**58.** The pharmaceutical composition according to any one of claims 38 to 45, wherein the other drug is a combination of rituximab and lenalidomide or a pharmacologically acceptable salt thereof.

**59.** The pharmaceutical composition according to any one of claims 38 to 45, wherein the other drug is a combination of rituximab and venetoclax or a pharmacologically acceptable salt thereof.

**60.** The pharmaceutical composition according to any one of claims 38 to 45, wherein the other drug is a drug relating to R-CHOP therapy.

**61.** The pharmaceutical composition according to any one of claims 38 to 45, wherein the other drug is a drug relating to R-CHP therapy.

**62.** The pharmaceutical composition according to any one of claims 38 to 61, wherein the anti-CD37 antibody-drug conjugate and the one or more other drugs selected from the group consisting of a molecular targeting drug, a drug relating to R-CHOP therapy, a drug relating to R-CHP therapy, an alkylating agent and an immunomodulatory drug are separately contained as active ingredients in different formulations and administered at the same time or different times.

**63.** The pharmaceutical composition according to any one of claims 38 to 61, wherein the anti-CD37 antibody-drug conjugate and the one or more other drugs selected from the group consisting of a molecular targeting drug, a drug relating to R-CHOP therapy, a drug relating to R-CHP therapy, an alkylating agent and an immunomodulatory drug are contained as active ingredients in one formulation.

**64.** The pharmaceutical composition according to any one of claims 38 to 63 for the treatment of at least one disease selected from the group consisting of diffuse large B-cell lymphoma, follicular lymphoma, mantle cell lymphoma, marginal zone lymphoma, Burkitt's lymphoma, chronic lymphocytic leukemia, peripheral T-cell lymphoma, cutaneous T-cell lymphoma, myelodysplastic syndrome and acute myeloid leukemia.

**65.** The pharmaceutical composition according to claim 64 for the treatment of diffuse large B-cell lymphoma.

**66.** The pharmaceutical composition according to claim 64 for the treatment of chronic lymphocytic leukemia.

**67.** The pharmaceutical composition according to claim 64 for the treatment of follicular lymphoma.

**68.** A method of treatment comprising administering an anti-CD37 antibody-drug conjugate and one or more other drugs selected from the group consisting of a molecular targeting drug, a drug relating to R-CHOP therapy, a drug relating to R-CHP therapy, an alkylating agent and an immunomodulatory drug in combination to an individual in need of treatment, wherein the anti-CD37 antibody-drug conjugate and the other drugs are described in any one of claims 1 to 31 and claims 38 to 61.

**69.** The method of treatment according to claim 68,
wherein the anti-CD37 antibody-drug conjugate and the one or more other drugs selected from the group consisting of a molecular targeting drug, a drug relating to R-CHOP therapy, a drug relating to R-CHP therapy, an alkylating agent and an immunomodulatory drug are separately contained as active ingredients in different formulations and administered at the same time or different times.

**70.** The method of treatment according to claim 68,
wherein the anti-CD37 antibody-drug conjugate and the one or more other drugs selected from the group consisting of a molecular targeting drug, a drug relating to R-CHOP therapy, a drug relating to R-CHP therapy, an alkylating agent

and an immunomodulatory drug are contained as active ingredients in one formulation.

**71.** The method of treatment according to any one of claims 68 to 70 for the treatment of at least one disease selected from the group consisting of diseases described in claim 34 or 64.

**72.** Use of an anti-CD37 antibody-drug conjugate for the production of a medicament for the treatment of a disease by administration in combination with one or more other drugs selected from the group consisting of a molecular targeting drug, a drug relating to R-CHOP therapy, a drug relating to R-CHP therapy, an alkylating agent and an immunomodulatory drug, wherein the anti-CD37 antibody-drug conjugate and the other drugs are described in any one of claims 1 to 31 and claims 38 to 61.

**73.** The use according to claim 72, wherein the anti-CD37 antibody-drug conjugate and the one or more other drugs selected from the group consisting of a molecular targeting drug, a drug relating to R-CHOP therapy, a drug relating to R-CHP therapy, an alkylating agent and an immunomodulatory drug are separately contained as active ingredients in different formulations and administered at the same time or different times.

**74.** The use according to claim 72, wherein the anti-CD37 antibody-drug conjugate and the one or more other drugs selected from the group consisting of a molecular targeting drug, a drug relating to R-CHOP therapy, a drug relating to R-CHP therapy, an alkylating agent and an immunomodulatory drug are contained as active ingredients in one formulation.

**75.** The use according to any one of claims 72 to 74 for the treatment of at least one disease selected from the group consisting of diseases described in claim 34 or 64.

**76.** A pharmaceutical product comprising an anti-CD37 antibody-drug conjugate and one or more other drugs selected from the group consisting of a molecular targeting drug, a drug relating to R-CHOP therapy, a drug relating to R-CHP therapy, an alkylating agent and an immunomodulatory drug for administration in combination, wherein the anti-CD37 antibody-drug conjugate and the other drugs are described in any one of claims 1 to 31 and claims 38 to 61.

**77.** The pharmaceutical product according to claim 76,
wherein the anti-CD37 antibody-drug conjugate and the one or more other drugs selected from the group consisting of a molecular targeting drug, a drug relating to R-CHOP therapy, a drug relating to R-CHP therapy, an alkylating agent and an immunomodulatory drug are separately contained as active ingredients in different formulations and administered at the same time or different times.

**78.** The pharmaceutical product according to claim 76,
wherein the anti-CD37 antibody-drug conjugate and the one or more other drugs selected from the group consisting of a molecular targeting drug, a drug relating to R-CHOP therapy, a drug relating to R-CHP therapy, an alkylating agent and an immunomodulatory drug are contained as active ingredients in one formulation.

**79.** The pharmaceutical product according to any one of claims 76 to 78 for the treatment of at least one disease selected from the group consisting of diseases described in claim 34 or 64.

**80.** Use of an anti-CD37 antibody-drug conjugate in combination with one or more other drugs selected from the group consisting of a molecular targeting drug, a drug relating to R-CHOP therapy, a drug relating to R-CHP therapy, an alkylating agent and an immunomodulatory drug for the treatment of a disease, wherein the anti-CD37 antibody-drug conjugate and the other drugs are described in any one of claims 1 to 31 and claims 38 to 61.

**81.** The use according to claim 80, wherein the anti-CD37 antibody-drug conjugate and the one or more other drugs selected from the group consisting of a molecular targeting drug, a drug relating to R-CHOP therapy, a drug relating to R-CHP therapy, an alkylating agent and an immunomodulatory drug are separately contained as active ingredients in different formulations and administered at the same time or different times.

**82.** The use according to claim 80, wherein the anti-CD37 antibody-drug conjugate and the one or more other drugs selected from the group consisting of a molecular targeting drug, a drug relating to R-CHOP therapy, a drug relating to R-CHP therapy, an alkylating agent and an immunomodulatory drug are contained as active ingredients in one formulation.

**83.** The use according to any one of claims 80 to 82 for the treatment of at least one disease selected from the group consisting of diseases described in claim 34 or 64.

# Fig. 1

SEQ ID NO: 1: Nucleotide sequence encoding hmAb-L11 light chain

ATGGTGCTGCAGACCCAGGTGTTCATCTCCCTGCTGCTGTGGATCTCCGGCGCGTACGGCGAT
ATCCAGATGACACAGAGCCCTAGCAGCCTGTCTGCCAGCGTGGGAGACAGAGTGACCATCACA
TGCAAGGCCAGCCAGGATGTGTCCACCGCCGTGGATTGGTATCAGCAGAAGCCTGGCAAGGCC
CCTAAGCTGCTGATCAACTGGGCCAGCACAAGACACACAGGCGTGCCCAGCAGATTTTCTGGC
AGCGGCTCTGGCACCGACTTCACCCTGACCATATCTAGCCTGCAGCCTGAGGACTTCGCCACC
TACTACTGCAGACAGCACTACAGCACCCCTTTCACCTTTGGCCAGGGCACCAAGGTGGAAATC
AAGCGTACGGTGGCCGCCCCCTCCGTGTTCATCTTCCCCCCCTCCGACGAGCAGCTGAAGTCC
GGCACCGCCTCCGTGGTGTGCCTGCTGAATAACTTCTACCCCAGAGAGGCCAAGGTGCAGTGG
AAGGTGGACAACGCCCTGCAGTCCGGGAACTCCCAGGAGAGCGTGACCGAGCAGGACAGCAA
GGACAGCACCTACAGCCTGAGCAGCACCCTGACCCTGAGCAAAGCCGACTACGAGAAGCACA
AGGTGTACGCCTGCGAGGTGACCCACCAGGGCCTGAGCTCCCCCGTCACCAAGAGCTTCAACA
GGGGGGAGTGT

Signal sequence (1－60), Light chain variable region (61－384), Light chain constant region (385－702)

# Fig. 2

SEQ ID NO: 2: Full-length amino acid sequence of hmAb-L11 light chain

MVLQTQVFISLLLWISGAYGDIQMTQSPSSLSASVGDRVTITCKASQDVSTAVDWYQQKPGKAPK
LLINWASTRHTGVPSRFSGSGSGTDFTLTISSLQPEDFATYYCRQHYSTPFTFGQGTKVEIKRTVA
APSVFIFPPSDEQLKSGTASVVCLLNNFYPREAKVQWKVDNALQSGNSQESVTEQDSKDSTYSL
SSTLTLSKADYEKHKVYACEVTHQGLSSPVTKSFNRGEC

Signal sequence (1－20), Light chain variable region (21－128), Light chain constant region (129－234)

# Fig. 3

SEQ ID NO: 3: Nucleotide sequence encoding hmAb-H11 heavy chain

ATGAAACACCTGTGGTTCTTCCTCCTGCTGGTGGCAGCTCCCAGATGGGTGCTGAGCGAAGTT
CAGCTGGTTCAGTCTGGCGCCGAAGTGAAGAAACCTGGCGCCTCTGTGAAGGTGTCCTGCAA
GGCCAGCGGCTACAGCTTCACCGACTACAACATGTACTGGGTCCGACAGGCCCCTGGCCAGTC
TCTTGAGTGGATGGGCTACATCGACCCCTACAACGGCGACACCACCTACAACCAGAAATTCCA
GGGCAGAGTGACCATCACCGCCGACACCTCTACAAGCACCGCCTACATGGAACTGAGCAGCCT
GAGAAGCGAGGACACCGCCGTGTACTACTGCGCCAGATCTCCTTACGGCCACTACGCCATGGA
TTACTGGGGCCAGGGAACCCTGGTCACAGTTAGCTCAGCCTCCACCAAGGGCCCAAGCGTCTT
CCCCCTGGCACCCTCCTCCAAGAGCACCTCTGGCGGCACAGCCGCCCTGGGCTGCCTGGTCAA
GGACTACTTCCCCGAACCCGTGACCGTGAGCTGGAACTCAGGCGCCCTGACCAGCGGCGTGC
ACACCTTCCCCGCTGTCCTGCAGTCCTCAGGACTCTACTCCCTCAGCAGCGTGGTGACCGTGC
CCTCCAGCAGCTTGGGCACCCAGACCTACATCTGCAACGTGAATCACAAGCCCAGCAACACCA
AGGTGGACAAGAAGGTTGAGCCCAAATCTTGTGACAAAACTCACACATGCCCACCCTGCCCAG
CACCTGAACTCCTGGGGGGACCCTCAGTCTTCCTCTTCCCCCCAAAACCCAAGGACACCCTCA
TGATCTCCCGGACCCCTGAGGTCACATGCGTGGTGGTGGACGTGAGCCACGAAGACCCTGAG
GTCAAGTTCAACTGGTACGTGGACGGCGTGGAGGTGCATAATGCCAAGACAAAGCCCCGGGA
GGAGCAGTACAACAGCACGTACCGGGTGGTCAGCGTCCTCACCGTCCTGCACCAGGACTGGCT
GAATGGCAAGGAGTACAAGTGCAAGGTCTCCAACAAAGCCCTCCCAGCCCCCATCGAGAAAAC
CATCTCCAAAGCCAAAGGCCAGCCCCGGGAACCACAGGTGTACACCCTGCCCCCATCCCGGGA
GGAGATGACCAAGAACCAGGTCAGCCTGACCTGCCTGGTCAAAGGCTTCTATCCCAGCGACAT
CGCCGTGGAGTGGGAGAGCAATGGCCAGCCCGAGAACAACTACAAGACCACCCCTCCCGTGC
TGGACTCCGACGGCTCCTTCTTCCTCTACAGCAAGCTCACCGTGGACAAGAGCAGGTGGCAGC
AGGGCAACGTCTTCTCATGCTCCGTGATGCATGAGGCTCTGCACAACCACTACACCCAGAAGA
GCCTCTCCCTGTCTCCCGGCAAA

Signal sequence (1 − 57), Heavy chain variable region (58 − 414), Heavy chain constant region (415 − 1404)

# Fig. 4

SEQ ID NO: 4: Full-length amino acid sequence of hmAb-H11 heavy chain

MKHLWFFLLLVAAPRWVLSEVQLVQSGAEVKKPGASVKVSCKASGYSFTDYNMYWVRQAPGQS
LEWMGYIDPYNGDTTYNQKFQGRVTITADTSTSTAYMELSSLRSEDTAVYYCARSPYGHYAMDY
WGQGTLVTVSSASTKGPSVFPLAPSSKSTSGGTAALGCLVKDYFPEPVTVSWNSGALTSGVHTFP
AVLQSSGLYSLSSVVTVPSSSLGTQTYICNVNHKPSNTKVDKKVEPKSCDKTHTCPPCPAPELLG
GPSVFLFPPKPKDTLMISRTPEVTCVVVDVSHEDPEVKFNWYVDGVEVHNAKTKPREEQYNST
YRVVSVLTVLHQDWLNGKEYKCKVSNKALPAPIEKTISKAKGQPREPQVYTLPPSREEMTKNQV
SLTCLVKGFYPSDIAVEWESNGQPENNYKTTPPVLDSDGSFFLYSKLTVDKSRWQQGNVFSCSV
MHEALHNHYTQKSLSLSPGK

Signal sequence (1 − 19), Heavy chain variable region (20 − 138), Heavy chain constant region (139 − 468)

# Fig. 5

SEQ ID NO: 5: Nucleotide sequence encoding hmAb-H541 heavy chain

ATGAAACACCTGTGGTTCTTCCTCCTGCTGGTGGCAGCTCCCAGATGGGTGCTGAGCGAAGTG
CAGCTGGTTCAGTCTGGCGCCGAAGTGAAAAAGCCTGGCGCCTCTGTGAAGGTGTCCTGCAA
GGCCAGCGGCTACAGCTTCACCGACTACAACATGTACTGGGTCCGACAGGCCCCTGGCCAGTC
TCTTGAGTGGATGGGCTACATCGACCCCTACAACGGCGACACCACCTACAACCAGAAATTCCA
GGGCAGAGTGACCATGACCAGAGACACCAGCATCAGCACCGCCTACATGGAACTGAGCCGGCT
GAGATCCGATGACACCGCCGTGTACTACTGCGCCAGATCTCCTTACGGCCACTACGCCATGGAT
TACTGGGGCCAGGGCACCACAGTGACAGTTAGCTCAGCCTCCACCAAGGGCCCAAGCGTCTTC
CCCCTGGCACCCTCCTCCAAGAGCACCTCTGGCGGCACAGCCGCCCTGGGCTGCCTGGTCAA
GGACTACTTCCCCGAACCCGTGACCGTGAGCTGGAACTCAGGCGCCCTGACCAGCGGCGTGC
ACACCTTCCCCGCTGTCCTGCAGTCCTCAGGACTCTACTCCCTCAGCAGCGTGGTGACCGTGC
CCTCCAGCAGCTTGGGCACCCAGACCTACATCTGCAACGTGAATCACAAGCCCAGCAACACCA
AGGTGGACAAGAAGGTTGAGCCCAAATCTTGTGACAAAACTCACACATGCCCACCCTGCCCAG
CACCTGAACTCCTGGGGGGACCCTCAGTCTTCCTCTTCCCCCCAAAACCCAAGGACACCCTCA
TGATCTCCCGGACCCCTGAGGTCACATGCGTGGTGGTGGACGTGAGCCACGAAGACCCTGAG
GTCAAGTTCAACTGGTACGTGGACGGCGTGGAGGTGCATAATGCCAAGACAAAGCCCCGGGA
GGAGCAGTACAACAGCACGTACCGGGTGGTCAGCGTCCTCACCGTCCTGCACCAGGACTGGCT
GAATGGCAAGGAGTACAAGTGCAAGGTCTCCAACAAAGCCCTCCCAGCCCCCATCGAGAAAAC
CATCTCCAAAGCCAAAGGCCAGCCCCGGGAACCACAGGTGTACACCCTGCCCCCATCCCGGGA
GGAGATGACCAAGAACCAGGTCAGCCTGACCTGCCTGGTCAAAGGCTTCTATCCCAGCGACAT
CGCCGTGGAGTGGGAGAGCAATGGCCAGCCCGAGAACAACTACAAGACCACCCCTCCCGTGC
TGGACTCCGACGGCTCCTTCTTCCTCTACAGCAAGCTCACCGTGGACAAGAGCAGGTGGCAGC
AGGGCAACGTCTTCTCATGCTCCGTGATGCATGAGGCTCTGCACAACCACTACACCCAGAAGA
GCCTCTCCCTGTCTCCCGGCAAA

Signal sequence (1−57), Heavy chain variable region (58−414), Heavy chain constant region (415−1404)

# Fig. 6

SEQ ID NO: 6: Full-length amino acid sequence of hmAb-H541 heavy chain

MKHLWFFLLLVAAPRWVLSEVQLVQSGAEVKKPGASVKVSCKASGYSFTDYNMYWVRQAPGQS
LEWMGYIDPYNGDTTYNQKFQGRVTMTRDTSISTAYMELSRLRSDDTAVYYCARSPYGHYAMD
YWGQGTTVTVSSASTKGPSVFPLAPSSKSTSGGTAALGCLVKDYFPEPVTVSWNSGALTSGVHT
FPAVLQSSGLYSLSSVVTVPSSSLGTQTYICNVNHKPSNTKVDKKVEPKSCDKTHTCPPCPAPEL
LGGPSVFLFPPKPKDTLMISRTPEVTCVVVDVSHEDPEVKFNWYVDGVEVHNAKTKPREEQYN
STYRVVSVLTVLHQDWLNGKEYKCKVSNKALPAPIEKTISKAKGQPREPQVYTLPPSREEMTKN
QVSLTCLVKGFYPSDIAVEWESNGQPENNYKTTPPVLDSDGSFFLYSKLTVDKSRWQQGNVFSC
SVMHEALHNHYTQKSLSLSPGK

Signal sequence (1−19), Heavy chain variable region (20−138), Heavy chain constant region (139−468)

# Fig. 7

SEQ ID NO: 7: Nucleotide sequence encoding hmAb-H551 heavy chain

ATGAAACACCTGTGGTTCTTCCTCCTGCTGGTGGCAGCTCCCAGATGGGTGCTGAGCGAAGTG
CAGCTGGTTCAGTCTGGCGCCGAAGTGAAAAAGCCTGGCGCCTCTGTGAAGGTGTCCTGCAA
GGCCAGCGGCTACAGCTTCACCGACTACAACATGTACTGGGTCCGACAGGCCCCTGGCCAGTC
TCTTGAGTGGATGGGCTACATCGACCCCTACAACGGCGACACCACCTACAACCAGAAATTCCA
GGGCAGAGTGACCATGACCAGAGACACCAGCAGCAGCACCGCCTACATGGAACTGAGCAGAC
TGAGAAGCGACGACACCGCCGTGTACTACTGCGCCAGATCTCCTTACGGCCACTACGCCATGG
ATTACTGGGGCCAGGGCACCACAGTGACAGTTAGCTCAGCCTCCACCAAGGGCCCAAGCGTCT
TCCCCCTGGCACCCTCCTCCAAGAGCACCTCTGGCGGCACAGCCGCCCTGGGCTGCCTGGTCA
AGGACTACTTCCCCGAACCCGTGACCGTGAGCTGGAACTCAGGCGCCCTGACCAGCGGCGTG
CACACCTTCCCCGCTGTCCTGCAGTCCTCAGGACTCTACTCCCTCAGCAGCGTGGTGACCGTG
CCCTCCAGCAGCTTGGGCACCCAGACCTACATCTGCAACGTGAATCACAAGCCCAGCAACACC
AAGGTGGACAAGAAGGTTGAGCCCAAATCTTGTGACAAAACTCACACATGCCCACCCTGCCCA
GCACCTGAACTCCTGGGGGGACCCTCAGTCTTCCTCTTCCCCCCAAAACCCAAGGACACCCTC
ATGATCTCCCGGACCCCTGAGGTCACATGCGTGGTGGTGGACGTGAGCCACGAAGACCCTGAG
GTCAAGTTCAACTGGTACGTGGACGGCGTGGAGGTGCATAATGCCAAGACAAAGCCCCGGGA
GGAGCAGTACAACAGCACGTACCGGGTGGTCAGCGTCCTCACCGTCCTGCACCAGGACTGGCT
GAATGGCAAGGAGTACAAGTGCAAGGTCTCCAACAAAGCCCTCCCAGCCCCCATCGAGAAAAC
CATCTCCAAAGCCAAAGGCCAGCCCCGGGAACCACAGGTGTACACCCTGCCCCCATCCCGGGA
GGAGATGACCAAGAACCAGGTCAGCCTGACCTGCCTGGTCAAAGGCTTCTATCCCAGCGACAT
CGCCGTGGAGTGGGAGAGCAATGGCCAGCCCGAGAACAACTACAAGACCACCCCTCCCGTGC
TGGACTCCGACGGCTCCTTCTTCCTCTACAGCAAGCTCACCGTGGACAAGAGCAGGTGGCAGC
AGGGCAACGTCTTCTCATGCTCCGTGATGCATGAGGCTCTGCACAACCACTACACCCAGAAGA
GCCTCTCCCTGTCTCCCGGCAAA

Signal sequence (1－57), Heavy chain variable region (58－414), Heavy chain constant region (415－1404)

# Fig. 8

SEQ ID NO: 8: Full-length amino acid sequence of hmAb-H551 heavy chain

MKHLWFFLLLVAAPRWVLSEVQLVQSGAEVKKPGASVKVSCKASGYSFTDYNMYWVRQAPGQS
LEWMGYIDPYNGDTTYNQKFQGRVTMTRDTSSSTAYMELSRLRSDDTAVYYCARSPYGHYAMD
YWGQGTTVTVSSASTKGPSVFPLAPSSKSTSGGTAALGCLVKDYFPEPVTVSWNSGALTSGVHT
FPAVLQSSGLYSLSSVVTVPSSSLGTQTYICNVNHKPSNTKVDKKVEPKSCDKTHTCPPCPAPEL
LGGPSVFLFPPKPKDTLMISRTPEVTCVVVDVSHEDPEVKFNWYVDGVEVHNAKTKPREEQYN
STYRVVSVLTVLHQDWLNGKEYKCKVSNKALPAPIEKTISKAKGQPREPQVYTLPPSREEMTKN
QVSLTCLVKGFYPSDIAVEWESNGQPENNYKTTPPVLDSDGSFFLYSKLTVDKSRWQQGNVFSC
SVMHEALHNHYTQKSLSLSPGK

Signal sequence (1－19), Heavy chain variable region (20－138), Heavy chain constant region (139－468)

# Fig. 9

SEQ ID NO: 9: Nucleotide sequence encoding hmAb-H11a heavy chain

ATGAAACACCTGTGGTTCTTCCTCCTGCTGGTGGCAGCTCCCAGATGGGTGCTGAGCGAAGTG
CAGCTGGTTCAGTCTGGCGCCGAAGTGAAAAAGCCTGGCGCCTCTGTGAAGGTGTCCTGCAA
GGCCAGCGGCTACAGCTTCACCGACTACAACATGTACTGGGTCCGACAGGCCCCTGGCCAGTC
TCTTGAGTGGATGGGCTACATCGACCCCTACAACGGCGACACCACCTACAACCAGAAATTCCA
GGGCAGAGTGACCATCACCGCCGACAAGAGCAAGAGCACCGCCTACATGGAACTGAGCAGCC
TGAGAAGCGAGGACACCGCCGTGTACTACTGCGCCAGATCTCCTTACGGCCACTACGCCATGG
ATTACTGGGGCCAGGGCACACTGGTTACCGTTAGCTCAGCCTCCACCAAGGGCCCAAGCGTCT
TCCCCCTGGCACCCTCCTCCAAGAGCACCTCTGGCGGCACAGCCGCCCTGGGCTGCCTGGTCA
AGGACTACTTCCCCGAACCCGTGACCGTGAGCTGGAACTCAGGCGCCCTGACCAGCGGCGTG
CACACCTTCCCCGCTGTCCTGCAGTCCTCAGGACTCTACTCCCTCAGCAGCGTGGTGACCGTG
CCCTCCAGCAGCTTGGGCACCCAGACCTACATCTGCAACGTGAATCACAAGCCCAGCAACACC
AAGGTGGACAAGAAGGTTGAGCCCAAATCTTGTGACAAAACTCACACATGCCCACCCTGCCCA
GCACCTGAACTCCTGGGGGGACCCTCAGTCTTCCTCTTCCCCCCAAAACCCAAGGACACCCTC
ATGATCTCCCGGACCCCTGAGGTCACATGCGTGGTGGTGGACGTGAGCCACGAAGACCCTGAG
GTCAAGTTCAACTGGTACGTGGACGGCGTGGAGGTGCATAATGCCAAGACAAAGCCCCGGGA
GGAGCAGTACAACAGCACGTACCGGGTGGTCAGCGTCCTCACCGTCCTGCACCAGGACTGGCT
GAATGGCAAGGAGTACAAGTGCAAGGTCTCCAACAAAGCCCTCCCAGCCCCCATCGAGAAAAC
CATCTCCAAAGCCAAAGGCCAGCCCCGGGAACCACAGGTGTACACCCTGCCCCCATCCCGGGA
GGAGATGACCAAGAACCAGGTCAGCCTGACCTGCCTGGTCAAAGGCTTCTATCCCAGCGACAT
CGCCGTGGAGTGGGAGAGCAATGGCCAGCCCGAGAACAACTACAAGACCACCCCTCCCGTGC
TGGACTCCGACGGCTCCTTCTTCCTCTACAGCAAGCTCACCGTGGACAAGAGCAGGTGGCAGC
AGGGCAACGTCTTCTCATGCTCCGTGATGCATGAGGCTCTGCACAACCACTACACCCAGAAGA
GCCTCTCCCTGTCTCCCGGCAAA

Signal sequence (1 − 57), Heavy chain variable region (58 − 414), Heavy chain constant region (415 − 1404)

# Fig. 10

SEQ ID NO: 10: Full-length amino acid sequence of hmAb-H11a heavy chain

MKHLWFFLLLVAAPRWVLSEVQLVQSGAEVKKPGASVKVSCKASGYSFTDYNMYWVRQAPGQS
LEWMGYIDPYNGDTTYNQKFQGRVTITADKSKSTAYMELSSLRSEDTAVYYCARSPYGHYAMDY
WGQGTLVTVSSASTKGPSVFPLAPSSKSTSGGTAALGCLVKDYFPEPVTVSWNSGALTSGVHTFP
AVLQSSGLYSLSSVVTVPSSSLGTQTYICNVNHKPSNTKVDKKVEPKSCDKTHTCPPCPAPELLG
GPSVFLFPPKPKDTLMISRTPEVTCVVVDVSHEDPEVKFNWYVDGVEVHNAKTKPREEQYNST
YRVVSVLTVLHQDWLNGKEYKCKVSNKALPAPIEKTISKAKGQPREPQVYTLPPSREEMTKNQV
SLTCLVKGFYPSDIAVEWESNGQPENNYKTTPPVLDSDGSFFLYSKLTVDKSRWQQGNVFSCSV
MHEALHNHYTQKSLSLSPGK

Signal sequence (1 − 19), Heavy chain variable region (20 − 138), Heavy chain constant region (139 − 468)

Fig. 11

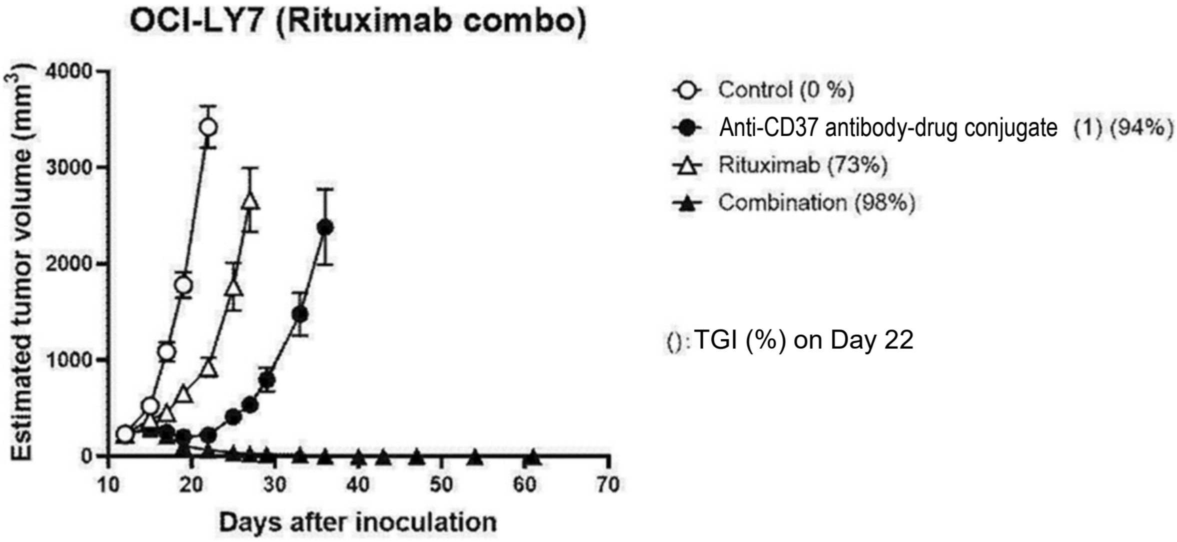
OCI-LY7 (Rituximab combo)
Estimated tumor volume (mm$^3$)
4000
3000
2000
1000
0
10
20
30
40
50
60
70
Days after inoculation
Control (0 %)
Anti-CD37 antibody-drug conjugate (1) (94%)
Rituximab (73%)
Combination (98%)
():TGI (%) on Day 22

Fig. 12

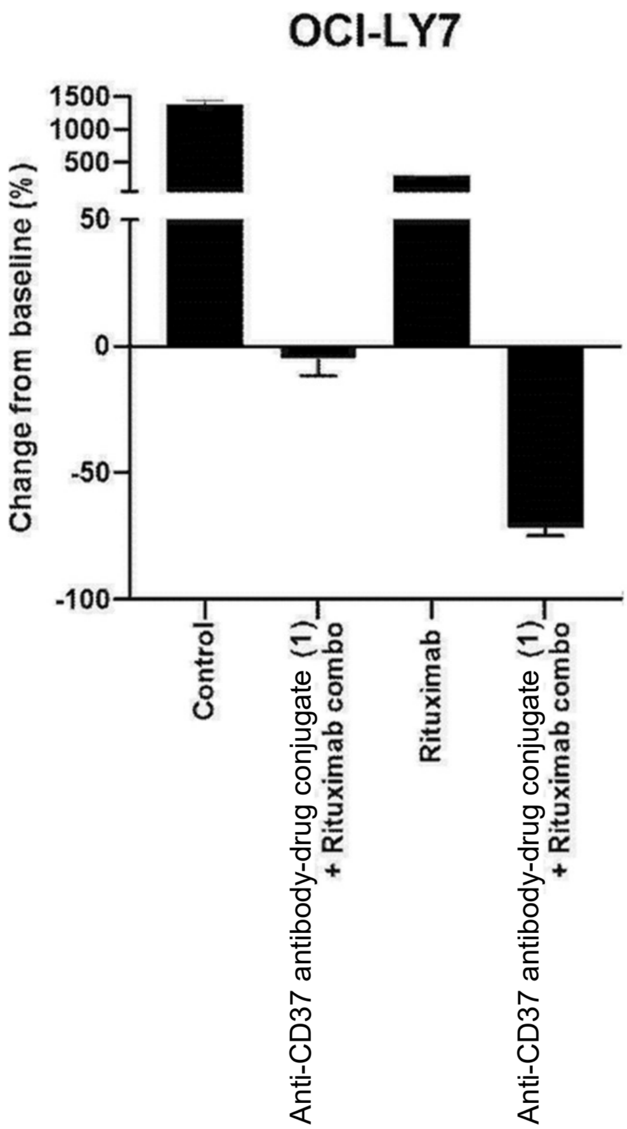
OCI-LY7
Change from baseline (%)
1500
1000
500
50
0
-50
-100
Control
Anti-CD37 antibody-drug conjugate (1) + Rituximab combo
Rituximab
Anti-CD37 antibody-drug conjugate (1) + Rituximab combo

Fig. 13

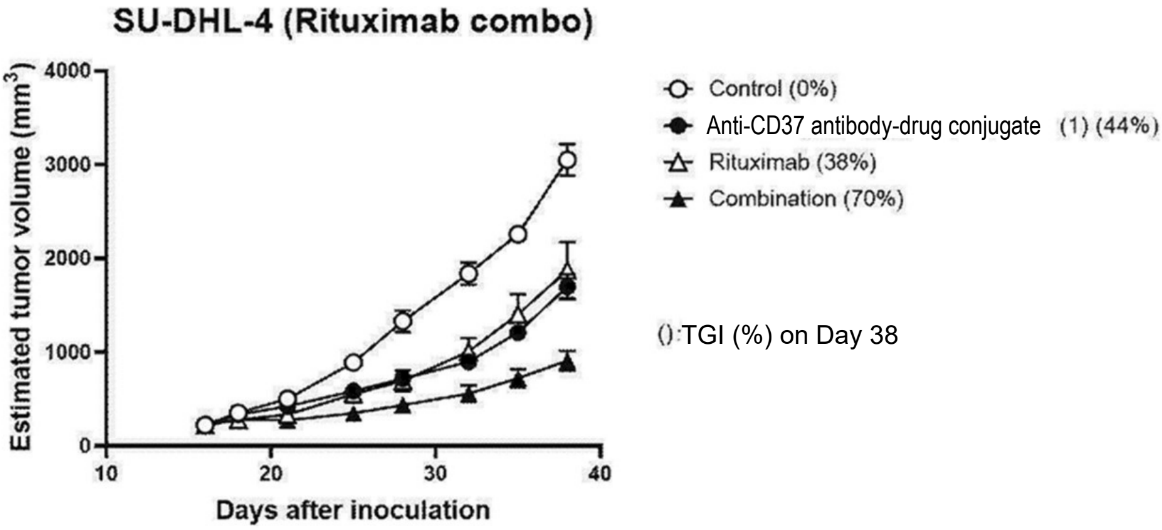


Fig. 14

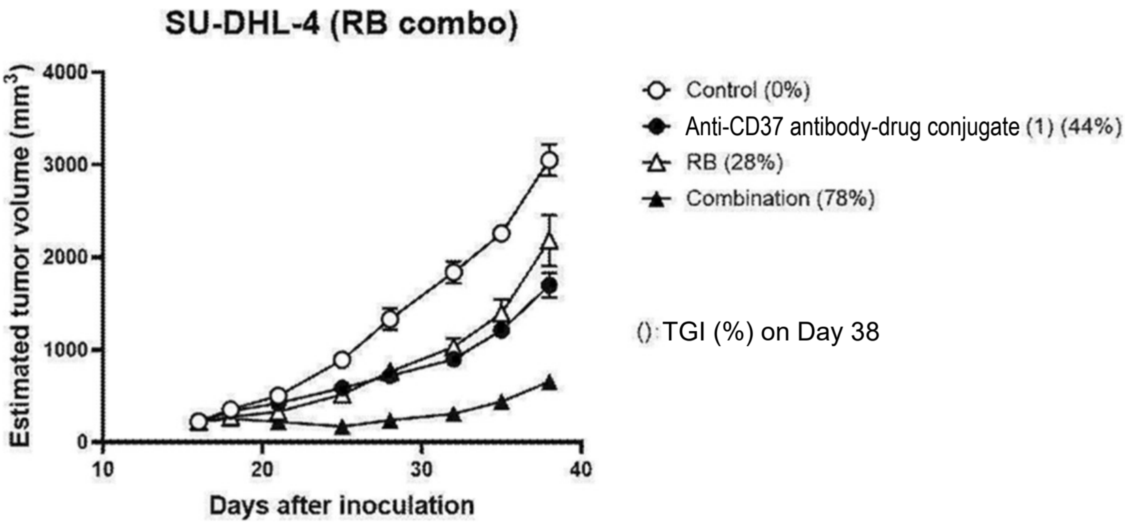


Fig. 15

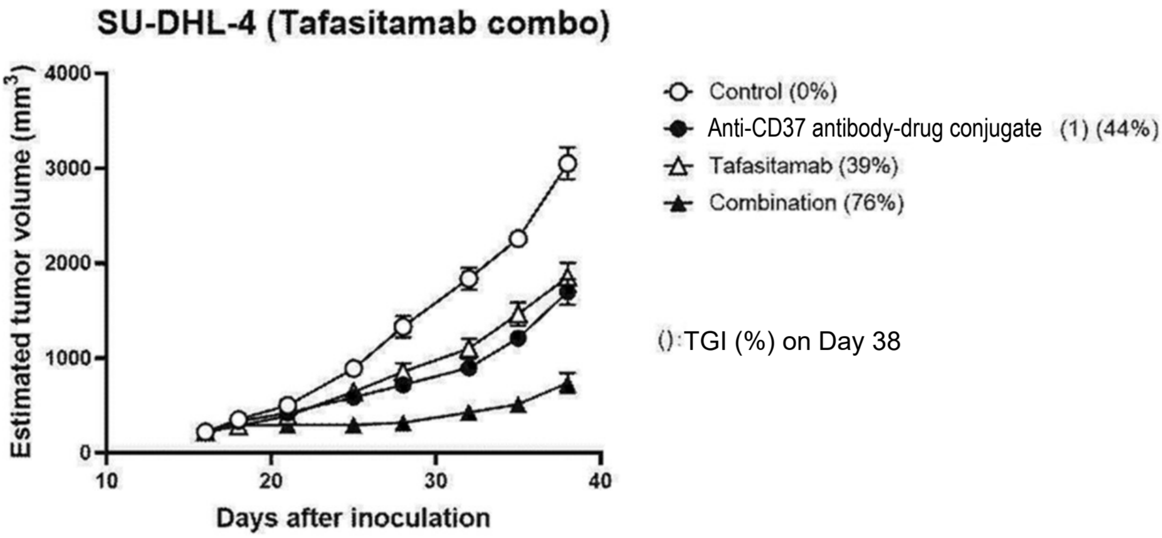

Fig. 16

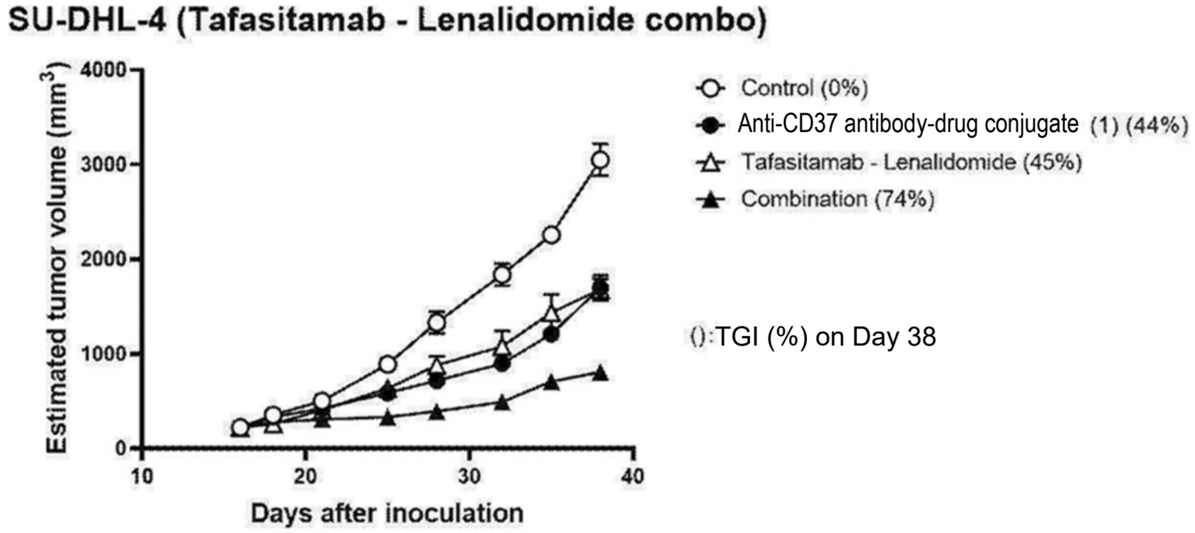
SU-DHL-4 (Tafasitamab - Lenalidomide combo)
Estimated tumor volume (mm3)
4000
3000
2000
1000
0
10
20
30
40
Days after inoculation
Control (0%)
Anti-CD37 antibody-drug conjugate (1) (44%)
Tafasitamab - Lenalidomide (45%)
Combination (74%)
():TGI (%) on Day 38

Fig. 17

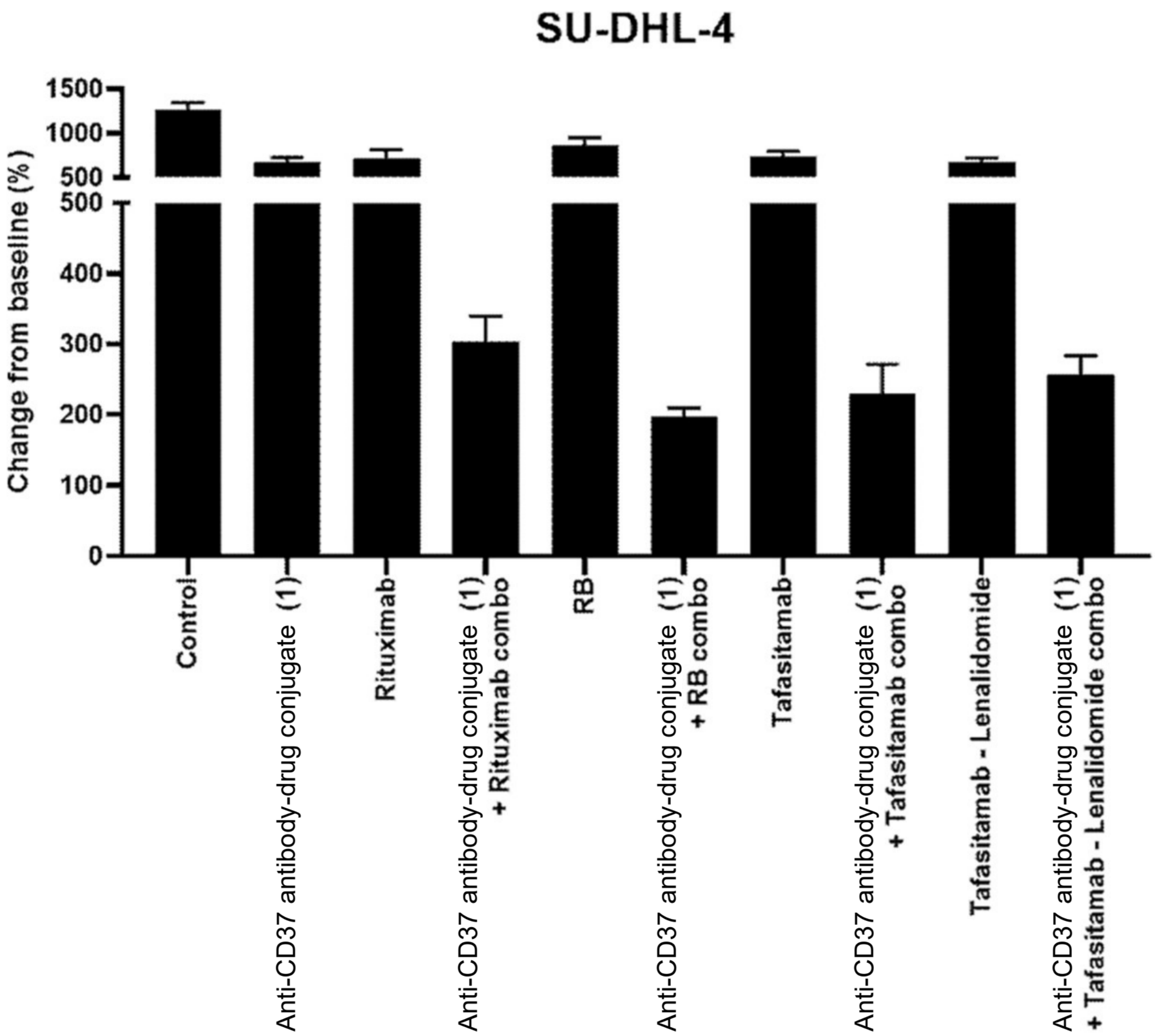
SU-DHL-4
Change from baseline (%)
1500
1000
500
500
400
300
200
100
0
Control
Anti-CD37 antibody-drug conjugate (1)
Rituximab
Anti-CD37 antibody-drug conjugate (1) + Rituximab combo
RB
Anti-CD37 antibody-drug conjugate (1) + RB combo
Tafasitamab
Anti-CD37 antibody-drug conjugate (1) + Tafasitamab combo
Tafasitamab - Lenalidomide
Anti-CD37 antibody-drug conjugate (1) + Tafasitamab - Lenalidomide combo

Fig. 18

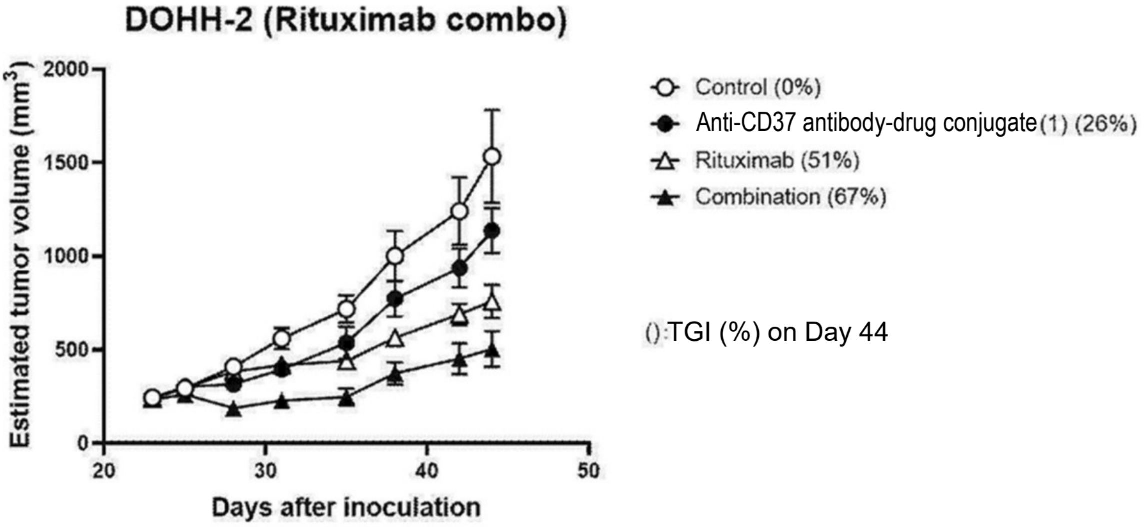


Fig. 19

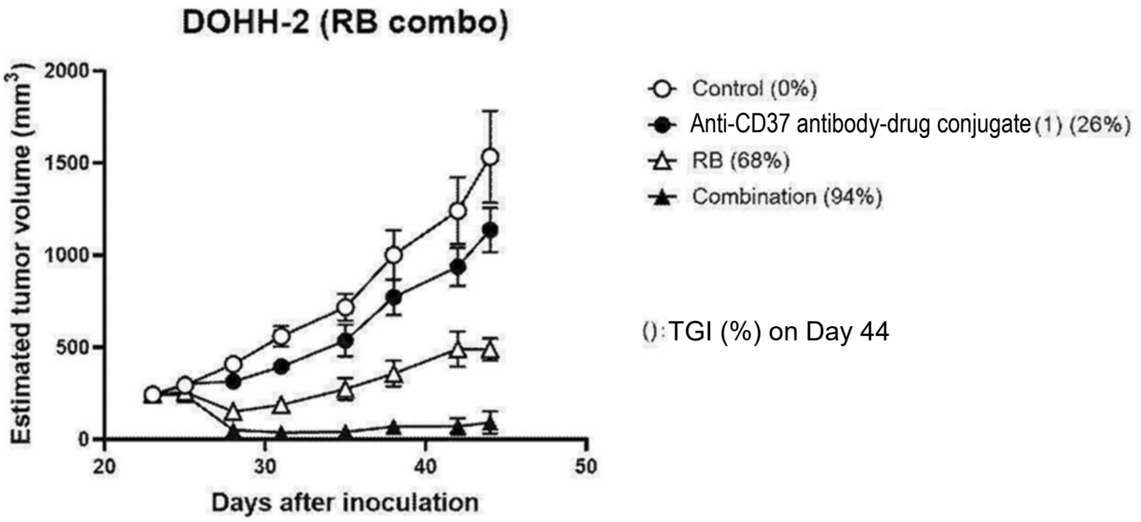


Fig. 20

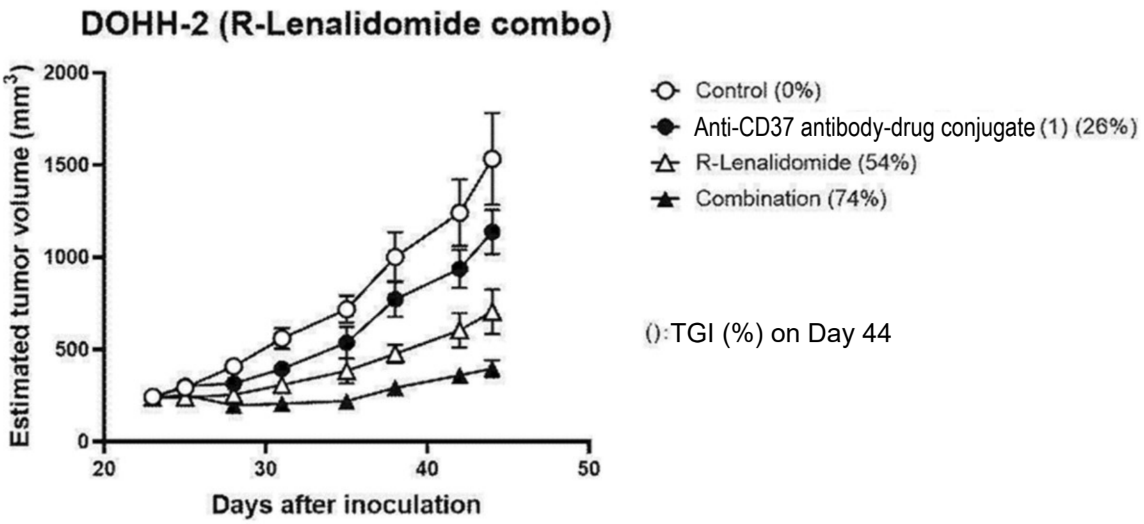

Fig. 21

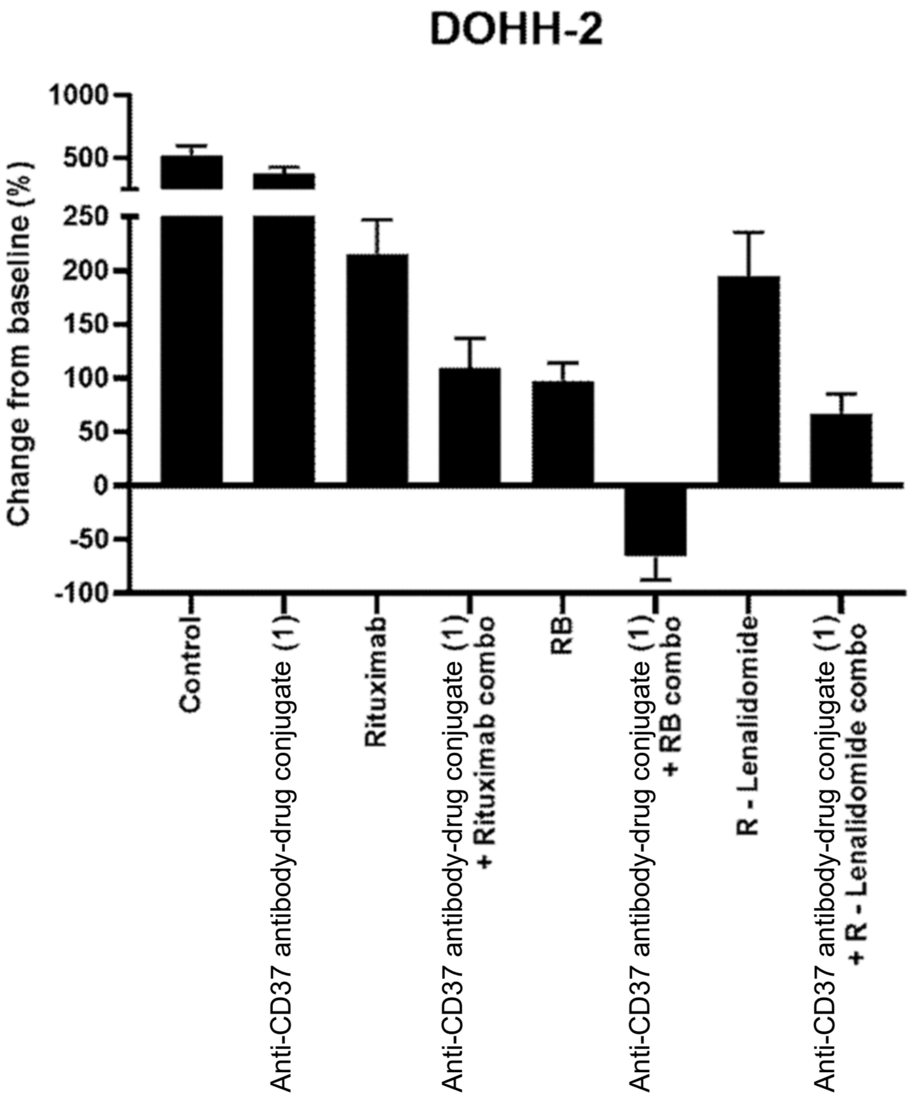
DOHH-2
Change from baseline (%)
1000
500
250
200
150
100
50
0
-50
-100
Control
Anti-CD37 antibody-drug conjugate (1)
Rituximab
Anti-CD37 antibody-drug conjugate (1) + Rituximab combo
RB
Anti-CD37 antibody-drug conjugate (1) + RB combo
R - Lenalidomide
Anti-CD37 antibody-drug conjugate (1) + R - Lenalidomide combo

Fig. 22

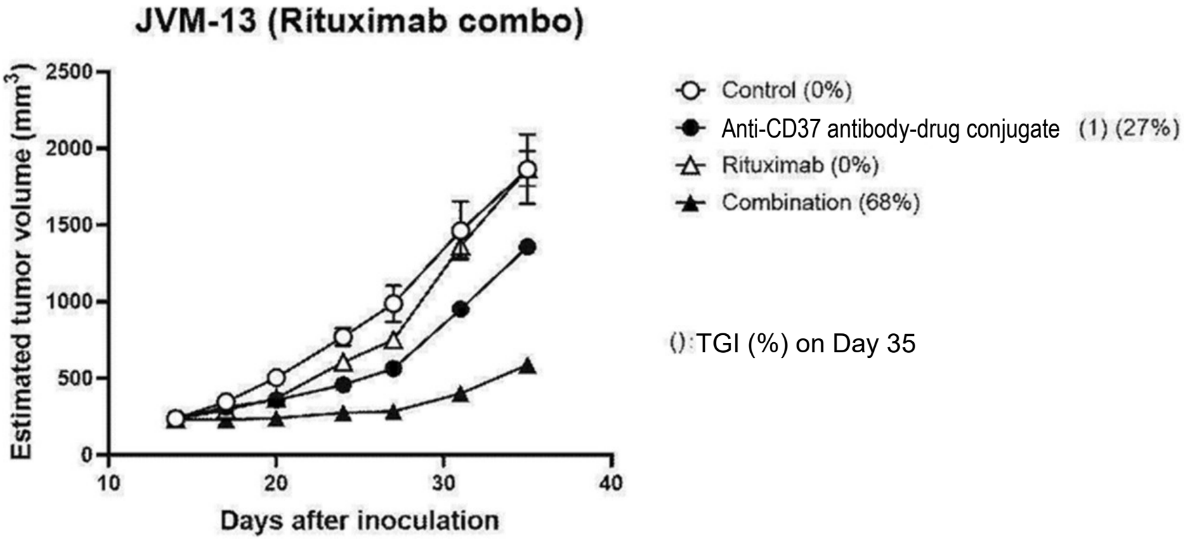
JVM-13 (Rituximab combo)
Estimated tumor volume (mm3)
2500
2000
1500
1000
500
0
10
20
30
40
Days after inoculation
Control (0%)
Anti-CD37 antibody-drug conjugate (1) (27%)
Rituximab (0%)
Combination (68%)
(): TGI (%) on Day 35

Fig. 23

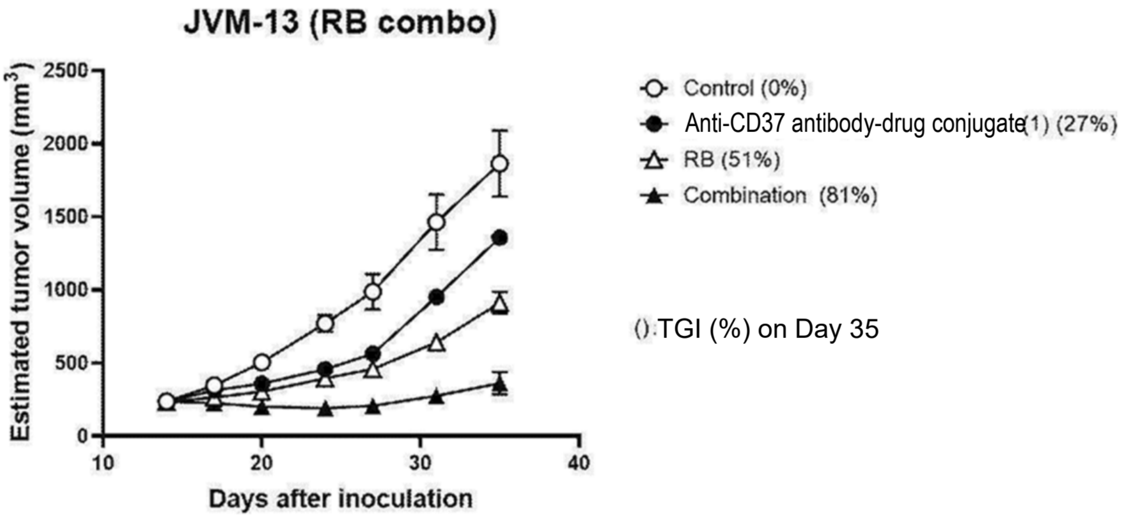


Fig. 24

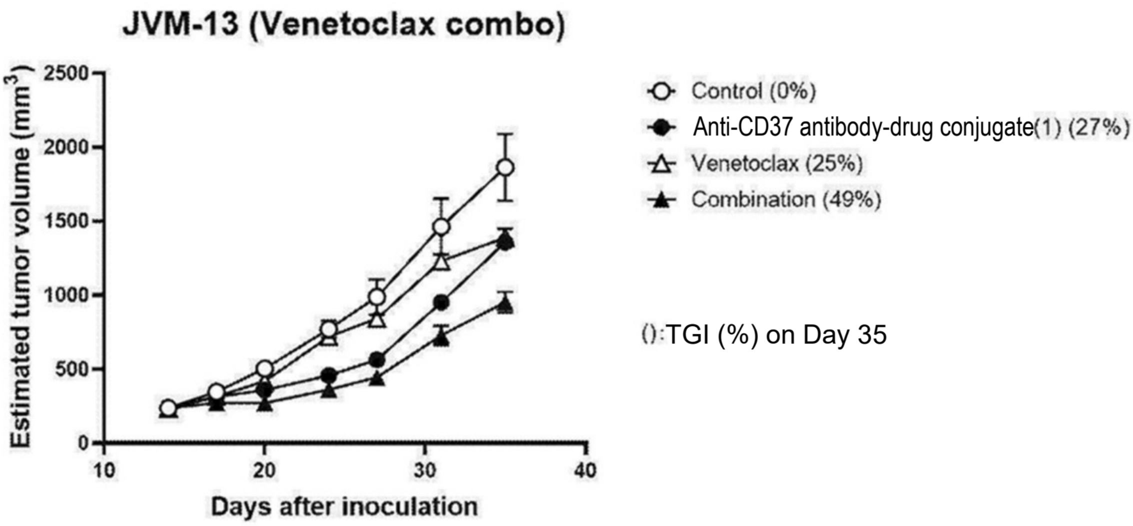


Fig. 25

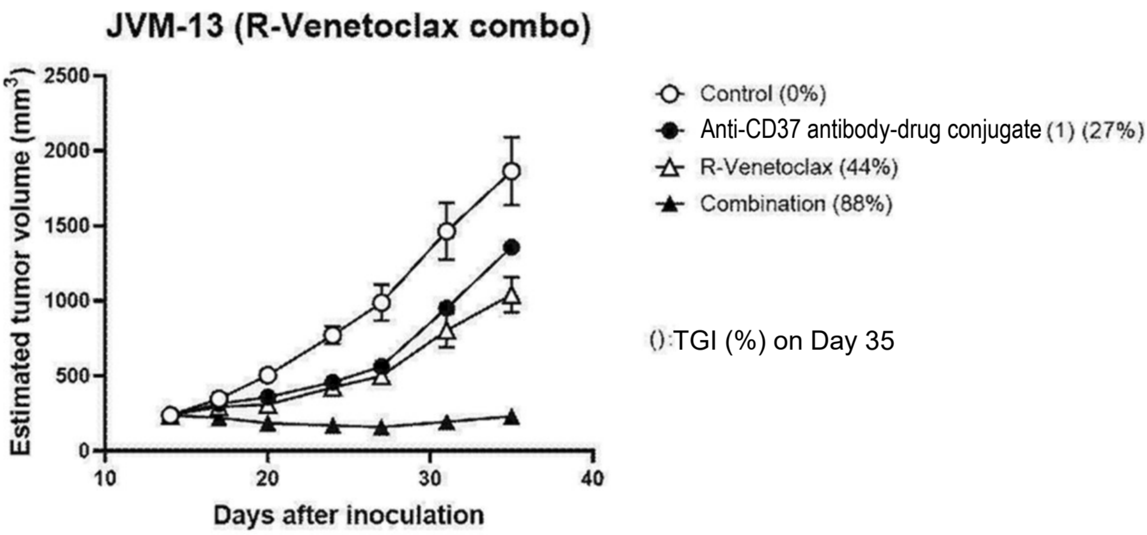

Fig. 26

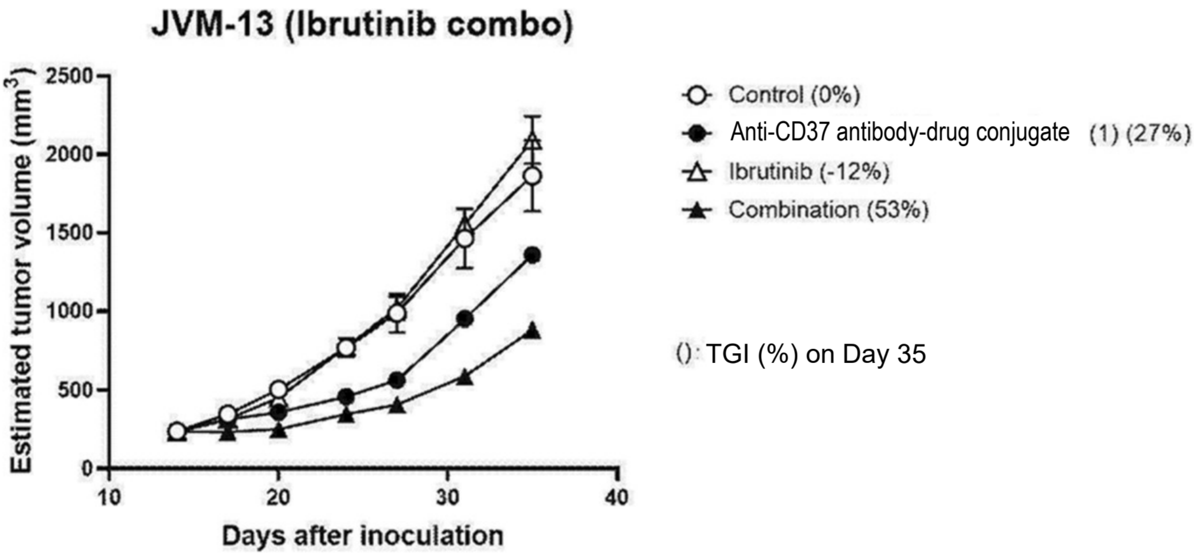
JVM-13 (Ibrutinib combo)
Estimated tumor volume (mm3)
2500
2000
1500
1000
500
0
10
20
30
40
Days after inoculation
Control (0%)
Anti-CD37 antibody-drug conjugate (1) (27%)
Ibrutinib (-12%)
Combination (53%)
(): TGI (%) on Day 35

Fig. 27

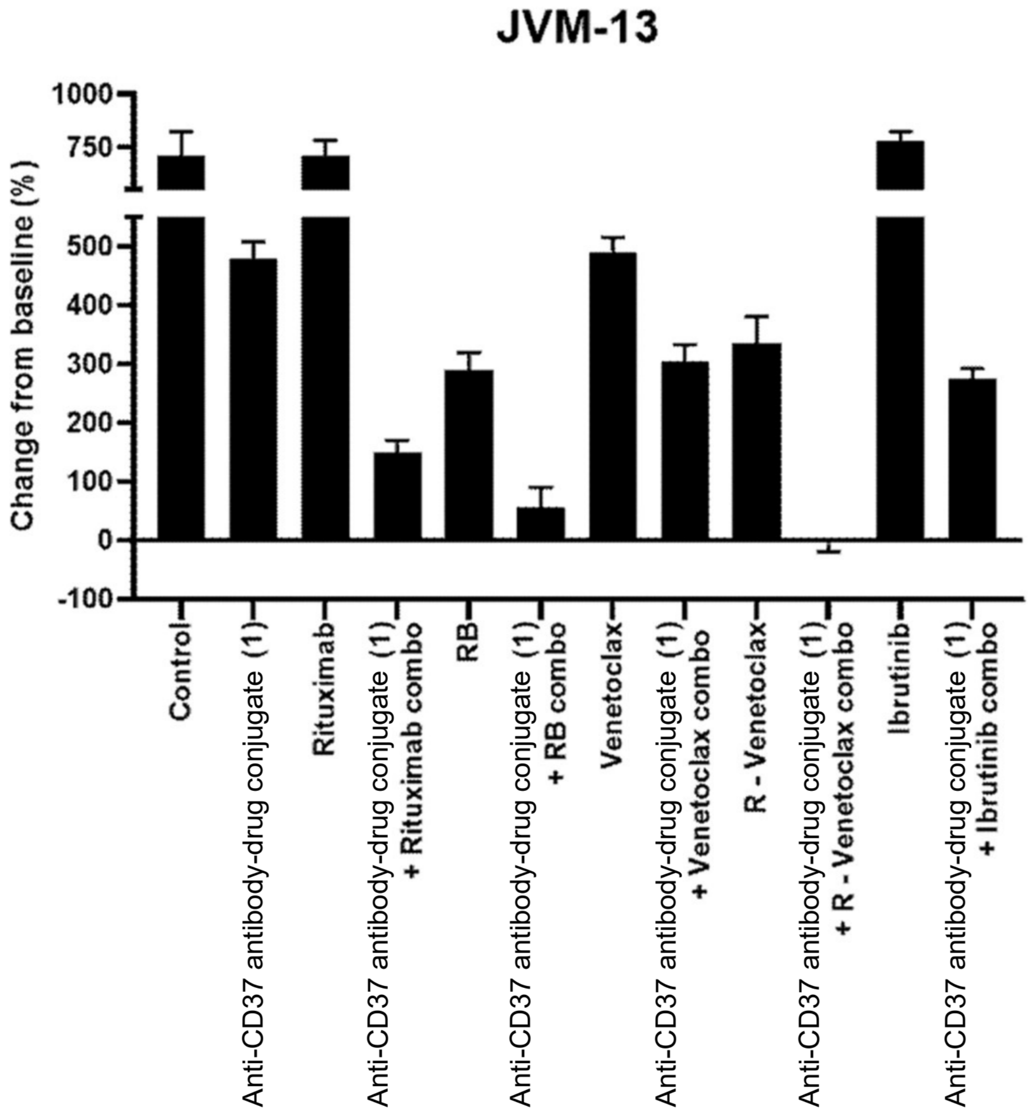
JVM-13
Change from baseline (%)
1000
750
500
400
300
200
100
0
-100
Control
Anti-CD37 antibody-drug conjugate (1)
Rituximab
Anti-CD37 antibody-drug conjugate (1) + Rituximab combo
RB
Anti-CD37 antibody-drug conjugate (1) + RB combo
Venetoclax
Anti-CD37 antibody-drug conjugate (1) + Venetoclax combo
R - Venetoclax
Anti-CD37 antibody-drug conjugate (1) + R - Venetoclax combo
Ibrutinib
Anti-CD37 antibody-drug conjugate (1) + Ibrutinib combo

Fig. 28

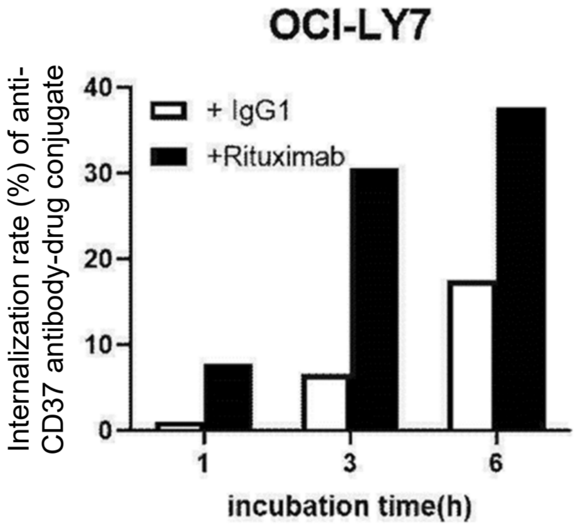


Fig. 29

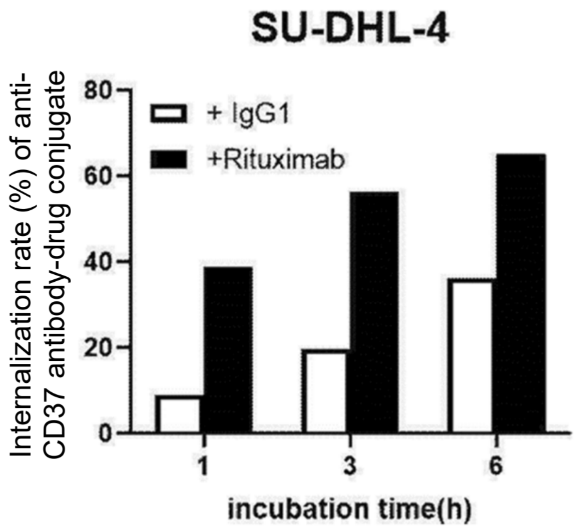


Fig. 30

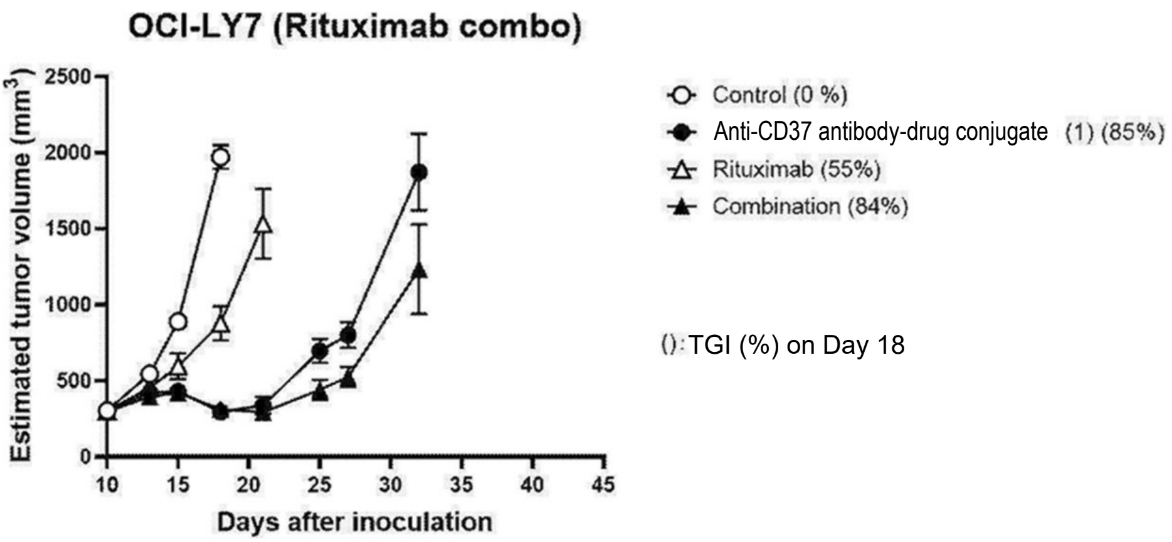

Fig. 31

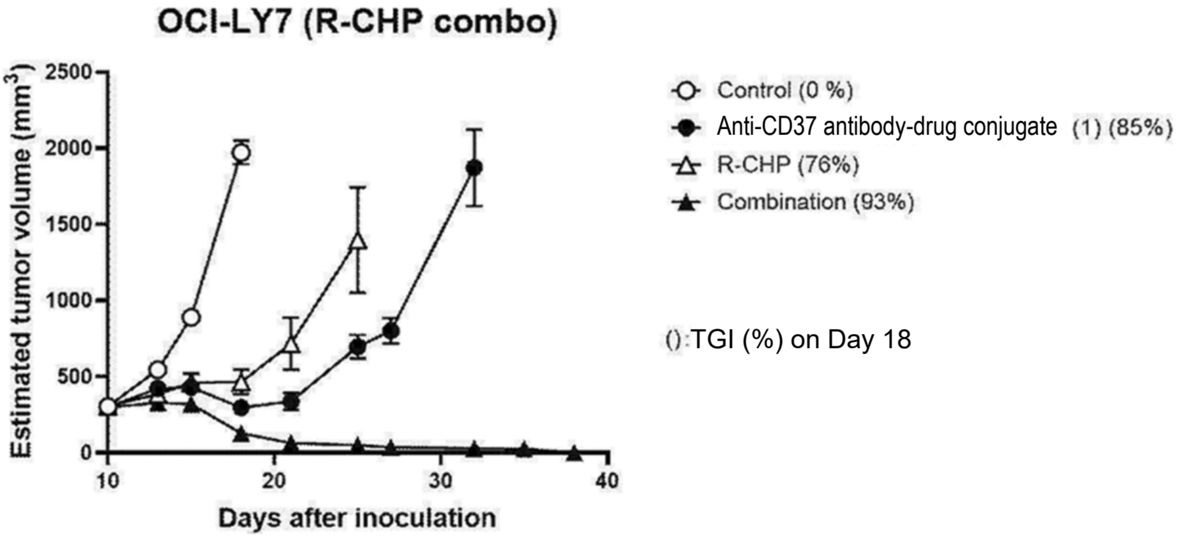
OCI-LY7 (R-CHP combo)
Estimated tumor volume (mm3)
2500
2000
1500
1000
500
0
10
20
30
40
Days after inoculation
Control (0 %)
Anti-CD37 antibody-drug conjugate (1) (85%)
R-CHP (76%)
Combination (93%)
():TGI (%) on Day 18

Fig. 32

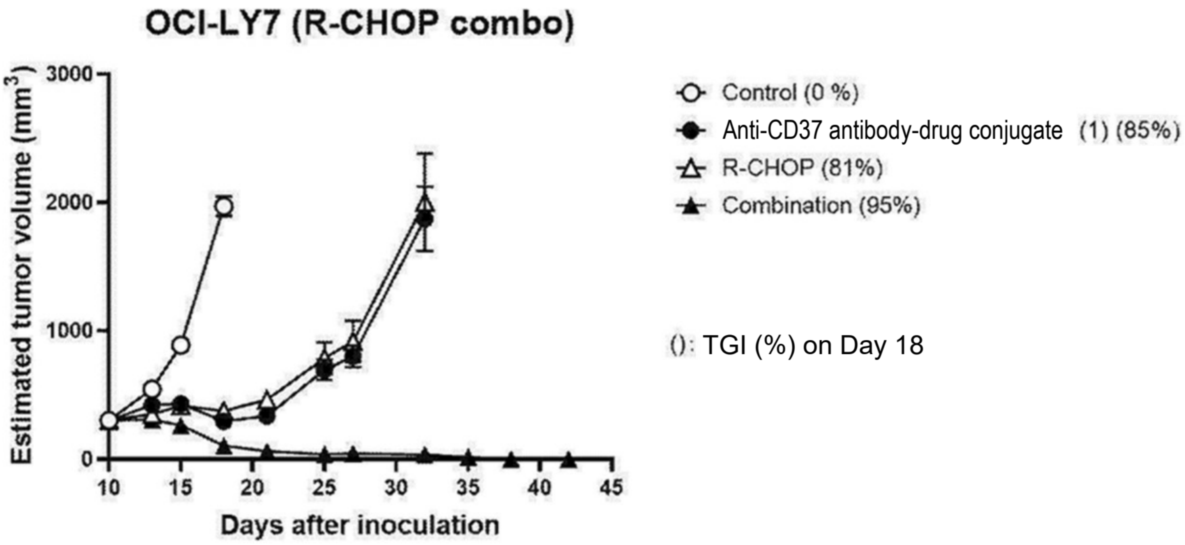
OCI-LY7 (R-CHOP combo)
Estimated tumor volume (mm3)
3000
2000
1000
0
10
15
20
25
30
35
40
45
Days after inoculation
Control (0 %)
Anti-CD37 antibody-drug conjugate (1) (85%)
R-CHOP (81%)
Combination (95%)
(): TGI (%) on Day 18

Fig. 33

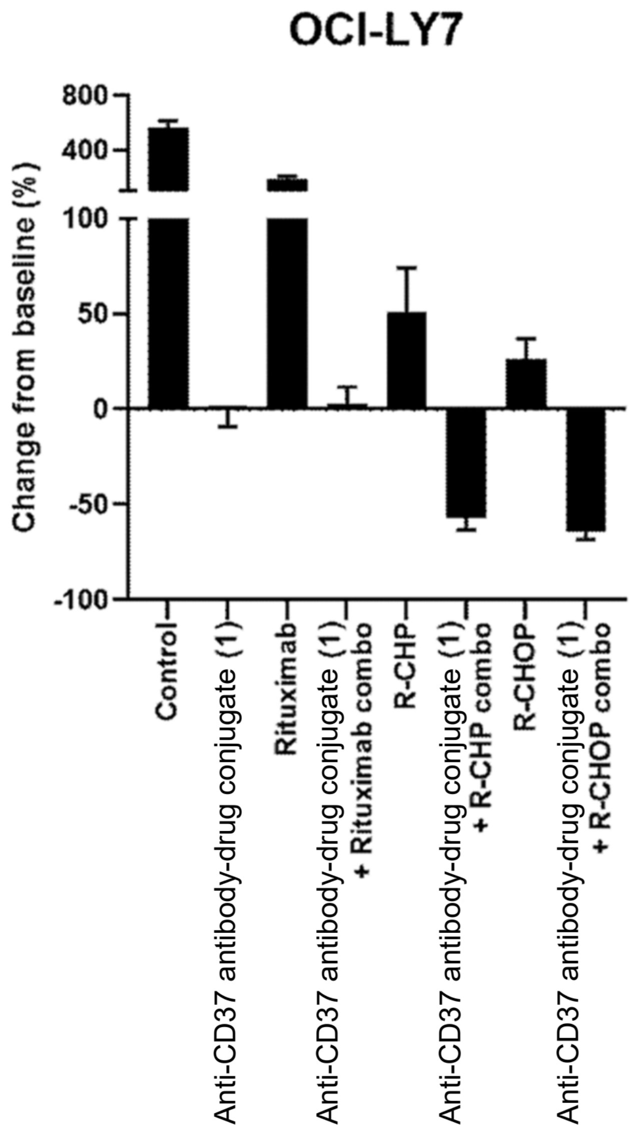
OCI-LY7
Change from baseline (%)
800
400
100
50
0
-50
-100
Control
Anti-CD37 antibody-drug conjugate (1)
Rituximab
Anti-CD37 antibody-drug conjugate (1) + Rituximab combo
R-CHP
Anti-CD37 antibody-drug conjugate (1) + R-CHP combo
R-CHOP
Anti-CD37 antibody-drug conjugate (1) + R-CHOP combo

Fig. 34

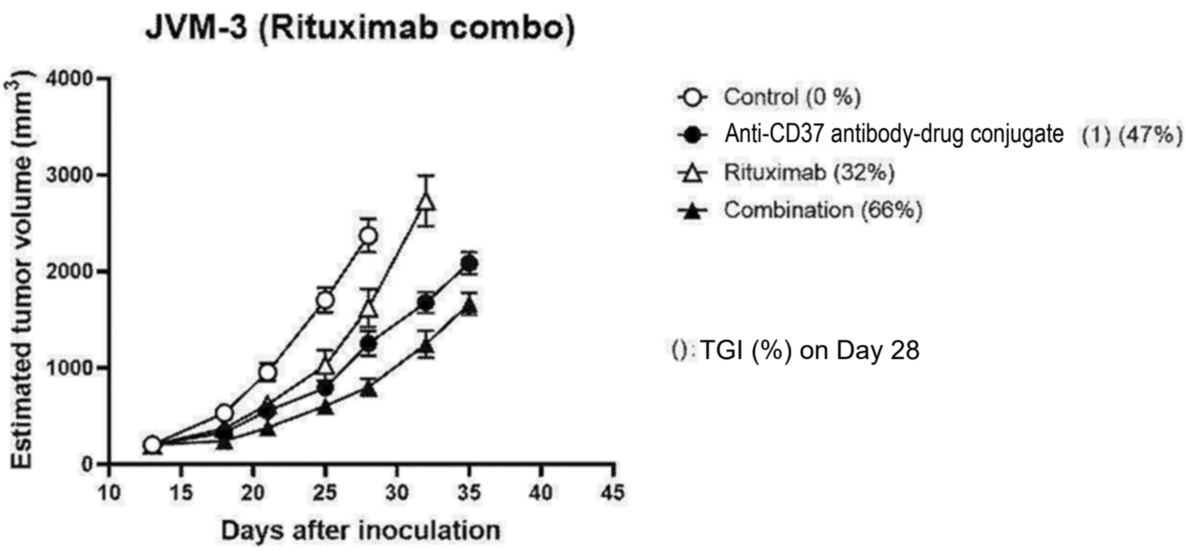
JVM-3 (Rituximab combo)
Estimated tumor volume ($mm^3$)
4000
3000
2000
1000
0
10
15
20
25
30
35
40
45
Days after inoculation
Control (0 %)
Anti-CD37 antibody-drug conjugate (1) (47%)
Rituximab (32%)
Combination (66%)
(): TGI (%) on Day 28

Fig. 35

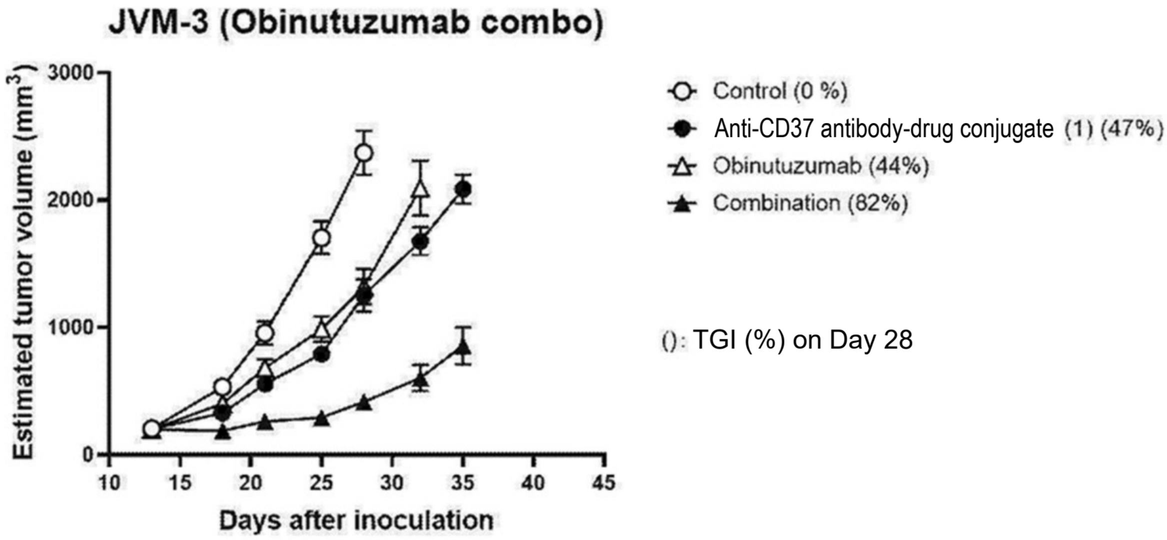
JVM-3 (Obinutuzumab combo)
Estimated tumor volume (mm3)
3000
2000
1000
0
10
15
20
25
30
35
40
45
Days after inoculation
Control (0 %)
Anti-CD37 antibody-drug conjugate (1) (47%)
Obinutuzumab (44%)
Combination (82%)
(): TGI (%) on Day 28

Fig. 36

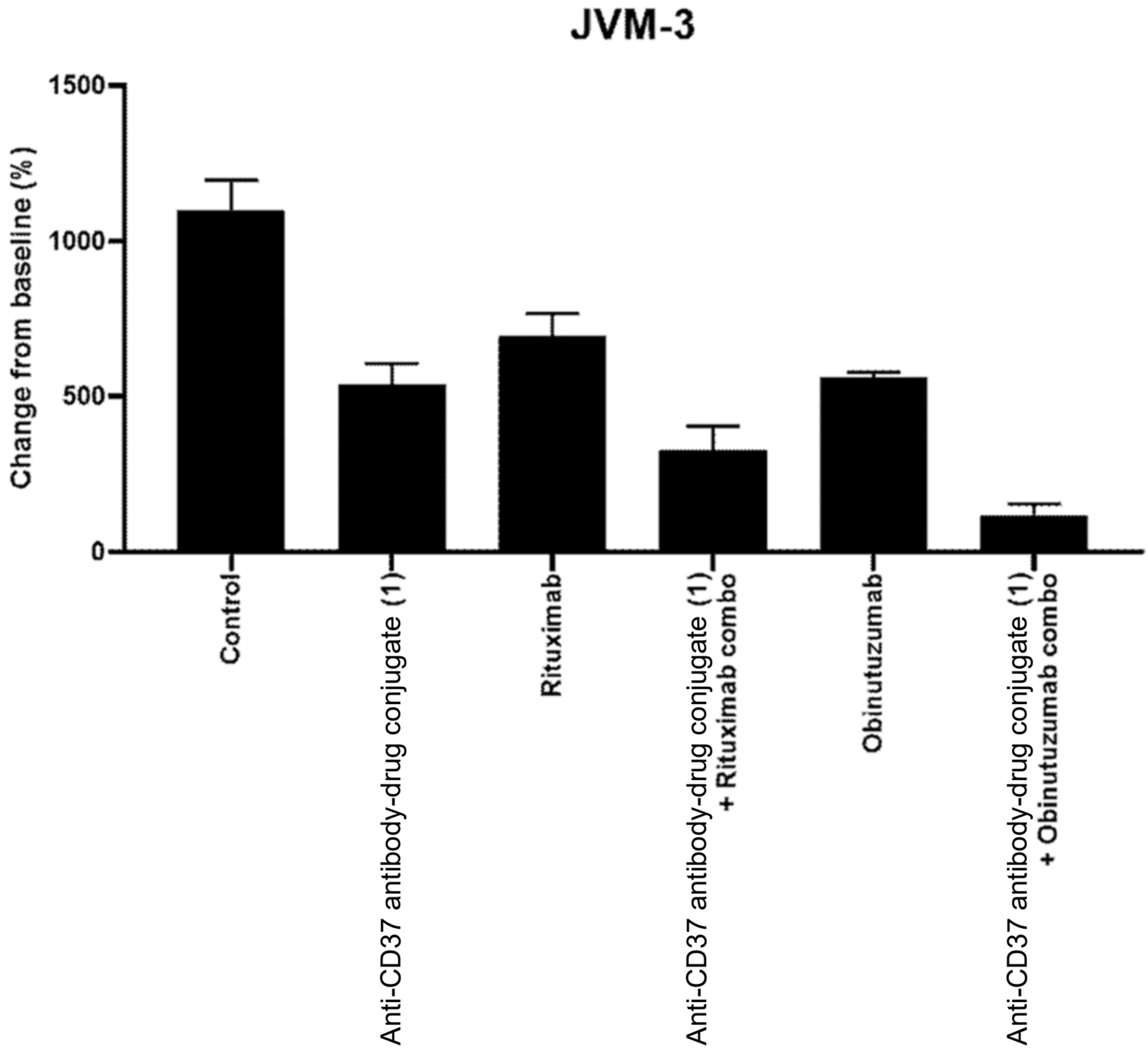
JVM-3
Change from baseline (%)
1500
1000
500
0
Control
Anti-CD37 antibody-drug conjugate (1)
Rituximab
Anti-CD37 antibody-drug conjugate (1) + Rituximab combo
Obinutuzumab
Anti-CD37 antibody-drug conjugate (1) + Obinutuzumab combo

Fig. 37

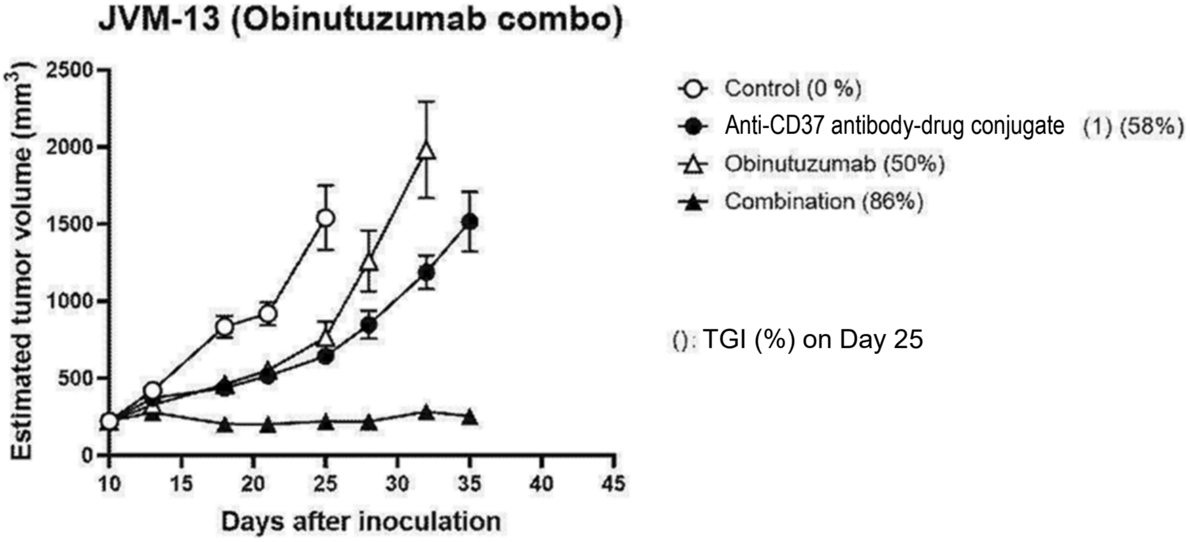
JVM-13 (Obinutuzumab combo)
Estimated tumor volume (mm3)
2500
2000
1500
1000
500
0
10
15
20
25
30
35
40
45
Days after inoculation
Control (0 %)
Anti-CD37 antibody-drug conjugate (1) (58%)
Obinutuzumab (50%)
Combination (86%)
(): TGI (%) on Day 25

Fig. 38

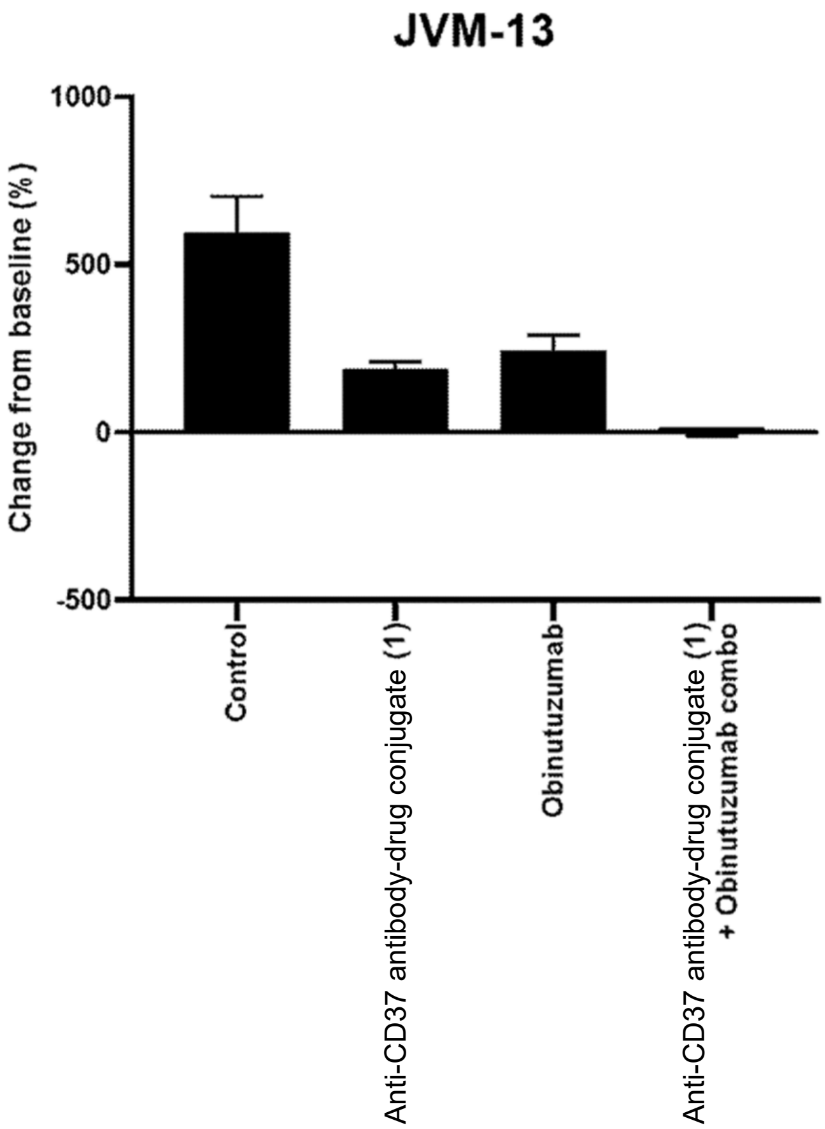
JVM-13
Change from baseline (%)
1000
500
0
-500
Control
Anti-CD37 antibody-drug conjugate (1)
Obinutuzumab
Anti-CD37 antibody-drug conjugate (1)
+ Obinutuzumab combo

## INTERNATIONAL SEARCH REPORT

International application No.
**PCT/JP2024/015357**

**A. CLASSIFICATION OF SUBJECT MATTER**

***A61K 47/68***(2017.01)i; ***A61K 31/4184***(2006.01)i; ***A61K 31/454***(2006.01)i; ***A61K 31/4745***(2006.01)i; ***A61K 31/496***(2006.01)i; ***A61K 31/519***(2006.01)i; ***A61K 31/573***(2006.01)i; ***A61K 31/664***(2006.01)i; ***A61K 31/704***(2006.01)i; ***A61K 39/395***(2006.01)i; ***A61K 45/00***(2006.01)i; ***A61P 7/00***(2006.01)i; ***A61P 35/00***(2006.01)i; ***A61P 35/02***(2006.01)i; ***A61P 43/00***(2006.01)i; ***C07K 7/06***(2006.01)i; ***C07K 16/28***(2006.01)i

FI: A61K47/68 ZNA; A61K45/00; A61P35/00; A61P35/02; A61P7/00; A61K39/395 E; A61K39/395 T; A61K39/395 C; A61K39/395 L; A61P43/00 121; A61K31/4745; A61K31/664; A61K31/704; A61K31/573; A61K31/496; A61K31/519; A61K31/4184; A61K31/454; C07K16/28; C07K7/06

According to International Patent Classification (IPC) or to both national classification and IPC

**B. FIELDS SEARCHED**

Minimum documentation searched (classification system followed by classification symbols)

A61K47/68; A61K31/4184; A61K31/454; A61K31/4745; A61K31/496; A61K31/519; A61K31/573; A61K31/664; A61K31/704; A61K39/395; A61K45/00; A61P7/00; A61P35/00; A61P35/02; A61P43/00; C07K7/06; C07K16/28

Documentation searched other than minimum documentation to the extent that such documents are included in the fields searched

Published examined utility model applications of Japan 1922-1996
Published unexamined utility model applications of Japan 1971-2024
Registered utility model specifications of Japan 1996-2024
Published registered utility model applications of Japan 1994-2024

Electronic data base consulted during the international search (name of data base and, where practicable, search terms used)

CAplus/REGISTRY/MEDLINE/EMBASE/BIOSIS (STN)

**C. DOCUMENTS CONSIDERED TO BE RELEVANT**

| Category* | Citation of document, with indication, where appropriate, of the relevant passages | Relevant to claim No. |
|---|---|---|
| Y | JP 2018-517714 A (DEBIOPHARM INTERNATIONAL, S. A) 05 July 2018 (2018-07-05) claims, examples 1-11 | 1-83 |
| Y | JP 2013-524777 A (IMMUNOGEN, INC.) 20 June 2013 (2013-06-20) claims, paragraph [0075], examples 1-17 | 1-83 |
| Y | WO 2020/130125 A1 (DAIICHI SANKYO COMPANY, LIMITED) 25 June 2020 (2020-06-25) claims, paragraphs [0201], [0278], each example | 1-83 |

☑ Further documents are listed in the continuation of Box C. ☑ See patent family annex.

* Special categories of cited documents:
"A" document defining the general state of the art which is not considered to be of particular relevance
"D" document cited by the applicant in the international application
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **11 June 2024** | **25 June 2024** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | Telephone No. |

Form PCT/ISA/210 (second sheet) (July 2022)

**INTERNATIONAL SEARCH REPORT**

International application No. **PCT/JP2024/015357**

**C. DOCUMENTS CONSIDERED TO BE RELEVANT**

| Category* | Citation of document, with indication, where appropriate, of the relevant passages | Relevant to claim No. |
|---|---|---|
| Y | WO 2018/110515 A1 (DAIICHI SANKYO COMPANY, LIMITED) 21 June 2018 (2018-06-21)<br>claims, paragraph [0096], each example | 1-83 |
| Y | 我妻利紀, トポイソメラーゼＩ阻害剤を搭載した新規ＡＤＣ技術開発, 薬学雑誌, 2017, vol. 137, no. 5, pp. 545-550, (AGATSUMA, Toshinori. Development of New ADC Technology with Topoisomerase I Inhibitor. YAKUGAKU ZASSHI.)<br>in particular, abstract, p. 546, left column to p. 549, left column, fig. 1 | 1-83 |
| Y | 一般社団法人　日本癌治療学会ホームページ，がん治療ガイドライン　造血器腫瘍診療ガイドライン， [online]. 16 June 2018, [retrieved on 13 June 2024], Internet<URL:https://web.archive.org/web/20180616194653/http://www.jsco-cpg.jp/item/12/index.html>, non-official translation (Japan Society of Clinical Oncology Website. Cancer Treatment Guidelines: Guidelines for treatment of hematopoietic tumors.)<br>in particular, sections "I. Leukemia, 5. Chronic lymphocytic leukaemia/Small lymphocytic lymphoma (CLL/SLL)", "II. Lymphoma, 1. Follicular lymphoma (FL)", "II. Lymphoma, 5. Diffuse large B-cell lymphoma (DLBCL, NOS)" | 1-83 |
| A | HICKS, Stuart W. et al. The antitumor Activity of IMGN529, a CD37-Targeting Antibody-Drug Conjugate, Is Potentiated by Rituximab in Non-Hodgkin Lymphoma Models. Neoplasia. 2017, vol. 19, no. 9, pp. 661-671 | 1-83 |
| A | JP 2015-516980 A (BOEHRINGER INGELHEIM INTERNATIONAL GMBH) 18 June 2015 (2015-06-18)<br>claims, each example | 1-83 |
| P, A | WO 2023/068226 A1 (DAIICHI SANKYO CO., LTD.) 27 April 2023 (2023-04-27)<br>claims, each example | 1-83 |

Form PCT/ISA/210 (second sheet) (July 2022)

**INTERNATIONAL SEARCH REPORT**

International application No.
**PCT/JP2024/015357**

**Box No. I Nucleotide and/or amino acid sequence(s) (Continuation of item 1.c of the first sheet)**

1. With regard to any nucleotide and/or amino acid sequence disclosed in the international application, the international search was carried out on the basis of a sequence listing:
   - a. ☑ forming part of the international application as filed.
   - b. ☐ furnished subsequent to the international filing date for the purposes of international search (Rule 13*ter*.1(a)),
     - ☐ accompanied by a statement to the effect that the sequence listing does not go beyond the disclosure in the international application as filed.

2. ☐ With regard to any nucleotide and/or amino acid sequence disclosed in the international application, this report has been established to the extent that a meaningful search could be carried out without a WIPO Standard ST.26 compliant sequence listing.

3. Additional comments:

Form PCT/ISA/210 (continuation of first sheet) (July 2022)

**INTERNATIONAL SEARCH REPORT**
**Information on patent family members**

International application No.
**PCT/JP2024/015357**

| Patent document cited in search report | Publication date (day/month/year) | Patent family member(s) | Publication date (day/month/year) |
|---|---|---|---|
| JP 2018-517714 A | 05 July 2018 | US 2017/0000900 A1<br>claims, examples 1-11<br>WO 2016/200676 A1<br>EP 3302561 A1<br>CA 2986437 A1<br>AU 2016276158 A1<br>KR 10-2018-0011315 A<br>CN 108472361 A | |
| JP 2013-524777 A | 20 June 2013 | US 2011/0256153 A1<br>claims, paragraph [0106], examples 1-17<br>WO 2011/112978 A1<br>EP 2544719 A1<br>TW 201206481 A<br>AU 2011226672 A1<br>CA 2792618 A1<br>CN 102971012 A<br>KR 10-2013-0016272 A | |
| WO 2020/130125 A1 | 25 June 2020 | US 2022/0040324 A1<br>claims, paragraphs [0117], [0203], each example<br>EP 3903828 A1<br>CN 113195000 A<br>KR 10-2021-0107069 A<br>AU 2019407426 A1<br>CA 3124330 A1<br>TW 202038957 A | |
| WO 2018/110515 A1 | 21 June 2018 | US 2019/0314362 A1<br>claims, paragraph [0192], each example<br>EP 3552626 A1<br>AU 2017377233 A1<br>CA 3046293 A1<br>CN 110049779 A<br>KR 10-2019-0095280 A<br>TW 201828993 A | |
| JP 2015-516980 A | 18 June 2015 | US 2013/0287797 A1<br>claims, each example<br>WO 2013/160396 A1<br>EP 2841099 A1 | |
| WO 2023/068226 A1 | 27 April 2023 | TW 202330041 A | |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2014057687 A
- WO 2014061277 A
- WO 2015098099 A
- WO 2015115091 A
- WO 2015146132 A
- WO 2015155976 A
- WO 2015155998 A
- WO 9007861 A
- WO 9201047 A
- WO 9220791 A
- WO 9306213 A
- WO 9311236 A
- WO 9319172 A
- WO 9501438 A
- WO 9515388 A
- WO 199954342 A
- WO 200061739 A
- WO 200231140 A
- WO 2007133855 A
- WO 2013120066 A
- US 20160297890 A
- US 2016297890 A
- WO 2011112978 A
- US 8765917 B
- WO 2015017552 A
- US 9925273 B
- WO 2013088363 A
- US 20140348745
- WO 2004056312 A
- WO 2009155386 A


### Non-patent literature cited in the description


- **GILLES S. et al.** *Adv Ther*, 2017, vol. 34 (10), 2232-2273
- **B COIFFIER. et al.** *Oncogene*, 2007, vol. 26 (25), 3603-3613
- **MUNAKATA W. et al.** *Expert Opin Drug Discov*, 2016, vol. 11 (11), 1123-1130
- **SMYTH E. et al.** *Cancer Treat Rev*, 2023, vol. 113, 102510
- **BRUCE D. CHESON. et al.** *Blood Cancer, J*, 2021, vol. 11 (4), 68
- **MORITZ BEWARDER. et al.** *Cancers (Basal)*, 2021, vol. 13 (10), 2468
- **LAURENT DUCRY. et al.** *Bioconjug Chem*, 2010, vol. 21 (1), 5-13
- **STEPEN C ALLEY. et al.** *Curr Opin ChemBiol*, 2010, vol. 14 (4), 529-37
- **NITIN K DAMLE. et al.** *Expert Opin Biol Ther*, 2004, vol. 4 (9), 1445-52
- **PETER D SENTER. et al.** *Nat Biotechnol*, 2012, vol. 30 (7), 631-7
- **HOWARD A. et al.** *J Clin Oncol*, 2011, vol. 29 (4), 398-405
- **LAURIE H SEHN.** *Blood Adv*, 2022, vol. 6 (2), 533-543
- **BO XU.** *European Journal of Clinical Pharmacology*, 2022, vol. 78, 707-719
- **OWEN STRETTON. et al.** *Lancet Haematol*, 2021, vol. 8 (8), e546-e547
- **OGITANI Y. et al.** *Clinical Cancer Research*, 2016, vol. 22 (20), 5097-5108
- **OGITANI Y. et al.** *Cancer Science*, 2016, vol. 107 (7), 1039-46
- **DOI T et al.** *Lancet Oncol*, 2017, vol. 18 (11), 1512-1522
- **TAKEGAWA N et al.** *Int. J. Cancer*, 2017, vol. 141 (8), 1682-1689
- **CHARRIN S. et al.** *J Cell Sci*, 2014, vol. 127, 3641-3648
- *Cell Death and Differentiation*, 2008, vol. 15 (2), 751-761
- *Molecular Biology of the Cell*, December 2004, vol. 15 (3), 5268-5282
- *Bio Techniques*, January 2000, vol. 28, 162-165
- **KOHLER** ; **MILSTEIN**. *Nature*, 1975, vol. 256, 495-497
- Monoclonal Antibodies. Plenum Press, 1980, 365-367
- **AIHARA H** ; **MIYAZAKI J.** *Nat Biotechnol.*, September 1998, vol. 16 (9), 867-70
- **MIR LM** ; **BUREAU MF** ; **GEHL J** ; **RANGARA R** ; **ROUY D** ; **CAILLAUD JM** ; **DELAERE P** ; **BRANELLEC D** ; **SCHWARTZ B** ; **SCHERMAN D.** *Proc Natl Acad Sci U S A.*, 13 April 1999, vol. 96 (8), 4262-7
- **MCMAHON JM1** ; **SIGNORI E** ; **WELLS KE** ; **FAZIO VM** ; **WELLS DJ.** *Gene Ther.*, August 2001, vol. 8 (16), 1264-70
- **SMELAND E et al.** *Scand J Immunol*, 1985, vol. 21 (3), 205-214

- *Proc. Natl. Acad. Sci. U.S.A.*, 1984, vol. 81, 6851-6855
- *Nature*, 1986, vol. 321, 522-525
- **ANDREW C. R. MARTIN** ; **JAMES ALLEN**. Handbook of Therapeutic Antibodies. *Bioinformatics Tools for Antibody Engineering*, 2007
- **ALTSCHUL** ; **STEPHEN F.** ; **THOMAS L. MADDEN** ; **ALEJANDRO A. SCHAFFER** ; **JINGHUI ZHANG** ; **ZHENG ZHANG** ; **WEBB MILLER** ; **DAVID J. LIPMAN**. Gapped BLAST and PSI-BLAST: a new generation of protein database search programs. *Nucleic Acids Res.*, 1997, vol. 25, 3389-3402
- **TOMIZUKA, K. et al.** *Nature Genetics*, 1997, vol. 16, 133-143
- **KUROIWA, Y. et al.** *Nucl. Acids Res.*, 1998, vol. 26, 3447-3448
- **YOSHIDA, H. et al.** *Animal Cell Technology: Basic and Applied Aspects*, vol. 10, 69-73
- Kluwer Academic Publishers. 1999
- **TOMIZUKA, K. et al.** *Proc. Natl. Acad. Sci. USA*, 2000, vol. 97, 722-727
- **WORMSTONE, I. M. et al.** *Investigative Ophthalmology & Visual Science.*, 2002, vol. 43 (7), 2301-2308
- **CARMEN, S. et al.** *Briefings in Functional Genomics and Proteomics*, 2002, vol. 1 (2), 189-203
- **SIRIWARDENA, D. et al.** *Ophthalmology*, 2002, vol. 109 (3), 427-431
- *Nature Biotechnology*, 2005, vol. 23 (9), 1105-1116
- *Annu. Rev. Immunol*, 1994, vol. 12, 433-455
- **LORI BURTON et al.** *Pharmaceutical Development and Technology*, 2007, vol. 12, 265-273
- **GLUZMAN, Y.** *Cell*, 1981, vol. 23, 175-182
- **URLAUB, G.** ; **CHASIN, L. A.** *Proc. Natl. Acad. Sci. U.S.A.*, 1980, vol. 77, 4126-4220
- *Journal of Chromatography A*, 1995, vol. 705, 129-134
- *Analytical Biochemistry*, 2007, vol. 360, 75-83
- Strategies for Protein Purification and Characterization: A Laboratory Course Manual. Cold Spring Harbor Laboratory Press, 1996
- Antibodies: A Laboratory Manual.. Cold Spring Harbor Laboratory, 1988
- **OGITANI Y. et al.** *Clinical Cancer Research*, 29 March 2016, vol. 22 (20), 5097-5108
- **OGITANI Y. et al.** *Cancer Science*, 2016, vol. 107, 1039-1046
- *Pharmacological Reviews*, 2016, vol. 68, 3-19
- Pharmacol Rev. *Protein Cell*, January 2016, vol. 68, 3-19
- **HERMANSON, G. T**. Bioconjugate Techniques. Academic Press, 1996, 56-136, 456-493
- *Protein Science*, 1995, vol. 4, 2411-2423
- *CHEMICAL ABSTRACTS*, 852808-04-9
- *Methods in Enzymology*, 1991, vol. 203, 121-153
- *Nuc. Acid Res.*, 2007, vol. 35, D301-D303
- *Proc. Natl. Acad. Sci. USA*, 1989, vol. 86, 10029-10033
- Sequences of Proteins of Immunological Interest. **KABAT et al.** Public Health Service National Institutes of Health. 1991
- **QUEEN et al.** *Proc. Natl. Acad. Sci. USA*, 1989, vol. 86, 10029-10033
- *Adv. Enzyme Regul.*, 1984, 2227-55